# EUROPÄISCHE PATENTSCHRIFT

(11) **EP 2 513 125 B1**
(45) Veröffentlichungstag und Bekanntmachung des Hinweises auf die Patenterteilung: **29.10.2014**
(21) Anmeldenummer: 10790441.9
(22) Anmeldetag: 13.12.2010
(51) Int. Cl.: C07F 15/00, H01L 51/50, H01L 51/00

(54) **METALLKOMPLEXE, ENTHALTEND DIAZABENZIMIDAZOLCARBEN-LIGANDEN UND DEREN VERWENDUNG IN OLEDS**
METAL COMPLEXES WITH DIAZABENZIMIDAZOLECARBENE LIGANDS AND THEIR USE IN OLEDS
COMPLEXES DES METAUX AVEC UN LIGAND CARBENE DIAZABENZIMIDAZOLE ET LEUR USAGE EN DISPOSITIFS D'ÉCLAIRAGE À DIODES ÉLECTROLUMINESCENTES ORGANIQUES

(30) Priorität: 14.12.2009 US 286046 P; 14.04.2010 US 323885 P; 11.10.2010 EP 10187176; 11.10.2010 US 391712 P
(43) Veröffentlichungstag der Anmeldung: 24.10.2012
(73) Patentinhaber: BASF SE, 67056 Ludwigshafen (DE)
(72) Erfinder: FUCHS, Evelyn, 68199 Mannheim (DE); LANGER, Nicolle, 64646 Heppenheim (DE); MOLT, Oliver, 69469 Weinheim (DE); DORMANN, Korinna, 67098 Bad Dürkheim (DE); SCHILDKNECHT, Christian, 68307 Mannheim (DE); WATANABE, Soichi, 68161 Mannheim (DE); WAGENBLAST, Gerhard, 67157 Wachenheim (DE); LENNARTZ, Christian, 67105 Schifferstadt (DE); SCHÄFER, Thomas, CH-4410 Liestal (CH); WOLLEB, Heinz, CH-4232 Fehren (CH); FIGUEIRA DUARTE, Teresa Marina, 55116 Mainz (DE); METZ, Stefan, 68165 Mannheim (DE); MURER, Peter, CH-4104 Oberwil (CH)
(86) Internationale Anmeldenummer: PCT/EP2010/069541
(87) Internationale Veröffentlichungsnummer: WO 2011/073149

(56) Entgegenhaltungen:
- WO-A1-2009/046266
- WO-A2-2006/056418

## Beschreibung

Die vorliegende Erfindung betrifft Metall-Carben-Komplexe umfassend ein Zentralatom ausgewählt aus Iridium und Platin, und Diazabenzimidazolcarben-Liganden, OLEDs *(Organic Light Emitting Diode, OLED),* die solche Komplexe enthalten, Licht-emittierende Schichten, enthaltend wenigstens einen solchen Metall-Carben-Komplex, eine Vorrichtung ausgewählt aus der Gruppe bestehend aus Beleuchtungselementen, stationären Bildschirmen und mobilen Bildschirmen enthaltend eine solche OLED, die Verwendung eines solchen Metall-Carben-Komplexes in OLEDs, beispielsweise als Emitter, Matrixmaterial, Ladungstransportmaterial und/oder Ladungs- oder Excitonenblocker.

In organischen Leuchtdioden *(Organic Light Emitting Diode, OLED)* wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen. Aufgrund der sehr kompakten Bauweise und des intrinsisch niedrigen Stromverbrauchs eignen sich Vorrichtungen enthaltend OLEDs insbesondere für mobile Anwendungen, z. B. für Anwendungen in Handys, Laptops usw. Des Weiteren bieten weiße OLEDs große Vorteile gegenüber den bisher bekannten Beleuchtungstechnologien insbesondere eine besonders hohe Effizienz.

Es werden im Stand der Technik zahlreiche Materialien vorgeschlagen, die bei Anregung durch elektrischen Strom Licht emittieren.

WO 2005/019373 offenbart Übergangsmetallkomplexe mit Carbenliganden als Emitter für organische Licht-emittierende Dioden (OLEDs). Die Liganden dieser Übergangsmetallkomplexe sind bevorzugt über eine Metall-Carben-Bindung sowie über eine Bindung zwischen dem Metallatom und einem aromatischen Rest angebunden. Es werden zahlreiche Heterozyklen offenbart, die über eine Carben-Bindung an das Metallatom angebunden sind, es werden jedoch keine Komplexe offenbart, die Diazabenzimidazolcarben-Liganden aufweisen.

WO 2006/056418 A2 offenbart die Verwendung von Übergangsmetall-Carbenkomplexen in organischen Licht-emittierenden Dioden. In den entsprechenden Übergangsmetallkomplexen wird ein Metallatom über wenigstens eine Metall-Carben-Bindung sowie über eine Bindung zwischen dem Metallatom und einem aromatischen Rest an die Liganden gebunden. Die Metall-Carben-Bindung erfolgt bevorzugt über einen Imidazol-Ring, an den gemäß der genannten Schrift auch aromatische Zyklen anelliert sein können. Es werden jedoch keine Komplexe offenbart, die Diazabenzimidazolcarben-Liganden aufweisen.

WO 2007/088093 A1 und WO 2007/185981 A1 offenbaren Übergangsmetallkomplexe, enthaltend Liganden, die über Metall-Carben-Bindungen angebunden sind. Als bevorzugter Carben-Ligand werden Imidazol-Liganden genannt. Diese können auch anellierte aromatische Sechsringe aufweisen, wobei 1 bis 4 der in dem aromatischen Sechsring vorliegenden Kohlenstoffatome durch Stickstoff ersetzt sein können. Die genannten Dokumente offenbaren nicht die Positionen der Stickstoffe im aromatischen Sechsring.

WO 2007/115970 A1 offenbart ebenfalls Übergangsmetall-Carben-Komplexe, wobei als Carben-Ligand Imidazoleinheiten bevorzugt sind. An diese Imidazol-Einheit kann ebenfalls ein aromatischer Sechsring anelliert sein, wobei in diesem 1 bis 4 Kohlenstoffatome durch Stickstoff-Atome ersetzt sein können. Auch dieses Dokument enthält keine Offenbarung bezüglich der Position der Stickstoffatome.

Obwohl bereits Verbindungen bekannt sind, die im sichtbaren, insbesondere im blauen Bereich des elektromagnetischen Spektrums Elektrolumineszenz zeigen, ist die Bereitstellung von Verbindungen, die lange Diodenlebensdauern zeigen, wünschenswert. Im Rahmen der vorliegenden Erfindung wird unter Elektrolumineszenz sowohl Elektrofluoreszenz als auch Elektrophosphoreszenz verstanden.

Aufgabe der vorliegenden Erfindung ist daher die Bereitstellung von alternativen Iridium- und Platin-Komplexen, die zur Elektrolumineszenz im sichtbaren, insbesondere im blauen, Bereich des elektromagnetischen Spektrums geeignet sind, wodurch die Herstellung von Vollfarben-Displays und weißen OLEDs ermöglicht wird. Des Weiteren ist es eine Aufgabe der vorliegenden Erfindung, entsprechende Komplexe bereitzustellen, die als Mischung mit einer Wirtverbindung (Matrixmaterial) als Licht-emittierende Schicht in OLEDs eingesetzt werden können. Des Weiteren ist es eine Aufgabe entsprechende Komplexe bereitzustellen, die eine hohe Quantenausbeute, sowie eine hohe Stabilität in Dioden aufweisen. Die Komplexe sollen als Emitter, Matrixmaterial, Ladungstransportmaterial, insbesondere Lochtransportmaterial, oder Ladungsblocker in OLEDs einsetzbar sein.

Diese Aufgaben werden erfindungsgemäß gelöst durch Metall-Carben-Komplexe der allgemeinen Formel (I) 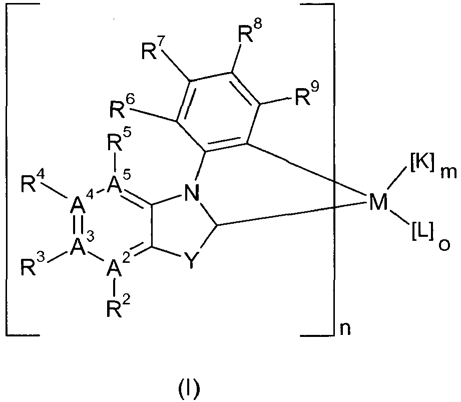 wobei M, n, Y, A², A³, A⁴, A⁵, R¹, R², R³, R⁴, R⁵, R⁶, R⁷, R⁸, R⁹, R¹⁰, K, L, m und o die folgenden Bedeutungen aufweisen:
- M: Ir oder Pt,
- n: ganze Zahl ausgewählt aus 1, 2 oder 3,
- Y: NR¹, O, S oder C(R¹⁰)₂,
- A², A³, A⁴, A⁵: unabhängig voneinander N oder C, wobei 2 A = N-Atome sind und im Ring zwischen zwei N-Atomen wenigstens ein C-Atom vorliegt,
- R¹: linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
- R², R³, R⁴, R⁵: falls A², A³, A⁴ und/oder A⁵ N bedeuten, freies Elektronenpaar oder, falls A², A³, A⁴ und/oder A⁵ C bedeuten, unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung
oder
R³ und R⁴ bilden zusammen mit A³ und A⁴ einen gegebenenfalls von wenigstens einem weiteren Heteroatom unterbrochenen, gegebenenfalls substituierten, ungesättigten Ring mit insgesamt 5 bis 18 Kohlenstoff- und/oder Heteroatomen,
- R⁶, R⁷, R⁸, R⁹: unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung,
oder
R⁶ und R⁷, R⁷ und R⁸ oder R⁸ und R⁹ bilden zusammen mit den C-Atomen, an welche Sie gebunden sind, einen gegebenenfalls von wenigstens einem Heteroatom unterbrochenen ungesättigten oder aromatischen, gegebenenfalls substituierten, Ring mit insgesamt 5 bis 18 Kohlenstoff- und/oder Heteroatomen,
und/oder
falls A⁵ C bedeutet, bilden R⁵ und R⁶ zusammen eine gesättigte oder ungesättigte, lineare oder verzweigte, gegebenenfalls Heteroatome, aromatische Einheit, heteroaromatische Einheit und/oder funktionelle Gruppen enthaltende Verbrückung mit insgesamt 1 bis 30 Kohlenstoff- und/oder Heteroatomen, an die gegebenenfalls ein substituierter oder unsubstituierter, fünf- bis achtgliedriger, Kohlenstoff- und/oder Heteroatome enthaltender Ring, annelliert ist,
- R¹⁰: unabhängig voneinander linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
- K: neutraler mono- oder bidentater Ligand,
- L: mono- oder dianionischer Ligand, bevorzugt monoanionischer Ligand, der mono- oder bidentat sein kann,
- m: 0, 1 oder 2, wobei bei m gleich 2 die Liganden K gleich oder verschieden sein können,
- o: 0, 1 oder 2, wobei bei o gleich 2 die Liganden L gleich oder verschieden sein können.

Bedeuten m und o jeweils 0, so liegen erfindungsgemäß homoleptische Metall-Carben-Komplexe der allgemeinen Formel (I) vor. Bedeuten wenigstens eines aus m und o 1 oder 2, so liegen erfindungsgemäß heteroleptische Metall-Carben-Komplexe der allgemeinen Formel (I) vor.

Im Sinne der vorliegenden Erfindung haben die Begriffe Arylrest, -einheit oder -gruppe, Heteroarylrest, -einheit oder -gruppe, Alkylrest, -einheit oder -gruppe und Cycloalkylrest, -einheit oder -gruppe die folgenden Bedeutungen - soweit keine anderen Bedeutungen angegeben sind:
Unter einem Arylrest, -einheit oder -gruppe ist ein Rest mit einem Grundgerüst von 6 bis 30 Kohlenstoffatomen, bevorzugt 6 bis 18 Kohlenstoffatomen zu verstehen, der aus einem aromatischen Ring oder mehreren kondensierten aromatischen Ringen aufgebaut ist. Geeignete Grundgerüste sind zum Beispiel Phenyl, Naphthyl, Anthracenyl oder Phenanthrenyl. Dieses Grundgerüst kann unsubstituiert sein, d. h., dass alle Kohlenstoffatome, die substituierbar sind, Wasserstoffatome tragen, oder an einer oder mehreren oder allen substituierbaren Positionen des Grundgerüsts substituiert sein.

Geeignete Substituenten sind zum Beispiel Alkylreste, bevorzugt Alkylreste mit 1 bis 8 Kohlenstoffatomen, besonders bevorzugt Methyl, Ethyl, i-Propyl, t-Butyl, Neopentyl, Arylreste, bevorzugt C₆-Arylreste, die wiederum substituiert oder unsubstituiert sein können, Heteroarylreste, bevorzugt Heteroarylreste, die mindestens ein Stickstoffatom enthalten, besonders bevorzugt Pyridylreste, Alkenylreste, bevorzugt Alkenylreste, die eine Doppelbindung tragen, besonders bevorzugt Alkenylreste mit einer Doppelbindung und 1 bis 8 Kohlenstoffatomen, oder Gruppen mit Donor- oder Akzeptorwirkung. Unter Gruppen mit Donorwirkung sind Gruppen zu verstehen, die einen +l- und/oder +M-Effekt aufweisen, und unter Gruppen mit Akzeptorwirkung sind Gruppen zu verstehen, die einen -|- und/oder -M-Effekt aufweisen. Geeignete Gruppen, mit Donor- oder Akzeptorwirkung sind Halogenreste, bevorzugt F, Cl, Br, besonders bevorzugt F, Alkylreste, Silylreste, Siloxyreste, Alkoxyreste, Aryloxyreste, Carbonylreste, Esterreste, Aminreste, Amidreste, CH₂F-Gruppen, CHF₂-Gruppen, GF₃-Gruppen, CN-Gruppen, Thiogruppen oder SCN-Gruppen. Ganz besonders bevorzugt tragen die Arylreste Substituenten ausgewählt aus der Gruppe bestehend aus Methyl, Ethyl, *iso-*Propyl, n-Propyl, n-Butyl, *iso*-Butyl, *tert*-Butyl, Neopentyl, CF₃, Aryloxy, Amin, Thiogruppen und Alkoxy, oder die Arylreste sind unsubstituiert. Bevorzugt ist der Arylrest oder die Arylgruppe ein C₆-Arylrest, der gegebenenfalls mit mindestens einem der vorstehend genannten Substituenten substituiert ist. Besonders bevorzugt weist der C₆-Arylrest keinen, einen, zwei oder drei der vorstehend genannten Substituenten auf.

Unter einem Heteroarylrest oder einer Heteroaryleinheit oder -gruppe sind Reste mit 5 bis 30 Kohlenstoff- und/oder Heteroatomen zu verstehen, die sich von den vorstehend genannten Arylresten dadurch unterscheiden, dass in dem Grundgerüst der Arylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüsts der Arylreste durch Heteroatome ersetzt. Insbesondere bevorzugt ist das Grundgerüst ausgewählt aus elektronenarmen Systemen wie Pyridyl, Pyrimidyl, Pyrazyl und Triazolyl, und fünfgliedrigen Heteroaromaten wie Pyrrol, Furan, Thiophen, Imidazol, Pyrazol, Triazol, Oxazol und Thiazol. Das Grundgerüst kann an einer, mehreren oder allen substituierbaren Positionen des Grundgerüsts substituiert sein. Geeignete Substituenten sind dieselben, die bereits bezüglich der Arylgruppen genannt wurden.

Unter einem Alkylrest oder einer Alkylgruppe ist ein Rest mit 1 bis 20 Kohlenstoffatomen, bevorzugt 1 bis 10 Kohlenstoffatomen, besonders bevorzugt 1 bis 8 Kohlenstoffatomen zu verstehen. Dieser Alkylrest kann verzweigt oder unverzweigt sein und gegebenenfalls mit einem oder mehreren Heteroatomen, bevorzugt N, O oder S unterbrochen sein. Des Weiteren kann dieser Alkylrest mit einem oder mehreren der bezüglich der Arylgruppen genannten Substituenten substituiert sein. Es ist ebenfalls möglich, dass der Alkylrest eine oder mehrere Arylgruppen trägt. Dabei sind alle der vorstehend aufgeführten Arylgruppen geeignet. Besonders bevorzugt sind die Alkylreste ausgewählt aus der Gruppe bestehend aus Methyl, Ethyl, i-Propyl, n-Propyl, i-Butyl, n-Butyl, t-Butyl, sec-Butyl, i-Pentyl, n-Pentyl, sec-Pentyl, neo-Pentyl, n-Hexyl, i-Hexyl und sec-Hexyl. Ganz besonders bevorzugt sind Methyl, i-Propyl, tert-Butyl und Neopentyl.

Unter einem Cycloalkylrest oder einer Cycloalkylgruppe ist ein cyclischer Rest mit 3 bis 20 Kohlenstoffatomen, bevorzugt 3 bis 10 Kohlenstoffatomen, besonders bevorzugt 3 bis 8 Kohlenstoffatomen zu verstehen. Dieser Cycloalkylrest kann gegebenenfalls mit einem oder mehreren Heteroatomen, bevorzugt N, O oder S unterbrochen sein. Des Weiteren kann dieser Cycloalkylrest unsubstituiert oder substituiert sein, d. h. mit einem oder mehreren der bezüglich der Arylgruppen genannten Substituenten substituiert sein. Es ist ebenfalls möglich, dass der Cycloalkylrest eine oder mehrere Arylgruppen trägt. Dabei sind alle vorstehend aufgeführten Arylgruppen geeignet.

Das bezüglich der Aryl-, Heteroaryl, Alkylreste und Cycloalkylreste Gesagte trifft erfindungsgemäß unabhängig voneinander auf die in der vorliegenden Anmeldung genannten Reste zu, wobei R², R³, R⁴ und R⁵ für den Fall, dass A², A³, A⁴ und/oder A⁵ gleich N ist, ein freies Elektronenpaar bedeuten, d. h., dass an diesen Ring-Stickstoffatomen kein Substituent ausgewählt aus der oben genannten Gruppe vorliegt. Für den Fall, dass A², A³, A⁴ und/oder A⁵ gleich C ist, liegen als R², R³, R⁴ und R⁵ unabhängig voneinander Wasserstoff und/oder die genannten Substituenten vor.

K bedeutet in der allgemeinen Formel (I) einen neutralen mono- oder bidentaten Ligand, und L bedeutet in der allgemeinen Formel (I) einen mono- oder dianionischen Ligand, bevorzugt einen monoanionischer Ligand, der mono- oder bidentat sein kann.

Unter einem bidentaten Liganden ist ein Ligand zu verstehen, der an zwei Stellen an das Übergangsmetallatom M koordiniert ist. Im Sinne der vorliegenden Anmeldung wird der Begriff "zweizähnig" synonym mit dem Begriff "bidentat" verwendet. Unter einem monodentaten Liganden ist ein Ligand zu verstehen, der an einer Stelle des Liganden mit dem Übergangsmetallatom M koordiniert.

Geeignete mono- oder dianionische Liganden L, bevorzugt monoanionische Liganden L, die mono- oder bidentat sein können, sind die üblicherweise als mono- oder bidentate mono- oder dianionische Liganden eingesetzten Liganden.

Geeignete monoanionische monodentate Liganden sind zum Beispiel Halogenide, insbesondere Cl⁻ und Br⁻, Pseudohalogenide, insbesondere CN⁻, Cyclopentadienyl (Cp⁻), Hydrid, Alkylreste, die mit dem Übergangsmetall M über eine Sigmabindung verknüpft sind, zum Beispiel CH₃, Alkylarylreste, die mit dem Übergangsmetall M über eine Sigmabindung verknüpft sind, zum Beispiel Benzyl.

Geeignete monoanionische bidentate Liganden sind zum Beispiel Acetylacetonat und dessen Derivate, Picolinat, Schiffsche Basen, Aminosäuren, Arylpyridine, z.B. Phenylpyridin sowie die weiteren in WO 02/15645 genannten bidentaten monoanionischen Liganden, Carbenliganden wie sie in WO2006056418 und in EP1658343 genannt sind, Arylazole, z.B. 2-Arylimidazole, wobei 2-Arylimidazole und Carbenliganden bevorzugt sind.

Geeignete dianionische bidentate Liganden sind zum Beispiel Dialkoholate, Dicarbonate, Dicarboxylate, Diamide, Diimide, Dithiolate, Biscyclopentadienyle, Bisphosphonate, Bissulfonate und 3-Phenylpyrazol.

Geeignete neutrale mono- oder bidentate Liganden K sind bevorzugt ausgewählt aus der Gruppe bestehend aus Phosphinen, sowohl Mono- als auch Bisphosphinen; Phosphonaten, sowohl Mono- als auch Bisphosphonaten, und Derivaten davon, Arsenaten, sowohl Mono- als auch Bisarsenaten, und Derivaten davon; Phosphiten, sowohl Mono- als auch Bisphosphiten; CO; Pyridinen, sowohl Mono- als auch Bispyridinen; Nitrilen, Dinitrilen, Allyl, Diiminen, nicht konjugierten Dienen und konjugierten Dienen, die einen π-Komplex mit M¹ bilden. Besonders bevorzugte neutrale mono- oder bidentate Liganden K sind ausgewählt aus der Gruppe bestehend aus Phosphinen, sowohl Mono- als auch Bisphosphinen, bevorzugt Trialkyl-, Triaryl-oder Alkylarylphosphinen, besonders bevorzugt PAr₃, wobei Ar ein substituierter oder unsubstituierter Arylrest ist und die drei Arylreste in PAr₃ gleich oder verscheiden sein können, besonders bevorzugt PPh₃, PEt₃, PnBu₃, PEt₂Ph, PMe₂Ph, PnBu₂Ph; Phosphonaten und Derivaten davon, Arsenaten und Derivaten davon, Phosphiten, CO; Pyridinen, sowohl Mono- als auch Bispyridinen, wobei die Pyridine mit Alkyl- oder Arylgruppen substituiert sein können; Nitrilen und Dienen, die einen π-Komplex mit M¹ bilden, bevorzugt η⁴-Diphenyl-1,3-butadien, η⁴-1,3-Pentadien, η⁴-1-Phenyl-1,3-pentadien, η⁴-1,4-Dibenzyl-1,3-butadien, η⁴-2,4-Hexadien, η⁴-3-Methyl-1,3-pentadien, η⁴-1,4-Ditolyl-1,3-butadien, η⁴-1,4-Bis(trimethylsilyl)-1,3-butadien und η²- oder η⁴-Cyclooctadien (je 1,3 und je 1,5), besonders bevorzugt 1,4-Diphenyl-1,3-butadien, 1-Phenyl-1,3-pentadien, 2,4-Hexadien, Butadien, η²-Cycloocten, η⁴-1,3-Cyclooctadien und η⁴-1,5-Cyclooctadien. Ganz besonders bevorzugte neutrale monodentate Liganden sind ausgewählt aus der Gruppe bestehend aus PPh₃, P(OPh)₃, AsPh₃, CO, Pyridin, Nitrilen und deren Derivaten. Geeignete neutrale mono- bzw. bidentate Liganden sind bevorzugt 1,4-Diphenyl-1,3-butadien, 1-Phenyl-1,3-pentadien, 2,4-Hexadien, η⁴-Cyclooctadien und η²-Cyclooctadien (je 1,3 und je 1,5).

Die Zahl o der monoanionischen Liganden L beträgt in dem vorstehend genannten Fall 0, 1, 2. Ist o > 1 können die Liganden L gleich oder verschieden sein, bevorzugt sind sie gleich.

Die Zahl m der neutralen Liganden K ist abhängig davon, ob die Koordinationszahl 6 des Ir(III) oder 4 des Pt(II) mit Hilfe der Carbenliganden und der Liganden L bereits erreicht wurde. Ist - in dem Fall, dass Ir(III) eingesetzt wird - n drei und werden drei monoanionische bidentate Carbenliganden eingesetzt, so ist m in dem vorstehend genannten Fall 0. Ist - in dem Fall, dass Pt(II) eingesetzt wird - n zwei und werden zwei monoanionische bidentate Carbenliganden eingesetzt, so ist m in diesem Fall ebenfalls 0.

In einer weiteren bevorzugten Ausführungsform betrifft die vorliegende Erfindung einen erfindungsgemäßen Metall-Carben-Komplex, wobei in der allgemeinen Formel (I) L Carbenligand der allgemeinen Formel (II) 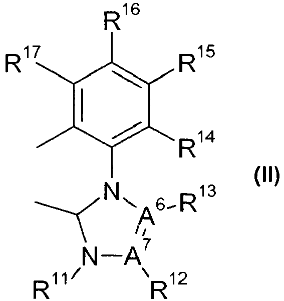 bedeutet, mit
- A⁶, A⁷: unabhängig voneinander C oder N
- R¹¹: linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
- R¹², R¹³: unabhängig voneinander, wenn A gleich N, freies Elektronenpaar, oder wenn A gleich C, Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung,
- R¹⁴ R¹⁵, R¹⁶, R¹⁷: unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung,
oder
R¹² und R¹³, R¹⁴ und R¹⁵, R¹⁵ und R¹⁶ oder R¹⁶ und R¹⁷ bilden zusammen mit A bzw. den C-Atomen, an welche sie gebunden sind, einen gegebenenfalls von wenigstens einem Heteroatom unterbrochenen ungesättigten oder aromatischen, gegebenenfalls substituierten, Ring mit insgesamt 5 bis 18 Kohlenstoff- und/oder Heteroatomen,
und/oder
falls A⁶ C bedeutet, bilden R¹³ und R¹⁴ zusammen eine gesättigte oder ungesättigte, lineare oder verzweigte, gegebenenfalls Heteroatome, aromatische Einheiten, heteroaromatische Einheiten und/oder funktionelle Gruppen enthaltende Verbrückung mit insgesamt 1 bis 30 Kohlenstoff- und/oder Heteroatomen, an die gegebenenfalls ein substituierter oder unsubstituierter, fünf- bis achtgliedriger, Kohlenstoff- und/oder Heteroatome enthaltender Ring, annelliert ist.

In einer weiteren bevorzugten Ausführungsform betrifft die vorliegende Erfindung einen erfindungsgemäßen Metall-Carben-Komplex, wobei in der allgemeinen Formel (I) L heterocyclischer Nicht-Carbenligand der allgemeinen Formel (III) 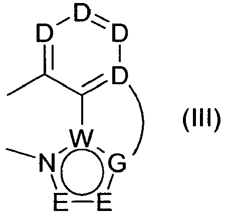 bedeutet, worin die Symbole in dem Liganden der allgemeinen Formel IV die folgenden Bedeutungen aufweisen:
- D: unabhängig voneinander CR¹⁸ oder N, bevorzugt CR¹⁸;
- W: C, N, bevorzugt C;
- E: unabhängig voneinander CR¹⁹, N, NR²⁰, bevorzugt CR¹⁹ oder N;
- G: CR²¹, N, NR²², S, O, bevorzugt NR²¹
- R¹⁸, R¹⁹ R²¹: unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung,
oder jeweils 2 Reste R¹⁸, R¹⁹ und R²¹ bilden zusammen mit den C-Atomen, an welche sie gebunden sind, einen gegebenenfalls von wenigstens einem Heteroatom unterbrochenen gesättigten, ungesättigten oder aromatischen, gegebenenfalls substituierten, Ring mit insgesamt 5 bis 18 Kohlenstoff- und/oder Heteroatomen,
- R²⁰, R²²: unabhängig voneinander linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung; bevorzugt o,o'-dialkylierter Arylrest
wobei die durchgezogene, gebogene Linie eine optionale Verbrückung zwischen einer der Gruppen D und der Gruppe G bedeutet; wobei die Verbrückung die folgenden Bedeutungen aufweisen kann:
Alkylen, Arylen, Heteroarylen, Alkinylen, Alkenylen, NR²³, O, S, SiR²⁴R²⁵, und (CR²⁶R²⁷)_{d}, wobei eine oder mehrere nicht benachbarte Gruppen (CR²⁶R²⁷) durch NR²³, O, S, SiR²⁴R²⁵, ersetzt sein können, wobei
   - d: 2 bis 10;
und
23
- R²⁴, R²⁵, R²⁶, R²⁷: H, Alkyl, Aryl, Heteroaryl, Alkenyl, Alkinyl.

Für die erfindungsgemäße Ausführungsform, dass jeweils 2 Reste R¹⁸, R¹⁹ und R²¹ zusammen mit den C-Atomen, an welche sie gebunden sind, einen gegebenenfalls von wenigstens einem Heteroatom unterbrochenen gesättigten, ungesättigten oder aromatischen, gegebenenfalls substituierten, Ring mit insgesamt 5 bis 18 Kohlenstoffund/oder Heteroatomen bilden, gilt, dass beispielsweise zwei Reste R¹⁸, zwei Reste R¹⁹ oder ein Rest R¹⁹ und ein Rest R²¹ einen entsprechenden Ring bilden.

Erfindungsgemäß ganz besonders bevorzugte Liganden L sind im Folgenden abgebildet:

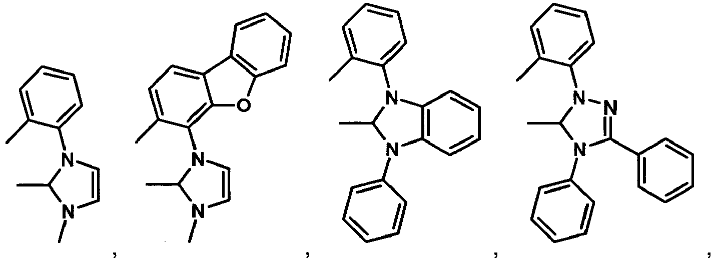

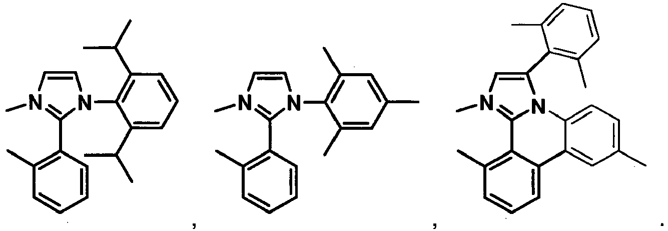

Weitere bevorzugte Liganden L:

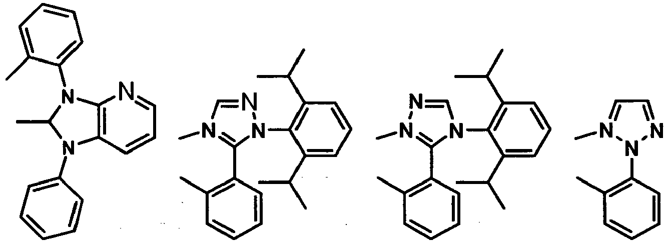

In einer bevorzugten Ausführungsform weisen in der allgemeinen Formel (I) M, n, Y, A², A³, A⁴, A⁵, R¹, R², R³, R⁴, R⁵, R⁶, R⁷, R⁸, R⁹, R¹⁰, K, L, n und o die folgenden Bedeutungen auf:
M bedeutet erfindungsgemäß Ir oder Pt, bevorzugt Ir. Ir liegt in den erfindungsgemäßen Komplexen in der Oxidationsstufe +3 vor. Pt liegt in den erfindungsgemäßen Komplexen in der Oxidationsstufe +2 vor.
n bedeutet im Allgemeinen 1, 2 oder 3. Falls M gleich Ir ist, ist n bevorzugt gleich 3. Falls M gleich Pt ist, ist n bevorzugt gleich 1.
Y bedeutet erfindungsgemäß NR¹, O, S oder C(R²⁵)₂, bevorzugt NR¹.
A², A³, A⁴ und A⁵ bedeuten erfindungsgemäß unabhängig voneinander C oder N, wobei 2 A = N-Atom sind und im Ring zwischen zwei N-Atomen wenigstens ein C-Atom liegt. Im Allgemeinen befinden sich zwischen zwei N-Atomen ein oder zwei C-Atome.

Erfindungsgemäß sind folgende Ausführungsformen bevorzugt:
1. A² und A⁵ sind N, und A³ und A⁴ sind C, d. h. die erfindungsgemäßen Metall-Carben-Komplexe enthalten in dieser bevorzugten Ausführungsform wenigstens eine über eine Metall-Carben-Bindung angebundene Pyrazinoimidazol-Einheit der allgemeinen Formel (la) 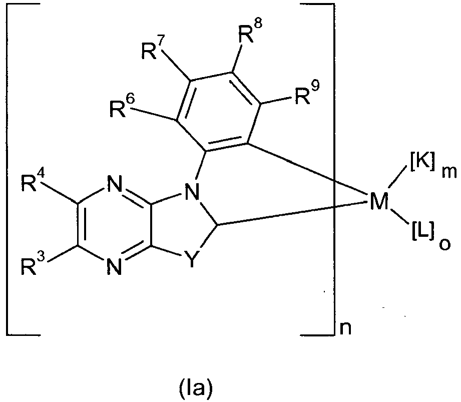
2. A² und A⁴ sind N, A³ und A⁵ sind C, d. h. die erfindungsgemäßen Metall-Carben-Komplexe enthalten in dieser bevorzugten Ausführungsform wenigstens eine über eine Metall-Carben-Bindung angebundene Pyrimidinoimidazol-Einheit der allgemeinen Formel (Ib) 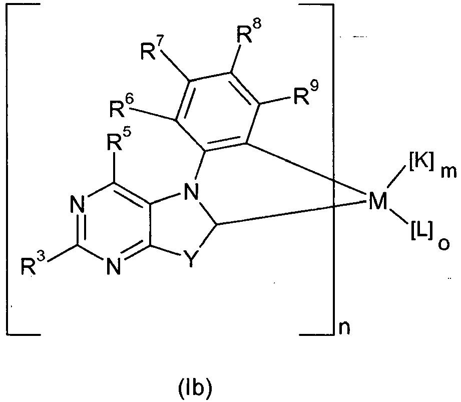
3. A³ und A⁵ sind N, A² und A⁴ sind C, d. h. die erfindungsgemäßen Metall-Carben-Komplexe enthalten in dieser bevorzugten Ausführungsform wenigstens eine über eine Metall-Carben-Bindung angebundene Pyrimidinoimidazol-Einheit der allgemeinen Formel (Ic)

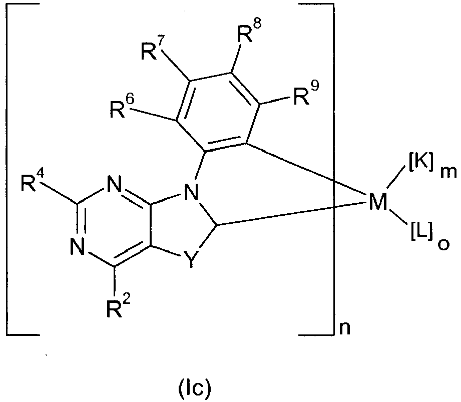

In den allgemeinen Formeln (la), (Ib) und (Ic) gelten die gleichen Bedeutungen wie in der allgemeinen Formel (I).

Für den bevorzugten Fall, dass Y NR¹ bedeutet, bedeutet R¹ in einer bevorzugten Ausführungsform linearer oder verzweigter Alkylrest mit 1 bis 6 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen.

Besonders bevorzugt bedeutet R¹ linearer oder verzweigter Alkylrest mit 1 bis 6 Kohlenstoffatomen, substituierter oder unsubstituierter Phenylrest, substituierter oder unsubstituierter Heteroarylrest mit insgesamt 5 oder 6 Kohlenstoff- und/oder Heteroatomen.

Ganz besonders bevorzugt ist R¹ ausgewählt aus Phenyl, Toluyl, Mesityl, Thiophenyl, Furanyl, Pyridyl, Methyl, iso-Propyl oder Neopentyl.

Die vorliegende Erfindung betrifft daher insbesondere einen erfindungsgemäßen Metall-Carben-Komplex, in dem Y NR¹ bedeutet, wobei R¹ ausgewählt ist aus der Gruppe bestehend aus Phenyl, Toluyl, Mesityl, Thiophenyl, Furanyl, Pyridyl, Methyl, iso-Propyl oder Neopentyl.

R², R³, R⁴, R⁵ bedeuten in einer bevorzugten Ausführungsform unabhängig voneinander Wasserstoff, einen linearen oder verzweigten Alkylrest mit 1 bis 20 Kohlenstoffatomen, einen substituierten oder unsubstituierten Arylrest mit 6 bis 30 Kohlenstoffatomen, einen substituierten oder unsubstituierten Heteroarylrest mit 5 bis 18 Kohlenstoff- und/oder Heteroatomen oder Silylrest.

R², R³, R⁴, R⁵ bedeuten in einer bevorzugten Ausführungsform, falls A², A³, A⁴ und/oder A⁵ N bedeuten, freies Elektronenpaar, oder falls A², A³, A⁴ und/oder A⁵ C bedeuten, unabhängig voneinander Wasserstoff, linearer oder verzweigter Alkylrest mit 1 bis 6 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen oder Silylrest
oder
R³ und R⁴ bilden zusammen mit A³ und A⁴ einen gegebenenfalls von wenigstens einem weiteren Heteroatom unterbrochenen, gegebenenfalls substituierten, ungesättigten Ring mit insgesamt 5 bis 18 Kohlenstoff- und/oder Heteroatomen.

Erfindungsgemäß wird unter einem ungesättigten Ring ein einfach, zweifach oder mehrfach ungesättigter, bevorzugt einfach ungesättigter, Ring verstanden. Ein aromatischer Ring fällt erfindungsgemäß nicht unter diese Definition. Insbesondere bilden R³ und R⁴ zusammen mit A³ und A⁴ keinen Phenylring.

Besonders bevorzugt bedeutet R² freies Elektronenpaar, falls A² N bedeutet, oder Wasserstoff, falls A² C bedeutet.

Besonders bevorzugt bedeutet R³ freies Elektronenpaar, falls A³ N bedeutet, oder Wasserstoff oder linearer oder verzweigter Alkylrest mit 1 bis 20 Kohlenstoffatomen oder gegebenenfalls substituierter, gesättigter, ungesättigter oder aromatischer Ring mit insgesamt 5 bis 18 Kohlenstoff- und/oder Heteroatomen, besonders bevorzugt verzweigter Alkylrest oder o,o'-dialkylierter Phenylring, falls A³ C bedeutet.

Besonders bevorzugt bedeutet R⁴ freies Elektronenpaar, falls A⁴ N bedeutet, oder Wasserstoff oder linearer oder verzweigter Alkylrest mit 1 bis 20 Kohlenstoffatomen oder gegebenenfalls substituierter, gesättigter, ungesättigter oder aromatischer Ring mit insgesamt 5 bis 18 Kohlenstoff- und/oder Heteroatomen, besonders bevorzugt verzweigter Alkylrest oder o,o'-dialkylierter Phenylring, falls A⁴ C bedeutet.

Ganz besonders bevorzugt bilden R³ und R⁴ zusammen mit A³ und A⁴, falls A³ und A⁴ gleich C sind, einen gegebenenfalls substituierten, ungesättigten Ring mit insgesamt 5 bis 18 Kohlenstoffatomen, wobei der Fall, dass A² und A⁵ N bedeuten und R³ und R⁴ zusammen mit A³ und A⁴ einen Phenylring bilden, erfindungsgemäß ausgeschlossen ist.

Besonders bevorzugt bedeutet R⁵ freies Elektronenpaar, falls A⁵ N bedeutet, oder Wasserstoff, falls A⁵ C bedeutet.

In einer weiteren bevorzugten Ausführungsform bedeuten R⁶, R⁷, R⁸, R⁹ unabhängig voneinander Wasserstoff oder linearer oder verzweigter Alkylrest mit 1 bis 20 Kohlenstoffatomen oder o,o'-dialkylierte Phenylreste, besonders bevorzugt Wasserstoff.

In einer weiteren bevorzugten Ausführungsform bilden R⁶ und R⁷ oder R⁷ und R⁸ oder R⁸ und R⁹ zusammen mit dem Phenylring, d. h. mit den C-Atomen, an welche die Reste angebunden sind, einen gegebenenfalls von wenigstens einem Heteroatom unterbrochenen ungesättigten oder aromatischen, gegebenenfalls substituierten, Ring mit insgesamt 5, 6 oder 7 Kohlenstoff- und/oder Heteroatomen. Besonders bevorzugt bilden die beiden jeweiligen Reste zusammen mit dem Phenylring folgende Heterocyclen: Dibenzofuran, Dibenzothiophen, Fluoren, Acridan, Xanthen, Thioxanthen, Phenazin oder Phenoxazin.

Alternativ oder zusätzlich können R⁵ und R⁶ zusammen eine gesättigte oder ungesättigte, lineare oder verzweigte, gegebenenfalls Heteroatome, aromatische Einheiten, heteroaromatische Einheiten und/oder funktionelle Gruppen enthaltende Verbrückung mit insgesamt 1 bis 30 Kohlenstoff- und/oder Heteroatomen, an die gegebenenfalls ein substituierter oder unsubstituierter, fünf- bis achtgliedriger, bevorzugt sechsgliedriger, Kohlenstoff- und/oder Heteroatome enthaltender Ring, annelliert ist, bilden.

R²⁵ bedeutet, falls vorhanden, erfindungsgemäß bevorzugt unabhängig voneinander, einen linearen oder verzweigten Alkylrest mit 1 bis 20 Kohlenstoffatomen, einen substituierten oder unsubstituierten Arylrest mit 6 bis 30 Kohlenstoffatomen, einen substituierten oder unsubstituierten Heteroarylrest mit 5 bis 18 Kohlenstoff- und/oder Heteroatomen, besonders bevorzugt einen linearen Alkylrest oder einen substituierten oder unsubstituierten Phenylrest.

In einer besonders bevorzugten Ausführungsform betrifft die vorliegende Erfindung einen erfindungsgemäßen Metall-Carben-Komplex, wobei M, n, Y, A², A³, A⁴, A⁵, R¹, R², R³, R⁴, R⁵, R⁶, R⁷, R⁸, R⁹, L, m und o die folgenden Bedeutungen aufweisen:
- M: Ir,
- n: 1, 2 oder 3,
- Y: NR¹,
- A², A³, A⁴, A⁵: unabhängig voneinander N oder C, wobei 2 A = N-Atome sind und im Ring zwischen zwei N-Atomen wenigstens ein C-Atom vorliegt,
- R¹: linearer oder verzweigter Alkylrest mit 1 bis 6 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
- R², R³, R⁴, R⁵: falls A², A³, A⁴ und/oder A⁵ N bedeuten freies Elektronenpaar, oder falls A², A³, A⁴ und/oder A⁵ C bedeuten, unabhängig voneinander Wasserstoff, linearer oder verzweigter Alkylrest mit 1 bis 6 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
oder
R³ und R⁴ bilden zusammen mit A³ und A⁴ einen gegebenenfalls von wenigstens einem weiteren Heteroatom unterbrochenen, gegebenenfalls substituierten, ungesättigten Ring mit insgesamt 5 bis 18 Kohlenstoff- und/oder Heteroatomen,
- R⁶, R⁷, R⁸, R⁹: unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
- L: monoanionischer bidentater Ligand,
- m: 0,
- o: 0, 1 oder 2.

Besonders bevorzugt betrifft die vorliegende Erfindung einen erfindungsgemäßen Metall-Carben-Komplex, wobei M, n, Y, A², A³, A⁴, A⁵, R¹, R², R³, R⁴, R⁵, R⁶, R⁷, R⁸, R⁹, L, m und o die folgenden Bedeutungen aufweisen:
- M: Ir,
- n: 1, 2 oder 3,
- Y: NR¹,
- A², A³, A⁴, A⁵: A² und A⁵ sind N und A³ und A⁴ sind C
oder
- A² und A⁴: sind N und A³ und A⁵ sind C
oder
- A³ und A⁵: sind N und A² und A⁴ sind C,
- R¹: linearer oder verzweigter Alkylrest mit 1 bis 6 Kohlenstoffatomen, substituierter oder unsubstituierter Phenylrest, substituierter oder unsubstituierter Heteroarylrest mit insgesamt 6 bis 18 Kohlenstoff- und/oder Heteroatomen,
- R², R³, R⁴, R⁵: falls A², A³, A⁴ und/oder A⁵ N bedeuten, freies Elektronenpaar oder, falls A², A³, A⁴ und/oder A⁵ C bedeuten, unabhängig voneinander Wasserstoff, linearer oder verzweigter Alkylrest mit 1 bis 6 Kohlenstoffatomen, substituierter, - insbesondere o,o'-dialkylierter, oder unsubstituierter Phenylrest,
oder
R³ und R⁴ bilden zusammen mit A³ und A⁴ einen gegebenenfalls substituierten, einfach ungesättigten Ring mit insgesamt 5 bis 7 Kohlenstoffatomen,
- R⁶, R⁷, R⁸, R⁹: unabhängig voneinander Wasserstoff, linearer oder verzweigter Alkylrest mit 1 bis 20 Kohlenstoffatomen, o,o'-dialkylierter Arylrest mit 6 bis 30 Kohlenstoffatomen,.
- L: monoanionischer bidentater Ligand,
- m: 0,
- o: 0, 1 oder 2.

Die weiteren oben genannten bevorzugten und besonders bevorzugten Ausführungsformen gelten entsprechend.

Ganz besonders bevorzugte erfindungsgemäße Metall-Carben-Komplexe der allgemeinen Formel (I) sind im Folgenden dargestellt.

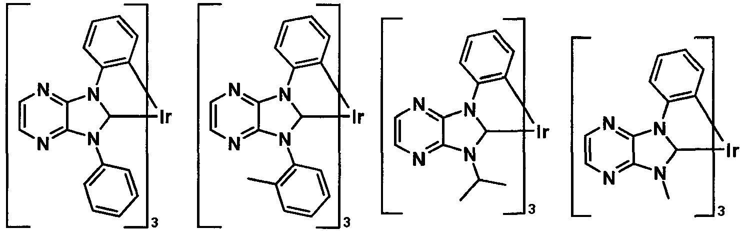

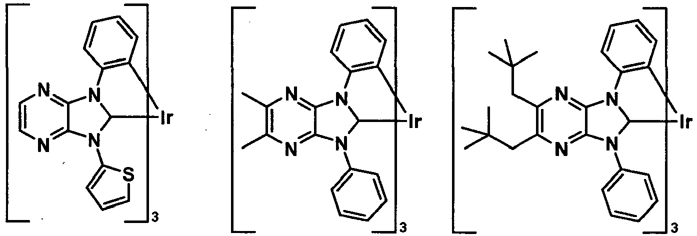

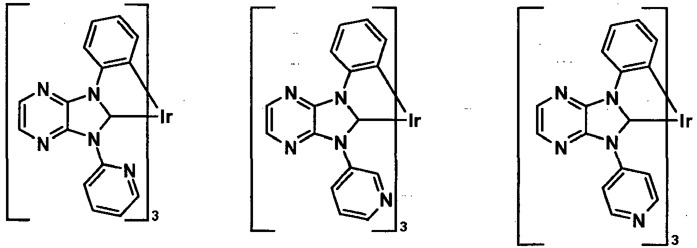

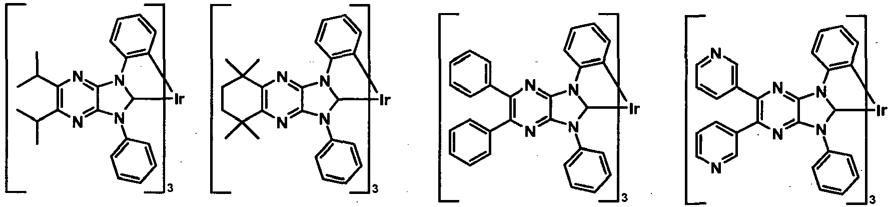

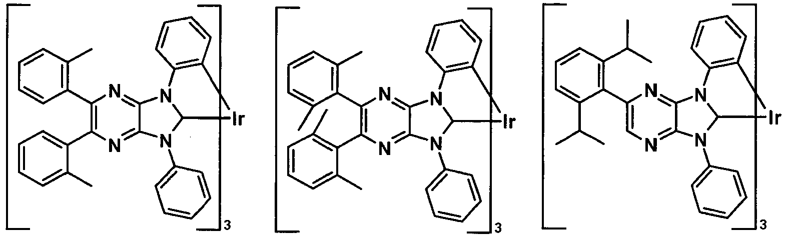

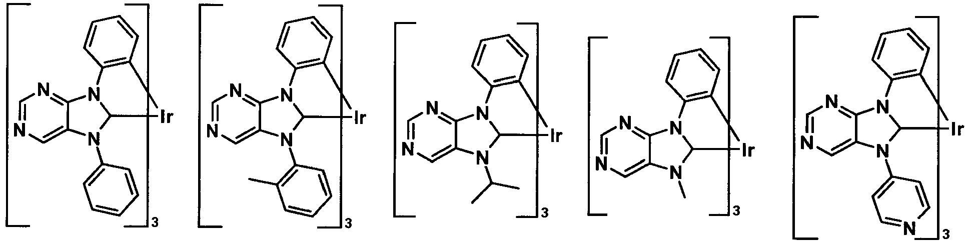

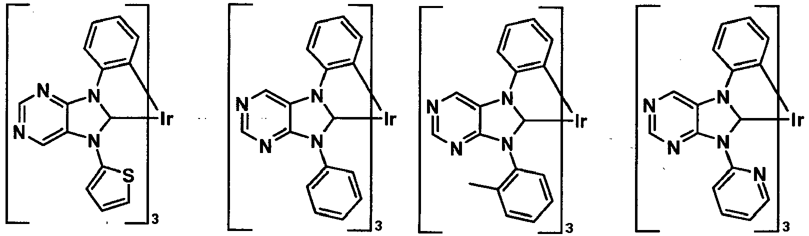

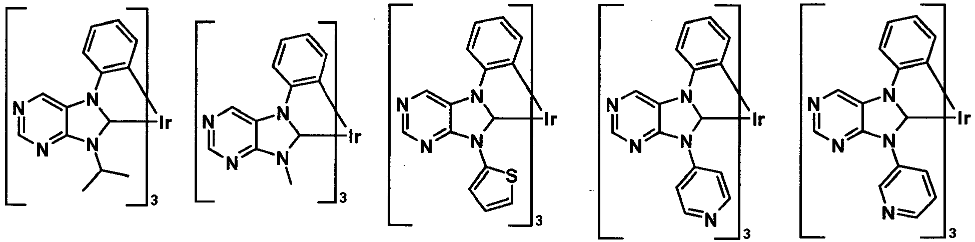

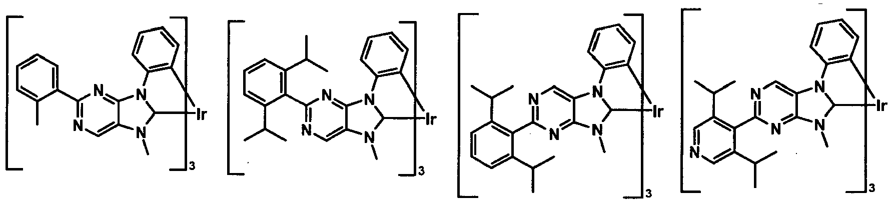

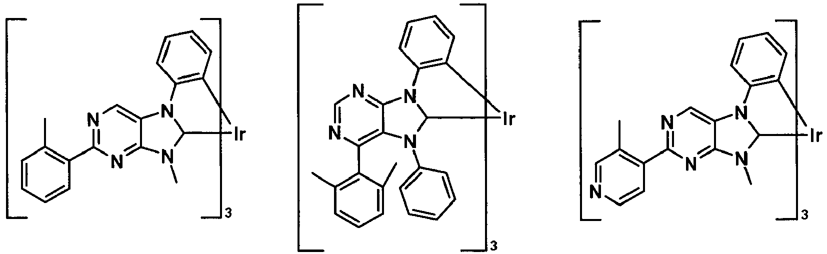

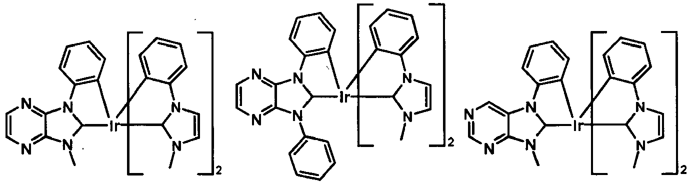

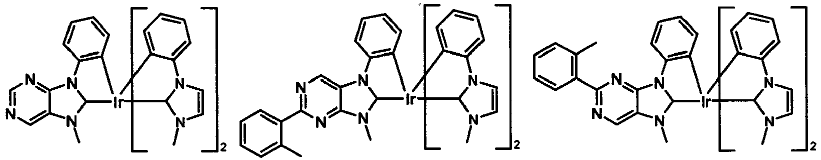

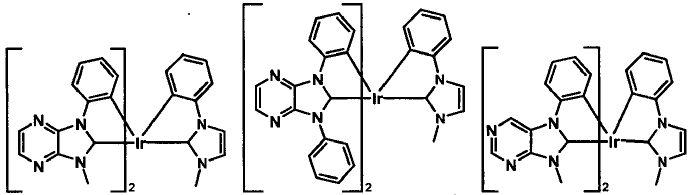

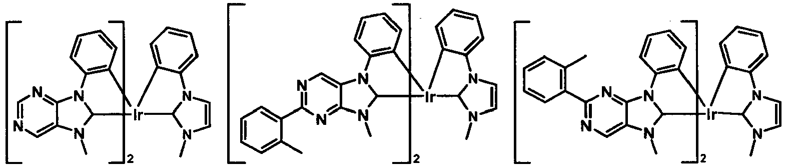

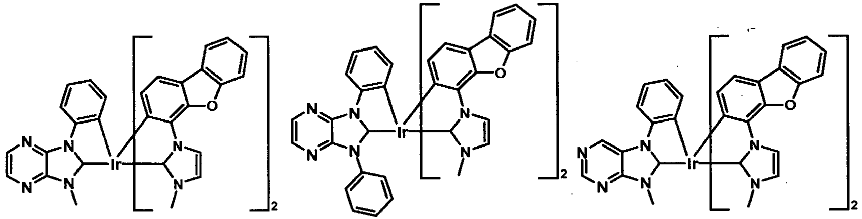

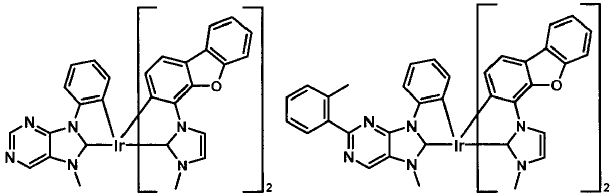

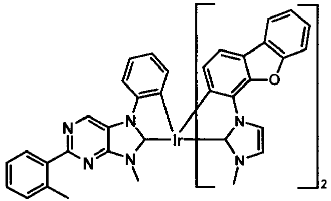

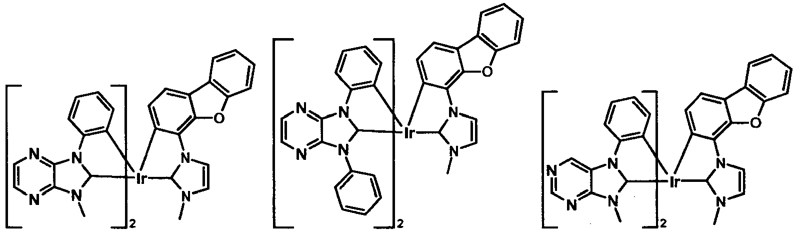

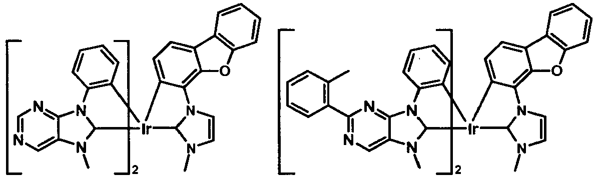

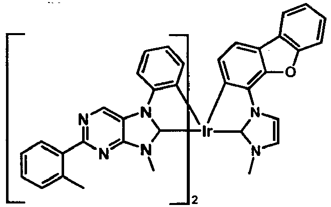

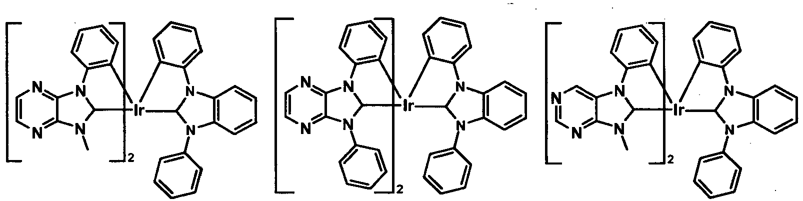

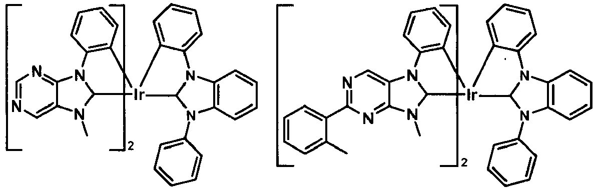

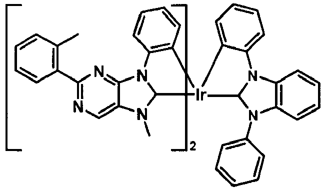

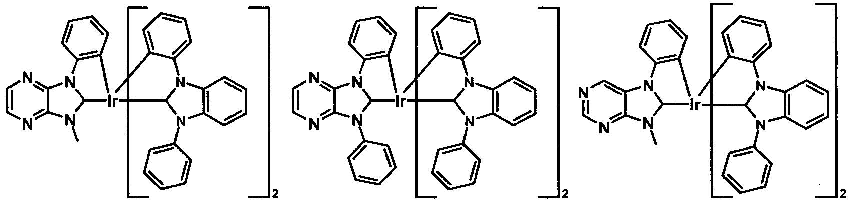

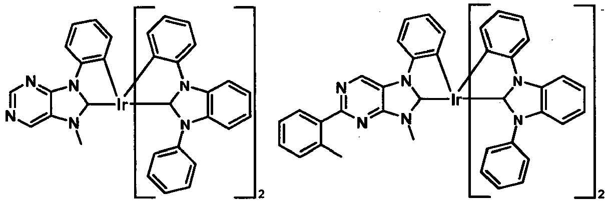

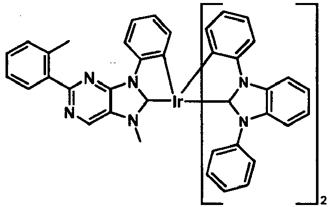

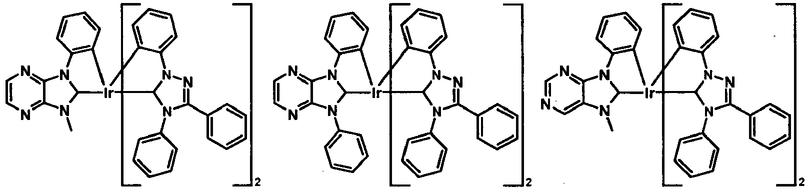

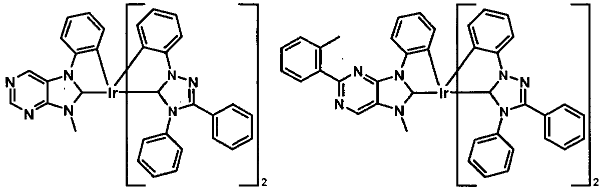

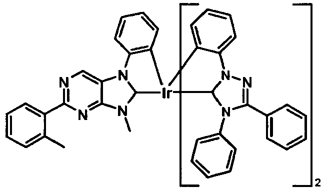

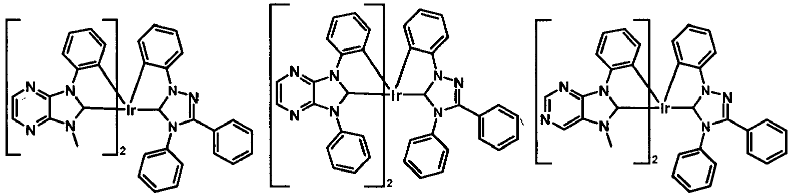

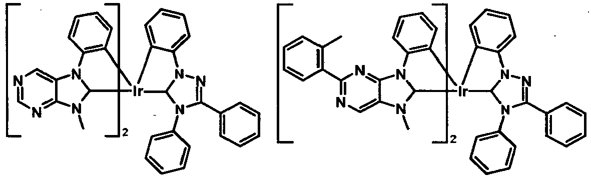

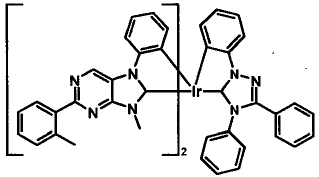

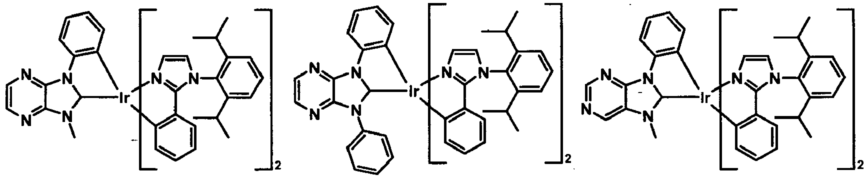

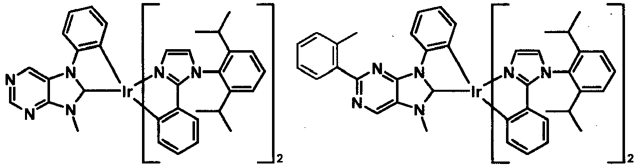

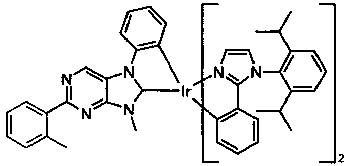

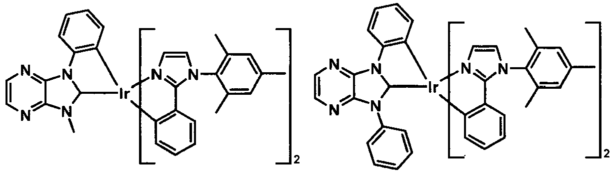

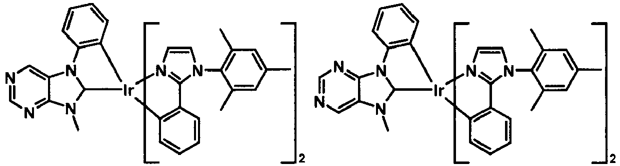

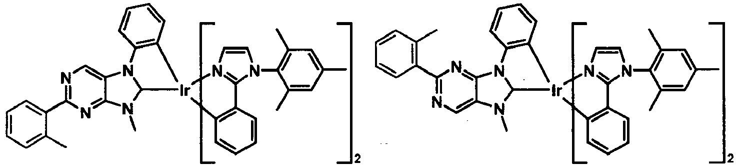

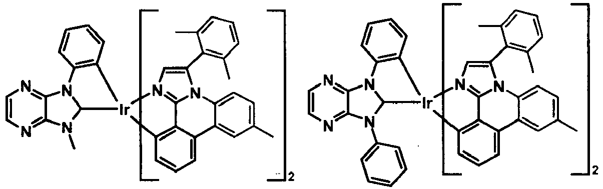

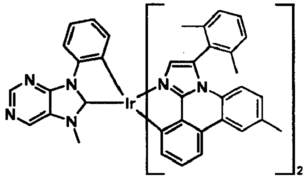

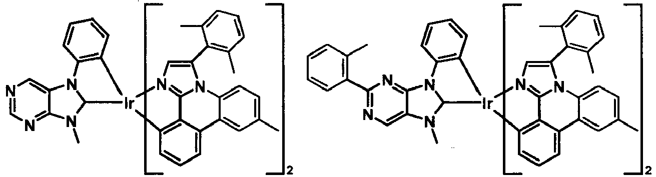

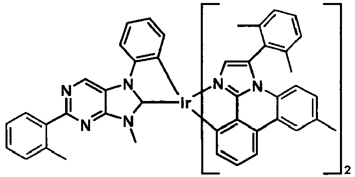

Die erfindungsgemäßen homoleptischen Metall-Carben-Komplexe können als faciale oder meridionale Isomere vorliegen, wobei die facialen Isomere bevorzugt sind.

Bei den heteroleptischen Metall-Carben-Komplexen können vier verschiedene Isomere vorliegen, wobei die pseudo-facialen Isomere bevorzugt sind.

Die vorliegende Erfindung betrifft des Weiteren auch ein Verfahren zur Herstellung der erfindungsgemäßen Metall-Carben-Komplexe durch Inkontaktbringen von geeigneten M enthaltenden Verbindungen mit den entsprechenden Liganden bzw. Ligandvorläufern.

In einer bevorzugten Ausführungsform des erfindungsgemäßen Verfahrens wird eine geeignete Verbindung enthaltend das entsprechende Metall M, d. h. Iridium oder Platin, bevorzugt Iridium, und entsprechende Carbenliganden, bevorzugt in deprotonierter Form als freies Carben oder in Form eines geschützten Carbens z. B. als Silber-Carbenkomplex, in Kontakt gebracht. Geeignete Vorläuferverbindungen enthalten die entsprechenden Substituenten R¹ bis R⁹ und R²⁵, die in den Komplexen der allgemeinen Formel (I) vorliegen sollen.

Daher betrifft die vorliegende Erfindung insbesondere das erfindungsgemäße Verfahren, wobei als Ligandvorläufer ein entsprechender Ag-Carben-Komplex eingesetzt wird.

In einer weiteren bevorzugten Ausführungsform des erfindungsgemäßen Verfahrens werden als Ligandvorläufer organische Verbindungen eingesetzt, die mit geeigneten M enthaltenden Verbindungen umgesetzt werden. Dabei kann das Freisetzen des Carbens durch Entfernen flüchtiger Substanzen, beispielsweise niedere Alkohole wie Methanol, Ethanol, beispielsweise bei erhöhter Temperatur und/oder erniedrigtem Druck und/oder Verwendung von Molekularsieben, welche die abgespaltenen Alkoholmoleküle binden, aus Vorläuferverbindungen der Carbenliganden erfolgen. Dieses Verfahren wird insbesondere bei Verwendung der Verbindungen der allgemeinen Formel (XII) durchgeführt. Entsprechende Verfahren sind dem Fachmann bekannt.

Daher betrifft die vorliegende Erfindung auch das erfindungsgemäße Verfahren, wobei als Ligandvorläufer eine Verbindung der allgemeinen Formel (IV) 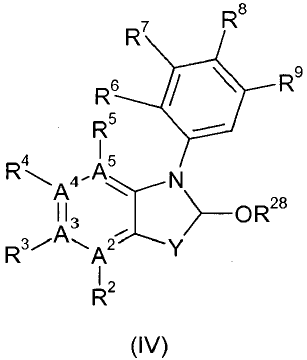 eingesetzt wird, wobei Y, A², A³, A⁴, A⁵, R², R³, R⁴, R⁵, R⁶, R⁷, R⁸ und R⁹ die gleichen Bedeutungen wie oben bezüglich der Verbindungen der allgemeinen Formel (I) definiert aufweisen, und R²⁸ die folgende Bedeutung aufweist:
- R²⁸: unabhängig voneinander SiR²⁹R³⁰R³¹, Aryl, Heteroaryl, Alkyl, Cycloalkyl oder Heterocycloalkyl,
- R²⁹, R³⁰, R³¹: unabhängig voneinander Aryl, Heteroaryl, Alkyl, Cycloalkyl oder Heterocycloalkyl.

Die Definitionen von Aryl, Heteroaryl, Alkyl, Cycloalkyl oder Heterocycloalkyl sind oben genannt.

In einer besonders bevorzugten Ausführungsform bedeutet R²⁸ Alkyl, insbesondere C₁-C₂₀-Alkyl, bevorzugt C₁-C₁₀-Alkyl, besonders bevorzugt C₁-C₈-Alkyl, beispielsweise Methyl, Ethyl, Propyl wie n-Propyl, iso-Propyl, Butyl wie n-Butyl, iso-Butyl, tert-Butyl, Pentyl, Hexyl, Heptyl oder Octyl.

Ganz besonders bevorzugt bedeutet R²⁸ in der Verbindung der allgemeinen Formel (IV) Methyl oder Ethyl.

Verbindungen der allgemeinen Formel (IV) sind im Allgemeinen nach dem Fachmann bekannten Verfahren zugänglich. Für den erfindungsgemäß besonders bevorzugten Fall, dass Y NR¹ bedeutet, können entsprechende Verbindungen der allgemeinen Formel (IV) durch Umsetzung von Verbindungen der allgemeinen Formel (V) 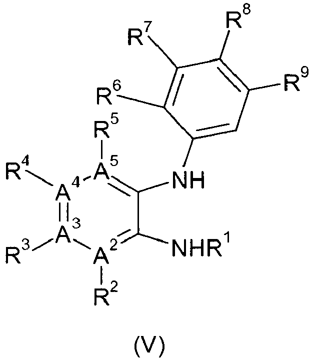 mit Verbindungen der allgemeinen Formel (VI)

HC(OR²⁸)₃ (VI),

erhalten werden, wobei A², A³, A⁴, A⁵, R¹, R², R³, R⁴, R⁵, R⁶, R⁷, R⁸, R⁹ und R²⁸ die gleichen Bedeutungen wie oben bezüglich der Verbindungen der allgemeinen Formel (I) bzw. (IV) definiert aufweisen.

Diese Herstellung der Verbindungen der allgemeinen Formel (IV) kann in Anwesenheit oder in Abwesenheit eines Lösungsmittels erfolgen. Geeignete Lösungsmittel sind weiter unten genannt. In einer bevorzugten Ausführungsform werden die Verbindungen der allgemeinen Formel (IV) in Substanz hergestellt, oder die Verbindung der allgemeinen Formel (VI) wird in einem Überschuss zugegeben, so dass diese als Lösungsmittel fungiert.

Verbindungen der allgemeinen Formeln (V) und (VI) sind kommerziell erhältlich und/oder durch dem Fachmann bekannte Verfahren zugänglich, beispielsweise sind Verbindungen der allgemeinen Formel (V) durch Umsetzung der entsprechenden Chloride mit den entsprechenden Aminen zugänglich.

Die Herstellung der Verbindungen der allgemeinen Formel (IV) erfolgt im Allgemeinen bei einer Temperatur von 10 bis 150 °C, bevorzugt 40 bis 120 °C, besonders bevorzugt 60 bis 110 °C.

Die Reaktionsdauer beträgt im Allgemeinen 2 bis 48 Stunden, bevorzugt 6 bis 24 Stunden, besonders bevorzugt 8 bis 16 Stunden.

Nach erfolgter Umsetzung kann das gewünschte Produkt nach üblichen, dem Fachmann bekannten Verfahren isoliert bzw. gereinigt werden, beispielsweise Filtration, Umkristallisation, Säulenchromatographie etc.

Entsprechende Verbindungen, insbesondere Komplexe, enthaltend das entsprechende Metall M, bevorzugt Iridium, sind dem Fachmann bekannt. besonders geeignete Verbindungen enthaltend Platin oder Iridium enthalten beispielsweise Liganden wie Halogenide, bevorzugt Chlorid, 1,5-Cyclooctadien (COD), Cycloocten (COE), Phosphine, Cyanide, Alkoholate, Pseudohalogenide und/oder Alkyl.

Besonders bevorzugte Komplexe enthaltend das entsprechende Metall, insbesondere Iridium, sind ausgewählt aus der Gruppe bestehend aus [Ir(COD)Cl]₂, [Ir(COE)₂Cl]₂ IrCl₃ x H₂O, Ir(acac)₃, Ir(COD)₂BF₄, Ir(COD)₂BARF (BARF = tetrakis-[3,5-bis(trifluormethyl)phenyl]borat)), Pt(COD)Cl₂, Pt(acac)₂, [Pt(C₆H₁₀)Cl₂]₂, K₂PtCl₆ und Mischungen davon.

Die Deprotonierung der Carbenliganden-Vorläufer, bevorzugt vor der Reaktion, erfolgt beispielsweise durch dem Fachmann bekannte basische Verbindungen, beispielsweise basische Metallate, basische Metallacetate, -acetylacetonate oder -alkoxylate oder Basen wie KO^{t}Bu, NaO^{t}Bu, LiO^{t}Bu, NaH, Silylamide, Ag₂O sowie Phosphazen-Basen. Besonders bevorzugt wird mit Ag₂O deprotoniert und das entsprechende Ag-Carben erhalten, welches mit der M enthaltenden Verbindung zu den erfindungsgemäßen Komplexen umgesetzt wird.

Das erfindungsgemäße Verfahren zur Herstellung der Komplexe der allgemeinen Formel (I) unter Verwendung der Verbindungen der allgemeinen Formel (IV) weist den Vorteil auf, dass die Verbindungen der allgemeinen Formel (IV) stabile Zwischenstufe darstellen, die unter normalen Laborbedingungen leicht handhabbar sind bzw. isoliert werden können. Des Weiteren sind die Verbindungen der allgemeinen Formel (IV) löslich in üblichen organischen Lösungsmitteln, so dass die Herstellung der erfindungsgemäßen Komplexe der allgemeinen Formel (I) in homogener Lösung möglich ist, so dass eine Aufarbeitung des gewünschten Produkts, d. h. der Komplexe der allgemeinen Formel (I), beispielsweise zur Isolierung und/oder Reinigung, leichter möglich ist.

Das Inkontaktbringen erfolgt bevorzugt in einem Lösungsmittel. Geeignete Lösungsmittel sind dem Fachmann an sich bekannt und sind bevorzugt ausgewählt aus der Gruppe bestehend aus aromatischen oder aliphatischen Lösungsmitteln, beispielsweise Benzol, Toluol, Xylol oder Mesitylen, cyclischen oder acyclischen Ethern, beispielsweise Dioxan oder THF, Alkoholen, Estern, Amiden, Ketonen, Nitrilen, halogenierten Verbindungen und Mischungen davon. Besonders bevorzugte Lösungsmittel sind Toluol, Xylole, Mesitylen und Dioxan.

Das molare Verhältnis von eingesetztem Metall-Nichtcarben-Komplex zu eingesetztem Carben-Ligandvorläufer beträgt im Allgemeinen 1 zu 10 bis 10 zu 1, bevorzugt 1 zu 1 bis 1 zu 6, besonders bevorzugt 1 zu 2 bis 1 zu 5.

Das Inkontaktbringen erfolgt im Allgemeinen bei einer Temperatur von 20 bis 200 °C, bevorzugt 50 bis 150 °C, besonders bevorzugt 60 bis 130 °C.

Die Reaktionsdauer ist abhängig von dem gewünschten Carben-Komplex und beträgt im Allgemeinen 0,02 bis 50 Stunden, bevorzugt 0,1 bis 24 Stunden, besonders bevorzugt 1 bis 12 Stunden.

Die nach der Reaktion erhaltenen Komplexe der allgemeinen Formel (I) können gegebenenfalls nach dem Fachmann bekannten Verfahren, beispielsweise Waschen, Kristallisation oder Chromatographie gereinigt werden und gegebenenfalls unter dem Fachmann ebenfalls bekannten Bedingungen, beispielsweise säurevermittelt, thermisch oder photochemisch, isomerisiert werden.

Die vorstehend genannten Metall-Carben-Komplexe und Mischungen davon sind hervorragend als Emittermoleküle in organischen Licht-emittierenden Dioden (OLEDs) geeignet. Durch Variationen der Liganden ist es möglich, entsprechende Komplexe bereitzustellen, die Elektrolumineszenz im roten, grünen sowie insbesondere im blauen Bereich des elektromagnetischen Spektrums zeigen. Die erfindungsgemäßen Metall-Carben-Komplexe der allgemeinen Formel (I) eignen sich daher hervorragend als Emittersubstanzen, da sie eine Emission (Elektrolumineszenz) im sichtbaren Bereich des elektromagnetischen Spektrums, beispielsweise bei 400 bis 800 nm, bevorzugt 400 bis 600 nm, aufweisen. Durch die erfindungsgemäßen Komplexe ist es möglich, Verbindungen bereitzustellen, die Elektrolumineszenz im roten, grünen sowie im blauen Bereich des elektromagnetischen Spektrums aufweisen. Somit ist es möglich, mithilfe der erfindungsgemäßen Komplexe als Emittersubstanzen technisch einsetzbare OLEDs bereitzustellen.

Des Weiteren können die erfindungsgemäßen Metall-Carben-Komplexe der allgemeinen Formel (I) als Matrixmaterial, Ladungstransportmaterial, insbesondere Lochtransportmaterial, und/oder Ladungsblocker eingesetzt werden.

Bevorzugt werden die erfindungsgemäßen Metall-Carben-Komplexe der allgemeinen Formel (I) als Emitter und/oder Lochtransportmaterial eingesetzt, besonders bevorzugt als Emitter.

Besondere Eigenschaften der erfindungsgemäßen Metall-Carben-Komplexe der allgemeinen Formel (I) sind besonders gute Effizienzen und lange Lebensdauern beim Einsatz in OLEDs.

Ein weiterer Gegenstand der vorliegenden Anmeldung ist daher eine OLED, enthaltend wenigstens einen erfindungsgemäßen Metall-Carben-Komplex der allgemeinen Formel (I). Der erfindungsgemäße Metall-Carben-Komplex der allgemeinen Formel (I) wird in der OLED bevorzugt als Emitter, Matrixmaterial, Ladungstransportmaterial, insbesondere Lochtransportmaterial, und/oder Ladungsblocker eingesetzt, besonders bevorzugt als Emitter und/oder Lochtransportmaterial, ganz besonders bevorzugt als Emitter.

Ein weiterer Gegenstand der vorliegenden Anmeldung ist auch die Verwendung der Metall-Carben-Komplexe der allgemeinen Formel (I) in OLEDs, bevorzugt als Emitter, Matrixmaterial, Ladungstransportmaterial, insbesondere Lochtransportmaterial, und/oder Ladungsblocker, besonders bevorzugt als Emitter und/oder Lochtransportmaterial, ganz besonders bevorzugt als Emitter.

Organische Leuchtdioden sind grundsätzlich aus mehreren Schichten aufgebaut, z.B.:
- Anode (1)
- Löcher-transportierende Schicht (2)
- Licht-emittierende Schicht (3)
- Elektronen-transportierende Schicht (4)
- Kathode (5)

Es ist jedoch auch möglich, dass die OLED nicht alle der genannten Schichten aufweist, zum Beispiel ist eine OLED mit den Schichten (1) (Anode), (3) (Licht-emittierende Schicht) und (5) (Kathode) ebenfalls geeignet, wobei die Funktionen der Schichten (2) (Löcher-transportierende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Die Metall-Carben-Komplexe der allgemeinen Formel (I) werden bevorzugt in der Licht-emittierenden Schicht (3) als Emittermoleküle und/oder Matrixmaterialien eingesetzt. Die erfindungsgemäßen Metall-Carben-Komplexe der allgemeinen Formel (I) können auch - zusätzlich zu dem Einsatz als Emittermoleküle und/oder Matrixmaferialien in der Licht-emittierenden Schicht (3) oder anstelle des Einsatzes in der Licht-emittierenden Schicht - als Ladungstransportmaterial in der Löcher-transportierenden Schicht (2) oder in der Elektronen-transportierenden Schicht (4) und/oder als Ladungsblocker eingesetzt werden, wobei der Einsatz als Ladungstransportmaterial in der Löcher-transportierenden Schicht (2) (Lochtransportmaterial) bevorzugt ist.

Ein weiterer Gegenstand der vorliegenden Anmeldung ist daher eine Licht-emittierende Schicht enthaltend mindestens einen der erfindungsgemäßen Metall-Carben-Komplexe der allgemeinen Formel (I), bevorzugt als Emittermolekül. Bevorzugte Metall-Carben-Komplexe der allgemeinen Formel (I) sind bereits vorstehend genannt.

Die erfindungsgemäß verwendeten Metall-Carben-Komplexe der allgemeinen Formel (I) können in Substanz, d. h. ohne weitere Zusätze, in der Licht-emittierenden Schicht vorliegen. Es ist jedoch auch möglich, dass neben den erfindungsgemäß eingesetzten Metall-Carben-Komplexen der allgemeinen Formel (I) weitere Verbindungen in der Licht-emittierenden Schicht vorliegen. Beispielsweise kann ein fluoreszierender Farbstoff anwesend sein, um die Emissionsfarbe des als Emittermoleküls eingesetzten Metall-Carben-Komplexes zu verändern. Des Weiteren kann ein Verdünnungsmaterial (Matrixmaterial) eingesetzt werden. Dieses Verdünnungsmaterial kann ein Polymer sein, z. B. Poly(N-vinylcarbazol) oder Polysilan. Das Verdünnungsmaterial kann jedoch ebenfalls ein kleines Molekül sein, z. B. 4,4'-N,N'-Dicarbazolbiphenyl (CDP) oder tertiäre aromatische Amine. Wenn ein Verdünnungsmaterial eingesetzt wird, beträgt der Anteil der erfindungsgemäßen Metall-Carben-Komplexe der allgemeinen Formel (I) in der Licht-emittierenden Schicht im Allgemeinen weniger als 40 Gew.-%, bevorzugt 3 bis 30 Gew.-%. Bevorzugt werden die erfindungsgemäßen Metall-Carben-Komplexe der allgemeinen Formel (I) in einer Matrix eingesetzt. Somit enthält die Licht-emittierende Schicht bevorzugt mindestens einen erfindungsgemäßen Metall-Carben-Komplex der allgemeinen Formel (I) und mindestens ein Matrixmaterial.

Als Matrixmaterialien sind - neben den vorstehend genannten Verdünnungsmaterialien - grundsätzlich die nachstehend als Loch- und Elektronentransportmaterialien genannten Materialien sowie Carbenkomplexe, z.B. die Carbenkomplexe der Formel (I) oder die in WO 2005/019373 genannten Carbenkomplexe, geeignet. Besonders geeignet sind Carbazolderivate, z.B. 4,4'-Bis(carbazol-9-yl)-2,2'-dimethyl-bipheny) (CDBP), 4,4'-Bis(carbazol-9-yl)-biphenyl (CBP), 1,3-Bis(N-carbazolyl)benzol (mCP), sowie die in den folgenden Anmeldungen genannten Matrixmaterialien: WO2008/034758, WO2009/003919.

Des Weiteren sind Dibenzofurane als Matrixmaterialien geeignet, z.B. die in US 2007/0224446 A1 offenbarten Dibenzofurane, z.B. solche Dibenzofurane, worin mindestens einer der Reste R1 bis R8 eine heterocyclische Gruppe darstellt, z.B. Verbindung A-15, sowie die in WO 2009/069442 A1, WO 2010/090077 A1 und JP 2006/321750 A offenbarten Dibenzofurane.

Weitere geeignete Matrixmaterialien, wobei es sich um kleine Moleküle oder (Co)Polymere von den genannten kleinen Molekülen handeln kann, sind in den nachstehenden Veröffentlichungen genannt:
WO2007108459 (H-1 bis H-37), bevorzugt H-20 bis H-22 und H-32 bis H-37, ganz besonders bevorzugt H-20, H-32, H-36, H-37, WO2008035571 A1 (Host 1 bis Host 6), JP2010135467 (Verbindungen 1 bis 46 und Host-1 bis Host-39 und Host-43), WO2009008100 Verbindungen No.1 bis No.67, bevorzugt No.3, No.4, No.7 bis No. 12, No.55, No.59, No. 63 bis No.67, besonders bevorzugt No. 4, No. 8 bis No. 12, No. 55, No. 59, No.64, No.65, und No. 67, WO2009008099 Verbindungen No. 1 bis No. 110, WO2008140114 Verbindungen 1-1 bis 1-50, WO2008090912 Verbindungen OC-7 bis OC-36 und die Polymeren von Mo-42 bis Mo-51, JP2008084913 H-1 bis H-70, WO2007077810 Verbindungen 1 bis 44, bevorzugt 1, 2, 4-6, 8, 19-22, 26, 28-30, 32, 36, 39-44, WO201001830 die Polymere der Monomeren 1-1 bis 1-9, bevorzugt von 1-3, 1-7, und 1-9, WO2008029729 die (Polymeren von) Verbindungen 1-1 bis 1-36, WO20100443342 HS-1 bis HS-101 und BH-1 bis BH-17, bevorzugt BH-1 bis BH-17, JP2009182298 die (Co-)Polymere basierend auf den Monomeren 1 bis 75, JP2009170764, JP2009135183 die (Co-)Polymere basierend auf den Monomeren 1-14, WO2009063757 bevorzugt die (Co-)Polymere basierend auf den Monomeren 1-1 bis 1-26, WO2008146838 die Verbindungen a-1 bis a-43 und 1-1 bis 1-46, JP2008207520 die (Co)-Polymere basierend auf den Monomeren 1-1 bis 1-26, JP2008066569 die (Co)-Polymere basierend auf den Monomeren 1-1 bis 1-16, WO2008029652 die (Co)-Polymere basierend auf den Monomeren 1-1 bis 1-52, WO2007114244 die (Co)-Polymere basierend auf den Monomeren 1-1 bis 1-18, JP2010040830 die Verbindungen HA-1 bis HA-20, HB-1 bis HB-16, HC-1 bis HC-23 und die (Co)-Polymere basierend auf den Monomeren HD-1 bis HD-12, JP2009021336, WO2010090077 die Verbindungen 1 bis 55, WO2010079678 die Verbindungen H1 bis H42, WO2010067746, WO2010044342 die Verbindungen HS-1 bis HS-101 und Poly-1 bis Poly-4, JP2010114180 die Verbindungen PH-1 bis PH-36, US2009284138 die Verbindungen 1 bis 111 und H1 bis H71, WO2008072596 die Verbindungen 1 bis 45, JP2010021336 die Verbindungen H-1 bis H-38, bevorzugt H-1, WO2010004877 die Verbindungen H-1 bis H-60, JP2009267255 die Verbindungen 1-1 bis 1-105, WO2009104488 die Verbindungen 1-1 bis 1-38, WO2009086028, US2009153034, US2009134784, WO2009084413 die Verbindungen 2-1 bis 2-56, JP2009114369 die Verbindungen 2-1 bis 2-40, JP2009114370 die Verbindungen 1 bis 67, WO2009060742 die Verbindungen 2-1 bis 2-56, WO2009060757 die Verbindungen 1-1 bis 1-76, WO2009060780 die Verbindungen 1-1 bis 1-70, WO2009060779 die Verbindungen 1-1 bis 1-42, WO2008156105 die Verbindungen 1 bis 54, JP2009059767 die Verbindungen 1 bis 20, JP2008074939 die Verbindungen 1 bis 256, JP2008021687 die Verbindungen 1 bis 50, WO2007119816 die Verbindungen 1 bis 37, WO2010087222 die Verbindungen H-1 bis H-31, WO2010095564 die Verbindungen HOST-1 bis HOST-61, WO2007108362, WO2009003898, WO2009003919, WO2010040777, US2007224446 und WO06128800.

In einer besonders bevorzugten Ausführungsform werden eine oder mehrere Verbindungen der nachstehend genannten allgemeinen Formel (X) als Matrixmaterial eingesetzt. Bevorzugte Ausführungsformen der Verbindungen der allgemeinen Formel (X) sind ebenfalls nachstehend genannt.

Die einzelnen der vorstehend genannten Schichten der OLED können wiederum aus zwei oder mehreren Schichten aufgebaut sein. Beispielsweise kann die Löcher-transportierende Schicht aus einer Schicht aufgebaut sein, in die aus der Elektrode Löcher injiziert werden und einer Schicht, die die Löcher von der Loch-injizierenden Schicht weg in die Licht-emittierende Schicht transportiert. Die Elektronen-transportierende Schicht kann ebenfalls aus mehreren Schichten bestehen, z. B. einer Schicht, worin Elektronen durch die Elektrode injiziert werden, und einer Schicht, die aus der Elektronen-injizierenden Schicht Elektronen erhält und in die Licht-emittierende Schicht transportiert. Diese genannten Schichten werden jeweils nach Faktoren wie Energieniveau, Temperaturresistenz und Ladungsträgerbeweglichkeit sowie Energiedifferenz der genannten Schichten mit den organischen Schichten oder den Metallelektroden ausgewählt. Der Fachmann ist in der Lage, den Aufbau der OLEDs so zu wählen, dass er optimal an die erfindungsgemäß als Emittersubstanzen verwendeten heteroleptischen Komplexe gemäß der vorliegenden Erfindung angepasst ist.

Um besonders effiziente OLEDs zu erhalten, sollte das HOMO (höchstes besetztes Molekülorbital) der Loch-transportierenden Schicht mit der Arbeitsfunktion der Anode angeglichen sein und das LUMO (niedrigstes unbesetztes Molekülorbital) der elektronentransportierenden Schicht sollte mit der Arbeitsfunktion der Kathode angeglichen sein.

Ein weiterer Gegenstand der vorliegenden Anmeldung ist eine OLED enthaltend mindestens eine erfindungsgemäße Licht-emittierende Schicht. Die weiteren Schichten in der OLED können aus einem beliebigen Material aufgebaut sein, das üblicherweise in solchen Schichten eingesetzt wird und dem Fachmann bekannt ist.

Geeignete Materialien für die vorstehend genannten Schichten (Anode, Kathode, Loch- und Elektroneninjektionsmaterialien, Loch- und Elektronentransportmaterialien und Loch- und Elektronenblockermaterialien, Matrixmaterialien, Fluoreszenz- und Phosphoreszenzemitter) sind dem Fachmann bekannt und z.B. in H. Meng, N. Herron, Organic Small Molecule Materials for Organic Light-Emitting Devices in Organic Light-Emitting Materials and Devices, Ed.: Z. Li, H. Meng, Taylor & Francis, 2007, Chapter 3, Seiten 295 bis 411 genannt.

Die Anode ist eine Elektrode, die positive Ladungsträger bereitstellt. Sie kann zum Beispiel aus Materialien aufgebaut sein, die ein Metall, eine Mischung verschiedener Metalle, eine Metalllegierung, ein Metalloxid oder eine Mischung verschiedener Metalloxide enthält. Alternativ kann die Anode ein leitendes Polymer sein. Geeignete Metalle umfassen die Metalle der Gruppen 11, 4, 5 und 6 des Periodensystems der Elemente sowie die Übergangsmetalle der Gruppen 8 bis 10. Wenn die Anode lichtdurchlässig sein soll, werden im Allgemeinen gemischte Metalloxide der Gruppen 12, 13 und 14 des Periodensystems der Elemente eingesetzt, zum Beispiel Indium-Zinn-Oxid (ITO). Es ist ebenfalls möglich, dass die Anode (1) ein organisches Material, zum Beispiel Polyanilin enthält, wie beispielsweise in Nature, Vol. 357, Seiten 477 bis 479 (11. Juni 1992) beschrieben ist. Zumindest entweder die Anode oder die Kathode sollten mindestens teilweise transparent sein, um das gebildete Licht auskoppeln zu können.

Geeignete Lochtransportmaterialien für die Schicht (2) des erfindungsgemäßen OLEDs sind zum Beispiel in Kirk-Othmer Encyclopedia of Chemical Technology, 4. Auflage, Vol. 18, Seiten 837 bis 860, 1996 offenbart. Sowohl Löcher transportierende Moleküle als auch Polymere können als Lochtransportmaterial eingesetzt werden. Üblicherweise eingesetzte Löcher transportierende Moleküle sind ausgewählt aus der Gruppe bestehend aus 4,4'-Bis[N-(1-naphthyl)-N-phenyl-amino]biphenyl (α-NPD), N, N'-Diphenyl-N, N'-Bis(3-methylphenyl)-[1,1'-biphenyl]-4,4'-diamin (TPD), 1,1-Bis[(di-4-tolylamino)phenyl]cyclohexan (TAPC), N, N'-Bis(4-methylphenyl)-N, N'-Bis(4-ethylphenyl)-[1,1'-(3,3'-dimethyl)biphenyl]-4,4'-diamin (ETPD), Tetrakis-(3-methylphenyl)-N,N,N',N'-2,5-phenylendiamin (PDA), α-Phenyl-4-N,N-diphenylamino-styrol (TPS), p-(Diethylamino)-benzaldehyddiphenylhydrazon (DEH), Triphenylamin (TPA), Bis[4-(N,N-diethylamino)-2-methylphenyl)(4-methyl-phenyl)methan (MPMP), 1-Phenyl-3-[p-(diethylamino)styryl]-5-[p-(diethylamino)phenyl]pyrazolin (PPR oder DEASP), 1,2-trans-Bis(9H-carbazol-9-yl)cyclobutan (DCZB), N,N,N',N'-tetrakis(4-methylphenyl)-(1,1'-biphenyl)-4,4'-diamin (TTB), Fluorenverbindungen wie 2,2',7,7'-Tetra(N, N-di-tolyl)amino-9,9-spiro-bifluoren (Spiro-TTB), N,N'-Bis(naphthalen-1-yl)-N,N'-bis(phenyl)-9,9-spirobifluoren (Spiro-NPB) und 9,9-Bis(4-(N,N-bis-biphenyl-4-yl-amino)phenyl-9H-fluoren, Benzidinverbindungen wie N,N'-Bis(naphthalen-1-yl)-N,N'-bis(phenyl)-benzidin und Porphyrinverbindungen wie Kupferphthalocyanine. Üblicherweise eingesetzte Löcher transportierende Polymere sind ausgewählt aus der Gruppe bestehend aus Polyvinylcarbazolen, (Phenylmethyl)polysilanen und Polyanilinen. Es ist ebenfalls möglich, Löcher transportierende Polymere durch Dotieren Löcher transportierender Moleküle in Polymere wie Polystyrol und Polycarbonat zu erhalten. Geeignete Löcher transportierende Moleküle sind die bereits vorstehend genannten Moleküle.

Weiterhin können - in einer Ausführungsform - Carbenkomplexe als Lochleitermaterialien eingesetzt werden, wobei die Bandlücke des mindestens einen Lochleitermaterials im Allgemeinen größer ist als die Bandlücke des eingesetzten Emittermaterials. Dabei ist unter Bandlücke im Sinne der vorliegenden Anmeldung die Triplett-Energie zu verstehen. Geeignete Carben-Komplexe sind z. B. die erfindungsgemäßen Carben-Komplexe der allgemeinen Formel (I), Carben-Komplexe wie sie in WO 2005/019373 A2, WO 2006/056418 A2, WO 2005/113704, WO 2007/115970, WO 2007/115981 und WO 2008/000727 beschrieben sind. Ein Beispiel für einen geeigneten Carben-Komplex ist Ir(DPBIC)₃ mit der Formel:

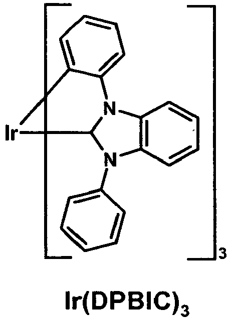

Die Löcher-transportierenden Schicht kann auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Die elektronische Dotierung ist dem Fachmann bekannt und zum Beipsiel in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo, Appl. Phys. Lett., Vol. 82, No. 25, 23 June 2003 und Pfeiffer et al., Organic Electronics 2003, 4, 89 - 103 und K. Walzer, B. Maennig, M. Pfeiffer, K. Leo, Chem. Soc. Rev. 2007, 107, 1233 offenbart. Beispielsweise ist es möglich, Mischungen in der Löcher-transportierenden Schicht einzusetzen, im Besonderen Mischungen, die zu einer elektrischen p-Dotierung der Löcher-transportierenden Schicht führen. Eine p-Dotierung wird durch das Hinzufügen von oxidierenden Materialien erreicht. Diese Mischungen können beispielsweise die folgenden Mischungen sein: Mischungen aus den oben genannten Lochtransportmaterialien mit mindestens einem Metalloxid, z.B. MoO₂, MoO₃, WOₓ, ReO₃ und/oder V₂O₅ , bevorzugt MoO₃ und/oder ReO₃, besonders bevorzugt ReO₃ oder Mischungen umfassend die vorstehend genannten Lochtransportmatierialien und eine oder mehrere Verbindungen ausgewählt aus 7,7,8,8-Tetracyanoquinodimethan (TCNQ), 2,3,5,6-Tetrafluoro-7,7,8,8-tetracyano-quinodimethan (F₄-TCNQ), 2,5-Bis(2-hydroxy-ethoxy)-7,7,8,8-tetracyanoquino-dimethan, Bis(tetra-n-butyl-ammonium)tetracyanodipheno-quino-dimethan, 2,5-Dimethyl-7,7,8,8-tetracyanoquinodimethan, Tetracyanoethylen, 11,11,12,12-Tetracyano-naphtho-2,6-quinodimethan, 2-Fluoro-7,7,8,8-tetracyanoquino-dimethan, 2,5-Difluoro-7,7,8,8-tetracyanoquinodimethan, Dicyanomethylen-1,3,4,5,7,8-hexafluoro-6H-naphthalen-2-yliden)-malononitril (F₆-TNAP), Mo(tfd)₃ (aus Kahn et al., J. Am. Chem. Soc. 2009, 131 (35), 12530-12531), Verbindungen wie sie in EP1988587 und in EP2180029 beschrieben sind und Chinonverbindungen wie sie in EP 09153776.1 erwähnt sind.

Geeignete Elektronen transportierende Materialien für die Schicht (4) der erfindungsgemäßen OLEDs umfassen mit oxinoiden Verbindungen chelatisierte Metalle wie Tris(8-hydroxychinolato)aluminium (Alq₃), Verbindungen auf Phenanthrolinbasis wie 2,9-Dimethyl-4,7-Diphenyl-1,10-phenanthrolin (DDPA = BCP), 4,7-Diphenyl-1,10-phenanthrolin (Bphen), 2,4,7,9-Tetraphenyl-1,10-phenanthrolin, 4,7-Diphenyl-1,10-phenanthrolin (DPA) oder Phenanthrolin-Derivate, die in EP1786050, in EP1970371, oder in EP1097981 offenbart sind, und Azolverbindungen wie 2-(4-Biphenylyl)-5-(4-t-butylphenyl)-1,3,4-oxadiazol (PBD) und 3-(4-Biphenylyl)-4-phenyl-5-(4-t-butylphenyl)-1,2,4-triazol (TAZ). Des Weiteren sind Dibenzofurane als Elektronen tansportierende Materialien geeignet, z.B. die in US 2007/0224446 A1 offenbarten Dibenzofurane, z.B. solche Dibenzofurane, worin mindestens einer der Reste R1 bis R8 eine heterocyclische Gruppe darstellt, z.B. Verbindung A-15, sowie die in WO 2009/069442 A1, WO 2010/090077 A1 und JP 2006/321750 A offenbarten Dibenzofurane. Dabei kann die Schicht (4) sowohl zur Erleichterung des Elektronentransports dienen als auch als Pufferschicht oder als Sperrschicht, um ein Quenchen des Excitons an den Grenzflächen der Schichten des OLEDs zu vermeiden. Vorzugsweise verbessert die Schicht (4) die Beweglichkeit der Elektronen und reduziert ein Quenchen des Excitons.

Es ist ebenfalls möglich Mischungen von mindestens zwei Materialien in der Elektronen-transportierenden Schicht einzusetzen, wobei mindestens ein Material elektronenleitend ist. Bevorzugt wird in solchen gemischten Elektronen-transportierenden Schichten mindestens eine Phenanthrolin-Verbindung eingesetzt, bevorzugt BCP, oder mindestens eine Pyridinverbindung gemäß der nachstehend genannten Formel (VIII), bevorzugt eine Verbindung der nachstehend genannten Formel (Vlllaa). Besonders bevorzugt werden in gemischten elektronentransportierenden Schichten neben mindestens einer Phenanthrolin-Verbindung zusätzlich Erdalkalimetall- oder Alkalimetall-hydroxychinolat-Komplexe wie beispielsweise Liq eingesetzt. Geeignete Erdalkalimetall- oder Alkalimetall-hydroxychinolat-Komplexe sind nachstehend genannt (Formel VII).

Die Elektronen transportierende Schicht kann auch elektronisch dotiert sein, um die Transporteigenschaften der eingesetzten Materialien zu verbessern, um einerseits die Schichtdicken großzügiger zu gestalten (Vermeidung von Pinholes/Kurzschlüssen) und um andererseits die Betriebsspannung des Devices zu minimieren. Die elektronische Dotierung ist dem Fachmann bekannt und zum Beipsiel in W. Gao, A. Kahn, J. Appl. Phys., Vol. 94, No. 1, 1 July 2003 (p-dotierte organische Schichten); A. G. Werner, F. Li, K. Harada, M. Pfeiffer, T. Fritz, K. Leo, Appl. Phys. Lett., Vol. 82, No. 25, 23 June 2003 und Pfeiffer et al., Organic Electronics 2003, 4, 89 - 103 und K. Walzer, B. Maennig, M. Pfeiffer, K. Leo, Chem. Soc. Rev. 2007, 107, 1233 offenbart. Beispielsweise können Mischungen eingesetzt werden, die zu einer elektrischen n-Dotierung der Elektronen transportierenden Schicht führen. Eine n-Dotierung wird durch das Hinzufügen von reduzierenden Materialien erreicht. Diese Mischungen können beispielsweise sein, Mischungen aus den oben genannten Elektrontransportmaterialien mit (Erd-) Alkalimetallen oder (Erd-)-Alkalimetallsalzen wie beispielsweise Li, Cs, Ca, Sr, Cs₂CO₃, mit Alkalimetall-Komplexen wie beispielsweise 8-Hydroxychinolato-lithium (Liq), sowie mit Y, Ce, Sm, Gd, Tb, Er, Tm, Yb, Li₃N, Rb₂CO₃, Dikaliumphthalat, W(hpp)₄ aus EP 1786050 oder mit Verbindungen, wie sie in EP1837926 B1 beschrieben sind.

Die vorliegende Erfindung betrifft daher auch eine erfindungsgemäße OLED, wobei sie eine Elektronen-transportierende Schicht enthält, umfassend mindestens zwei verschiedene Materialien, wovon mindestens ein Material elektronenleitend ist.

In einer bevorzugten Ausführungsform enthält die Elektronen-transportierende Schicht mindestens eine Verbindung der allgemeinen Formel (VII) 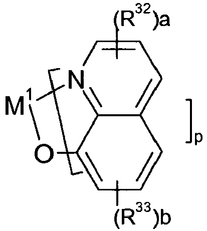 worin bedeuten
- R³² und R³³: unabhängig voneinander F, C₁-C₈-Alkyl, oder C₆-C₁₄-Aryl, das gegebenenfalls mit einer oder mehreren C₁-C₈-Alkyl Gruppen substituiert ist, oder
zwei Substituenten R³² und/oder R³³ bilden gemeinsam einen kondensierten Benzolring, der gegebenenfalls mit einer oder mehreren C₁-C₈-Alkyl Gruppen substituiert ist;
- a und b: unabhängig voneinander 0, oder 1, 2 oder 3,
- M¹: Alkalimetallatom oder Erdalkalimetallatom,
- p: bedeutet 1, wenn M¹ ein Alkalimetallatom ist, p bedeutet 2, wenn M¹ ein Erd-alkalimetallatom ist.

Eine ganz besonders bevorzugte Verbindung der Formel (VII) ist 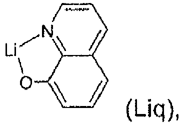 das als einzelne Spezies vorliegen kann, oder in anderen Formen wie LigQg, worin g eine ganze Zahl ist, zum Beispiel LigQ₆. Q ist ein 8-Hydroxyquinolat-Ligand oder ein 8-Hydroxyquinolat-Derivat.

In einer weiteren bevorzugten Ausführungsform enthält die Elektronen-transportierende Schicht mindestens eine Verbindung der Formel (VIII), 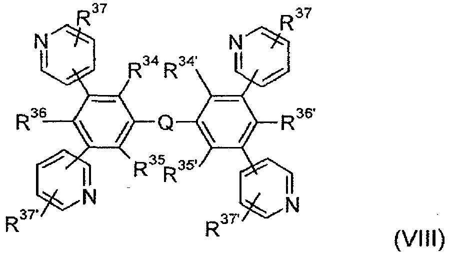 worin bedeuten
- R³⁴, R³⁵, R³⁶, R³⁷, R^{34'}, R^{35'}, R^{36'} und R^{37'}: unabhängig voneinander H, C₁-C₁₈-Alkyl, C₁-C₁₈-Alkyl, das mit E substituiert ist und/oder von D unterbrochen ist, C₆-C₂₄-Aryl, C₆-C₂₄-Aryl, das mit G substituiert ist, C₂-C₂₀-Heteroaryl, oder C₂-C₂₀-Heteroaryl, das mit G substituiert ist,
- Q: eine Arylen- oder Heteroarylengruppe, die jeweils gegebenenfalls mit G substituiert sind;
- D: -CO-; -COO-; -S-; -SO-; -SO₂₇; -O-; -NR⁴⁰-; -SiR⁴⁵R⁴⁶-; -POR⁴⁷-; -CR³⁸=CR³⁹-; oder -C≡C-; und
- E: -OR⁴⁴; -SR⁴⁴; -NR⁴⁰R⁴¹; -COR⁴³; -COOR⁴²; -CONR⁴⁰R⁴¹; -CN; oder F;
- G: E, C₁-C₁₈alkyl, C₁-C₁₈-Alkyl, das durch D unterbrochen ist, C₁-C₁₈-Perfluoroalkyl, C₁-C₁₈-Alkoxy, oder C₁-C₁₈-Alkoxy das mit E substituiert ist und/oder durch D unterbrochen ist,
wherein bedeuten
- R³⁸ und R³⁹: unabhängig voneinander H, C₆-C₁₈-Aryl; C₆-C₁₈-Aryl, das mit C₁-C₁₈-Alkyl oder C₁-C₁₈-Alkoxy substituiert ist; C₁-C₁₈-Alkyl; oder C₁-C₁₈-Alkyl, das durch -O- unterbrochen ist;
- R⁴⁰ und R⁴¹: unabhängig voneinander C₆-C₁₈-Aryl; C₆-C₁₈-Aryl, das mit C₁-C₁₈-Alkyl oder C₁-C₁₈-Alkoxy substituiert ist; C₁-C₁₈-Alkyl; oder C₁-C₁₈-Alkyl, das durch -O-unterbrochen ist; oder
- R⁴⁰ und R⁴¹: bilden gemeinsam einen 6-gliedrigen Ring;
- R⁴² und: R⁴³bedeuten unabhängig voneinander C₆-C₁₈-Aryl; C₆-C₁₈-Aryl das mit C₁-C₁₈-Alkyl, oder C₁-C₁₈-Alkoxy substituiert ist; C₁-C₁₈-Alkyl; oder C₁-C₁₈-Alkyl, das durch -O- unterbrochen ist,
- R⁴⁴: C₆-C₁₈-Aryl; C₆-C₁₈-Aryl, das mit C₁-C₁₈-Alkyl oder C₁-C₁₈-Alkoxy substituiert ist; C₁-C₁₈-Alkyl; oder C₁-C₁₈-Alkyl, das durch -O- unterbrochen ist,
- R⁴⁵ und R⁴⁶: unabhängig voneinander C₁-C₁₈-Alkyl, C₆-C₁₈-Aryl oder C₆-C₁₈-Aryl, das mit C₁-C₁₈-Alkyl substituiert ist,
- R⁴⁷: C₁-C₁₈-Alkyl, C₆-C₁₈-Aryl oder C₆-C₁₈-Aryl, das mit C₁-C₁₈-Alkyl substituiert ist.

Bevorzugte Verbindungen der Formel (VIII) sind Verbindungen der Formel (VIIIa) 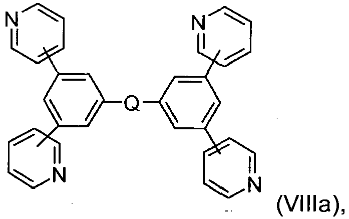 worin Q bedeutet: 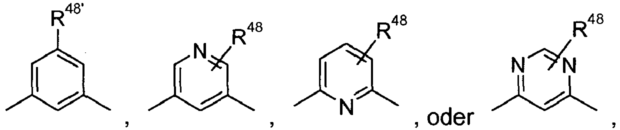 R⁴⁸ bedeutet H, oder C₁-C₁₈-Alkyl und R^{48'} bedeutet H, C₁-C₁₈-Alkyl oder

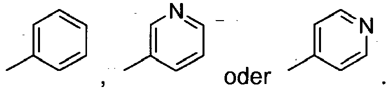

Besonders bevorzugt ist eine Verbindung der Formel (VIIIaa) 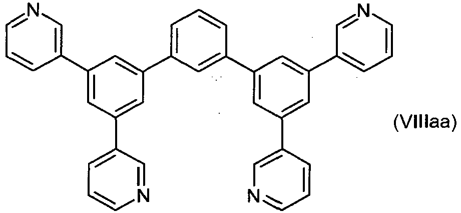 In einer weiteren ganz besonders bevorzugten Ausführungsform enthält die Elektronen-transportierende Schicht eine Verbindung der Formel 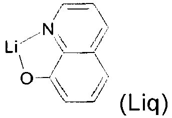 und eine Verbindung der Formel

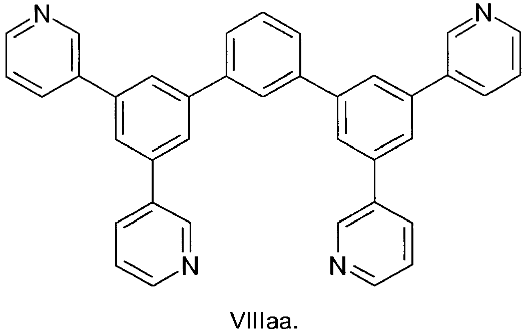

Die Elektronen-transportierende Schicht enthält in einer bevorzugten Ausführungsform die Verbindung der Formel (VII) in einer Menge von 99 bis 1 Gew.-%, bevorzugt 75 bis 25 Gew.-%, besonders bevorzugt etwa 50 Gew.-%, wobei die Menge der Verbindungen der Formeln (VII) und die Menge der Verbindungen der Formeln (VIII) insgesamt 100 Gew.-% ergibt.

Die Herstellung der Verbindungen der Formel (VIII) ist in J. Kido et al., Chem. Commun. (2008) 5821-5823, J. Kido et al., Chem. Mater. 20 (2008) 5951-5953 and JP2008-127326 beschrieben, oder die Verbindungen können analog zu den in den vorstehend genannten Dokumenten offenbarten Verfahren hergestellt werden.

Die Herstellung der Verbindungen der Formel (VII) ist z.B. in Christoph Schmitz et al. Chem. Mater. 12 (2000) 3012-3019 and WO00/32717 beschrieben, oder die Verbindungen können analog zu den in den vorstehend genannten Dokumenten offenbarten Verfahren hergestellt werden.

In einer bevorzugten Ausführungsform betrifft die vorliegende Erfindung eine erfindungsgemäße OLED, wobei die Elektronen-transportierende Schicht mindestens ein Phenanthrolin-Derivat und/oder Pyridin-Derivat enthält.

In einer weiteren bevorzugten Ausführungsform betrifft die Erfindung eine erfindungsgemäße OLED, wobei die Elektronen-transportierende Schicht mindestens ein Phenanthrolin-Derivat und/oder Pyridin-Derivat und mindestens einen Alkalimetall-Hydroxychinolat-Komplex enthält.

In einer weiteren bevorzugten Ausführungsform betrifft die Erfindung eine erfindungsgemäße OLED, wobei die Elektronen-transportierende Schicht mindestens ein Phenanthrolin-Derivat und/oder Pyridin-Derivat und 8-Hydroxychinolato-lithium enthält.

Von den vorstehend als Lochtransportmaterialien und Elektronen-transportierende Materialien genannten Materialien können einige mehrere Funktionen erfüllen. Zum Beispiel sind einige der Elektronen- transportierenden Materialien gleichzeitig Löcher blockende Materialien, wenn sie ein tief liegendes HOMO aufweisen.

Die Kathode (5) ist eine Elektrode, die zur Einführung von Elektronen oder negativen Ladungsträgern dient. Die Kathode kann jedes Metall oder Nichtmetall sein, das eine geringere Arbeitsfunktion aufweist als die Anode. Geeignete Materialien für die Kathode sind ausgewählt aus der Gruppe bestehend aus Alkalimetallen der Gruppe 1, zum Beispiel Li, Cs, Erdalkalimetallen der Gruppe 2, Metallen der Gruppe 12 des Periodensystems der Elemente, umfassend die Seltenerdmetalle und die Lanthanide und Aktinide. Des Weiteren können Metalle wie Aluminium, Indium, Calcium, Barium, Samarium und Magnesium sowie Kombinationen davon eingesetzt werden. Weiterhin können Lithium enthaltende organometallische Verbindungen wie 8-Hydroxychinolato-lithium (Liq), CsF, NaF, KF, Cs₂CO₃ oder LiF zwischen der organischen Schicht und der Kathode als Elektroneninjektionsschicht aufgebracht werden, um die Betriebsspannung (Operating Voltage) zu vermindern.

Die OLED gemäß der vorliegenden Erfindung kann zusätzlich weitere Schichten enthalten, die dem Fachmann bekannt sind. Beispielsweise kann zwischen der Schicht (2) und der Licht emittierenden Schicht (3) eine Schicht aufgebracht sein, die den Transport der positiven Ladung erleichtert und/oder die Bänderlücke der Schichten aneinander anpasst. Alternativ kann diese weitere Schicht als Schutzschicht dienen. In analoger Weise können zusätzliche Schichten zwischen der Licht emittierenden Schicht (3) und der Schicht (4) vorhanden sein, um den Transport der negativen Ladung zu erleichtern und/oder die Bänderlücke zwischen den Schichten aneinander anzupassen. Alternativ kann diese Schicht als Schutzschicht dienen.

In einer bevorzugten Ausführungsform enthält die erfindungsgemäße OLED zusätzlich zu den Schichten (1) bis (5) mindestens eine der im Folgenden genannten weiteren Schichten:
- eine Loch-Injektionsschicht zwischen der Anode (1) und der Löcher-transportierenden Schicht (2);
- eine Blockschicht für Elektronen zwischen der Löcher-transportierenden Schicht (2) und der Licht-emittierenden Schicht (3);
- eine Blockschicht für Löcher zwischen der Licht-emittierenden Schicht (3) und der Elektronen-transportierenden Schicht (4);
- eine Elektronen-Injektionsschicht zwischen der Elektronen-transportierenden Schicht (4) und der Kathode (5).

Wie bereits vorstehend erwähnt, ist es jedoch auch möglich, dass die OLED nicht alle der genannten Schichten (1) bis (5) aufweist, zum Beispiel ist ein OLED mit den Schichten (1) (Anode), (3) (Licht-emittierende Schicht) und (5) (Kathode) ebenfalls geeignet, wobei die Funktionen der Schichten (2) (Löcher-transportierende Schicht) und (4) (Elektronen-transportierende Schicht) durch die angrenzenden Schichten übernommen werden. OLEDs, die die Schichten (1), (2), (3) und (5) bzw. die Schichten (1), (3), (4) und (5) aufweisen, sind ebenfalls geeignet.

Dem Fachmann ist bekannt, wie er (zum Beispiel auf Basis von elektrochemischen Untersuchungen) geeignete Materialien auswählen muss. Geeignete Materialien für die einzelnen Schichten sind dem Fachmann bekannt und z. B. in WO 00/70655 offenbart.

Des Weiteren ist es möglich, dass einige oder alle der Schichten (1), (2), (3), (4) und (5) oberflächenbehandelt sind, um die Effizienz des Ladungsträgertransports zu erhöhen. Die Auswahl der Materialien für jede der genannten Schichten ist bevorzugt dadurch bestimmt, eine OLED mit einer hohen Effizienz zu erhalten.

Die Herstellung der erfindungsgemäßen OLED kann nach dem Fachmann bekannten Methoden erfolgen. Im Allgemeinen wird die OLED durch aufeinanderfolgende Dampfabscheidung (Vapor deposition) der einzelnen Schichten auf ein geeignetes Substrat hergestellt. Geeignete Substrate sind zum Beispiel Glas, anorganische Materialien wie ITO oder IZO oder Polymerfilme. Zur Dampfabscheidung können übliche Techniken eingesetzt werden wie thermische Verdampfung, Chemical Vapor Deposition (CVD), Physical Vapor Deposition (PVD) und andere.

In einem alternativen Verfahren können die organischen Schichten aus Lösungen oder Dispersionen in geeigneten Lösungsmitteln beschichtet werden, wobei dem Fachmann bekannte Beschichtungstechniken angewendet werden. Geeignete Beschichtungstechniken sind beispielsweise das Spin-coating, die Casting-Methode, die Langmuir-Blodgett ("LB")-Methode, die Tintenstrahldruck-Methode, Dip-coating, Letterpress-Druck, Screen-Druck, Doctor-blade-printing, slit-coating, Roller-printing, Reverse-Roller-printing, Offset-Lithography-Druck, flexographischer Druck, Webprinting, Sprühbeschichtung, Beschichtung durch einen Pinsel oder Padprinting und ähnliche. Unter den genannten Verfahren sind - neben der vorstehend erwähnten Dampfabscheidung - das Spin-coating, die Tintenstrahldruck-Methode und die Casting-Methode bevorzugt, da sie besonders einfach und kostengünstig durchzuführen sind. Für den Fall, dass Schichten der OLED durch die Spin-Coating-Methode, die Casting-Methode oder die Tintenstrahldruckmethode erzeugt werden, kann die Beschichtung unter Verwendung einer Lösung, hergestellt durch Auflösen der Zusammensetzung in einer Konzentration von 0,0001 bis 90 Gew.-% in einem geeigneten organischen Lösungsmittel wie Benzol, Toluol, Xylol, Tetrahydrofuran, Methyltetrahydrofuran, N,N-Dimethylformamid, Aceton, Acetonitril, Anisol, Dichlormethan, Dimethylsulfoxid, Wasser und Mischungen davon, erhalten werden.

Es ist möglich, dass die Schichten der OLED alle mit derselben Beschichtungsmethode hergestellt werden. Des Weiteren ist es ebenfalls möglich, dass zur Herstellung der Schichten der OLED zwei oder mehr verschiedene Beschichtungsmethoden durchgeführt werden.

Im Allgemeinen haben die verschiedenen Schichten folgende Dicken: Anode (2) 500 bis 5000 Å (Angstrom), bevorzugt 1000 bis 2000 Å; Löcher-transportierende Schicht (3) 50 bis 1000 Å, bevorzugt 200 bis 800 Å, Licht-emittierende Schicht (4) 10 bis 1000 Å, bevorzugt 100 bis 800 Å, Elektronen transportierende Schicht (5) 50 bis 1000 Å, bevorzugt 200 bis 800 Å, Kathode (6) 200 bis 10.000 Å, bevorzugt 300 bis 5000 Å. Des Weiteren ist es ebenfalls möglich mehrere Schichten durch Vermischen zu vereinigen. Beispielsweise kann das Löcher-transportierende Material mit den Materialien der Licht-emittierenden Schicht gemischt werden und dann gemeinsam aufgebracht werden. Die Lage der Rekombinationszone von Löchern und Elektronen in der erfindungsgemäßen OLED und somit das Emissionsspektrum der OLED können durch die relative Dicke und Konzentrationsverhältnisse jeder Schicht beeinflusst werden. Das bedeutet, die Dicke der Elektronentransportschicht sollte bevorzugt so gewählt werden, dass die Elektronen/Löcher Rekombinationszone in der Licht-emittierenden Schicht liegt. Das Verhältnis der Schichtdicken der einzelnen Schichten in der OLED ist von den eingesetzten Materialien abhängig. Die Schichtdicken von gegebenenfalls eingesetzten zusätzlichen Schichten sind dem Fachmann bekannt.

In einer bevorzugten Ausführungsform betrifft die vorliegende Erfindung eine OLED enthaltend mindestens einen erfindungsgemäßen Metall-Carben-Komplex, sowie mindestens eine Verbindung der allgemeinen Formel (X) 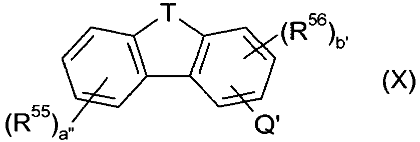 worin bedeuten
- T: NR⁵⁷, S, O oder PR⁵⁷, bevorzugt S oder O, besonders bevorzugt O;
- R⁵⁷: Aryl, Heteroaryl, Alkyl, Cycloalkyl oder Heterocycloalkyl;
- Q': -NR⁵⁸R⁵⁹, -P(O)R⁶⁰R⁶¹, -PR⁶²R⁶³, -S(O)₂R⁶⁴, -S(O)R⁶⁵, -SR⁶⁶ oder -OR⁶⁷, bevorzugt -NR⁵⁸R⁵⁹; besonders bevorzugt 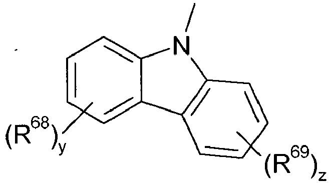 worin bedeuten
R⁶⁸, R⁶⁹ unabhängig voneinander Alkyl, Cycloalkyl, Heterocycloalkyl, Aryl oder Heteroaryl; bevorzugt Methyl, Carbazolyl, Dibenzofuryl oder Dibenzothienyl;
y, z unabhängig voneinander 0, 1, 2, 3 oder 4, bevorzugt 0 oder 1;
R⁵⁵, R⁵⁶ unabhängig voneinander Alkyl, Cycloalkyl, Heterocycloalkyl, Aryl, Heteroaryl, SiR⁷⁰R⁷¹R⁷², eine Gruppe Q' oder eine Gruppe mit Donor- oder Akzeptorwirkung;
- a": 0, 1, 2, 3 oder 4;
- b': 0, 1, 2 oder 3;
- R⁵⁸, R⁵⁹: bilden gemeinsam mit dem N-Atom einen cyclischen Rest mit 3 bis 10 Ringatomen, der unsubstituiert oder mit einem oder mehreren Substituenten ausgewählt aus Alkyl, Cycloalkyl, Heterocycloalkyl, Aryl, Heteroaryl und einer Gruppe mit Donor- oder Akzeptorwirkungsubstituiert sein kann und/oder mit einem oder mehreren weiteren cyclischen Resten mit 3 bis 10 Ringatomen anelliert sein kann, wobei die anellierten Reste unsubstituiert oder mit einem oder mehreren Substituenten ausgewählt aus Alkyl, Cycloalkyl, Heterocycloalkyl, Aryl, Heteroaryl, und einer Gruppe mit Donor- oder Akzeptorwirkung substituiert sein können;
- R⁷⁰, R⁷¹, R⁷², R⁶⁰, R⁶¹, R⁶², R⁶³, R⁶⁴, R⁶⁵, R⁶⁶, R⁶⁷: unabhängig voneinander Aryl, Heteroaryl, Alkyl, Cycloalkyl oder Heterocycloalkyl,
oder
zwei Einheiten der allgemeinen Formel (X) sind über eine lineare oder verzweigte, gesättigte oder ungesättigte, gegebenenfalls durch wenigstens ein Heteroatom unterbrochene Verbrückung, über eine Bindung oder über O miteinander verbrückt.

Bevorzugt sind Verbindungen der Formel (X), worin bedeuten:
- T: S oder O, bevorzugt O, und
- Q': 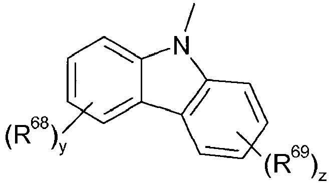 worin bedeuten
- R⁶⁸, R⁶⁹: unabhängig voneinander Alkyl, Cycloalkyl, Heterocycloalkyl, Aryl oder Heteroaryl; bevorzugt Methyl, Carbazolyl, Dibenzofuryl oder Dibenzothienyl;
- y, z: unabhängig voneinander 0, 1, 2, 3 oder 4, bevorzugt 0 oder 1.

Besonders bevorzugte Verbindungen der Formel (X) weisen die folgende Formel (Xa) auf: 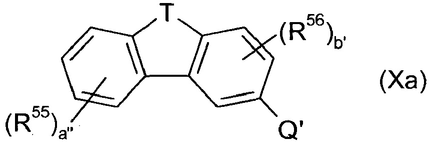 worin die Symbole und Indizes Q', T, R⁵⁵, R⁵⁶, a" und b' die vorstehend genannten Bedeutungen aufweisen.

Ganz besonders bevorzugte Verbindungen der Formel (X) weisen die Formel (Xaa) auf: 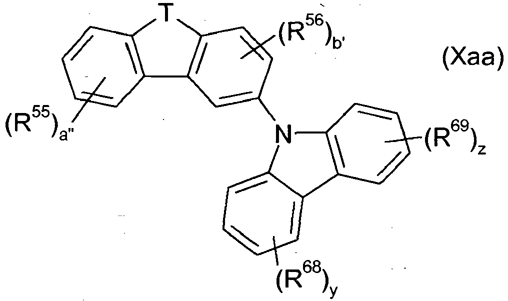 worin die Symbole und Indizes R⁶⁸, R⁶⁹ y, z, T, R⁵⁵, R⁵⁶, a" und b' die vorstehend genannten Bedeutungen aufweisen.

In einer ganz besonders bevorzugten Ausführungsform bedeuten in Formel (Xaa):
- T: O oder S, bevorzugt O;
- a": 1;
- b': 0;
- y, z: unabhängig voneinander 0 oder 1; und
- R⁶⁸, R⁶⁹: unabhängig voneinander Methyl, Carbazolyl, Dibenzofuryl oder Dibenzothienyl
- R⁵⁵: substituiertes Phenyl, Carbazolyl, Dibenzofuryl oder Dibenzothienyl.

In einer weiteren bevorzugten Ausführungsform weisen die Verbindungen der Formel (X) die Formel (XI) oder (XI*) auf: 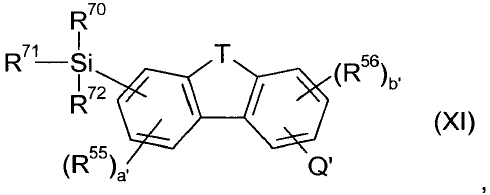 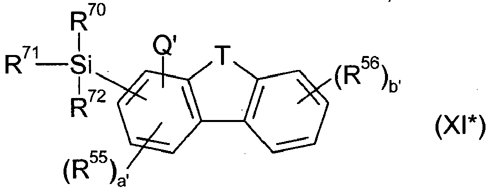 worin bedeuten
- T: NR⁵⁷, S, O oder PR⁵⁷;
- R⁵⁷: Aryl, Heteroaryl, Alkyl, Cycloalkyl oder Heterocycloalkyl;
- Q': -NR⁵⁸R⁵⁹, -P(O)R⁶⁰R⁶¹, -PR⁶²R⁶³, -S(O)₂R⁶⁴, -S(O)R⁶⁵, -SR⁶⁶ oder -OR⁶⁷;
- R⁷⁰, R⁷¹, R⁷²: unabhängig voneinander Aryl, Heteroaryl, Alkyl, Cycloalkyl, Heterocycloalkyl, wobei wenigstens einer der Reste R⁷⁰, R⁷¹, R⁷² mindestens zwei Kohlenstoffatome enthält oder OR⁷³,
- R⁵⁵, R⁵⁶: unabhängig voneinander Alkyl, Cycloalkyl, Heterocycloalkyl, Aryl, Heteroaryl, eine Gruppe Q oder eine Gruppe mit Donor- oder Akzeptorwirkung;
- a', b': für die Verbindung der Formel (XI): unabhängig voneinander 0, 1, 2, 3; für die Verbindung der Formel (XI*) bedeuten a' 0, 1, 2 und b' 0, 1, 2, 3, 4;
- R⁵⁸, R⁵⁹: bilden gemeinsam mit dem N-Atom einen cyclischen Rest mit 3 bis 10 Ringatomen, der unsubstituiert oder mit einem oder mehreren Substituenten ausgewählt aus Alkyl, Cycloalkyl, Heterocycloalkyl, Aryl, Heteroaryl und einer Gruppe mit Donor- oder Akzeptorwirkung substituiert sein kann und/oder mit einem oder mehreren weiteren cyclischen Resten mit 3 bis 10 Ringatomen anelliert sein kann, wobei die anellierten Reste unsubstituiert oder mit einem oder mehreren Substituenten ausgewählt aus Alkyl, Cycloalkyl, Heterocycloalkyl, Aryl, Heteroaryl, und einer Gruppe mit Donor- oder Akzeptorwirkung substituiert sein können;
- R⁷³: unabhängig voneinander SiR⁷⁴R⁷⁵R⁷⁶, Aryl, Heteroaryl, Alkyl, Cycloalkyl oder Heterocycloalkyl, gegebenenfalls substituiert mit einer Gruppe OR⁷⁷
- R⁷⁷: unabhängig voneinander SiR⁷⁴R⁷⁵R⁷⁶, Aryl, Heteroaryl, Alkyl, Cycloalkyl oder Heterocycloalkyl,
- R⁶⁰, R⁶¹, R⁶², R⁶³, R⁶⁴, R⁶⁵, R⁶⁶, R⁶⁷, R⁷⁴, R⁷⁵, R⁷⁶: unabhängig voneinander Aryl, Heteroaryl, Alkyl, Cycloalkyl oder Heterocycloalkyl,
oder
zwei Einheiten der allgemeinen Formeln (XI) und/oder (XI*) sind über eine lineare oder verzweigte, gesättigte oder ungesättigte, gegebenenfalls durch wenigstens ein Heteroatom unterbrochene Verbrückung oder über O miteinander verbrückt, wobei diese Verbrückung in den allgemeinen Formeln (XI) und/oder (XI*) jeweils anstelle von R⁷¹ an die Si-Atome angebunden ist.

Die Verbindungen der allgemeinen Formel (X) können als Matrix (Verdünnungsmaterial), Loch-/Excitonenblocker, Elektronen-/Excitonenblocker, Elektronentransportmaterial oder Lochtransportmaterial in Kombination mit den beanspruchten Komplexen, die dann als Emitter dienen, eingesetzt werden. Erfindungsgemäße OLEDs, die sowohl mindestens eine Verbindung der Formel (X) als auch eine Verbindung der Formel (I) beinhalten, zeigen besonders gute Effizienzen und Lebensdauern. Je nachdem in welcher Funktion die Verbindung der Formel (X) eingesetzt wird, liegt sie rein oder in verschiedenen Mischungsverhältnissen vor. In einer besonders bevorzugten Ausführungsform werden eine oder mehrere Verbindungen der Formel (X) als Matrixmaterial in der Licht-emittierenden Schicht eingesetzt.

Für die Verbindungen der allgemeinen Formel (X), insbesondere für die Reste R⁵⁵ bis R⁷⁷, gilt:
Die Begriffe Arylrest oder -gruppe, Heteroarylrest oder -gruppe, Alkylrest oder - gruppe, Cycloalkylrest oder -gruppe, Heterocycloalkylrest oder -gruppe, Alkenylrest oder -gruppe, Alkinylrest oder -gruppe, und Gruppen mit Donor- und/oder Akzeptorwirkung haben die folgenden Bedeutungen:
   Unter einem Arylrest (oder -gruppe) ist ein Rest mit einem Grundgerüst von 6 bis 30 Kohlenstoffatomen, bevorzugt 6 bis 18 Kohlenstoffatomen zu verstehen, der aus einem aromatischen Ring oder mehreren kondensierten aromatischen Ringen aufgebaut ist. Geeignete Grundgerüste sind zum Beispiel Phenyl, Naphthyl, Anthracenyl oder Phenanthrenyl, Indenyl oder Fluorenyl. Dieses Grundgerüst kann unsubstituiert sein (d. h., dass alle Kohlenstoffatome, die substituierbar sind, Wasserstoffatome tragen), oder an einer, mehreren oder allen substituierbaren Positionen des Grundgerüsts substituiert sein.

Geeignete Substituenten sind zum Beispiel Deuterium, Alkoxyreste, Aryloxyreste, Alkylaminogruppen, Arylaminogruppen, Carbazoylgruppen, Silylgruppen, SiR⁷⁸R⁷⁹R⁸⁰, wobei geeignete Silylgruppen SiR⁷⁸R⁷⁹R⁸⁰ nachstehend genannt sind, Alkylreste, bevorzugt Alkylreste mit 1 bis 8 Kohlenstoffatomen, besonders bevorzugt Methyl, Ethyl oder i-Propyl, Arylreste, bevorzugt C₆-Arylreste, die wiederum substituiert oder unsubstituiert sein können, Heteroarylreste, bevorzugt Heteroarylreste, die mindestens ein Stickstoffatom enthalten, besonders bevorzugt Pyridylreste und Carbazolylreste, Alkenylreste, bevorzugt Alkenylreste, die eine Doppelbindung tragen, besonders bevorzugt Alkenylreste mit einer Doppelbindung und 1 bis 8 Kohlenstoffatomen, Alkinylreste, bevorzugt Alkinylreste mit einer Dreifachbindung, besonders bevorzugt Alkinylreste mit einer Dreifachbindung und 1 bis 8 Kohlenstoffatomen oder Gruppen mit Donor- oder Akzeptorwirkung. Geeignete Gruppen, mit Donor- oder Akzeptorwirkung sind nachstehend genannt. Ganz besonders bevorzugt tragen die substituierten Arylreste Substituenten ausgewählt aus der Gruppe bestehend aus Methyl, Ethyl, Isopropyl, Alkoxy, Heteroaryl, Halogen, Pseudohalogen und Amino, bevorzugt Arylamino. Bevorzugt ist der Arylrest oder die Arylgruppe ein C₆-C₁₈-Arylrest, besonders bevorzugt ein C₆-Arylrest, der gegebenenfalls mit mindestens einem oder mehreren der vorstehend genannten Substituenten substituiert ist. Besonders bevorzugt weist der C₆-C₁₈-Arylrest, bevorzugt C₆-Arylrest, keinen, einen, zwei, drei oder vier, ganz besonders bevorzugt keinen, einen oder zwei der vorstehend genannten Substituenten auf.

Unter einem Heteroarylrest oder einer Heteroarylgruppe sind Reste zu verstehen, die sich von den vorstehend genannten Arylresten dadurch unterscheiden, dass in dem Grundgerüst der Arylreste mindestens ein Kohlenstoffatom durch ein Heteroatom ersetzt ist, sowie dadurch, dass das Grundgerüst der Heteroarylreste bevorzugt 5 bis 18 Ringatome aufweist. Bevorzugte Heteroatome sind N, O und S. Besonders bevorzugt geeignete Heteroarylreste sind stickstoffhaltige Heteroarylreste. Ganz besonders bevorzugt sind ein oder zwei Kohlenstoffatome des Grundgerüsts durch Heteroatome, bevorzugt Stickstoff, ersetzt. Insbesondere bevorzugt ist das Grundgerüst ausgewählt aus Systemen wie Pyridin, Pyrimidin und fünfgliedrigen Heteroaromaten wie Pyrrol, Furan, Pyrazol, Imidazol, Thiophen, Oxazol, Thiazol, Triazol. Des Weiteren kann es sich bei den Heteroarylresten um kondensierte Ringsysteme handeln, z. B. um Benzofuryl,- Benzothienyl-, Benzopyrrolyl-, Dibenzofuryl-, Dibenzothienyl-, Phenanthrolinyl-, Carbazolylreste, Azacarbazolylreste oder Diazacarbazoylreste. Das Grundgerüst kann an einer, mehreren oder allen substituierbaren Positionen des Grundgerüsts substituiert sein. Geeignete Substituenten sind dieselben, die bereits bezüglich der Arylgruppen genannt wurden.

Unter einem Alkylrest oder einer Alkylgruppe ist ein Rest mit 1 bis 20 Kohlenstoffatomen, bevorzugt 1 bis 10 Kohlenstoffatomen, besonders bevorzugt 1 bis 8, ganz besonders bevorzugt 1 bis 4 Kohlenstoffatomen zu verstehen. Dieser Alkylrest kann verzweigt oder unverzweigt sein und gegebenenfalls mit einem oder mehreren Heteroatomen, bevorzugt Si, N, O oder S, besonders bevorzugt N, O oder S, unterbrochen sein. Des Weiteren kann dieser Alkylrest mit einem oder mehreren der bezüglich der Arylgruppen genannten Substituenten substituiert sein. Des Weiteren können die erfindungsgemäß vorliegenden Alkylreste wenigstens ein Halogenatom, beispielsweise F, Cl, Br oder I, insbesondere F, aufweisen. In einer weiteren Ausführungsform können die erfindungsgemäß vorliegenden Alkyreste vollständig fluoriert sein. Es ist ebenfalls möglich, dass der Alkylrest eine oder mehrere (Hetero-)-Arylgruppen trägt. Im Sinne der vorliegenden Anmeldung stellen z. B. Benzylreste somit substituierte Alkylreste dar. Dabei sind alle der vorstehend aufgeführten (Hetero-)Arylgruppen geeignet. Besonders bevorzugt sind die Alkylreste ausgewählt aus der Gruppe bestehend aus Methyl, Ethyl, iso-Propyl, n-Propyl, n-Butyl, iso-Butyl und tert-Butyl, ganz besonders bevorzugt sind Methyl und Ethyl.

Unter einem Cycloalkylrest oder einer Cycloalkylgruppe ist ein Rest mit 3 bis 20 Kohlenstoffatomen, bevorzugt 3 bis 10 Kohlenstoffatomen, besonders bevorzugt 3 bis 8 Kohlenstoffatomen zu verstehen. Dieses Grundgerüst kann unsubstituiert sein (d. h., dass alle Kohlenstoffatome, die substituierbar sind, Wasserstoffatome tragen), oder an einer, mehreren oder allen substituierbaren Positionen des Grundgerüsts substituiert sein. Geeignete Substituenten sind die bereits vorstehend bezüglich der Arylreste genannten Gruppen. Es ist ebenfalls möglich, dass der Cycloalkylrest eine oder mehrere (Hetero-)Arylgruppen trägt. Beispiele für geeignete Cycloalkylreste sind Cyclopropyl, Cyclopentyl und Cyclohexyl.

Unter einem Heterocycloalkylrest oder einer Heterocycloalkylgruppe sind Reste zu verstehen, die sich von den vorstehend genannten Cycloalkylresten dadurch unterscheiden, dass in dem Grundgerüst der Cycloalkylreste mindestens ein Kohlenstoffatom durch ein Heteroatom ersetzt ist. Bevorzugte Heteroatome sind N, O und S. Ganz besonders bevorzugt sind ein oder zwei Kohlenstoffatome des Grundgerüsts der Cycloalkylreste durch Heteroatome ersetzt. Beispiele für geeignete Heterocycloalkylreste sind Reste abgeleitet von Pyrrolidin, Piperidin, Piperazin, Tetrahydrofuran, Dioxan.

Unter einem Alkenylrest oder einer Alkenylgruppe ist ein Rest zu verstehen, der den vorstehend genannten Alkylresten mit mindestens zwei Kohlenstoffatomen entspricht, mit dem Unterschied, dass mindestens eine C-C-Einfachbindung des Alkylrests durch eine C-C-Doppelbindung ersetzt ist. Bevorzugt weist der Alkenylrest eine oder zwei Doppelbindungen auf.

Unter einem Alkinylrest oder einer Alkinylgruppe ist ein Rest zu verstehen, der den vorstehend genannten Alkylresten mit mindestens zwei Kohlenstoffatomen entspricht, mit dem Unterschied, dass mindestens eine C-C-Einfachbindung des Alkylrests durch eine C-C-Dreifachbindung ersetzt ist. Bevorzugt weist der Alkinylrest eine oder zwei Dreifachbindungen auf.

Unter einer Gruppe SiR⁷⁸R⁷⁹R⁸⁰ ist ein Silylrest zu verstehen, worin
R⁷⁸, R⁷⁹ und R⁸⁰ unabhängig voneinander Alkyl, Cycloalkyl, Heterocycloalkyl, Aryl, Heteroaryl oder OR⁷³ bedeuten.

Unter einer Gruppe SiR⁷⁴R⁷⁵R⁷⁶ ist ein Silylrest zu verstehen, worin
R⁷⁴, R⁷⁵ und R⁷⁶ unabhängig voneinander Alkyl, Cycloalkyl, Heterocycloalkyl, Aryl, Heteroaryl oder OR⁷³ bedeuten.

Unter einer Gruppe oder einem Substituenten mit Donor- oder Akzeptorwirkung sind im Sinne der vorliegenden Anmeldung die folgenden Gruppen zu verstehen:
Unter Gruppen mit Donorwirkung sind Gruppen zu verstehen, die einen +I- und/oder +M-Effekt aufweisen, und unter Gruppen mit Akzeptorwirkung sind Gruppen zu verstehen, die einen -I- und/oder -M-Effekt aufweisen. Bevorzugte geeignete Gruppen sind ausgewählt aus C₁-C₂₀-Alkoxy, C₆-C₃₀-Aryloxy, C₁-C₂₀-Alkylthio, C₆-C₃₀-Arylthio, SiR⁸¹R⁸²R⁸³, OR⁷³, Halogenresten, halogenierten C₁-C₂₀-Alkylresten, Carbonyl (-CO(R⁸¹)), Carbonylthio (- C = O (SR⁸¹)), Carbonyloxy (- C = O(OR⁸¹)), Oxycarbonyl (-OC = O(R⁸¹)), Thiocarbonyl (- SC = O(R⁸¹)), Amino (-NR⁸¹R⁸²), Pseudohalogenresten, Amido (- C = O (NR⁸¹)), -NR⁸¹C = O (R⁸³), Phosphonat (- P(O) (OR⁸¹)₂, Phosphat (-OP(O) (OR⁸¹)₂), Phosphin (-PR⁸¹R⁸²), Phosphinoxid (-P(O)R⁸¹₂), Sulfat (-OS(O)₂OR⁸¹), Sulfoxid (-S(O)R⁸¹), Sulfonat (-S(O)₂OR⁸¹), , Sulfonyl (-S(O)₂R⁸¹), Sulfonamid (-S(O)₂NR⁸¹R⁸²), NO₂, Boronsäureestern (-OB(OR⁸¹)₂), Imino (-C = NR⁸¹R⁸²)), Boranresten, Stannanresten, Hydrazinresten, Hydrazonresten, Oximresten, Nitroso-Gruppen, Diazo-Gruppen, Vinylgruppen, Sulfoximinen, Alanen, Germanen, Boroximen und Borazinen.

Die in den vorstehend genannten Gruppen mit Donor- oder Akzeptorwirkung erwähnten Reste R⁸¹, R⁸² und R⁸³ bedeuten unabhängig voneinander:
Substituiertes oder unsubstituiertes C₁-C₂₀-Alkyl oder substituiertes oder unsubstituiertes C₆-C₃₀-Aryl, oder OR⁷⁶ wobei geeignete und bevorzugte Alkyl- und Arylreste vorstehend genannt sind. Besonders bevorzugt bedeuten die Reste R⁸¹, R⁸² und R⁸³ C₁-C₆-Alkyl, z. B. Methyl, Ethyl oder i-Propyl oder Phenyl. In einer bevorzugten Ausführungsform - im Falle von SiR⁸¹R⁸²R⁸³ - bedeuten R⁸¹, R⁸² und R⁸³ bevorzugt unabhängig voneinander substituiertes oder unsubstituiertes C₁-C₂₀-Alkyl oder substituiertes oder unsubstituiertes Aryl, bevorzugt Phenyl.

Bevorzugte Substituenten mit Donor- oder Akzeptorwirkung sind ausgewählt aus der Gruppe bestehend aus:
C₁- bis C₂₀-Alkoxy, bevorzugt C₁-C₆-Alkoxy, besonders bevorzugt Ethoxy oder Methoxy; C₆-C₃₀-Aryloxy, bevorzugt C₆-C₁₀-Aryloxy, besonders bevorzugt Phenyloxy; SiR⁸¹R⁸²R⁸³,wobei R⁸¹,R⁸² und R⁸³ bevorzugt unabhängig voneinander substituiertes oder unsubstituiertes Alkyl oder substituiertes oder unsubstituiertes Aryl. bevorzugt Phenyl, bedeuten; besonders bevorzugt ist mindestens einer der Reste R⁸¹ ,R⁸² oder R⁸³ substituiertes oder unsubstituiertes Phenyl, wobei geeignete Substituenten vorstehend genannt sind; Halogenresten, bevorzugt F, Cl, besonders bevorzugt F, halogenierten C₁-C₂₀-Alkylresten, bevorzugt halogenierten C₁-C₆-Alkylresten, ganz besonders bevorzugt fluorierten C₁-C₆-Alkylresten, z. B. CF₃, CH₂F, CHF₂ oder C₂F₅; Amino, bevorzugt Dimethylamino, Diethylamino oder Diarylamino, besonders bevorzugt Diarylamino; Pseudohalogenresten, bevorzugt CN, -C(O)OC₁-C₄-Alkyl, bevorzugt -C(O)OMe, P(O)R₂, bevorzugt P(O)Ph₂.

Ganz besonders bevorzugte Substituenten mit Donor- oder Akzeptorwirkung sind ausgewählt aus der Gruppe bestehend aus Methoxy, Phenyloxy, halogeniertem C₁-C₄-Alkyl, bevorzugt CF₃, CH₂F, CHF₂, C₂F₅, Halogen, bevorzugt F, CN, SiR⁸¹R⁸²R⁸³, wobei geeignete Reste R⁸¹, R⁸² und R⁸³ bereits genannt sind, Diarylamino (NR⁸⁴R⁸⁵, wobei R⁸⁴, R⁸⁵ jeweils C₆-C₃₀-Aryl bedeuten), -C(O)OC₁-C₄-Alkyl, bevorzugt -C(O)OMe, P(O)Ph₂.

Unter Halogengruppen sind bevorzugt F, Cl und Br, besonders bevorzugt F und Cl, ganz besonders bevorzugt F, zu verstehen.

Unter Pseudohalogengruppen sind bevorzugt CN, SCN und OCN, besonders bevorzugt CN, zu verstehen.

Die vorstehend genannten Gruppen mit Donor- oder Akzeptorwirkung schließen nicht aus, dass weitere der in der vorliegenden Anmeldung genannte Reste und Substituenten, die nicht in der vorstehenden Liste der Gruppen mit Donor- oder Akzeptorwirkung aufgeführt sind, eine Donor- oder Akzeptorwirkung aufweisen.

Die Arylreste oder -gruppen, Heteroarylreste oder -gruppen, Alkylreste oder -gruppen, Cycloalkylreste oder -gruppen, Heterocycloalkylreste oder -gruppen, Alkenylreste oder -gruppen und Gruppen mit Donor- und/oder Akzeptorwirkung können - wie vorstehend erwähnt - substituiert oder unsubstituiert sein. Unter einer unsubstituierten Gruppe ist im Sinne der vorliegenden Anmeldung eine Gruppe zu verstehen, worin die substituierbaren Atome der Gruppe Wasserstoffatome tragen. Unter einer substituierten Gruppe ist im Sinne der vorliegenden Anmeldung eine Gruppe zu verstehen, worin ein oder mehrere substituierbare Atom(e) mindestens an einer Position anstelle eines Wasserstoffatoms einen Substituenten tragen. Geeignete Substituenten sind die vorstehend bezüglich der Arylreste oder -gruppen genannten Substituenten.

Kommen Reste mit denselben Nummerierungen mehrfach in den Verbindungen gemäß der vorliegenden Anmeldung vor, so können diese Reste jeweils unabhängig voneinander die genannten Bedeutungen aufweisen.

Der Rest T in den Verbindungen der Formel (X) bedeutet NR⁵⁷, S, O oder PR⁵⁷, bevorzugt NR⁵⁷, S oder O, besonders bevorzugt O oder S, ganz besonders bevorzugt O.

Der Rest R⁵⁷ bedeutet Aryl, Heteroaryl, Alkyl, Cycloalkyl oder Heterocycloalkyl, bevorzugt Aryl, Heteroaryl oder Alkyl, besonders bevorzugt Aryl, wobei die vorstehend genannten Reste unsubstituiert oder substituiert sein können. Geeignete Substituenten sind vorstehend genannt. Besonders bevorzugt bedeutet R⁶⁵ Phenyl, das mit den vorstehend genannten Substituenten substituiert oder unsubstituiert sein kann. Ganz besonders bevorzugt bedeutet R⁵⁷ unsubstituiertes Phenyl.

Die Gruppe Q' in den Verbindungen der Formel (X) bedeutet -NR⁵⁸R⁵⁹, -P(O)R⁶⁰R⁶¹,-PR⁶²R⁶³, -S(O)₂R⁶⁴, -S(O)R⁶⁵, -SR⁶⁶ oder -OR⁶⁷; bevorzugt NR⁵⁸R⁵⁹, -P(O)R⁶⁰R⁶¹ oder - OR⁶⁷, besonders bevorzugt -NR⁵⁸R⁵⁹.

Die Reste R⁵⁸ bis R⁶⁷ und R⁷⁴ bis R⁷⁶ weisen dabei die folgenden Bedeutungen auf:
- R⁵⁸, R⁵⁹: bilden gemeinsam mit dem N-Atom einen cyclischen Rest mit 3 bis 10 Ringatomen, der unsubstituiert oder mit einem oder mehreren Substituenten ausgewählt aus Alkyl, Cycloalkyl, Heterocycloalkyl, Aryl, Heteroaryl und einer Gruppe mit Donor- oder Akzeptorwirkung substituiert sein kann und/oder mit einem oder mehreren weiteren cyclischen Resten mit 3 bis 10 Ringatomen anelliert sein kann, wobei die anellierten Reste unsubstituiert oder mit einem oder mehreren Substituenten ausgewählt aus Alkyl, Cycloalkyl, Heterocycloalkyl, Aryl, Heteroaryl, und einer Gruppe mit Donor- oder Akzeptorwirkung substituiert sein können;
- R⁶⁰, R⁶¹, R⁶², R⁶³, R⁶⁴, R⁶⁵, R⁶⁶, R⁶⁷, R⁷⁴, R⁷⁵, R⁷⁶: unabhängig voneinander Aryl, Heteroaryl, Alkyl, Cycloalkyl oderHeterocycloalkyl, bevorzugt Aryl oder Heteroaryl, wobei die Resteunsubstituiert oder mit einem oder mehreren der Reste ausgewählt ausAlkyl, Cycloalkyl, Heterocycloalkyl, Aryl, Heteroaryl und einer Gruppe mitDonor oder Akzeptorwirkung substituiert sein können, besonders bevorzugtunsubstituiertes oder substituiertes Phenyl, wobei geeignete Substituentenvorstehend genannt sind, z.B. Tolyl oder eine Gruppe der Formel 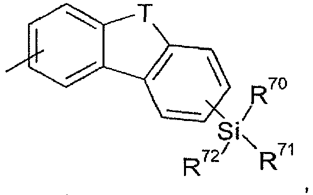 worin die Gruppe T und die Reste R⁷⁰, R⁷¹ und R⁷² unabhängig voneinander die bezüglich der Verbindungen der Formel (XI) oder (XI*) genannten Bedeutungen aufweisen.

Ganz besonders bevorzugt bedeuten R⁶⁰, R⁶¹, R⁶², R⁶³, R⁶⁴, R⁶⁵, R⁶⁶ und R⁶⁷ unabhängig voneinander Phenyl, Tolyl oder eine Gruppe der Formel 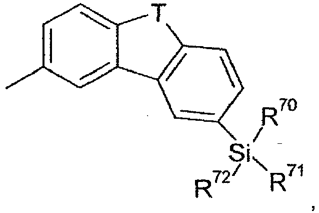 worin T NPh, S oder O bedeutet.

Beispiele für bevorzugt geeignete Gruppen -NR⁵⁸R⁵⁹ sind ausgewählt aus der Gruppe bestehend aus Pyrrolyl, 2,5-Dihydro-1-pyrrolyl, Pyrrolidinyl, Indolyl, Indolinyl, Isoindolinyl, Carbazolyl, Azacarbazolyl, Diazacarbazolyl, Imidazolyl, Imidazolinyl, Benzimidazolyl, Pyrazolyl, Iridazolyl, 1,2,3-Triazolyl, Benzotriazolyl, 1,2,4-Triazolyl, Tetrazolyl, 1,3-Oxazolyl, 1,3-Thiazolyl, Piperidyl, Morpholinyl, 9,10-Dihydroacridinyl und 1,4-Oxazinyl, wobei die vorstehend genannten Gruppen unsubstituiert oder mit einem oder mehreren Substituenten ausgewählt aus Alkyl, Cycloalkyl, Heterocycloalkyl, Aryl, Heteroaryl und einer Gruppe mit Donor- oder Akzeptorwirkung substituiert sein können, bevorzugt ist die Gruppe -NR⁵⁸R⁵⁹ ausgewählt aus Carbazolyl, Pyrrolyl, Indolyl, Imidazolyl, Benzimidazolyl, Azacarbazolyl und Diazacarbazolyl, wobei die vorstehend genannten Gruppen unsubstituiert oder mit einem oder mehreren Substituenten ausgewählt aus Alkyl, Cycloalkyl, Heterocycloalkyl, Aryl, Heteroaryl und einer Gruppe mit Donor- oder Akzeptorwirkung substituiert sein können, besonders bevorzugt bedeutet die Gruppe -NR⁵⁸R⁵⁹ Carbazolyl, das unsubstituiert oder mit einem oder mehreren Substituenten ausgewählt aus Alkyl, Cycloalkyl, Heterocycloalkyl, Aryl, Heteroaryl und einer Gruppe mit Donor- oder Akzeptorwirkung substituiert sein kann.

Besonders bevorzugte Gruppen -NR⁵⁸R⁵⁹ sind: 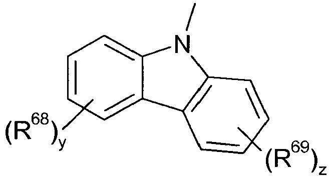 worin bedeuten
- R⁶⁸, R⁶⁹: unabhängig voneinander Alkyl, Cycloalkyl, Heterocycloalkyl, Aryl oder Heteroaryl; bevorzugt Methyl, Carbazolyl, Dibenzofuryl oder Dibenzothienyl;
- y, z: unabhängig voneinander 0, 1, 2, 3 oder 4, bevorzugt 0 oder 1;
zum Beispiel: 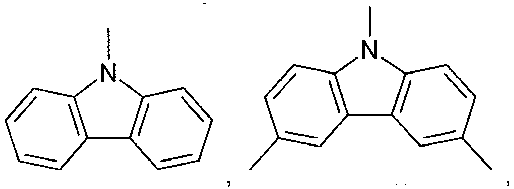 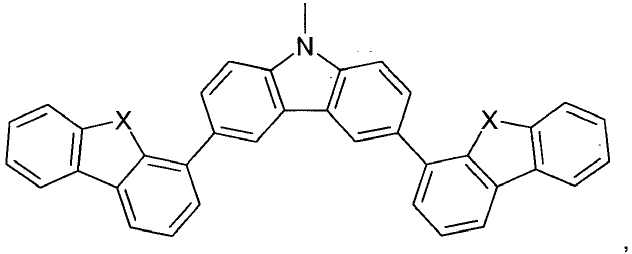 worin X NPh, S oder O bedeutet; 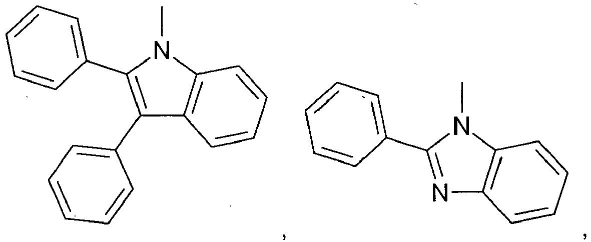 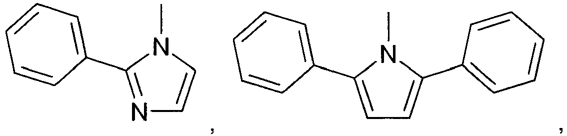 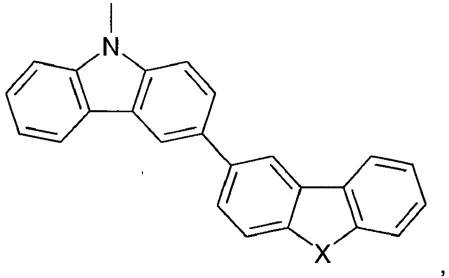 worin X NPh, S oder O bedeutet,

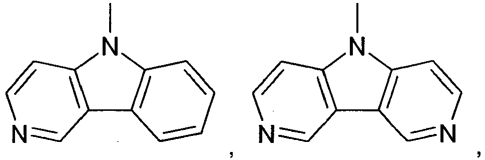

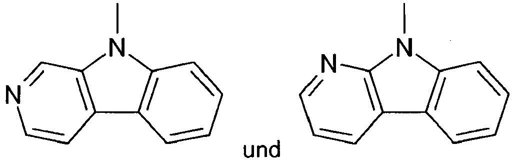

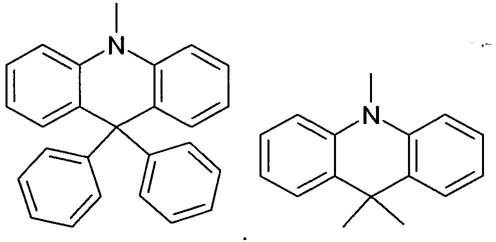

Besonders bevorzugte Gruppen -P(O)R⁶⁰R⁶¹ sind: 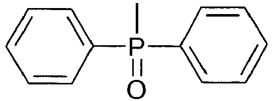 und

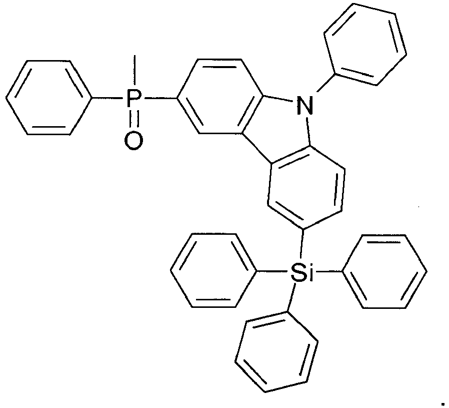

Eine besonders bevorzugte Gruppe PR⁶²R⁶³ ist:

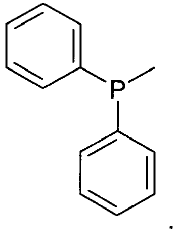

Besonders bevorzugte Gruppen -S(O)₂R⁶⁴ und -S(O)R⁶⁵ sind:

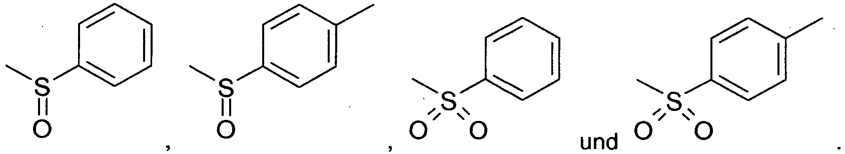

Besonders bevorzugte Gruppen -SR⁶⁶ und -OR⁶⁷ sind: 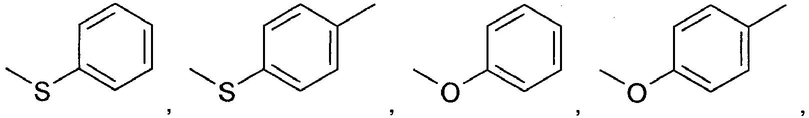 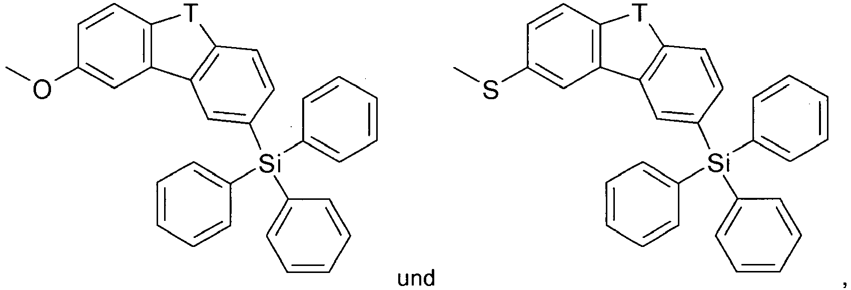 worin T jeweils NPh, S oder O bedeutet.

R⁵⁵, R⁵⁶ in den Verbindungen der Formel (X) bedeuten unabhängig voneinander Alkyl, Cycloalkyl, Heterocycloalkyl, Aryl, Heteroaryl, eine weitere Gruppe A oder eine Gruppe mit Donor- oder Akzeptorwirkung; bevorzugt unabhängig voneinander Alkyl, Aryl, Heteroaryl oder eine Gruppe mit Donor- oder Akzeptorwirkung. Beispielsweise können R⁵⁵ oder R⁵⁶ unabhängig voneinander bedeuten: 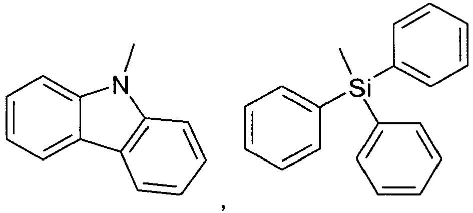 oder 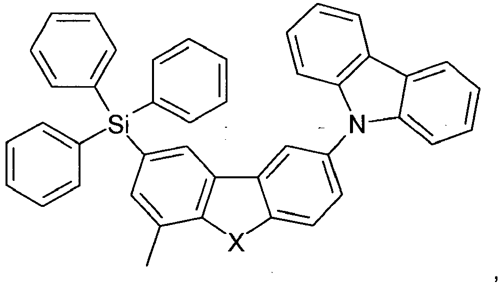 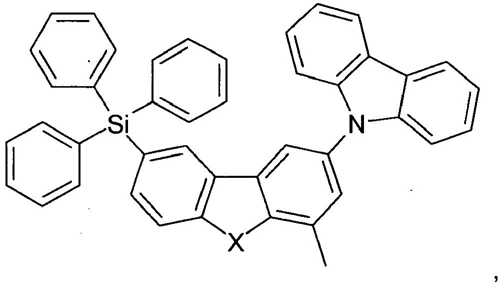 worin X NPh, S oder O bedeutet.

Dabei können in den Verbindungen der Formel (X) a" Gruppen R⁵⁵ und/oder b' Gruppen R⁵⁶ vorliegen, wobei a" und b' bedeuten:
- a": 0, 1, 2, 3 oder 4; bevorzugt unabhängig voneinander 0, 1 oder 2;
- b': 0, 1, 2, oder 3; bevorzugt unabhängig voneinander 0, 1 oder 2.

Ganz besonders bevorzugt ist mindestens a" oder b' 0, insbesondere ganz besonders bevorzugt sind a" und b' 0 oder a" bedeutet 1 und b' bedeutet 0.

R⁷³ bedeutet in den Verbindungen der allgemeinen Formel (XI) im Allgemeinen unabhängig voneinander SiR⁷⁴R⁷⁵R⁷⁶, Aryl, Heteroaryl, Alkyl, Cycloalkyl oder Heterocycloalkyl, gegebenenfalls substituiert mit einer Gruppe OR⁷⁷.

R⁷⁷ bedeutet in Verbindungen der allgemeinen Formel (XI) im Allgemeinen unabhängig voneinander Aryl, Heteroaryl, Alkyl, Cycloalkyl oder Heterocycloalkyl.

Der gegebenenfalls vorliegende Substituent OR⁷⁷ kann in den genannten Resten im Allgemeinen an allen dem Fachmann für geeignet erscheinenden Stellen vorliegen.

In einer weiteren Ausführungsform sind zwei Einheiten der allgemeinen Formel (XI) und/oder (XI*) über eine lineare oder verzweigte, gesättigte oder ungesättigte, gegebenenfalls durch wenigstens ein Heteroatom unterbrochene Verbrückung oder über O miteinander verbrückt, wobei diese Verbrückung in der allgemeinen Formel (XI) und/oder (XI*) jeweils anstelle von R⁷¹ an die Si-Atome angebunden ist.

Bevorzugt ist diese Verbrückung ausgewählt aus der Gruppe bestehend aus -CH₂-,-C₂H₄-, -C₃H₆-, -C₄H₈-, -C₆H₁₂-, -C₈H₁₆-, -C₉H₁₈-, -CH(C₈H₁₇)CH₂-, -C₂H₄(CF₂)₈ C₂H₄-,-C≡C-, -1,4-(CH₂)₂-Phenyl-(CH₂)₂-, 1,3-(CH₂)₂-Phenyl-(CH₂)₂-, -1,4-Phenyl-, -1,3-Phenyl-, -O-, -O-Si(CH₃)₂-O-, -O-Si(CH₃)₂-O- Si(CH₃)₂-O-, -O-

In einer bevorzugten Ausführungsform der vorliegenden Anmeldung weisen die Verbindungen der allgemeinen Formel (X) die allgemeine Formel (Xla), (XIb), (Xlc), (Xld) oder (Xle) auf, d.h. sie sind bevorzugte Ausführungsformen der Verbindungen der allgemeinen Formel (XI) oder (XI*): 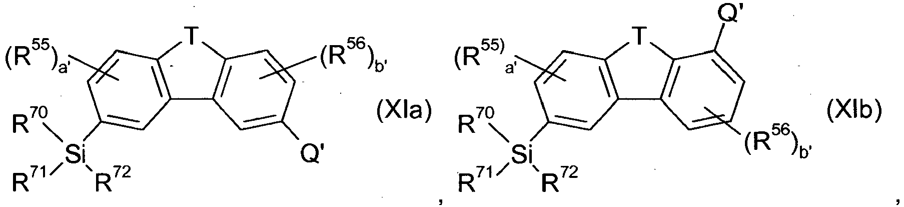 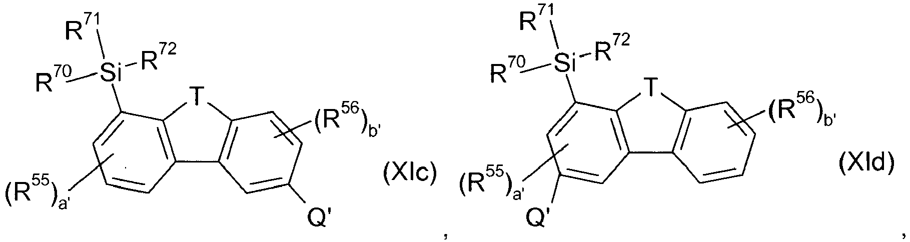 oder 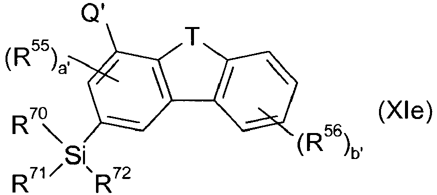 worin die Reste und Gruppen Q', T, R⁷⁰, R⁷¹, R⁷², R⁵⁵, R⁵⁶ sowie a' und b' die vorstehend genannten Bedeutungen aufweisen.

In einer weiteren erfindungsgemäß bevorzugten Ausführungsform bedeuten in den Verbindungen der allgemeinen Formel (XI) oder (XI*) R⁷⁰, R⁷¹ oder R⁷² aromatische Einheiten der allgemeinen Formeln (Xli) und/oder (XIi*) bedeuten 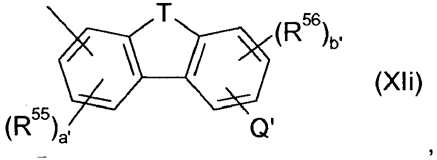 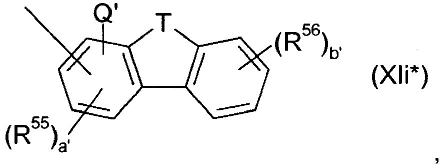 wobei R⁵⁵, R⁵⁶, Q', T, a' und b' die gleichen Bedeutungen wie oben angegeben.

Daher betrifft die vorliegende Erfindung in einer Ausführungsform eine erfindungsgemäße OLED, wobei in den Verbindungen der allgemeinen Formel (XI) oder (XI*) R⁷⁰, R⁷¹ oder R⁷² aromatische Einheiten der allgemeinen Formeln (Xli) und/oder (XIi*) bedeuten 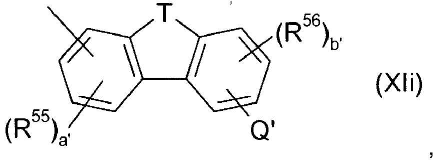 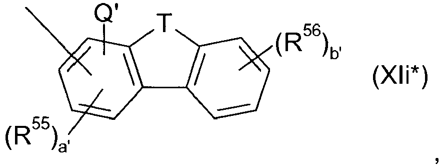 wobei R⁵⁵, R⁵⁶, Q', T, a' und b' die gleichen Bedeutungen wie oben angegeben.

In einer bevorzugten Ausführungsform betrifft die vorliegende Erfindung eine OLED, wobei die Verbindung der allgemeinen Formel (XI) oder (XI*) ausgewählt ist aus der folgenden Gruppe:

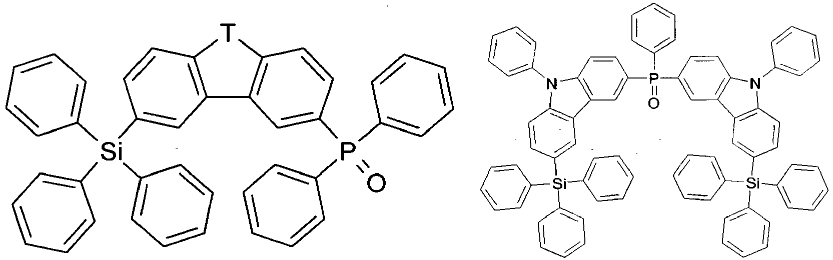

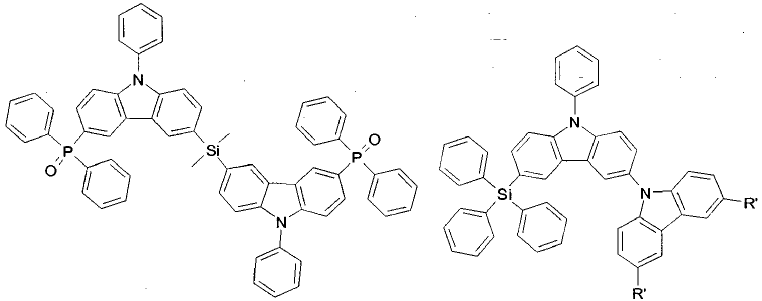

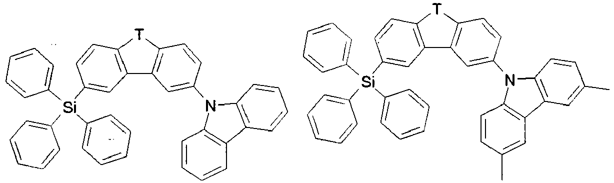

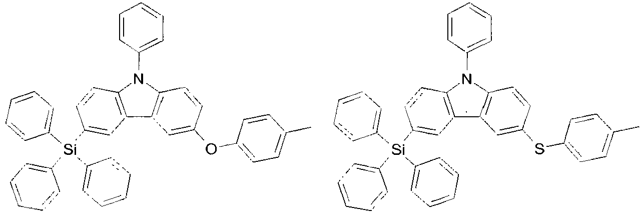

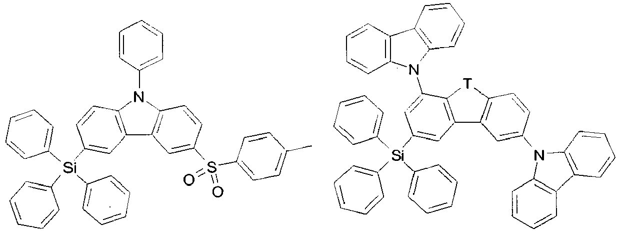

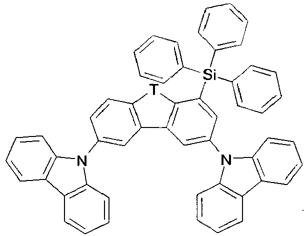

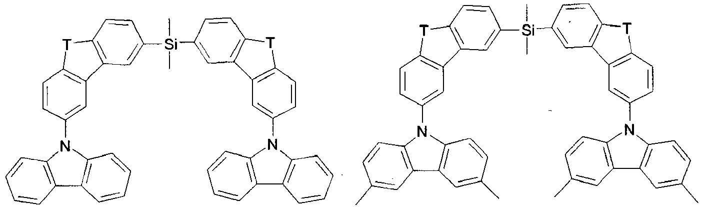

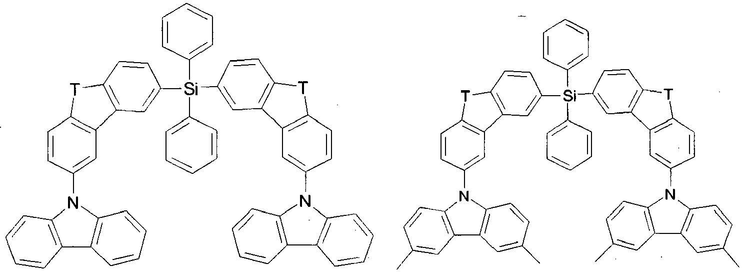

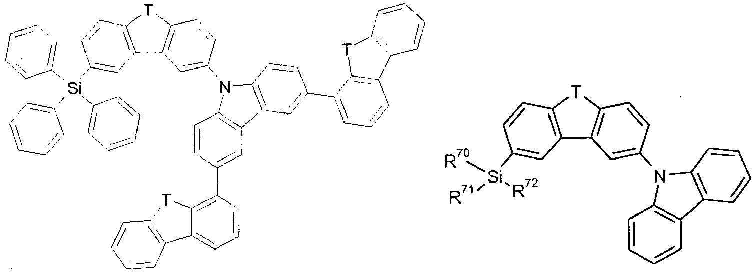

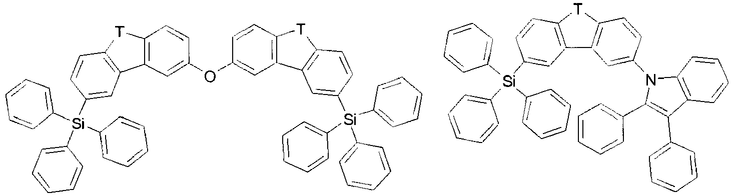

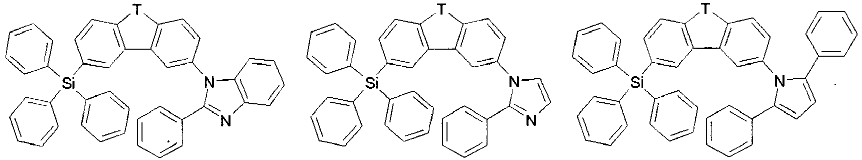

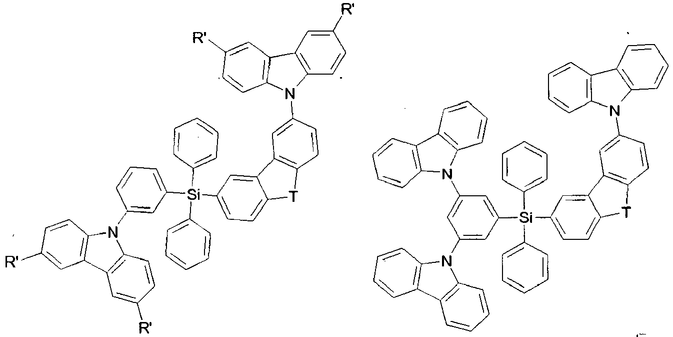

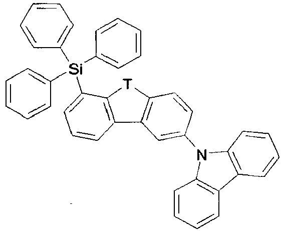

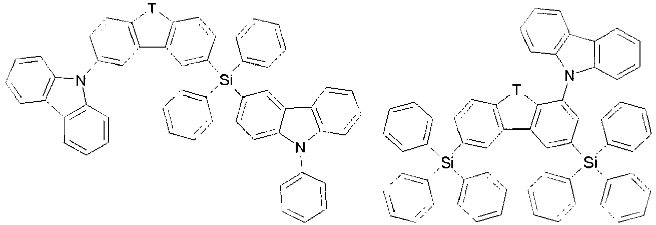

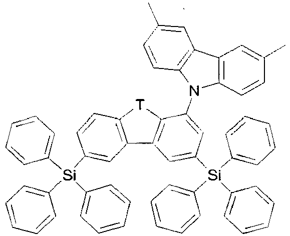

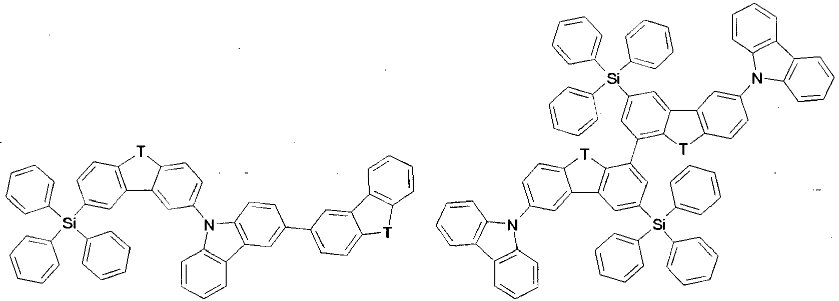

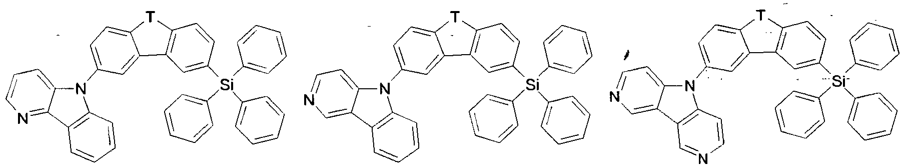

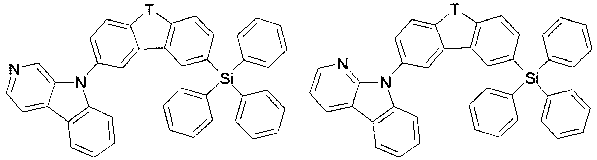

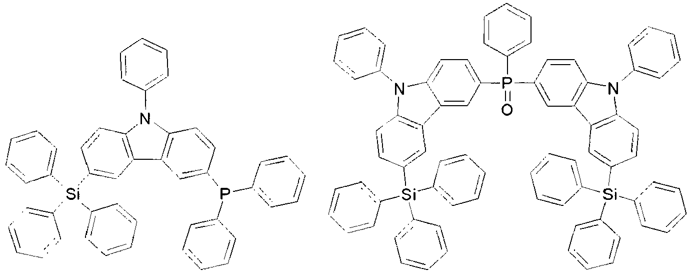

In diesen besonders bevorzugten Verbindungen der allgemeinen Formel (XI) oder (XI*) bedeuten:
- T: S oder O, und
- R': H oder CH₃; und
- R⁷⁰, R⁷¹, R⁷²: Phenyl, Carbazolyl, Dibenzofuran oder Dibenzothiophen.

Weitere besonders geeignete Verbindungen der allgemeinen Formel (XI) oder (XI*) sind:

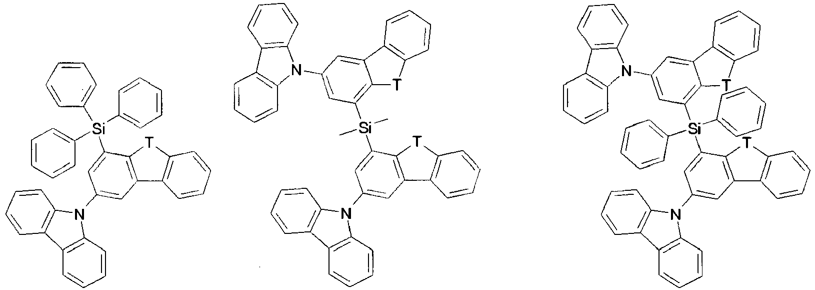

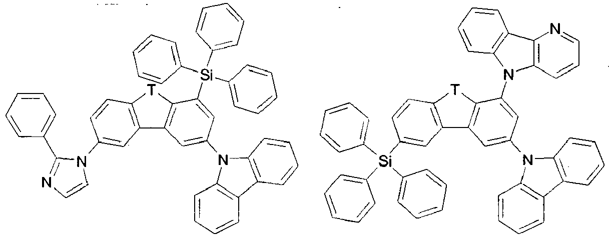

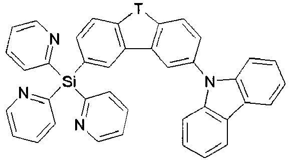

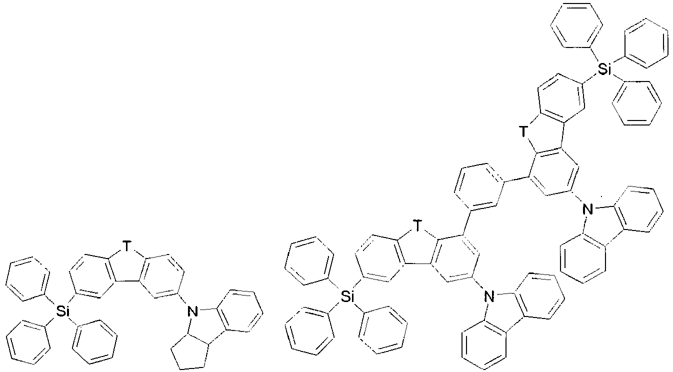

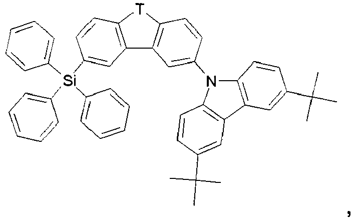

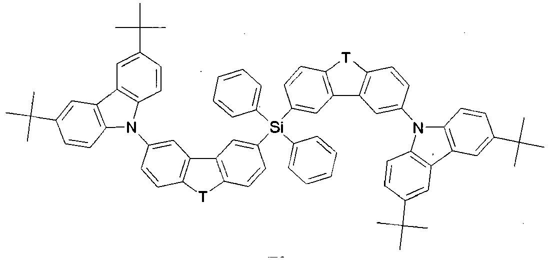

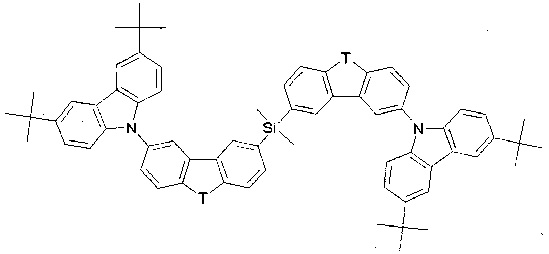

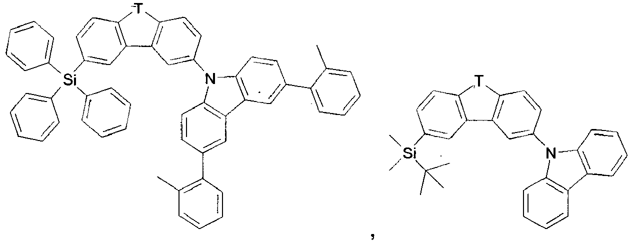

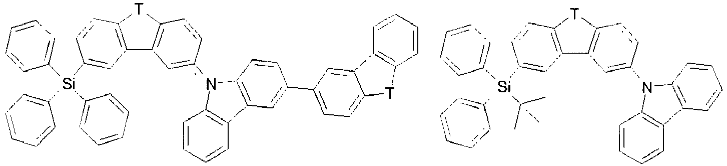

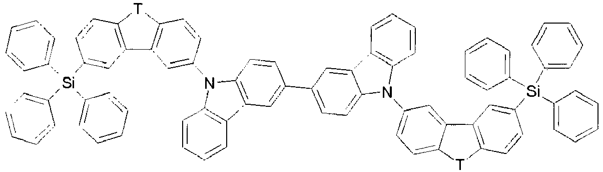

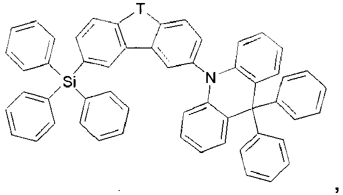

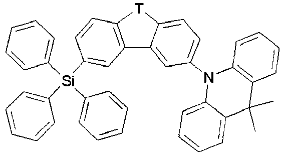

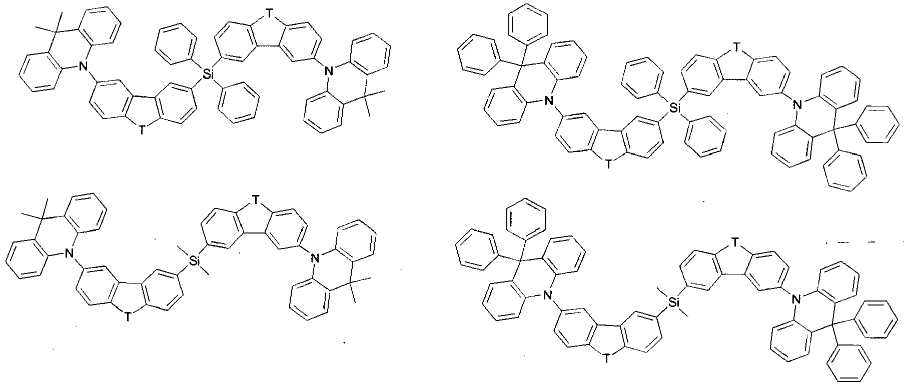

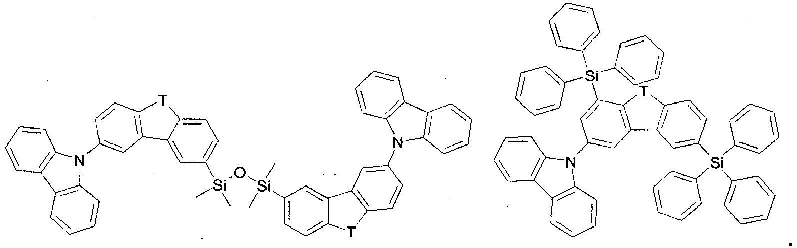

Auch in diesen besonders bevorzugten Verbindungen der allgemeinen Formel (XI) oder (XI*) bedeutet T O oder S, bevorzugt O.

Weitere erfindungsgemäße Verbindungen der allgemeinen Formel (XI) oder (XI*) entsprechen der folgenden Formel (XII)

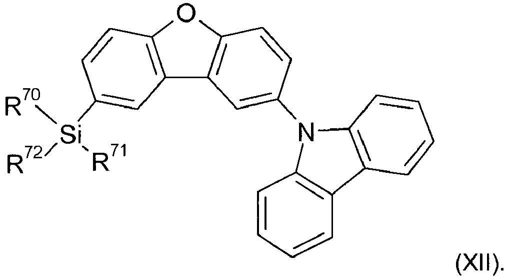

In der allgemeinen Formel (XII) haben R⁷⁰, R⁷¹, R⁷² die folgenden Bedeutungen:

| **Nr** | **R⁷⁰** | **R⁷¹** | **R⁷²** |
|---|---|---|---|
| **1** | Methyl | Methyl | Ethyl |
| **2** | Methyl | Methyl | i-Propyl |
| **3** | Methyl | Methyl | n-Propyl |
| **4** | Methyl | Methyl | n-Butyl |
| **5** | Methyl | Methyl | i-Butyl |
| **6** | Methyl | Methyl | t-Butyl |
| **7** | Methyl | Methyl | n-Pentyl |
| **8** | Methyl | Methyl | n-Hexyl |
| **9** | Methyl | Methyl | -CH₂CH₂C(CH₃)₃ |
| **10** | Methyl | Methyl | n-C₈H₁₇ |
| **11** | Methyl | Methyl | i-C₈H₁₇ |
| **12** | Methyl | Methyl | n-C₁₀H₂₁ |
| **13** | Methyl | Methyl | n-C₁₂H₂₅ |
| **16** | Methyl | Methyl | n-C₁₈H₃₇ |
| **17** | Methyl | Methyl | n-C₃₀H₆₁ |
| **19** | Methyl | Methyl | Cyclohexyl |
| **20** | Methyl | Methyl | C(CH₃)₂Ph |
| **21** | Methyl | Methyl | -C(CH₃)₂CH(CH₃)₂ |
| **22** | Methyl | Methyl | -CCH₂CH(CH₃)(C₂H₅) |
| **23** | Methyl | Methyl | -CH₂CH(C₁₀H₂₁)₂ |
| **24** | Methyl | Methyl | -CH₂CH(C₁₂H₂₅)₂ |
| **25** | Methyl | Methyl | -CH₂CH₂(C₃F₆)CF₃ |
| **26** | Methyl | Methyl | -CH₂CH₂(C₇F₁₄)CF₃ |
| **27** | Methyl | Methyl | -CH₂CH₂(C₅F₁₀)CF₃ |
| **29** | Methyl | Methyl | -CH₂CH₂CF₃ |
| **30** | Methyl | Methyl | Phenyl |
| **31** | Methyl | Methyl | 2-Biphenyl |
| **32** | Methyl | Methyl | p-Tolyl |
| **33** | Methyl | Methyl | C₆F₅ |
| **34** | Methyl | Methyl | 3,5-(CF₃)₂Phenyl |
| **35** | Methyl | Methyl | -CH₂C(CH₃)₂Phenyl |
| **36** | Methyl | Methyl | 9-Fluorenyl |
| **37** | Methyl | Methyl | 3,6-Di(tert-butyl)-9-Fluorenyl |
| **15** | Methyl | Methyl | R⁸⁶ |
| **38** | Methyl | Methyl | -OMe |
| **39** | Methyl | Methyl | -OEt |
| **40** | Methyl | Methyl | 2,4,6-t-Butylphenoxy |
| **41** | Methyl | Methyl | -O-tBu (tert-Butoxy) |
| **42** | Methyl | Methyl | -OSiEt₃ |
| **43** | Methyl | Ethyl | Ethyl |
| **44** | Methyl | Ethyl | Phenyl |
| **45** | Methyl | Ethyl | R⁸⁶ |
| **46** | Methyl | n-Propyl | n-Propyl |
| **47** | Methyl | n-Propyl | Phenyl |
| **48** | Methyl | n-Propyl | R⁸⁶ |
| **49** | Methyl | i-Propyl | i-Propyl |
| **50** | Methyl | i-Propyl | Phenyl |
| **51** | Methyl | i-Propyl | R⁸⁶ |
| **52** | Methyl | n-Butyl | n-Butyl |
| **53** | Methyl | n-Butyl | Phenyl |
| **54** | Methyl | n-Butyl | R⁸⁶ |
| **55** | Methyl | i-Butyl | i-Butyl |
| **56** | Methyl | i-Butyl | Phenyl |
| **57** | Methyl | i-Butyl | R⁸⁶ |
| **58** | Methyl | t-Butyl | t-Butyl |
| **59** | Methyl | t-Butyl | Phenyl |
| **60** | Methyl | t-Butyl | R⁸⁶ |
| **61** | Methyl | n-Pentyl | n-Pentyl |
| **62** | Methyl | n-Pentyl | n-Hexyl |
| **63** | Methyl | n-Pentyl | Phenyl |
| **64** | Methyl | n-Pentyl | R⁸⁶ |
| **65** | Methyl | n-Hexyl | Hexyl |
| **66** | Methyl | n-Hexyl | Phenyl |
| **67** | Methyl | n-Hexyl | R⁸⁶ |
| **68** | Methyl | n-Heptyl | R⁸⁶ |
| **69** | Methyl | n-Octyl | R⁸⁶ |
| **70** | Methyl | n-Decyl | R⁸⁶ |
| **71** | Methyl | n-C₁₂H₂₅ | R⁸⁶ |
| **72** | Methyl | n-C₁₈H₃₇ | R⁸⁶ |
| **73** | Methyl | n-C₂₂H₄₅ | R⁸⁶ |
| **74** | Methyl | n-C₃₀H₆₁ | R⁸⁶ |
| **75** | Methyl | Cyclopentyl | Cyclopentyl |
| **76** | Methyl | Cyclopentyl | Phenyl |
| **77** | Methyl | Cyclopentyl | R⁸⁶ |
| **78** | Methyl | Cyclohexyl | Cyclohexyl |
| **79** | Methyl | Cyclohexyl | Phenyl |
| **80** | Methyl | Cyclohexyl | R⁸⁶ |
| **81** | Methyl | -CF₂CHF₂ | R⁸⁶ |
| **82** | Methyl | -CH₂CH₂CF₃ | R⁸⁶ |
| **83** | Methyl | -CH₂CH₂(CF₂)₃CF₃ | R⁸⁶ |
| **84** | Methyl | -CH₂CH₂(CF₂)₅CF₃ | R⁸⁶ |
| **85** | Methyl | -CH₂CH₂(CF₂)₇CF₃ | R⁸⁶ |
| **86** | Methyl | Phenyl | Phenyl |
| **87** | Methyl | Phenyl | p-Tolyl |
| **89** | Methyl | Phenyl | Mesityl |
| **90** | Methyl | Phenyl | R⁸⁶ |
| **91** | Methyl | p-Tolyl | p-Tolyl |
| **92** | Methyl | p-Tolyl | R⁸⁶ |
| **93** | Methyl | Mesityl | Mesityl |
| **94** | Methyl | Mesityl | R5 |
| **95** | Methyl | R⁸⁶ | R⁸⁶ |
| **96** | Methyl | Methoxy | Methoxy |
| **97** | Methyl | Ethoxy | Ethoxy |
| **98** | Methyl | -OSiEt₃ | -OSiEt₃ |
| **99** | Methyl | -O-SiMe₂-CH₂CH₂(CF₂)₄CF₃ | -O-SiMe₂-CH₂CH₂(CF₂)₄CF₃ |
| **100** | Ethyl | Ethyl | Ethyl |
| **101** | Ethyl | Ethyl | n-Propyl |
| **102** | Ethyl | Ethyl | i-Propyl |
| **103** | Ethyl | Ethyl | n-Butyl |
| **104** | Ethyl | Ethyl | i-Butyl |
| **105** | Ethyl | Ethyl | t-Butyl |
| **106** | Ethyl | Ethyl | Phenyl |
| **107** | Ethyl | Ethyl | R5 |
| **108** | Ethyl | Phenyl | Phenyl |
| **109** | Ethyl | Phenyl | R⁸⁶ |
| **110** | Ethyl | R⁸⁶ | R⁸⁶ |
| **111** | Ethyl | Ethoxy | Ethoxy |
| **112** | n-Propyl | n-Propyl | n-Propyl |
| **113** | n-Propyl | n-Propyl | Phenyl |
| **114** | n-Propyl | n-Propyl | R⁸⁶ |
| **115** | n-Propyl | Phenyl | Phenyl |
| **116** | n-Propyl | Phenyl | R⁸⁶ |
| **117** | n-Propyl | R⁸⁶ | R⁸⁶ |
| **118** | i-Propyl | i-Propyl | i-Propyl |
| **119** | i-Propyl | i-Propyl | Phenyl |
| **120** | i-Propyl | i-Propyl | R⁸⁶ |
| **121** | i-Propyl | i-Propyl | 2-Biphenyl |
| **122** | i-Propyl | i-Propyl | Ethoxy |
| **123** | i-Propyl | Phenyl | Phenyl |
| **124** | i-Propyl | Phenyl | R⁸⁶ |
| **125** | i-Propyl | R⁸⁶ | R⁸⁶ |
| **126** | n-Butyl | n-Butyl | n-Butyl |
| **127** | n-Butyl | n-Butyl | Phenyl |
| **128** | n-Butyl | n-Butyl | R⁸⁶ |
| **129** | n-Butyl | n-Hexyl | R⁸⁶ |
| **130** | n-Butyl | Phenyl | Phenyl |
| **131** | n-Butyl | Phenyl | R⁸⁶ |
| **132** | n-Butyl | R⁸⁶ | R⁸⁶ |
| **133** | sec-Butyl | sec-Butyl | sec-Butyl |
| **134** | sec-Butyl | sec-Butyl | Phenyl |
| **135** | sec-Butyl | sec-Butyl | R⁸⁶ |
| **136** | sec-Butyl | Phenyl | Phenyl |
| **137** | sec-Butyl | Phenyl | R⁸⁶ |
| **138** | sec-Butyl | R⁸⁶ | R⁸⁶ |
| **139** | i-Butyl | i-Butyl | i-Butyl |
| **140** | i-Butyl | i-Butyl | n-C₈H₁₇ |
| **141** | i-Butyl | i-Butyl | n-C₁₈H₃₇ |
| **142** | i-Butyl | i-Butyl | Phenyl |
| **143** | i-Butyl | i-Butyl | R⁸⁶ |
| **144** | i-Butyl | Phenyl | Phenyl |
| **145** | i-Butyl | Phenyl | R⁸⁶ |
| **146** | i-Butyl | R⁸⁶ | R⁸⁶ |
| **147** | t-Butyl | t-Butyl | t-Butyl |
| **148** | t-Butyl | t-Butyl | n-C₈H₁₇ |
| **149** | t-Butyl | t-Butyl | Phenyl |
| **150** | t-Butyl | t-Butyl | R⁸⁶ |
| **151** | t-Butyl | Phenyl | Phenyl |
| **152** | t-Butyl | Phenyl | R5 |
| **153** | t-Butyl | R⁸⁶ | R⁸⁶ |
| **154** | n-Pentyl | n-Pentyl | n-Pentyl |
| **155** | n-Pentyl | n-Pentyl | Phenyl |
| **156** | n-Pentyl | n-Pentyl | R⁸⁶ |
| **157** | n-Pentyl | Phenyl | Phenyl |
| **158** | n-Pentyl | Phenyl | R⁸⁶ |
| **159** | n-Pentyl | R⁸⁶ | R⁸⁶ |
| **160** | Cyclopentyl | Cyclopentyl | Cyclopentyl |
| **161** | Cyclopentyl | Cyclopentyl | Phenyl |
| **162** | Cyclopentyl | Cyclopentyl | R⁸⁶ |
| **163** | Cyclopentyl | Phenyl | Phenyl |
| **164** | Cyclopentyl | Phenyl | R⁸⁶ |
| **165** | Cyclopentyl | R⁸⁶ | R⁸⁶ |
| **166** | n-Hexyl | n-Hexyl | n-Hexyl |
| **167** | n-Hexyl | n-Hexyl | Phenyl |
| **168** | n-Hexyl | n-Hexyl | R⁸⁶ |
| **169** | n-Hexyl | Phenyl | Phenyl |
| **170** | n-Hexyl | Phenyl | R⁸⁶ |
| **171** | n-Hexyl | R⁸⁶ | R⁸⁶ |
| **172** | -CH₂CH₂C(CH₃)₃ | -CH₂CH₂C(CH₃)₃ | -CH₂CH₂C(CH₃)₃ |
| **173** | -CH₂CH₂C(CH₃)₃ | -CH₂CH₂C(CH₃)₃ | R⁸⁶ |
| **174** | -CH₂CH₂C(CH₃)₃ | R⁸⁶ | R⁸⁶ |
| **175** | t-Hexyl | t-Hexyl | t-Hexyl |
| **176** | t-Hexyl | t-Hexyl | R⁸⁶ |
| **177** | t-Hexyl | R⁸⁶ | R⁸⁶ |
| **178** | n-Heptyl | n-Heptyl | n-Heptyl |
| **179** | n-Heptyl | n-Heptyl | R⁸⁶ |
| **180** | n-Heptyl | R⁸⁶ | R⁸⁶ |
| **181** | n-Octyl | n-Octyl | n-Octyl |
| **182** | n-Octyl | n-Octyl | R⁸⁶ |
| **183** | n-Octyl | R⁸⁶ | R⁸⁶ |
| **184** | i-Octyl | i-Octyl | i-Octyl |
| **185** | i-Octyl | i-Octyl | R⁸⁶ |
| **186** | i-Octyl | R⁸⁶ | R⁸⁶ |
| **187** | n-Nonyl | n-Nonyl | n-Nonyl |
| **188** | n-Nonyl | n-Nonyl | R⁸⁶ |
| **189** | n-Nonyl | R⁸⁶ | R⁸⁶ |
| **190** | Cyclohexyl | Cyclohexyl | Cyclohexyl |
| **191** | Cyclohexyl | Cyclohexyl | R⁸⁶ |
| **192** | Cyclohexyl | R⁸⁶ | R⁸⁶ |
| **193** | Cyclooctyl | Cyclooctyl | Cyclooctyl |
| **194** | Cyclooctyl | Cyclooctyl | R⁸⁶ |
| **195** | Cyclooctyl | R⁸⁶ | R⁸⁶ |
| **196** | n-C₁₀H₂₁ | n-C₁₀H₂₁ | n-C₁₀H₂₁ |
| **197** | n-C₁₀H₂₁ | n-C₁₀H₂₁ | R⁸⁶ |
| **198** | n-C₁₀H₂₁ | R⁸⁶ | R⁸⁶ |
| **199** | n-C₁₁H₂₃ | n-C₁₁H₂₃ | n-C₁₁H₂₃ |
| **200** | n-C₁₁H₂₃ | n-C₁₁H₂₃ | R⁸⁶ |
| **201** | n-C₁₁H₂₃ | R⁸⁶ | R⁸⁶ |
| **202** | n-C₁₂H₂₅ | n-C₁₂H₂₅ | n-C₁₂H₂₅ |
| **203** | n-C₁₂H₂₅ | n-C₁₂H₂₅ | R⁸⁶ |
| **204** | n-C₁₂H₂₅ | R⁸⁶ | R⁸⁶ |
| **205** | n-C₁₄H₂₉ | n-C₁₄H₂₉ | n-C₁₄H₂₉ |
| **206** | n-C₁₄H₂₉ | n-C₁₄H₂₉ | R⁸⁶ |
| **207** | n-C₁₄H₂₉ | R⁸⁶ | R⁸⁶ |
| **208** | n-C₁₆H₃₃ | n-C₁₆H₃₃ | n-C₁₆H₃₃ |
| **209** | n-C₁₆H₃₃ | n-C₁₆H₃₃ | R⁸⁶ |
| **210** | n-C₁₆H₃₃ | R⁸⁶ | R⁸⁶ |
| **211** | n-C₁₈H₃₇ | n-C₁₈H₃₇ | R⁸⁶ |
| **212** | n-C₁₈H₃₇ | R⁸⁶ | R⁸⁶ |
| **213** | n-C₁₈H₃₇ | OEt | OEt |
| **214** | n-C₁₈H₃₇ | R⁸⁶ | OMe |
| **215** | n-C₂₀H₄₁ | n-C₂₀H₄₁ | n-C₂₀H₄₁ |
| **216** | n-C₂₀H₄₁ | n-C₂₀H₄₁ | R⁸⁶ |
| **217** | n-C₂₀H₄₁ | R⁸⁶ | R⁸⁶ |
| **218** | n-C₂₂H₄₅ | n-C₂₂H₄₅ | n-C₂₂H₄₅ |
| **219** | n-C₂₂H₄₅ | n-C₂₂H₄₅ | R⁸⁶ |
| **220** | n-C₂₂H₄₅ | R⁸⁶ | R⁸⁶ |
| **221** | n-C₂₆H₅₃ | n-C₂₆H₅₃ | n-C₂₆H₅₃ |
| **222** | n-C₂₆H₅₃ | n-C₂₆H₅₃ | R⁸⁶ |
| **223** | n-C₂₆H₅₃ | R⁸⁶ | R⁸⁶ |
| **224** | n-C₃₀H₆₁ | n-C₃₀H₆₁ | n-C₃₀H₆₁ |
| **225** | n-C₃₀H₆₁ | n-C₃₀H₆₁ | R⁸⁶ |
| **226** | n-C₃₀H₆₁ | R⁸⁶ | R⁸⁶ |
| **227** | -CH₂-Cyclohexyl | -CH₂-Cyclohexyl | R⁸⁶ |
| **228** | -CH₂CH₂CF₃ | -CH₂CH₂CF₃ | -CH₂CH₂CF₃ |
| **229** | -CH₂CH₂CF₃ | -CH₂CH₂CF₃ | R⁸⁶ |
| **230** | -CH₂CH₂CF₃ | R⁸⁶ | R⁸⁶ |
| **231** | -CH₂CH₂(CF₂)₃CF₃ | -CH₂CH₂(CF₂)₃CF₃ | -CH₂CH₂(CF₂)₃CF₃ |
| **232** | -CH₂CH₂(CF₂)₃CF₃ | -CH₂CH₂(CF₂)₃CF₃ | R⁸⁶ |
| **233** | -CH₂CH₂(CF₂)₃CF₃ | R⁸⁶ | R⁸⁶ |
| **234** | -CH₂CH₂(CF₂)₅CF₃ | -CH₂CH₂(CF₂)₅CF₃ | -CH₂CH₂(CF₂)₅CF₃ |
| **235** | -CH₂CH₂(CF₂)₅CF₃ | -CH₂CH₂(CF₂)₅CF₃ | R⁸⁶ |
| **236** | -CH₂CH₂(CF₂)₅CF₃ | R⁸⁶ | R⁸⁶ |
| **237** | -CH₂CH₂(CF₂)₇CF₃ | -CH₂CH₂(CF₂)₇CF₃ | -CH₂CH₂(CF₂)₇CF₃ |
| **238** | -CH₂CH₂(CF₂)₇CF₃ | -CH₂CH₂(CF₂)₇CF₃ | R⁸⁶ |
| **239** | -CH₂CH₂(CF₂)₇CF₃ | R⁸⁶ | R⁸⁶ |
| **240** | -CH₂CH₂(CF₂)₉CF₃ | -CH₂CH₂(CF₂)₉CF₃ | -CH₂CH₂(CF₂)₉CF₃ |
| **241** | -CH₂CH₂(CF₂)₉CF₃ | -CH₂CH₂(CF₂)₉CF₃ | R⁸⁶ |
| **242** | -CH₂CH₂(CF₂)₉CF₃ | R⁸⁶ | R⁸⁶ |
| **243** | -CH₂CH₂(CF₂)₁₁CF₃ | -CH₂CH₂(CF₂)₁₁CF₃ | -CH₂CH₂(CF₂)₁₁CF₃ |
| **244** | -CH₂CH₂(CF₂)₁₁CF₃ | -CH₂CH₂(CF₂)₁₁CF₃ | R⁸⁶ |
| **245** | -CH₂CH₂(CF₂)₁₁CF₃ | R⁸⁶ | R⁸⁶ |
| **246** | -CF₂CHF₂ | -CF₂CHF₂ | -CF₂CHF₂ |
| **247** | -CF₂CHF₂ | -CF₂CHF₂ | R⁸⁶ |
| **248** | -CF₂CHF₂ | R⁸⁶ | R⁸⁶ |
| **249** | -(CF₂)₃CHF₂ | -(CF₂)₃CHF₂ | -(CF₂)₃CHF₂ |
| **250** | -(CF₂)₃CHF₂ | -(CF₂)₃CHF₂ | R⁸⁶ |
| **251** | -(CF₂)₃CHF₂ | R⁸⁶ | R⁸⁶ |
| **14** | Phenyl | Phenyl | Phenyl |
| **252** | Phenyl | Phenyl | p-Tolyl |
| **253** | Phenyl | Phenyl | m-Tolyl |
| **254** | Phenyl | Phenyl | o-Tolyl |
| **255** | Phenyl | Phenyl | 2-Xylyl |
| **256** | Phenyl | Phenyl | 5-Xylyl |
| **257** | Phenyl | Phenyl | Mesityl |
| **258** | Phenyl | Phenyl | 9-Fluorenyl |
| **18** | Phenyl | Phenyl | R⁸⁶ |
| **259** | Phenyl | Phenyl | -O-tBu (tert-Butoxy) |
| **260** | Phenyl | p-Tolyl | p-Tolyl |
| **261** | Phenyl | m-Tolyl | m-Tolyl |
| **262** | Phenyl | o-Tolyl | o-Tolyl |
| **263** | Phenyl | 2-Xylyl | 2-Xylyl |
| **264** | Phenyl | 5-Xylyl | 5-Xylyl |
| **265** | Phenyl | Mesityl | Mesityl |
| **266** | Phenyl | R⁸⁶ | R⁸⁶ |
| **267** | Phenyl | Ethoxy | Ethoxy |
| **268** | p-Tolyl | p-Tolyl | p-Tolyl |
| **269** | p-Tolyl | p-Tolyl | R⁸⁶ |
| **270** | p-Tolyl | R⁸⁶ | R⁸⁶ |
| **271** | m-Tolyl | m-Tolyl | m-Tolyl |
| **272** | m-Tolyl | m-Tolyl | R⁸⁶ |
| **273** | o-Tolyl | o-Tolyl | o-Tolyl |
| **274** | o-Tolyl | o-Tolyl | R⁸⁶ |
| **275** | 2-Xylyl | 2-Xylyl | 2-Xylyl |
| **276** | 2-Xylyl | 2-Xylyl | R⁸⁶ |
| **277** | 5-Xylyl | 5-Xylyl | 5-Xylyl |
| **278** | 5-Xylyl | 5-Xylyl | R⁸⁶ |
| **279** | Mesityl | Mesityl | Mesityl |
| **280** | Mesityl | Mesityl | R⁸⁶ |
| **281** | C₆F₅ | C₆F₅ | C₆F₅ |
| **282** | C₆F₅ | C₆F₅ | R⁸⁶ |
| **283** | C₆F₅ | R⁸⁶ | R⁸⁶ |
| **284** | R⁸⁶ | R⁸⁶ | R⁸⁶ |
| **285** | R⁸⁶ | Ethoxy | Ethoxy |
| **286** | R⁸⁶ | n-Butoxy | n-Butoxy |
| **287** | R⁸⁶ | R⁸⁶ | Methoxy |
| **288** | R⁸⁶ | R⁸⁶ | Ethoxy |
| **289** | R⁸⁶ | R⁸⁶ | OSiMe₃ |
| **290** | R⁸⁶ | R⁸⁶ | -(CH₂)₁₁O-(CH₂)₂OCH₃ |
| **291** | Methoxy | Methoxy | Methoxy |
| **292** | Ethoxy | Ethoxy | Ethoxy |
| **293** | i-Propoxy | i-Propoxy | i-Propoxy |
| **294** | t-Butoxy | t-Butoxy | t-Butoxy |
| **295** | OSiMe₃ | OSiMe₃ | OSiMe₃ |
| **296** | Cyclobutyl | | Methyl |
| **297** | Cyclobutyl | | R⁸⁶ |
| **298** | Cyclobutyl | | p-Methoxyphenyl |
| **299** | Cyclopentyl | | Methyl |
| **300** | Cyclopentyl | | R⁸⁶ |
| **301** | Cyclohexyl | | Methyl |
| **302** | Cyclohexyl | | R⁸⁶ |

In dieser Tabelle ist

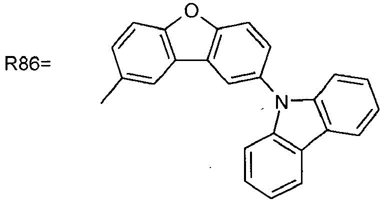

Besonders bevorzugte Verbindungen, in denen zwei Einheiten der allgemeinen Formeln (XI) und/oder (XI*) über eine lineare oder verzweigte, gesättigte oder ungesättigte, gegebenenfalls durch wenigstens ein Heteroatom unterbrochene Verbrückung oder über O miteinander verbrückt sind, wobei diese Verbrückung in den allgemeinen Formeln (XI) und/oder (XI*) jeweils anstelle von R⁷¹ an die Si-Atome angebunden ist, entsprechen der allgemeinen Formel (XIII)

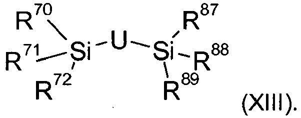

In Formel (XIII) haben U, R⁷⁰, R⁷¹, R⁷², R⁸⁷, R⁸⁸ und R⁸⁹ die folgenden Bedeutungen:

| **Nr.** | **R70** | **R71** | **R72** | **R87** | **R88** | **R89** | **U** |
|---|---|---|---|---|---|---|---|
| 303 | Methyl | R⁸⁶ | R⁸⁶ | Methyl | R5 | R⁸⁶ | -CH₂- |
| 304 | Methyl | Methyl | R⁸⁶ | Methyl | Methyl | R⁸⁶ | -CH₂- |
| 305 | R⁸⁶ | R⁸⁶ | R⁸⁶ | R⁸⁶ | R⁸⁶ | R⁸⁶ | -CH₂- |
| 306 | Methyl | R⁸⁶ | R⁸⁶ | Methyl | R5 | R⁸⁶ | -C₂H₄- |
| 307 | Methyl | Methyl | R⁸⁶ | Methyl | Methyl | R⁸⁶ | -C₂H₄- |
| 308 | R⁸⁶ | R⁸⁶ | R⁸⁶ | R⁸⁶ | R⁸⁶ | R⁸⁶ | -C₂H₄- |
| 309 | Methyl | R⁸⁶ | R⁸⁶ | Methyl | R⁸⁶ | R⁸⁶ | -C₃H₆- |
| 310 | Methyl | Methyl | R⁸⁶ | Methyl | Methyl | R⁸⁶ | -C₃H₆- |
| 311 | R⁸⁶ | R⁸⁶ | R⁸⁶ | R⁸⁶ | R⁸⁶ | R⁸⁶ | -C₃H₆- |
| 312 | Methyl | R⁸⁶ | R⁸⁶ | Methyl | R⁸⁶ | R⁸⁶ | -C₄H₈- |
| 313 | Methyl | Methyl | R⁸⁶ | Methyl | Methyl | R⁸⁶ | -C₄H₈- |
| 314 | R⁸⁶ | R⁸⁶ | R⁸⁶ | R⁸⁶ | R⁸⁶ | R⁸⁶ | -C₄H₈- |
| 315 | Methyl | R⁸⁶ | R⁸⁶ | Methyl | R⁸⁶ | R⁸⁶ | -C₆H₁₂- |
| 316 | Methyl | Methyl | R⁸⁶ | Methyl | Methyl | R⁸⁶ | -C₆H₁₂- |
| 317 | R⁸⁶ | R⁸⁶ | R⁸⁶ | R⁸⁶ | R⁸⁶ | R⁸⁶ | -C₆H₁₂- |
| 318 | Methyl | R⁸⁶ | R⁸⁶ | Methyl | R⁸⁶ | R⁸⁶ | -C₈H₁₆- |
| 319 | Methyl | Methyl | R⁸⁶ | Methyl | Methyl | R⁸⁶ | -C₈H₁₆- |
| 320 | R⁸⁶ | R⁸⁶ | R⁸⁶ | R⁸⁶ | R⁸⁶ | R⁸⁶ | -C₈H₁₆- |
| 321 | Methyl | R⁸⁶ | R⁸⁶ | Methyl | R⁸⁶ | R⁸⁶ | -C₉H₁₈- |
| 322 | Methyl | Methyl | R⁸⁶ | Methyl | Methyl | R⁸⁶ | -C₉H₁₈- |
| 323 | R⁸⁶ | R⁸⁶ | R⁸⁶ | R⁸⁶ | R⁸⁶ | R⁸⁶ | -C₉H₁₈- |
| 324 | R⁸⁶ | R⁸⁶ | R⁸⁶ | R⁸⁶ | R⁸⁶ | R⁸⁶ | - CH(C₈H₁₇)CH ₂- |
| 325 | Methyl | R⁸⁶ | R⁸⁶ | Methyl | R⁸⁶ | R⁸⁶ | -C₂H₄(CF₂)₈ C₂H₄- |
| 326 | Methyl | Methyl | R⁸⁶ | Methyl | Methyl | R⁸⁶ | -C₂H₄(CF₂)₈ C₂H₄- |
| 327 | R⁸⁶ | R⁸⁶ | R⁸⁶ | R⁸⁶ | R⁸⁶ | R⁸⁶ | -C₂H₄(CF₂)₈ C₂H₄- |
| 328 | Methyl | R⁸⁶ | R⁸⁶ | Methyl | R⁸⁶ | R⁸⁶ | -C≡C- |
| 329 | Methyl | Methyl | R⁸⁶ | Methyl | Methyl | R⁸⁶ | -C≡C- |
| 330 | R⁸⁶ | R⁸⁶ | R⁸⁶ | R⁸⁶ | R⁸⁶ | R⁸⁶ | -C≡C- |
| 331 | Methyl | R⁸⁶ | R⁸⁶ | Methyl | R⁸⁶ | R⁸⁶ | -1,4-(CH₂)₂-Phenyl-(CH₂)₂- |
| 332 | Methyl | Methyl | R⁸⁶ | Methyl | Methyl | R⁸⁶ | -1,4-(CH₂)₂-Phenyl-(CH₂)₂- |
| 333 | R⁸⁶ | R⁸⁶ | R⁸⁶ | R⁸⁶ | R⁸⁶ | R⁸⁶ | -1,4-(CH₂)₂-Phenyl-(CH₂)₂- |
| 334 | Methyl | R⁸⁶ | R⁸⁶ | Methyl | R⁸⁶ | R⁸⁶ | -1,3-(CH₂)₂-Phenyl-(CH₂)₂- |
| 335 | Methyl | Methyl | R⁸⁶ | Methyl | Methyl | R⁸⁶ | -1,3-(CH₂)₂-Phenyl-(CH₂)₂- |
| 336 | R⁸⁶ | R⁸⁶ | R⁸⁶ | R⁸⁶ | R⁸⁶ | R⁸⁶ | -1,3-(CH₂)₂-Phenyl-(CH₂)₂- |
| 337 | Methyl | R⁸⁶ | R⁸⁶ | Methyl | R⁸⁶ | R⁸⁶ | -1,4-(CH₂)₃-Phenyl-(CH₂)₃- |
| 338 | Methyl | Methyl | R⁸⁶ | Methyl | Methyl | R⁸⁶ | -1,4-(CH₂)₃-Phenyl-(CH₂)₃- |
| 339 | R⁸⁶ | R⁸⁶ | R⁸⁶ | R⁸⁶ | R⁸⁶ | R⁸⁶ | -1,4-(CH₂)₃-Phenyl-(CH₂)₃- |
| 340 | Methyl | R⁸⁶ | R⁸⁶ | Methyl | R⁸⁶ | R⁸⁶ | -1,3-(CH₂)₃-Phenyl-(CH₂)₃- |
| 341 | Methyl | Methyl | R⁸⁶ | Methyl | Methyl | R⁸⁶ | -1,3-(CH₂)₃-Phenyl-(CH₂)₃-- |
| 342 | R⁸⁶ | R⁸⁶ | R⁸⁶ | R⁸⁶ | R⁸⁶ | R⁸⁶ | -1,3-(CH₂)₃-Phenyl-(CH₂)₃- |
| 343 | Methyl | R⁸⁶ | R⁸⁶ | Methyl | R⁸⁶ | R⁸⁶ | -1,4-Phenyl- |
| 344 | Methyl | Methyl | R⁸⁶ | Methyl | Methyl | R⁸⁶ | -1,4-Phenyl- |
| 345 | R⁸⁶ | R⁸⁶ | R⁸⁶ | R⁸⁶ | R⁸⁶ | R⁸⁶ | -1,4-Phenyl- |
| 346 | Methyl | R⁸⁶ | R⁸⁶ | Methyl | R⁸⁶ | R⁸⁶ | -1,3-Phenyl- |
| 347 | Methyl | Methyl | R⁸⁶ | Methyl | Methyl | R⁸⁶ | -1,3-Phenyl- |
| 348 | R⁸⁶ | R⁸⁶ | R⁸⁶ | R⁸⁶ | R⁸⁶ | R⁸⁶ | -1,3-Phenyl- |
| 28 | Methyl | Methyl | R⁸⁶ | Methyl | Methyl | R⁸⁶ | -O- |
| 349 | Methyl | R⁸⁶ | R⁸⁶ | Methyl | R⁸⁶ | R⁸⁶ | -O- |
| 350 | Methyl | Methyl | R⁸⁶ | Methyl | Methyl | R⁸⁶ | -O-Si(CH₃)₂-O- |
| 351 | Methyl | Methyl | R⁸⁶ | Methyl | Methyl | R⁸⁶ | -O-Si(CH₃)(Ph)-O- |
| 352 | Methyl | Methyl | R⁸⁶ | Methyl | Methyl | R⁸⁶ | -O-Si(CH₃)₂-O-Si(CH₃)₂-O- |
| 353 | Methyl | Methyl | R⁸⁶ | Methyl | Methyl | R⁸⁶ | -O-Si(CH₃)₂-O-Si(CH₃)₂-O- Si(CH₃)₂-O- |
| 354 | Methyl | -OSiMe₃ | R⁸⁶ | Methyl | -OSiMe₃ | R⁸⁶ | -O- |
| 355 | Methyl | Phenyl | R⁸⁶ | Methyl | Phenyl | R⁸⁶ | -O- |
| 356 | i-Propyl | i-Propyl | R⁸⁶ | i-Propyl | i-Propyl | R⁸⁶ | -O- |
| 357 | Cyclopen tyl | Cyclopentyl l | R⁸⁶ | Cyclopentyl | Cyclopentyl | R⁸⁶ | -O- |
| 358 | Phenyl | Phenyl | R⁸⁶ | Phenyl | Phenyl | R⁸⁶ | -O- |
| 359 | Phenyl | R⁸⁶ | R⁸⁶ | Phenyl | R⁸⁶ | R⁸⁶ | -O- |
| 360 | R⁸⁶ | R⁸⁶ | R⁸⁶ | R⁸⁶ | R⁸⁶ | R⁸⁶ | -O- |

In dieser Tabelle ist

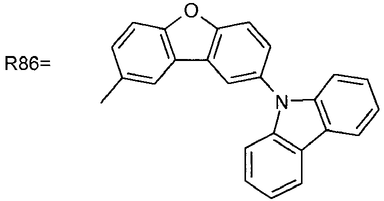

Weitere geeignete Verbindungen der Formel (XI) und/oder (XI*) sind im Folgenden genannt. Darin bedeutet R jeweils unabhängig voneinander Me, Phenyl oder R⁸⁶, wobei mindestens ein Rest R R⁸⁶ ist:

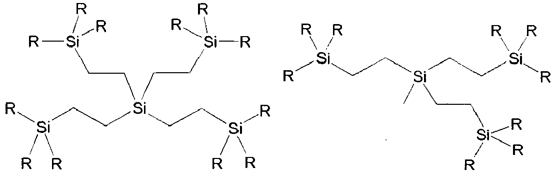

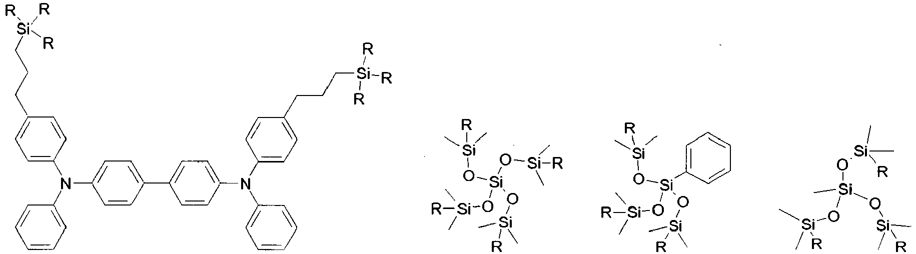

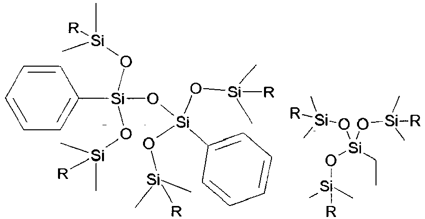

In einer ganz besonders bevorzugten Ausführungsform betrifft die vorliegende Erfindung eine OLED, die neben mindestens einem Metall-Carben-Komplex der allgemeinen Formel (I) mindestens eine Verbindung der allgemeinen Formel (X) enhält, wobei es sich bei der Verbindung der Formel (X) ganz besonders bevorzugt um mindestens eine der nachstehend genannten Verbindungen handelt:

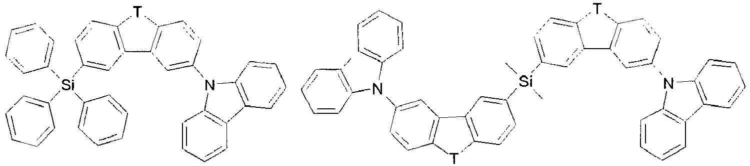

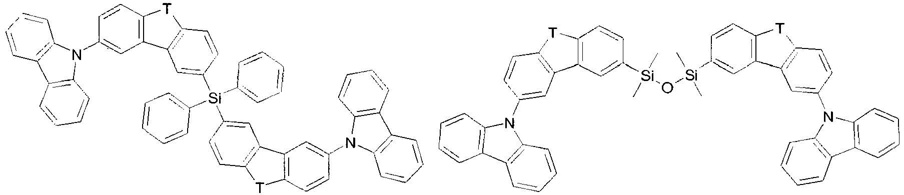

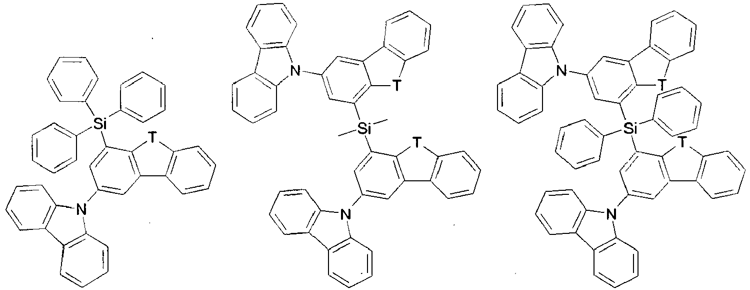

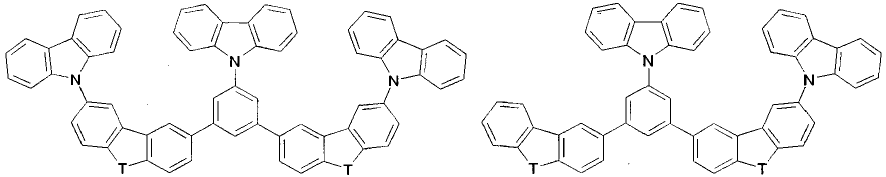

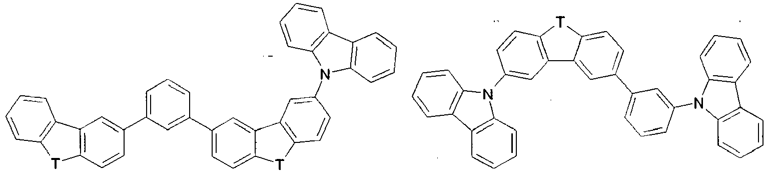

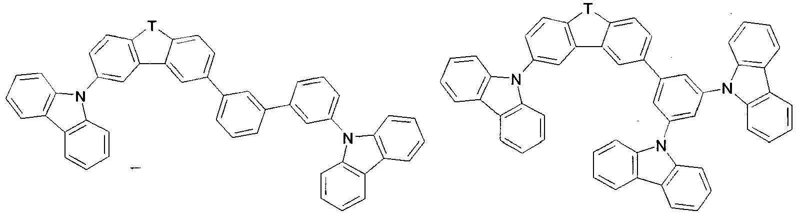

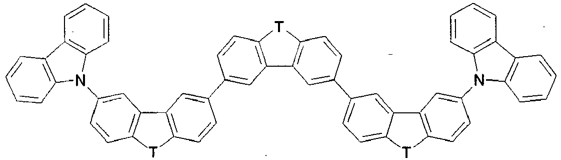

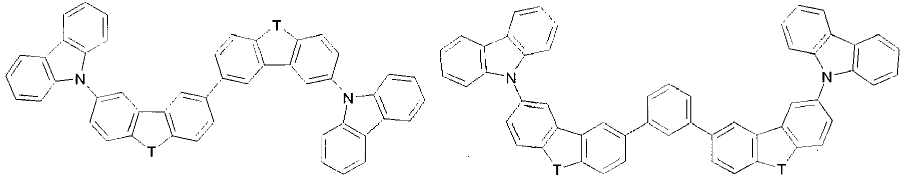

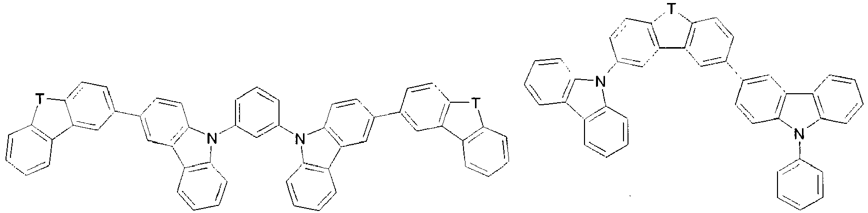

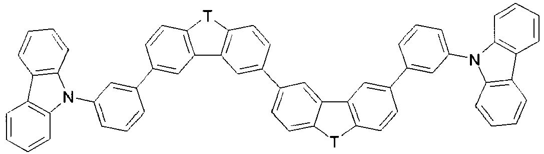

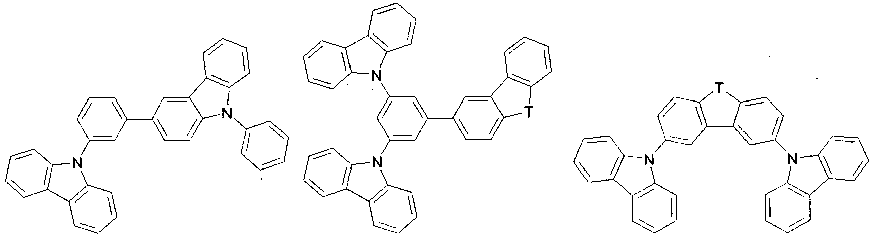

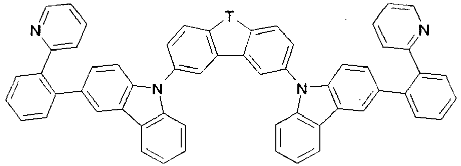

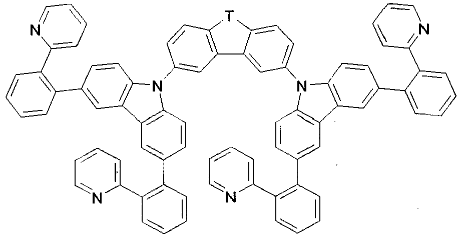

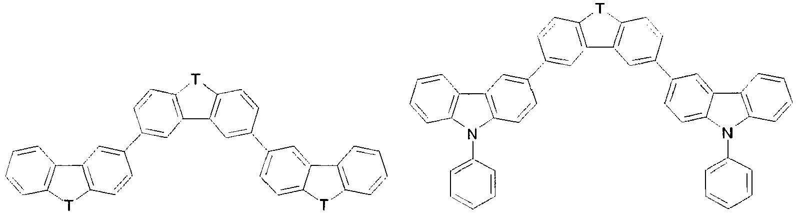

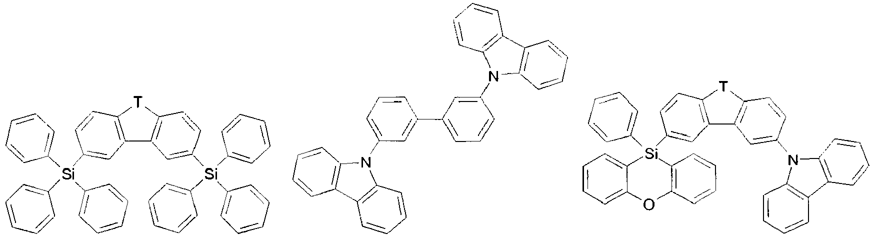

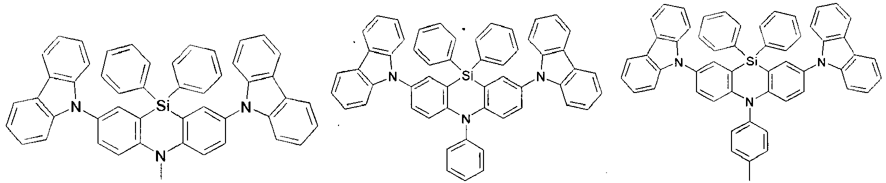

In den vorstehend genannten Verbindungen bedeutet T O oder S, bevorzugt O. Wenn mehr als ein T im Molekül vorkommt, haben alle Gruppen T die gleiche Bedeutung.

Neben den Verbindungen der Formel (X) können gemäß der vorliegenden Erfindung auch vernetzte oder polymere Materialien umfassend Wiederholungseinheiten basierend auf der allgemeinen Formel (X) in vernetzter oder polymerisierter Form gemeinsam mit mindestens einem Metall-Carben-Komplex der allgemeinen Formel (I) eingesetzt werden. Diese werden - wie die Verbindungen der allgemeinen Formel (X) - bevorzugt als Matrixmaterialien eingesetzt.

Die vernetzten oder polymeren Materialien weisen eine hervorragende Löslichkeit in organischen Lösungsmitteln, hervorragende Film-formende Eigenschaften und relativ hohe Glasübergangstemperaturen auf. Zusätzlich können hohe Ladungsträgermobilitäten, hohe Stabilitäten der Farbemission und lange Betriebszeiten der entsprechenden Bauteile beobachtet werden, wenn vernetzte oder polymere Materialien gemäß der vorliegenden Erfindung in organischen Licht-emittierenden Dioden (OLEDs) verwendet werden.

Die ernetzten oder polymersierten Materialien eignen sich besonders als Beschichtungen oder in dünnen Filmen, da sie thermisch und mechanisch stabil und relativ defektfrei sind.

Die vernetzten oder polymerisierten Materialien umfassend Wiederholungseinheiten basierend auf der allgemeinen Formel (X) können durch ein Verfahren umfassend die Schritte (a) und (b) hergestellt werden:
(a) Herstellen einer vernetzbaren oder polymerisierbaren Verbindung der allgemeinen Formel (X), wobei wenigstens einer der a" Reste R⁵⁵ oder wenigstens einer der b' Reste R⁵⁶ eine über einen Spacer angebundene vernetzbare oder polymerisierbare Gruppe bedeutet, und
(b) Vernetzung oder Polymerisation der aus Schritt (a) erhaltenen Verbindung der allgemeinen Formel (X).

Bei den vernetzten oder polymerisierten Materialien kann es sich um Homopolymere handeln, d. h. ausschließlich Einheiten der allgemeinen Formel (X) liegen in vernetzter oder polymerisierter Form vor. Es kann sich auch Copolymere handeln, d. h. neben den Einheiten der allgemeinen Formel (X) liegen weitere Monomere, beispielsweise Monomere mit lochleitenden und/oder elektronenleitenden Eigenschaften, in vernetzter oder polymerisierter Form vor.

In einer weiteren bevorzugten Ausführungsform der erfindungsgemäßen OLED enthält diese eine Emissionsschicht umfassend mindestens einen erfindungsgemäßen Metall-Carben-Komplex der allgemeinen Formel (I), mindestens Matrixmaterial gemäß Formel (X), und gegebenenfalls mindestens ein weiteres lochtransportierendes Matrixmaterial.

Die erfindungsgemäßen OLEDs können in allen Vorrichtungen eingesetzt werden, worin Elektrolumineszenz nützlich ist. Geeignete Vorrichtungen sind bevorzugt ausgewählt aus stationären und mobilen Bildschirmen und Beleuchtungsmitteln. Die vorliegende Erfindung betrifft daher auch eine Vorrichtung ausgewählt aus der Gruppe bestehend aus stationären Bildschirmen und mobilen Bildschirmen und Beleuchtungsmitteln, enthaltend ein erfindungsgemäßes OLED.

Stationäre Bildschirme sind z. B. Bildschirme von Computern, Fernsehern, Bildschirme in Druckern, Küchengeräten sowie Reklametafeln, Beleuchtungen und Hinweistafeln. Mobile Bildschirme sind z.B. Bildschirme in Handys, Laptops, Digitalkameras, mp-3 Playern, Smartphones, Fahrzeugen sowie Zielanzeigen an Bussen und Bahnen.

Weiterhin können die erfindungsgemäßen Metall-Carben-Komplexe der allgemeinen Formel (I) in OLEDs mit inverser Struktur eingesetzt werden. Bevorzugt werden die erfindungsgemäßen Komplexe in diesen inversen OLEDs wiederum in der Licht-emittierenden Schicht, eingesetzt. Der Aufbau von inversen OLEDs und die üblicherweise darin eingesetzten Materialien sind dem Fachmann bekannt.

Ein weiterer Gegenstand der vorliegenden Erfindung ist eine weiße OLED enthaltend mindestens einen erfindungsgemäßen Metall-Carben-Komplex der allgemeinen Formel (I). In einer bevorzugten Ausführungsform wird der Metall-Carben-Komplex der allgemeinen Formel (I) in der weißen OLED als Emittermaterial eingesetzt. Bevorzugte Ausführungsformen des Metall-Carben-Komplexes der allgemeinen Formel (I) sind vorstehend genannt. Neben dem mindestens einen Metall-Carben-Komplex der allgemeinen Formel (I) kann die weiße OLED
(i) mindestens eine Verbindung der Formel (X) enthalten. Die Verbindung der Formel (X) wird bevorzugt als Matrixmaterial eingesetzt. Bevorzugte Verbindungen der Formel (X) sind vorstehend genannt; und/oder
(ii) mindestens eine Verbindung der Formel (VII) und/oder (IX) enthalten. Die Verbindungen der Formel (VII) und/oder (IX) werden bevorzugt als Elektronentransportmaterial eingesetzt. Bevorzugte Verbindungen der Formeln (VII) und (IX) sind vorstehend genannt.

Um weißes Licht zu erzeugen, muss die OLED Licht erzeugen, das den gesamten sichtbaren Bereich des Spektrums abdeckt. Normalerweise emittieren organische Emitter jedoch nur in einem begrenztem Teil des sichtbaren Spektrums - sind also farbig. Weißes Licht kann durch die Kombination von verschiedenen Emittern erzeugt werden. Üblicherweise werden rote, grüne und blaue Emitter kombiniert Im Stand der Technik sind jedoch auch andere Methoden zur Ausbildung weißer OLEDs bekannt, z.B. der Triplett-Harvesting Ansatz. Geeignete Aufbauten für weiße OLEDs bzw. Methoden zur Ausbildung weißer OLEDs sind dem Fachmann bekannt.

In einer Ausführungsweise einer weißen OLED werden mehrere Farbstoffe in der licht-emittierenden Schicht einer OLED übereinander geschichtet und damit kombiniert (layered device). Das kann durch Mischen aller Farben oder durch ein direktes Hintereinanderschalten verschieden farbiger Schichten erreicht werden. sein. Der Ausdruck "Layered OLED" und geeignete Ausführungsformen sind dem Fachmann bekannt.

Im Allgemeinen haben die verschiedenen Schichten dann folgende Dicken: Anode (2) 500 bis 5000 Å (Angstrom), bevorzugt 1000 bis 2000 Å; Löcher-transportierende Schicht (3) 50 bis 1000 Å, bevorzugt 200 bis 800 Å, entweder eine Licht-emittierende Schicht enthaltend eine Mischung verschiedener Emitter (4): 10 bis 1000 Å, bevorzugt 100 bis 800 Å, oder mehrere Licht-emittierende Schichten hintereinander, wobei jede einzelne einen anderen Emitter enthält (4a,b,c,...): jeweils 10 bis 1000 Å, bevorzugt jeweils 50 bis 600 Å, Elektronen transportierende Schicht (5) 50 bis 1000 Å, bevorzugt 200 bis 800 Å, Kathode (6) 200 bis 10.000 Å, bevorzugt 300 bis 5000 Å.

In einer weiteren Ausführungsweise einer weißen OLED werden mehrere unterschiedlich farbige OLEDs übereinander gestapelt (stacked device). Für das Stapeln zweier OLEDs wird eine sogenannte charge-generation-layer (CG-layer) verwendet. Diese CG-layer kann beispielsweise aus einer elektrisch n-dotierten und einer elektrisch p-dotierten Transportschicht aufgebaut sein. Der Ausdruck "Stacked OLED" und geeignete Ausführungsformen sind dem Fachmann bekannt.

Im Allgemeinen haben die verschiedenen Schichten dann folgende Dicken: Anode (2) 500 bis 5000 Å (Angstrom), bevorzugt 1000 bis 2000 Å; erste Löcher-transportierende Schicht (3) 50 bis 1000 Å, bevorzugt 200 bis 800 Å, erste Licht-emittierende Schicht (4) 10 bis 1000 Å, bevorzugt 50 bis 600 Å, erste Elektronen transportierende Schicht (5) 50 bis 1000 Å, bevorzugt 200 bis 800 Å, elektrisch n-dotierte Schicht 50 bis 1000 Å, bevorzugt 100 bis 800 Å, elektrisch p-dotierte Schicht 50 bis 1000 Å, bevorzugt 100 bis 800 Å, zweite Löcher-transportierende Schicht (3) 50 bis 1000 Å, bevorzugt 200 bis 800 Å, zweite Licht-emittierende Schicht (4) 10 bis 1000 Å, bevorzugt 50 bis 600 Å, zweite Elektronen transportierende Schicht (5) 50 bis 1000 Å, bevorzugt 200 bis 800 Å, elektrisch n-dotierte Schicht 50 bis 1000 Å, bevorzugt 100 bis 800 Å, elektrisch p-dotierte Schicht 50 bis 1000 Å, bevorzugt 100 bis 800 Å, dritte Löcher-transportierende Schicht (3) 50 bis 1000 Å, bevorzugt 200 bis 800 Å, dritte Licht-emittierende Schicht (4) 10 bis 1000 Å, bevorzugt 50 bis 600 Å, dritte Elektronen transportierende Schicht (5) 50 bis 1000 Å, bevorzugt 200 bis 800 Å, Kathode (6) 200 bis 10.000 Å, bevorzugt 300 bis 5000 Å.

In weiteren Ausführungen dieses "Gestapeltes-Device-Konzepts" können auch nur zwei OLEDs gestapelt werden oder mehr als drei OLEDs gestapelt werden.

In einer weiteren Ausführung weißer OLEDs können die beiden genannten Konzepte zur Weißlichterzeugung auch kombiniert werden. Beispielsweise kann eine einfarbige OLED (beispielsweise Blau) mit einer mehrfarbigen geschichteten OLED (beispielsweise Rot-Grün) gestapelt werden. Weitere Kombinationen beider Konzepte sind denkbar und dem Fachmann bekannt.

Der erfindungsgemäße Metall-Carben-Komplex der Formel (I) kann dabei in jeder der vorstehend in weißen OLEDs erwähnten Schichten eingesetzt werden. In einer bevorzugten Ausführungsform wird er in einer oder mehreren oder allen Licht-emittierenden Schicht(en) der OLED(s) eingesetzt, wobei die Struktur des erfindungsgemäßen Metall-Carben-Komplexes in Abhängigkeit des Einsatzes der Komplexes variiert wird. Geeignete und bevorzugte Komponenten für die weiteren Schichten der weißen OLED(s) oder als Matrixmaterial in der oder den Licht-emittierenden Schicht(en) geeignete Materialien und bevorzugte Matrixmaterialien sind ebenfalls vorstehend genannt..

Die vorliegende Erfindung betrifft des Weiteren ein organisches Elektronikbauteil, bevorzugt eine Organische Licht-Emittierende Diode (OLED), Organische Photovoltaik-Zelle (OPV), Organischer Feldeffekttransistor (OFET) oder Licht-Emittierende Elektrochemische Zelle (LEEC), enthaltend wenigstens einen erfindungsgemäßen Metall-Carben-Komplex der allgemeinen Formel (I).

### Beispiele

Die folgenden Beispiele, insbesondere die in den Beispielen aufgeführten Methoden, Materialien, Bedingungen, Prozessparameter, Apparate und Ähnliches sollen die vorliegende Erfindung stützen, den Umfang der vorliegenden Erfindung aber nicht beschränken.

Alle Versuche werden in Schutzgasatmosphäre ausgeführt.

Die in den nachstehenden Beispielen erwähnten %-Angaben und Verhältnisse sind-soweit nicht anders erwähnt - Gew.-% und Gewichtsverhältnisse.

### Beispiel 1:

### 2,3-Bis(N-phenylamino)pyrazin

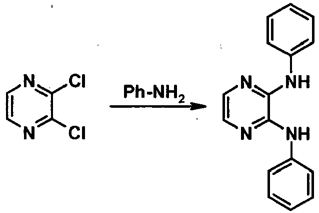

Eine Mischung von 2,3-Dichlorpyrazin (10.0 g, 67 mmol) in Anilin (35.3 mL, 376 mmol) wird bei 105 °C über Nacht gerührt. Nach Abkühlung auf 80 °C wird Wasser (100 mL) zugegeben. Die Mischung wird mit 50%iger Natronlauge auf pH 11 eingestellt und mit Dichlormethan und Essigester extrahiert. Die vereinten organischen Phasen werden zur Trockene eingeengt und das Rohprodukt wird säulenchromatographisch (Kieselgel, n-Hexan/Essigester4:1) gereinigt. Ausbeute: 16.2 g (92%).

¹H-NMR (CD₂Cl₂, 500 MHz): δ = 6.27 (br s, 2H), 7.00 (m_{c}, 2H), 7.26-7.30 (m, 8H), 7.72 (s, 2H).

### 1,3-Diphenylpyrazinoimidazolium-iodid

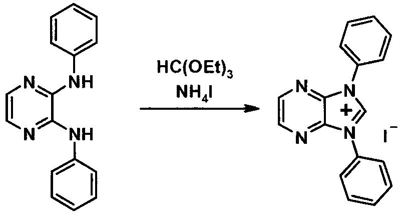

Eine Mischung von 2,3-Bis(N-phenylamino)pyrazin (15.5 g, 59 mmol) in Triethylorthoformiat (100 mL) wird mit Ammoniumiodid (17.1 g, 118 mmol) versetzt und bei 80 °C über Nacht gerührt. Nach Abkühlung auf Raumtemperatur wird der Feststoff abgesaugt, mit Petrolether gewaschen und im Vakuumtrockenschrank bei 70 °C getrocknet. Ausbeute: 19.5 g (82%).

¹H-NMR (d₆-DMSO, 500 MHz): δ = 7.77 (m_{c}, 2H), 7.84 (m_{c}, 4H), 8.03-8.08 (m, 4H), 9.08 (s, 2H), 11.20 (s, 1H).

### Komplex fac-Em1:

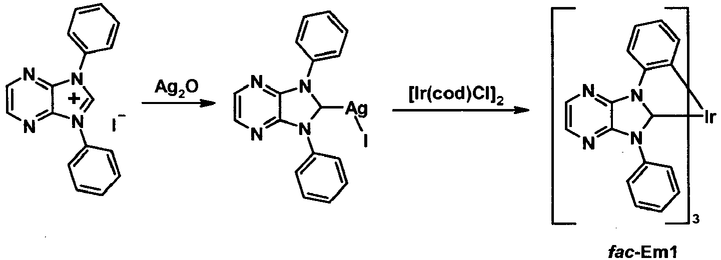

Eine Suspension von 1,3-Diphenylpyrazinoimidazolium-iodid (1.6 g, 4.0 mmol) in Dioxan (95 mL) wird mit Molsieb (14 g) und Silber(I)oxid (742 mg, 3.2 mmol) versetzt und über Nacht bei Raumtemperatur gerührt. Anschließend wird eine Lösung von Chloro-(1,5-cyclooctadiene)-iridium(I)-dimer (269 mg, 0.4 mmol) in o-Xylol (130 mL) zugegeben. Die Mischung wird über Nacht bei Rückfluss gerührt. Nach Abkühlung auf 80 °C wird der Rückstand abgesaugt und mit o-Xylol gewaschen. Die vereinten Filtrate werden zur Trockene eingeengt und der Rückstand wird bei 75 °C in Toluol (40 mL) gelöst. Die Lösung wird aufkonzentriert und über Nacht bei Raumtemperatur stehen gelassen. Der Niederschlag wird abfiltriert, mit Cyclohexan gewaschen und im Vakuumtrockenschrank bei 65°C getrocknet. Ausbeute: 0.8 g (95%).

¹H-NMR (d₆-DMSO, 500 MHz, 100°C): δ = 6.51-6.59 (m, 9H), 6.70 (m_{c}, 3H), 6.81 (m_{c}, 3H), 6.90 (br m, 6H), 7.09 (m_{c}, 3H), 8.16 (d, 3H), 8.42 (d, 3H), 8.66 (dd, 3H).

Photolumineszenz (2% im PMMA-Film):
λₘₐₓ = 474 nm, CIE: (0.16;0.24), QA = 93%

### Beispiel 2:

### 2-N-Isopropylamino-3-chlorpyrazin

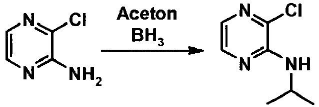

Eine Lösung von 2-Amino-3-chlorpyrazin (2.7 g, 20.8 mmol) in Dichlormethan (54 mL) und Eisessig (27 mL) wird bei 1 °C mit Aceton (4.1 mL, 56.3 mmol) und Boran-Dimethylsulfid-Komplex (2.2 mL, 22.9 mmol) versetzt und über Nacht bei Raumtemperatur gerührt. Eine zweite Portion Boran-Dimethylsulfid-Komplex (0.8 mL) wird bei 1 °C zugegeben und die Mischung wird wieder über Nacht bei Raumtemperatur gerührt. Abschließend wird eine dritte Portion Boran-Dimethylsulfid-Komplex (0.6 mL) und Aceton (1.0 mL) bei 1 °C zugegeben und die Mischung wird wieder über Nacht bei Raumtemperatur gerührt. Die Lösung wird bei < 10 °C mit Ammoniakwasser (38 mL) auf pH 9 eingestellt. Die organische Phase wird abgetrennt und mit Wasser gewaschen. Nach Trocknung über Natriumsulfat wird die Lösung aufkonzentriert und über Nacht stehen gelassen. Der entstandene Niederschlag wird abfiltriert und mit wenig Dichlormethan gewaschen. Die vereinten Filtrate werden zur Trockene eingeengt und im Vakuumtrockenschrank bei 40 °C getrocknet. Ausbeute: 2.3 g (64%).

¹H-NMR (CD₂Cl₂, 500 MHz): δ = 1.22 (d, 6H), 4.16 (sept, 1 H), 5.00 (br s, 1 H), 7.46 (d, 1H), 7.88 (d, 1H).

### 2-N-Phenylamino-3-N-isopropylaminopyrazin

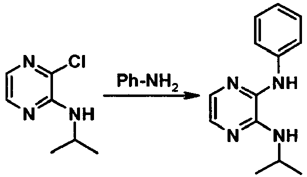

Eine Mischung von 2-Isopropylamino-3-chlorpyrazin (5.1 g, 30 mmol) in Anilin (9 mL, 99 mmol) wird bei 125 °C über Nacht gerührt. Nach Abkühlung auf 90 °C wird Wasser zugegeben. Bei Raumtemperatur wird Dichlormethan zugegeben und die Lösung mit Ammoniakwasser auf pH 9 eingestellt. Die organische Phase wird abgetrennt, mit Wasser gewaschen, über Natriumsulfat getrocknet und zur Trockene eingeengt. Das Rohprodukt wird säulenchromatographisch (Kieselgel, Toluol/Essigester 8:1) aufgereinigt. Ausbeute: 4.6 g (68%).

¹H-NMR (CD₂Cl₂, 500 MHz): δ = 1.23 (d, 6H), 4.15 (br s, 1 H), 4.18 (sept, 1 H), 5.95 (br s, 1H), 6.96-7.02 (m, 1H), 7.23-7.32 (m, 4H), 7.45 (d, 1H), 7.64 (d, 1H).

### 1-Phenyl-3-isopropyl pyrazinoimidazolium-iodid

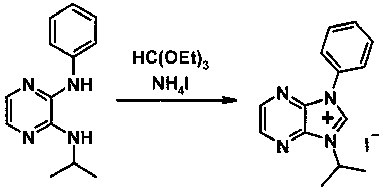

Eine Mischung von 2-N-Phenylamino-3-N-isopropylaminopyrazin (1.2 g, 5.2 mmol) in Triethylorthoformiat (6 mL) wird mit Ammoniumiodid (0.8 g, 5.7 mmol) versetzt und bei 80 °C über Nacht gerührt. Nach Abkühlung auf Raumtemperatur wird der Feststoff abgesaugt, mit wenig Ethanol und Petrolether gewaschen und im Vakuumtrockenschrank bei 50 °C getrocknet. Ausbeute: 1.1 g (60%).

¹H-NMR (d₆-DMSO, 500 MHz): δ = 1.76 (d, 6H), 5.23 (sept, 1H), 7.67-7.84 (m, 3H), 7.91-8.02 (m, 2H), 8.98 (d, 1H), 9.02 (d, 1H), 10.72 (s, 1H).

### Komplex mer-Em2:

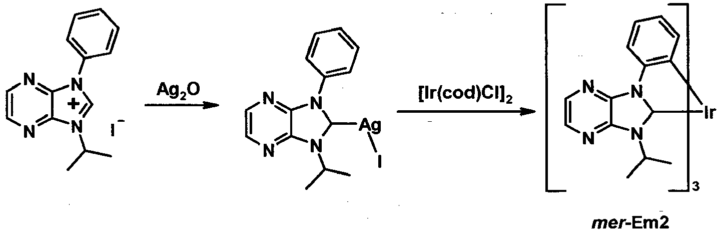

Eine Suspension von 1-Phenyl-3-isopropylpyrazinoimidazolium-iodid (166 mg, 0.48 mmol) in Dioxan (8 mL) wird mit Molsieb (1 g) und Silber(I)oxid (89 mg, 0.38 mmol) versetzt und über Nacht bei Raumtemperatur gerührt. Chloro-(1,5-cyclooctadiene)-iridium(I)-dimer (30 mg, 0.05 mmol) wird zugegeben und die Mischung wird über Nacht bei Rückfluss gerührt. Nach Abkühlung auf Raumtemperatur wird der Rückstand abgesaugt und mit Dichlormethan gewaschen. Die vereinten Filtrate werden zur Trockene eingeengt und der Rückstand wird säulenchromatographisch (Kieselgel, Toluol/Essigester 4:1) aufgereinigt. Ausbeute: 65 mg (80%).

¹H-NMR (CD₂Cl₂, 500 MHz): δ = 0.72 (d, 3H), 0.77 (d, 3H), 0.87 (d, 3H), 1.36 (d, 3H), 1.45 (d, 3H), 1.77 (d, 3H), 4.37-4.53 (m, 2H), 4.65 (sept, 1 H), 6.57 (dd, 1 H), 6.65-6.74 (m, 3H), 6.94-7.24 (m, 5H), 8.17 (d, 1 H), 8.22 (d, 1 H), 8.23 (d, 1 H), 8.28 (d, 1 H), 8.30 (d, 1 H), 8.33 (d, 1 H), 8.60 (dd, 1 H), 8.63 (dd, 1 H), 8.67 (dd, 1 H).

Photolumineszenz (2% im PMMA-Film):
λₘₐₓ = 516 nm, CIE: (0.29;0.51), QA = 66%

### Komplex fac-Em2:

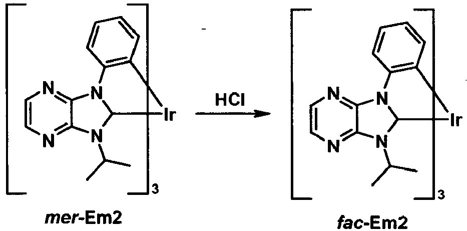

Eine Lösung von *mer*-**Em2** (720 mg, 0.80 mmol) in Butanon (70 mL) wird mit Salzsäure (1 N, 10 mL) versetzt und über Nacht unter Rückfluss gerührt. Die Lösung wird aufkonzentriert, mit Dichlormethan verdünnt und mit Wasser gewaschen. Die organische Phase wird über Natriumsulfat getrocknet und zur Trockene eingeengt. Der Rückstand wird säulenchromatographisch (Kieselgel, Toluol/Essigester 10:1) gereinigt. Ausbeute: 327 mg (45%).

¹H-NMR (CD₂Cl₂, 500 MHz, 100°C): δ = 0.86 (d, 9H), 1.70 (d, 9H), 4.54 (sept, 3H), 6.36 (d, 3H), 6.64 (dd, 3H), 7.02 (dd, 3H), 8.17 (d, 3H), 8.28 (d, 3H), 8.60 (d, 3H).

Photolumineszenz (2% im PMMA-Film):
λₘₐₓ = 474 nm, CIE: (0.16;0.27), QA = 90%

### Beispiel 3:

### 2-N-Phenylamino-3-aminopyrazin

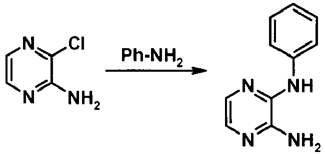

Eine Mischung von 2-Amino-3-chlorpyrazin (5.0 g, 39 mmol) in Anilin (12 mL, 131 mmol) wird bei 100 °C zweimal über Nacht gerührt. Nach Abkühlung auf Raumtemperatur wird die Mischung mit Dichlormethan (80 mL) verdünnt, über Nacht gerührt und filtriert. Der Feststoff wird in Dichlormethan (200 mL) gelöst und mit gesättigter wässriger Natriumhydrogencarbonat-Lösung gewaschen. Die organische Phase wird zur Trockene eingeengt und der Rückstand im Vakuumtrockenschrank bei 70 °C getrocknet. Ausbeute: 5.3 g (74%).

¹H-NMR (CD₂Cl₂, 500 MHz): δ = 4.43 (br s, 2H), 6.23 (br s, 1 H), 7.03 (dd, 1 H), 7.32 (dd, 2H), 7.40 (d, 2H), 7.59 (d, 1H), 7.62 (d, 1H).

### 1-Phenylpyrazinoimidazol

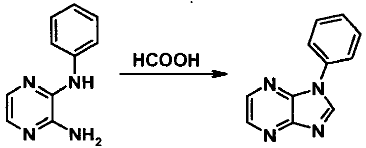

Eine Lösung von 2-N-Phenylamino-3-aminopyrazin (5.3 g, 29 mmol) in Ameisensäure (160 mL) wird über Nacht unter Rückfluss gerührt und anschließend zur Trockene eingeengt. Der Rückstand wird in Dichlormethan (100 mL) aufgenommen und mit gesättigter wässriger Natriumhydrogencarbonatlösung gewaschen. Die wässrige Phase wird mit Dichlormethan (2x 50 mL) extrahiert und die vereinten organischen Phasen werden zur Trockene eingeengt. Ausbeute: 5.6 g (98%)

¹H-NMR (CD₂Cl₂, 500 MHz): δ = 7.49 (dd, 1H), 7.60 (dd, 2H), 7.76 (d, 2H), 8.39 (d, 1 H), 8.56 (d, 1 H), 8.63 (s, 1 H).

### 1-Phenyl-3-methylpyrazinoimidazolium-tetrafluoroborat

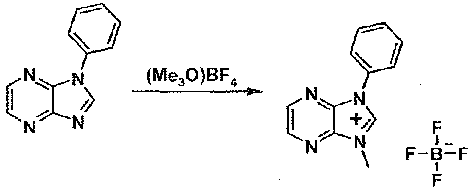

Eine Lösung von 1-Phenylpyrazinoimidazol (5.5 g, 28 mmol) in Dichlormethan (400 mL) wird mit Trimethyloxoniumtetrafluoroborat (4.2 g, 28 mmol) versetzt und über Nacht bei Rückfluss gerührt. Der Niederschlag wird abfiltriert und getrocknet. Das Produkt wird in einer Reinheit von ca. 75% erhalten. Ausbeute: 7.3 g (87%).

¹H-NMR (d₆-DMSO, 500 MHz): δ = 4.19 (s, 3H), 7.71 (dd, 1H), 7.78 (dd, 2H), 7.91 (d, 2H), 8.99 (d, 1H), 9.04 (d, 1H), 10.70 (s, 1H).

### 1-Phenyl-3-methylpyrazinoimidazolium-iodid

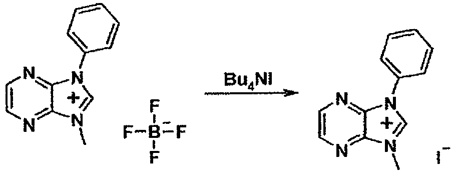

Eine Lösung von 1-Phenyl-3-methylpyrazinoimidazolium-tetrafluoroborat (7.3 g, 25 mmol, Reinheit: 75%) in Acetonitril (70 mL) wird mit einer Lösung von Tetrabutylammoniumiodid (27.2 g, 74 mmol) in Acetonitril (67 mL) versetzt und über Nacht bei Raumtemperatur gerührt. Der Niederschlag wird abgesaugt und mit Petrolether gewaschen. Das Produkt wird in einer Reinheit von ca. 75% erhalten. Ausbeute: 6.0 g (72%).

¹H-NMR (d₆-DMSO, 500 MHz): δ = 4.19 (s, 3H), 7.71 (dd, 1H), 7.78 (dd, 2H), 7.92 (d, 2H), 8.99 (d, 1 H), 9.04 (d, 1H), 10.72 (s, 1H).

### Komplex mer-Em3:

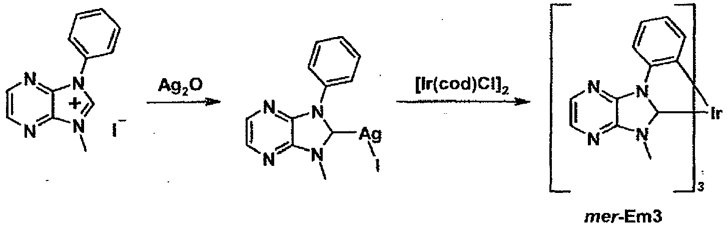

Eine Suspension von 1-Phenyl-3-methylpyrazinoimidazolium-iodid (1.0 g, 2.3 mmol) in Dioxan (60 mL) wird mit Molsieb (8.4 g) und Silber(I)oxid (0.4 g, 1.8 mmol) versetzt und über Nacht bei Raumtemperatur gerührt. Eine Lösung von Chloro-(1,5-cyclooctadiene)-iridium(I)-dimer (153 mg, 0.2 mmol) in o-Xylol (83 mL) wird zugegeben und die Mischung wird über Nacht bei Rückfluss gerührt. Nach Abkühlung auf Raumtemperatur wird der Rückstand abgesaugt und mit Aceton gewaschen. Die vereinten Filtrate werden zur Trockene eingeengt und der Rückstand wird säulenchromatographisch (Kieselgel, Dichlormethan) aufgereinigt. Ausbeute: 44 mg (13%).

¹H-NMR (CD₂Cl₂, 500 MHz): δ = 3.22 (s, 3H), 3.36 (s, 3H), 3.37 (s, 3H), 6.60 (dd, 1H), 6.70-6.78 (m, 3H), 6.87 (dd, 1H), 6.92 (dd, 1H), 7.01-7.10 (m, 3H), 8.22 (d, 1H), 8.27 (d, 1 H), 8.28 (d, 1 H), 8.32 (d, 1 H), 8.35 (d, 1 H), 8.36 (d, 1 H), 8.60 (dd, 1 H), 8.67 (dd, 1H), 8.68 (dd, 1 H).

Photolumineszenz (2% im PMMA-Film):
λₘₐₓ = 515 nm, CIE: (0.29;0.50), QA = 74%

### Komplex fac-Em3:

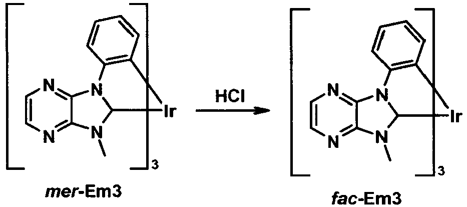

Eine Lösung von *mer*-**Em3** in Butanon wird mit Salzsäure versetzt und über Nacht unter Rückfluss gerührt. Die Lösung wird aufkonzentriert, mit Dichlormethan verdünnt und mit Wasser gewaschen. Die organische Phase wird über Natriumsulfat getrocknet und zur Trockene eingeengt. Der Rückstand wird säulenchromatographisch gereinigt.

### Beispiel 4

### µ-Dichlorodimer D1:

*N*-(2,6-diisopropylphenyl)-2-phenylimidazol wird analog Beispiel 14 in WO2006/121811 synthetisiert.

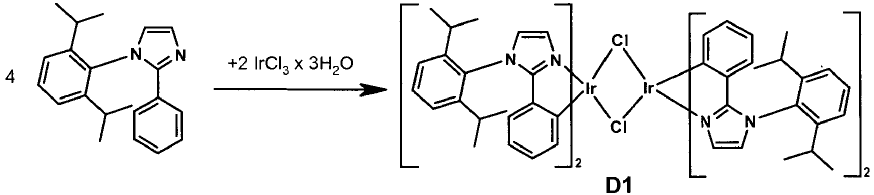

3.50 g (11.5 mmol) 1-(2,6-Diisopropyl-phenyl)-2-phenyl-1 H-imidazol werden in 200 ml 2-Ethoxyethanol/Wasser (Verhältnis 3/1) vorgelegt und mit 1.84 g (5.2 mmol) Iridium(III)chlorid-Trihydrat versetzt. Das Reaktionsgemisch wird 18 h am Rückfluss erhitzt. Nach dem Abkühlen werden 50 ml destilliertes Wasser zugegeben. Der Niederschlag wird abfiltriert, mit destilliertem Wasser gewaschen und getrocknet. Man erhält 3.50 g (80%) µ-Dichlorodimer **D1** als gelbes Pulver.

¹H-NMR (CD₂Cl₂, 400 MHz):
δ= 0.95 (d, 12H), 1.18 (d, 12H), 1.27 (d, 12H), 1.34 (d, 12H), 2.80-2.91 (m, 8H), 6.08 (d, 4H), 6.24 (d, 4H), 6.39 (pt, 4H), 6.53 (pt, 4H), 6.97 (d, 4H), 7.39-7.45 (m, 8H), 7.59 (t, 4H), 7.67 (d, 4H).

### Komplex mer-Em7:

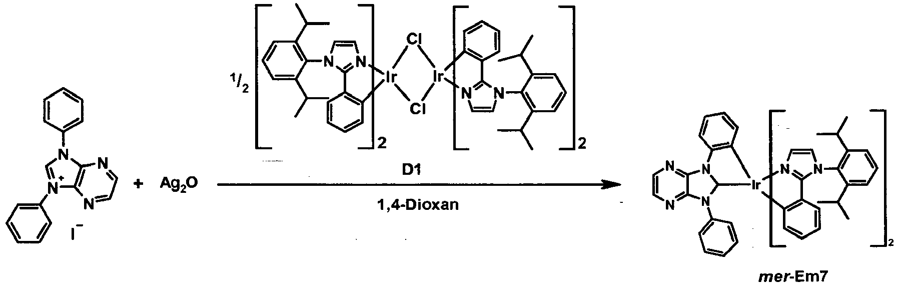

Eine Suspension von 1,3-Diphenylpyrazinoimidazolium-iodid (0.5 g, 1.25 mmol) in wasserfreiem Dioxan (100 ml) wird mit Molsieb (10 g) und Silber(I)oxid (0.19 g, 0.81 mmol) versetzt und über Nacht bei Raumtemperatur gerührt. Anschließend wird eine Lösung aus Chlorodimer D1 (0.52 g, 0.31 mmol) in Dioxan (74 ml) zugetropft. Danach wird eine Stunde unter Rückfluss gerührt. Die Reaktionsmischung wird abgekühlt und filtriert. Das Filtrat wird im Vakuum vom Lösungsmittel befreit, mit Methanol gewaschen und anschließend säulenchromatographisch (Kieselgel, Laufmittel Cyclohexan/Aceton = 1/0.25) aufgereinigt. Man erhält 0.40 g *mer***-Em7** als orangefarbenes Pulver (63%).

¹H-NMR (CD₂Cl₂, 500 MHz):
δ = 0.89 (d, 3H), 0.90 (d, 6H), 0.92 (d, 3H), 1.09 (d, 3H), 1.16 (d, 3H), 1.21 (d, 3H), 1.24 (d, 3H), 2.13 (sept, 1H), 2.66 (m, 2H), 2.73 (sept, 1H), 6.08-6.23 (m, 5H), 6.45-6.48 (m, 3H), 6.66 (d, 1H), 6.70-6.76 (m, 3H), 6.85-6.92 (m, 2H), 6.98 (m, 4H), 7.08-7.13 (m, 2H), 7.31 (t, 2H), 7.35 (d, 1H), 7.39 (d, 1H), 7.50 (t, 1H), 7.55 (t, 1H), 8.21 (d, 1H), 8.41 (d, 1 H), 8.80 (d, 1 H).

Photolumineszenz (2% im PMMA-Film):
λₘₐₓ = 565 nm, CIE: (0.44;0.49)

### Komplex fac-Em7:

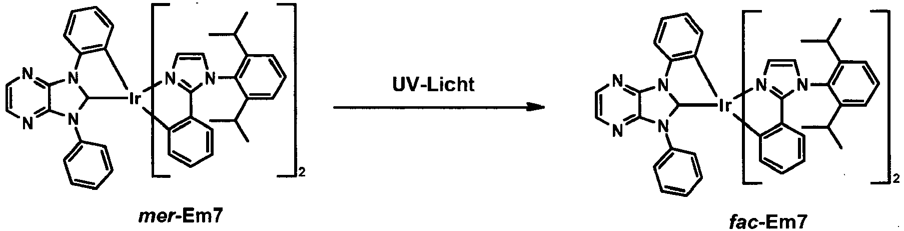

Der Komplex *fac***-Em7** (Isomer zu *mer***-Em7**) wird durch Bestrahlung einer Lösung von *mer*-**Em7** in 3-Methoxypropionitril mit einer Blacklight-Blue-Lampe (Osram, L18W/73, λₘₐₓ = 370-380 nm) und anschließender säulenchromatographischer Aufreinigung erhalten.

### Beispiel 5:

### Komplex K1:

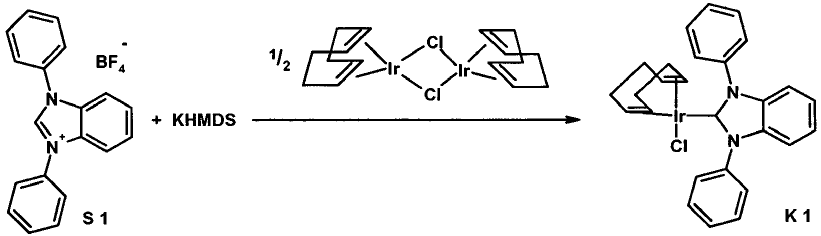

5.00 g (14.0 mmol) Benzimidazoliumsalz **S1** werden in 80 ml wasserfreiem Toluol suspendiert und auf -8°C abgekühlt. Dann werden 28 ml Bis(trimethylsilyl)kaliumamid (KHMDS, 0.5M in Toluol, 14.0 mmol) innerhalb von 10 min zugesetzt. Das Gemisch wird eine Stunde bei Raumtemperatur gerührt und danach innerhalb von 15 min bei-78°C zu einer Lösung von 4.70 g (7.0 mmol) [(µ-Cl)Ir(η⁴-1,5-COD)]₂ in 120 ml Toluol getropft. Die Reaktionsmischung wird 1.5 h bei Raumtemperatur gerührt und anschließend 19 h am Rückfluss erhitzt. Nach dem Abkühlen wird der Niederschlag abfiltriert und mit Toluol gewaschen. Die vereinigten Toluolphasen werden zur Trockene eingeengt und säulenchromatographisch (Kieselgel, Laufmittel Methylenchlorid) gereinigt. Man erhält 5.8 g (68 %) **K1** als gelbes Pulver.

¹H-NMR (CD₂Cl₂, 500 MHz):
δ = 1.17 (m, 2H), 1.34 (m, 4H), 1.61 (m, 2H), 2.43 (m, 2H), 4.31 (m, 2H), 7.18 (m, 2H), 7.25 (m, 2H), 7.51 (m, 6H), 7.96 (m, 4H).

### Komplex Em8:

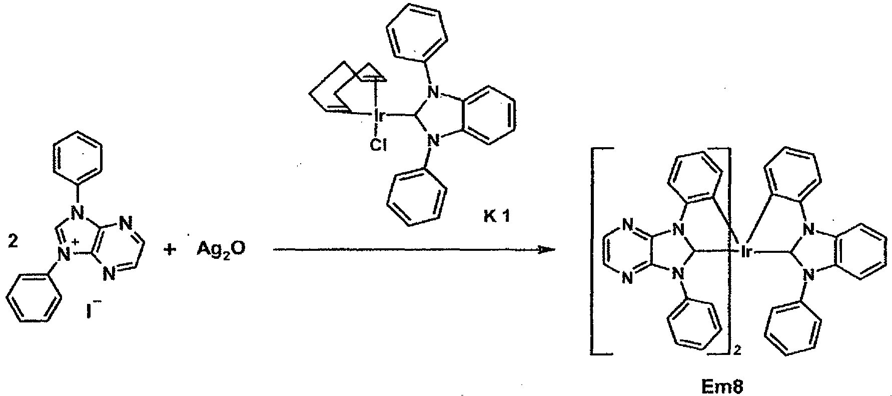

Eine Suspension von 1,3-Diphenylpyrazinoimidazolium-iodid (3.17 g, 7.92 mmol) in wasserfreiem 1,4-Dioxan (140 ml) wird mit Molsieb (15 g) und Silber(I)oxid (1.48 g, 6.34 mmol) versetzt und über Nacht bei Raumtemperatur gerührt. Anschließend wird eine Lösung aus Komplex **K1** (1.60 g, 2.64 mmol) in wasserfreiem o-Xylol (200 ml) zugetropft. Danach wird 24 Stunden unter Rückfluss gerührt. Die Reaktionsmischung wird abgekühlt und filtriert. Das Filtrat wird im Vakuum vom Lösungsmittel befreit und säulenchromatographisch (Kieselgel, Laufmittel Ethylacetat/Cyclohexan = 1/2) aufgereinigt. Man erhält 0.80 g **Em8** (30%).

¹H-NMR (CD₂Cl₂, 500 MHz):
δ = 6.20-7.40 (sehr flaches breites Signal, 8H), 6.26 (d, 1 H), 6.36-6.42 (m, 3H), 6.59 (d, 1H), 6.66 (t, 2H), 6.76-6.85 (m, 6H), 7.06-7.17 (m, 4H), 7.29 (t, 1H), 7.33 (t, 1H), 8.01 (m, 2H), 8.04 (d, 1 H), 8.18 (d, 1 H), 8.27 (d, 1 H), 8.31 (d, 1 H), 8.70 (d, 1 H), 8.77 (d, 1 H).

Photolumineszenz (2% im PMMA-Film):
λₘₐₓ = 480 nm, CIE: (0.17;0.31)

### Beispiel 6:

### Komplex D2:

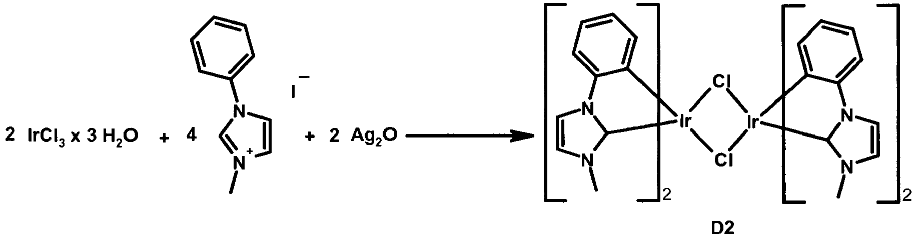

4.29 g (18.5 mmol) Silberoxid, 9.47g (33.1 mmol) Imidazoliumiodid und 3.56 g (10.1 mmol) Iridiumtrichloridtrihydrat werden in 350 ml 2-Ethoxyethanol suspendiert und 15 h bei 120°C im Dunkeln gerührt. Danach entfernt man das Lösungsmittel im Vakuum und extrahiert den Rückstand mit Methylenchlorid. Der Extrakt wird auf ca. ein Viertel seines Volumens eingeengt und mit Methanol versetzt. Der ausfallende Niederschlag wird abfiltriert und getrocknet. Man erhält 1.7 g Komplex D2 (31%).

¹H-NMR (CD₂Cl₂, 500 MHz):
δ = 7.59 (d, 4H), 7.17 (d, 4H), 6.99 (d, 4H), 6.73 (pt, 4H), 6.45 (pt, 4H), 6.09 (d, 4H), 3.91 (s, 12H).

### Komplex Em9:

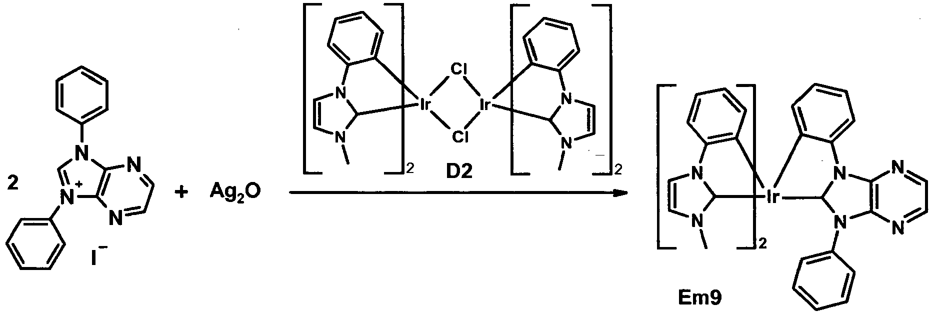

Eine Suspension von 1,3-Diphenylpyrazinoimidazolium-iodid (0.59 g, 1.48 mmol) in wasserfreiem 1,4-Dioxan (35 ml) wird mit Molsieb (5 g) und Silber(I)oxid (0.28 g, 1.18 mmol) versetzt und über Nacht bei Raumtemperatur gerührt. Anschließend wird eine Suspension aus Komplex D2 (0.40 g, 0.37 mmol) in wasserfreiem o-Xylol (50 ml) zugegeben. Danach wird 3.5 Stunden unter Rückfluss gerührt. Die Reaktionsmischung wird abgekühlt und filtriert. Das Filtrat wird im Vakuum vom Lösungsmittel befreit und säulenchromatographisch (Kieselgel, Laufmittel Dichlormethan) aufgereinigt.

Man erhält eine Fraktion 1 von 0.11 g (20%, orangefarbenes Pulver, R_{F}-Wert = 0.39, 20/80-Isomerengemisch) und eine Fraktion 2 von 0.23 g (40%, gelbes Pulver, R_{F}-Wert = 0.31, 40/60-Isomerengemisch) als Gemische von jeweils zwei unterschiedlichen Isomeren von **Em9.**
Fraktion 1:
MS (Maldi):
m/e = 779 (M+H⁺)
Photolumineszenz (2% im PMMA-Film):
λₘₐₓ = 547 nm, CIE: (0.40;0.54)
Fraktion 2:
MS (Maldi):
m/e = 779 (M+H⁺)
Photolumineszenz (2% im PMMA-Film):
λₘₐₓ = 526 nm, CIE: (0.33;0.55)

### Beispiel 7:

### Komplex K2:

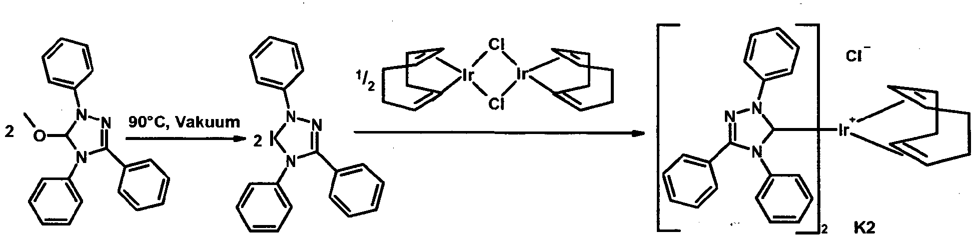

3.10 g (9.41 mmol) 5-Methoxy-1,3,4-triphenyl-4,5-dihydro-1H-[1,2,4]-triazol werden 16 h auf 90°C unter Vakuum (10⁻³mbar) erhitzt. Nach dem Abkühlen wird der Rückstand in 120 ml wasserfreiem Toluol aufgenommen und mit einer Lösung von 1.38 g (2.03 mmol) µ-Chloro-(1,5-cyclooctadien)iridium(I)-Dimer in 120 ml wasserfreiem Toluol versetzt. Das Reaktionsgemisch wird langsam auf 50°C erhitzt und 1 h bei dieser Temperatur gerührt. Nach dem Abkühlen wird der orangerote Niederschlag abfiltriert, mit Toluol gewaschen und getrocknet. Man erhält 2.68 g **K2** (70%).

¹H-NMR (CD₂Cl₂, 500 MHz):
δ = 1.19 (m, 2H), 1.29 (m, 2H), 2.14 (m, 2H), 2.26 (m, 2H), 2.77 (m, 2H), 4.95 (m, 2H), 7.25-7.44 (m, 26H), 7.82 (d, 4H).

### Komplex Em10:

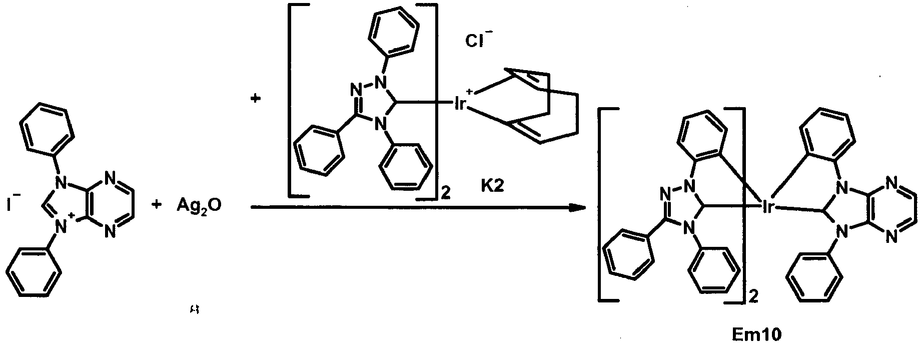

Eine Suspension von 0.5 g (1.25 mmol) 1,3-Diphenylpyrazinoimidazolium-iodid in trockenem Dioxan (40 ml) wird mit 10 g Molsieb und 0.22 g (0.95 mmol) Silber(I)oxid versetzt und über Nacht bei Raumtemperatur gerührt. Anschließend werden portionsweise 0.94 g (1.01 mmol) Komplex **K2** zugegeben. Das Reaktionsgemisch wird über Nacht unter Rückfluss erhitzt. Nach dem Abkühlen wird der Niederschlag abfiltriert. Das Filtrat wird zur Trockenen eingeengt, säulenchromatographisch aufgereinigt (Kieselgel, Laufmittel: Cyclohexan/Aceton) und mit etwas Methanol gewaschen. Man erhält 0.21 g Emitter **Em10** (20%).

### Beispiel 8:

### Komplex K3:

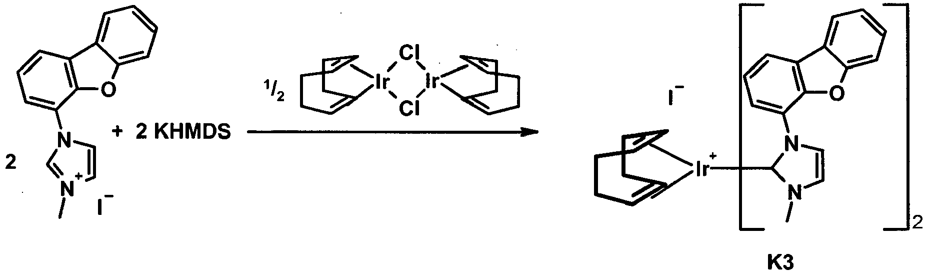

10.4 g (27.6 mmol) 3-Dibenzofuran-4-yl-1-methyl-3H-imidazol-1-ium-iodid in 230 ml wasserfreiem Toluol werden bei Raumtemperatur langsam mit 55.3 ml Bis-(trimethylsilyl)-kaliumamid-Lösung (KHMDS, 0.5M in Toluol, 27.6 mmol) versetzt und eine Stunde gerührt. Anschließend wird eine Lösung von 4.68 g (6.9 mmol) µ-Chloro-(1,5-cyclooctadien)iridium(I)-Dimer in 230 ml wasserfreiem Toluol zugetropft. Das Reaktionsgemisch wird über Nacht am Rückfluss erhitzt. Nach dem Abkühlen wird der Niederschlag abfiltriert, mit wenig Wasser gewaschen und mit Ethanol extrahiert. Nach Trocknen des Extraktes erhält man 6.8 g **K3** (53%) als orange-rotes Pulver.

¹H-NMR (CD₂Cl₂, 500 MHz):
δ =1.49 (m, 2H), 1.84 (m, 2H), 2.02 (m, 2H), 2.16 (m, 2H), 3.29 (m, 2H), 4.85 (m, 2H), 6.53 (s, 2H), 6.93 (s, 2H), 7.37 (d, 2H), 7.47-7.62 (m, 8H), 8.14 (d, 2H), 8.25 (d, 2H):

### Komplex Em11:

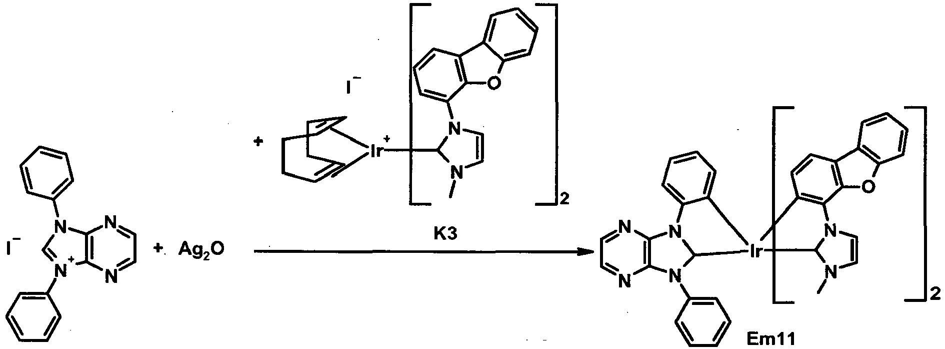

Die Synthese von **Em11** erfolgt analog zur Synthese von **Em10.**

### Beispiel 9:

### Herstellung einer OLED - Vergleich verschiedener Emitter

Das als Anode verwendete ITO-Substrat wird zuerst mit kommerziellen Reinigungsmitteln für die LCD-Produktion (Deconex^{®} 20NS und Neutralisationsmittel 25ORGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs. Als nächstes wird die Lochinjektionsschicht AJ20-1000 der Firma Plexcore aus Lösung aufgesponnen.

Danach werden die nachfolgend genannten organischen Materialien mit einer Rate von ca. 0.5-5 nm/min bei etwa 10⁻⁷-10⁻⁹ mbar auf das gereinigte Substrat aufgedampft. Als Lochleiter und Excitonenblocker wird Ir(DPBIC)₃ mit einer Dicke von 45 nm auf das Substrat aufgebracht, wovon die ersten 35 nm zur Verbesserung der Leitfähigkeit mit MoOₓ (10% für Diode mit **CEm** und ***fac*-Em2,** 50% für Diode mit ***fac*-Em1**) dotiert sind. 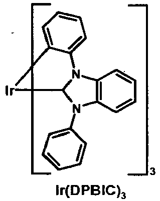 (zur Herstellung von Ir(DPBIC)₃ siehe Ir-Komplex (7) in der Anmeldung PCT/EP/04/ 09269).

Anschließend wird eine Mischung aus Emitter (10% für **CEm,** 20% für ***fac*-Em1** und **fac-Em2)** und der Verbindung **Ma1** (90% und 80%, resp.), mit einer Dicke von 40 nm aufgedampft, wobei letztere Verbindung als Matrixmaterial fungiert. Anschließend wird das Material **Ma1** (für ***fac*-Em1)** mit einer Dicke von 5 nm oder **Ma5** (5 nm für **Em1,** 10 nm für ***fac*-Em2)** als Excitonen- und Lochblocker aufgedampft.

Die Synthese von **Ma1** wird in WO2010079051 beschrieben, die von Ma5 in WO2009003898.

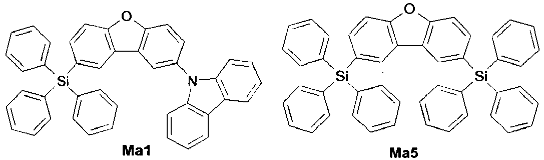

Als Vergleichsemitter des Standes der Technik wird **CEm** eingesetzt:

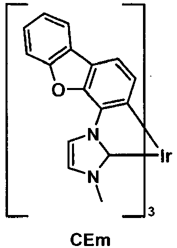

Als nächstes wird als Elektronentransporter eine Mischung von Liq und BCP (2,9-Dimethyl-4,7-diphenyl-1,10-phenantrolin) (50:50 für ***fac*-Em1,** 0:100 für **CEm** und ***fac-***Em2) in einer Dicke von 40 nm für ***fac*-Em1,** 30 nm für **CEm** und ***fac*-Em2,** eine 1,0 nm dicke Liq-Schicht für ***fac*-Em1,** eine 0.7 nm LiF-Schicht für **CEm** und ***fac*-Em2** und abschließend eine 100 nm dicke Al-Elektrode aufgedampft. Alle Bauteile werden in einer inerten Stickstoffatmosphäre mit einem Glasdeckel verklebt.

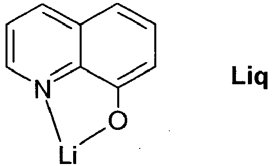

Zur Charakterisierung der OLED werden Elektrolumineszenz-Spektren bei verschiedenen Strömen bzw. Spannungen aufgenommen. Weiterhin wird die Strom-Spannungs-Kennlinie in Kombination mit der abgestrahlten Lichtleistung gemessen. Die Lichtleistung kann durch Kalibrierung mit einem Luminanzmeter in photometrische Größen umgerechnet werden. Die Lebensdauer t_{1/2} der Diode ist definiert durch die Zeit, die vergeht bis die Luminanz auf 50% ihres initialen Werts abgesunken ist. Die Lebensdauermessung wird bei einem konstanten Strom durchgeführt.

Für die verschiedenen Emitter in dem oben beschriebenen OLED-Aufbau ergeben sich die folgenden elektrooptischen Daten:

| Emitter | CIE | Im/W @ 300nits | t_{1/2} @ 300nits (normiert auf den Wert von **CEm**) |
|---|---|---|---|
| **CEm** | 0.15,0.15 | 6.5 | 100% |
| ***fac*-Em2** | 0.17,0.30 | 6.4 | 714% |
| ***fac*-Em1** | 0.17,0.28 | 4.5 | 5300% |

### Beispiel 10:

### Einfluss des Matrixmaterials (Teil 1)

Die Emissionsschicht des in Beispiel 9 beschriebenen Aufbaus wird variiert. Der Dotiergrad von MoOₓ beträgt 50%. Eine Mischung aus Emitter *fac***-Em1** (20%) und verschiedenen Matrixmaterialien (80%) wird mit einer Dicke von 20 nm aufgedampft. Als Loch/Excitonenblocker (5 nm) wird jeweils das Matrixmaterial eingesetzt. Als Elektronenleiter wird eine Mischung aus BCP und Liq (50:50) in einer Dicke von 40 nm eingesetzt. Als Elektroninjektor wird Liq (1 nm) benutzt. Neben **Ma1** werden folgende Materialien eingesetzt:

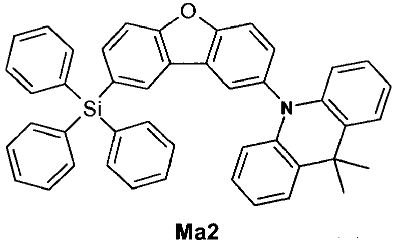

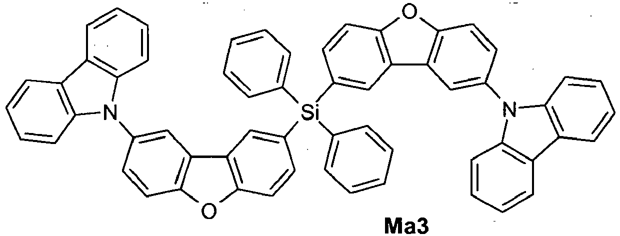

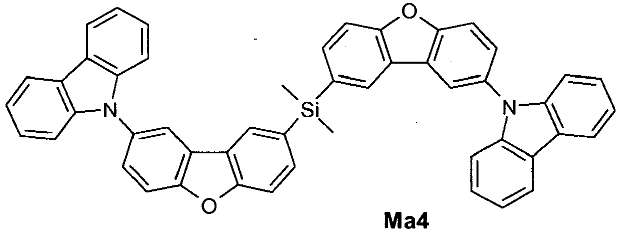

Die Synthesen von **Ma2, Ma3** und **Ma4** werden in WO2010079051 beschrieben.

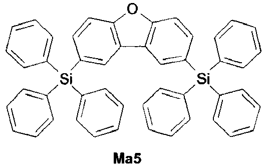

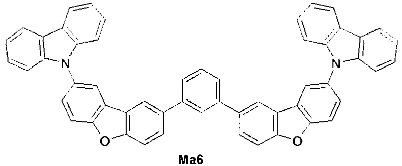

**Ma6** kann nach einem für einen Fachmann bekannten Verfahren hergestellt werden. 9-(8-Bromo-dibenzofuran-2-yl)-9H-carbazol (s. WO2010079051) wird mit n-BuLi in THF und 2-Isopropoxy-4,4,5,5-tetramethyl-1,3,2-dioxaborolan zu 9-[8-(4,4,5,5-Tetramethyl-[1,3,2]dioxaborolan-2-yl)-dibenzofuran-2-yl]-9H-carbazol umgesetzt (analog an Stufe 1 von Beispiel 18), was dann in einer Fachmann bekannten Suzuki-Kupplung mit 1,3-Diiodbenzol zu **Ma6** umgesetzt wird.

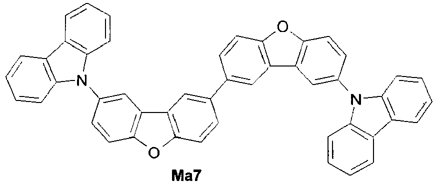

Die Synthese von **Ma7** ist in Beispiel 19 beschrieben.

Für die verschiedenen Matrixmaterialien in dem oben beschriebenen OLED-Aufbau ergeben sich die folgenden elektrooptischen Daten (normiert auf den Wert von **Ma5):**

| Matrix | CIE | EQE @ 300nits | Im/W @ 300nits | Spannung @ 300nits |
|---|---|---|---|---|
| **Ma2**^{a,b,c,d,e} | 0.17,0.28 | 98% | 120% | 95% |
| **Ma3**^{f} | 0.17,0.28 | 106% | 160% | 77% |
| **Ma1** | 0.17,0.26 | 119% | 164% | 80% |
| **Ma4**^{f,g} | 0.17,0.28 | 109% | 176% | 72% |
| **Ma5** | 0.16,0.23 | 100% | 100% | 100% |
| **Ma6**^{a} | 0.17,0.29 | 120% | 171% | 83% |
| **Ma7**^{a, f, h} | 0.17,0.31 | 133% | 275% | 60% |

| | | | | |
|---|---|---|---|---|
| ^{a} 10% MoOₓ Dotierung ^{b} Emissionsschicht 40 nm ^{c} **Ma1** als Loch/Excitonenblocker 10 nm ^{d} Elektronenleiter: 20 nm BCP**:Ma1** 80:20 ^{e} Elektroninjektor: LiF (0.7 nm) ^{f} Dotiergrad 30% Emitter in 70% Matrix ^{g} Elektronenleiterschicht 35 nm ^{h} Loch/excitonenblocker 10 nm, Elektronenleiter 20 nm, Elektroninjektor CsF | | | | |

### Beispiel 10a:

Die Emissionsschicht des in Beispiel 10 beschriebenen Aufbaus wird variiert. Als Emissionschicht wird eine Mischung aus *fac*-**Em1** (30%) und Matrix (s. unterstehende Tabelle, 70%) benutzt. Als Lochleiter wird eine Schicht von 40 nm Ir(DPBIC)₃ verwendet, wovon die ersten 35 nm mit ReO₃ (5%) dotiert wurden. Als Loch/Excitonenblocker wird eine Schicht von 5 nm **Ma7** verwendet. Als Elektronenleiter wird eine Mischung aus Liq und **ETM1** (50:50, 40 nm) verwendet. Als Elektroneninjektor wird KF in einer Dicke von 4 nm verwendet. 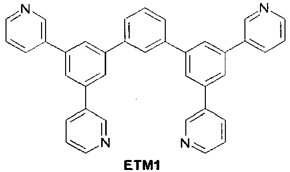 **ETM1** ist kommerziell erhältlich.

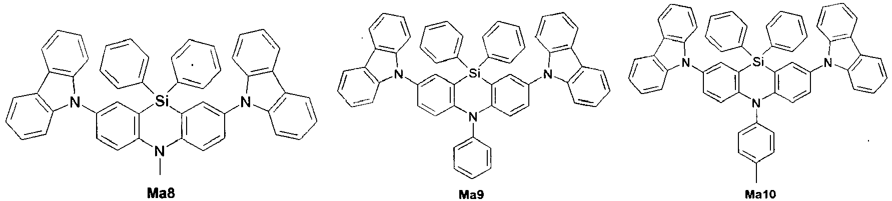

Die Synthesen von **Ma8, Ma9,** und **Ma10** werden in JP2006083167 beschrieben.

Für die verschiedenen Matrixmaterialien in dem oben beschriebenen OLED-Aufbau ergeben sich die folgenden elektrooptischen Daten (normiert auf den Wert von **Ma8):**

| Matrix | CIE | EQE @ 300nits |
|---|---|---|
| **Ma8** | 0.18,0.31 | 100% |
| **Ma9** | 0.17,0.30 | 145% |
| **Ma10** | 0.17,0.31 | 128% |
| **Ma7*** | 0.16,0.26 | 154% |

| | | |
|---|---|---|
| *Dotiergrad Emitter 10% | | |

### Beispiel 10b

Eine Diode mit dem Emitter **Em8** wird analog zu Beispiel 10 aufgebaut. Die Lochleiterschicht ist 40 nm dick. **Em8** wird mit 30% in **Ma4** dotiert. Als Elektronenleiterschicht wird **ETM1** in einer Dicke von 40 nm benutzt. Als Elektroneninjektor wird KF (2 nm) eingesetzt.

Die Diode zeigt die Farbkoordinaten CIE 0.19, 0.37. Bei 300 cd/m² beträgt die Spannung 3.5 V und die Leuchteffizienz 11 lm/W

### Beispiel 10c

Eine Diode wird analog zu Beispiel 10a für **Ma7** aufgebaut. Als Emitter wird **Em8** statt *fac***-Em1** benutzt.

Die Diode zeigt die Farbkoordinaten CIE 0.17, 0.31.

### Beispiel 10d

Eine Diode wird analog zu Beispiel 10a für **Ma7** aufgebaut. Als Emitter wird **Em8** statt *fac***-Em1** benutzt. Die Matrix wird variiert. Für die verschiedenen Matrixmaterialien in dem oben beschriebenen OLED-Aufbau ergeben sich die folgenden elektrooptischen Daten (normiert auf den Wert von **Ma10):**

| Matrix | CIE | EQE @ 300nits | lm/W @ 300nits | Spannung @ 300nits |
|---|---|---|---|---|
| **Ma10** | 0.17,0.32 | 100% | 100% | 100% |
| **Ma8** | 0.17,0.30 | 128% | 133% | 94% |
| **Ma9** | 0.17,0.30 | 149% | 156% | 92% |

### Beispiel 10e

Eine Diode wird analog zu Beispiel 10a aufgebaut. Als Emitter wird *fac***-Em12** statt fac-**Em1** in einer Dotierkonzentration von 20% in der Matrix **Ma4** (80%) benutzt. Der Loch/Excitonenblocker wird in 10 nm Dicke aufgebracht.

Die Diode zeigt die Farbkoordinaten CIE 0.19, 0.36. Bei 300 cd/m² beträgt die Spannung 4.0 V und die externe Quanteneffizienz 13%.

### Beispiel 10f

Eine Diode wird analog zu Beispiel 10a aufgebaut. Als Emitter wird *fac***-Em14** statt fac-**Em1** in einer Dotierkonzentration von 20% in der Matrix **Ma4** (80%) benutzt. Der Loch/Excitonenblocker wird in 10 nm Dicke aufgebracht.

Die Diode zeigt die Farbkoordinaten CIE 0.20, 0.27.

### Beispiel 10g

Eine Diode wird analog zu Beispiel 10a aufgebaut. Als Lochleiter und Excitonenblocker wird Ir(DPBIC)₃ mit einer Dicke von 45 nm auf das Substrat aufgebracht, wovon die ersten 35 nm zur Verbesserung der Leitfähigkeit mit MoOₓ (10%) dotiert sind. Der Loch/Excitonenblocker wird in 10 nm Dicke aufgebracht. Als Emitter wird *fac*-**Em13** statt *fac***-Em1** in einer Dotierkonzentration von 20% in der Matrix **Ma4** (80%) benutzt.

Die Diode zeigt die Farbkoordinaten CIE 0.14, 0.22. Bei 300 cd/m² beträgt die Spannung 3.7 V.

### Beispiel 10h

Eine Diode wird analog an Beispiel 10g aufgebaut. Als Emitter wird *fac*-**Em13** in einer Dotierkonzentration von 20% in der Matrix **Ma7** (80%) benutzt.

Die Diode zeigt die Farbkoordinaten CIE 0.14, 0.22. Bei 300 cd/m² beträgt die Spannung 3.6 V und die externe Quanteneffizienz 16%

### .Beispiel 11:

### Einfluss des Matrixmaterials (Teil 2)

Die Emissionsschicht des in Beispiel 10 beschriebenen Aufbaus wird variiert. Eine Mischung aus dem Emitter *fac***-Em1** (20%) und einem bzw. zwei Matrixmaterialien wird mit einer Dicke von 20 nm aufgedampft. Wenn zwei Matrixmaterialien benutzt werden, werden beide Matrixmaterialien in gleichen Verhältnissen eingesetzt. Als Loch/Excitonenblocker wird jeweils **Ma1** eingesetzt.

Es ergeben sich die folgenden elektrooptischen Daten:

| Matrix | CIE | Spannung @ 300nits (normiert auf den Wert von **Ma1**) |
|---|---|---|
| **Ma1** | 0.17,0.26 | 100% |
| **Ir(DPBIC)₃** | 0.16,0.24 | 83% |
| **Ma1** + **Ir(DPBIC)₃** | 0.16,0.23 | 77% |
| **Ma11** | 0.17,0.29 | 93% |
| **Ma11** + **Ir(DPBIC)₃** | 0,16,0.25 | 73% |

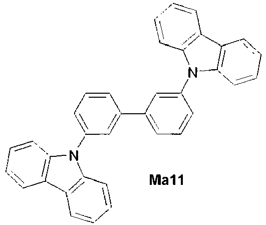

**Ma11** ist kommerziell erhältlich.

### Beispiel 11a:

Aufbau wie in Beispiel 10a. Als Emissionschicht wird eine Mischung aus Ir(DPBIC)₃ (35%), *fac***-Em1** (30%) und Matrix (35%, s. unterstehende Tabelle) benutzt. Als Loch/Excitonenblocker wird jeweils das Matrixmaterial benutzt.

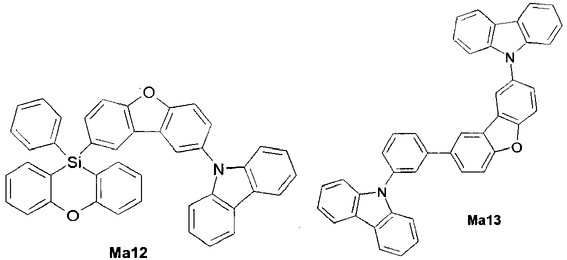

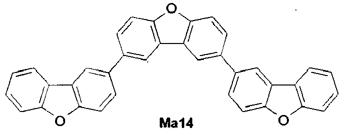

Die Synthese von **Ma13** wird in WO2009008100 beschrieben.

Die Synthese von **Ma12** ist in Beispiel 20 beschrieben und die Synthese von **Ma14** ist in Beispiel 18 beschrieben.

Für die verschiedenen Matrixmaterialien in dem oben beschriebenen OLED-Aufbau ergeben sich die folgenden elektrooptischen Daten (normiert auf den Wert von **Ma9):**

| Matrix | CIE | EQE @ 300nits | lm/W @ 300nits | Spannung @ 300nits |
|---|---|---|---|---|
| **Ma9*** | 0.18,0.32 | 100% | 100% | 100% |
| **Ma7** | 0.18,0.33 | 105% | 112% | 97% |
| **Ma4** | 0.17,0.32 | 102% | 107% | 94% |
| **Ma12^{a}** | 0.17,0.30 | 117% | 124% | 91% |
| **Ma13** | 0.17,0.31 | 116% | 107% | 106% |
| **Ma14** | 0.17,0.31 | 104% | 121% | 86% |

| | | | | |
|---|---|---|---|---|
| *Als Loch/Excitonenblocker wird **Ma7** benutzt ^{a} Elektroneninjektionsschicht 2 nm | | | | |

### Beispiel 11 b

Aufbau wie in Beispiel 10a. Als Emissionschicht wird eine Mischung aus Ir(DPBIC)₃ (45%), **Em8** (10%) und Matrix (45%, s. unterstehende Tabelle) benutzt. Für die verschiedenen Matrixmaterialien in dem oben beschriebenen OLED-Aufbau ergeben sich die folgenden elektrooptischen Daten (normiert auf den Wert von **Ma9):**

| Matrix | CIE | EQE @ 300nits | lm/W @ 300nits | Spannung @ 300nits |
|---|---|---|---|---|
| **Ma9*** | 0.17,0.31 | 100% | 100% | 100% |
| **Ma4** | 0.17,0.34 | 107% | 131% | 87% |
| **Ma7** | 0.17,0.35 | 106% | 129% | 87% |
| **Ma13**^{a} | 0.17,0.32 | 93% | 118% | 79% |

| | | | | |
|---|---|---|---|---|
| *Als Loch/Excitonenblocker wird **Ma7** benutzt ^{a}Als Elektroneninjektor wird CsF (4 nm) benutzt | | | | |

### Beispiel 11 c

Aufbau wie in Beispiel 11a. Als Lochleiter und Excitonenblocker wird Ir(DPBIC)₃ mit einer Dicke von 40 nm auf das Substrat aufgebracht, wovon die ersten 35 nm zur Verbesserung der Leitfähigkeit mit MoOₓ (50%) dotiert sind. Als Emissionschicht wird eine Mischung aus Ir(DPBIC)₃ (40%), *fac***-Em12** (20%), und **Ma4** (40%) benutzt. Als Elektronenleiterschicht wird eine Mischung von **ETM1:Liq** (75:25) in einer Dicke von 40 nm benutzt. Als Elektroneninjektor wird KF (2 nm) eingesetzt.

Die Diode zeigt die Farbkoordinaten CIE 0.20, 0.40. Bei 300 cd/m² beträgt die Spannung 3.0 V und die externe Quanteneffizienz 13%.

### Beispiel 11 d

Aufbau wie in Beispiel 11b. Als Emissionschicht wird eine Mischung aus Ir(DPBIC)₃ (45%), *fac*-Em14 (10%) und **Ma4** (45%) benutzt.

Die Diode zeigt die Farbkoordinaten CIE 0.26, 0.39. Bei 300 cd/m² beträgt die Spannung 3.0 V.

### Beispiel 11e

Eine Diode wird analog zu Beispiel 11a aufgebaut. Als Lochleiter und Excitonenblocker wird Ir(DPBIC)₃ mit einer Dicke von 45 nm auf das Substrat aufgebracht, wovon die ersten 35 nm zur Verbesserung der Leitfähigkeit mit MoOₓ (10%) dotiert sind. Als Loch/Excitonenblocker wird **Ma7** (5 nm) benutzt. Als Emissionschicht wird eine Mischung aus Ir(DPBIC)₃ (40%), ***fac*-Em13** (30%) und **Ma4** (30%) benutzt.

Die Diode zeigt die Farbkoordinaten CIE 0.15, 0.26. Bei 300 cd/m² beträgt die Spannung 3.4 V.

### Beispiel 11f

Eine Diode wird analog zu Beispiel 11a aufgebaut. Als Lochleiter und Excitonenblocker wird Ir(DPBIC)₃ mit einer Dicke von 45 nm auf das Substrat aufgebracht, wovon die ersten 35 nm zur Verbesserung der Leitfähigkeit mit MoOₓ (10%) dotiert sind. Der Loch/Excitonenblocker wird in 10 nm Dicke aufgebracht. Als Emissionschicht wird eine Mischung aus Ir(DPBIC)₃ (40%), ***fac-*Em13** (30%) und **Ma7** (30%) benutzt.

Die Diode zeigt die Farbkoordinaten CIE 0.14, 0.25. Bei 300 cd/m² beträgt die externe Quanteneffizienz 15%.

### Beispiel 11g

Eine Diode wird analog zu Beispiel 11a aufgebaut. Als Emissionschicht wird eine Mischung aus Ir(DPBIC)₃ (40%), ***fac*-Em13** (30%) und **Ma15** (30%) benutzt.

Die Diode zeigt die Farbkoordinaten CIE 0.14, 0.23. Bei 300 cd/m² beträgt die externe Quanteneffizienz 17%. 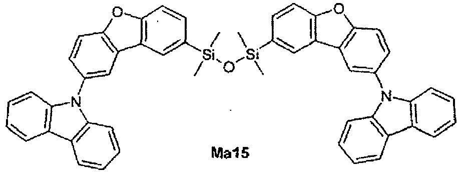 Synthese von **Ma15** wird in WO2010079051 beschrieben.

### Beispiel 11 h

Eine Diode wird analog zu Beispiel 11a für **Ma12** aufgebaut. Statt **Ma12** wird **Ma16** (s. Synthese in JP2009267255) benutzt.

Die Diode zeigt die Farbkoordinaten CIE 0.18, 0.34 und eine Spannung von 3.3 V bei 300 cd/m².

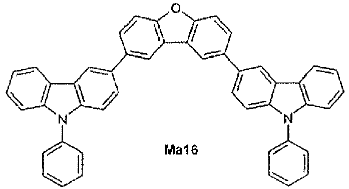

### Beispiel 11i:

### Weiße Dioden

Das als Anode verwendete ITO-Substrat wird zuerst mit kommerziellen Reinigungsmitteln für die LCD-Produktion (Deconex^{®} 20NS und Neutralisationsmittel 25ORGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs. Als nächstes wird die Lochinjektionsschicht AJ20-1000 der Firma Piexcore aus Lösung aufgesponnen.

### Layered white (Beispiel 11 ia):

Nach der Lochinjektionsschicht werden die nachfolgend genannten organischen Materialien mit einer Rate von ca. 0.5-5 nm/min bei etwa 10⁻⁷-10⁻⁹ mbar auf das gereinigte Substrat aufgedampft. Als Lochleiter und Excitonenblocker wird Ir(DPBIC)₃ mit einer Dicke von 20 nm auf das Substrat aufgebracht, wovon die ersten 10 nm zur Verbesserung der Leitfähigkeit mit 10% MoOₓ dotiert sind.

Anschließend wird eine Mischung aus 10% Emitter **Rot1** und 90% der Verbindung **Ma7** mit einer Dicke von 6 nm aufgedampft, wobei letztere Verbindung als Matrixmaterial fungiert. In einer weiteren Ausführung 11ib wird die vorstehend genannte Mischung durch eine Mischung aus 10% Emitter **Rot1,** 60% der Matrix **Ma7** und 30% der Matrix Ir(DPBIC)₃ ersetzt:

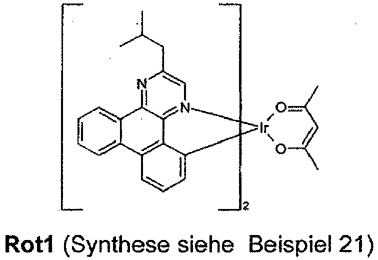

Anschließend wird eine Mischung der Materialien ***fac*-Em1, Ma7** und Ir(DPBIC)₃ mit einer Schichtdicke von 30 nm aufgedampft. Das Mischungsverhältnis beträgt 40% Emitter ***fac*-Em1,** 30% der Matrix **Ma7** und 30% der zweiten Matrix Ir(DPBIC)₃. Anschließend wird das Material **Ma7** mit einer Schichtdicke von 10 nm als Loch- und Excitonen-Blocker aufgebracht. Als folgende Elektronenleiterschicht dient eine mit Cs₂CO₃ dotierte BCP Schicht mit einer Schichtdicke von 30 nm. Eine Aluminiumkathode von 100 nm Dicke schließt die Diode ab.

Alle Bauteile werden in einer inerten Stickstoffatmosphäre mit einem Glasdeckel verklebt.

### Stacked white (Beispiel 11ic):

Nach der Lochinjektionsschicht werden die nachfolgend genannten organischen Materialien mit einer Rate von ca. 0.5-5 nm/min bei etwa 10⁻⁷-10⁻⁹ mbar auf das gereinigte Substrat aufgedampft. Als Lochleiter und Excitonenblocker wird Ir(DPBIC)₃ mit einer Dicke von 20 nm auf das Substrat aufgebracht, wovon die ersten 10 nm zur Verbesserung der Leitfähigkeit mit 10% MoOₓ dotiert sind.

Anschließend wird eine Mischung der Materialien ***fac*-Em1, Ma1** und Ir(DPBIC)₃ mit einer Dicke von 20 nm aufgedampft. Das Mischungsverhältnis beträgt 30% Emitter ***fac-*Em1,** 40% der Matrix **Ma1** und 30% der Matrix Ir(DPBIC)₃. Anschließend wird eine reine Schicht 10 nm **Ma1** als Excitonen- und Lochblocker aufgebracht.

Die folgende Kombination aus 20 nm BCP dotiert mit Cs₂CO₃ und 60 nm Ir(DPBIC)₃ (in einer weiteren Ausführung 11 id mit 90 nm Ir(DPBIC)₃) dotiert mit 10% MoOₓ dient als ladungsgenerierende Schicht.

Anschließend wird Ir(DPBIC)₃ als Lochleiter und Excitonenblocker mit einer Dicke von 10 nm auf das Substrat aufgebracht. Darauf folgend wird eine Mischung der Materialien **Rot1,** NPD und **Ma17** mit einer Dicke von 20 nm aufgebracht. Das Mischungsverhältnis beträgt 10% Emitter **Rot1,** 40% der Matrix NPD und 50% der zweiten Matrix **Ma17.**

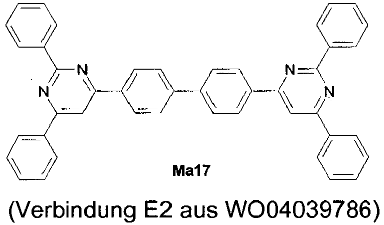

Anschließend wird eine reine Schicht 10 nm BAlq als Excitonen- und Lochblocker aufgebracht.

Als folgende Elektronenleiterschicht dient eine mit Cs₂CO₃ dotierte BCP Schicht mit einer Schichtdicke von 50 nm. Eine Aluminiumkathode von 100 nm Dicke schließt die Diode ab.

Alle Bauteile werden in einer inerten Stickstoffatmosphäre mit einem Glasdeckel verklebt.

Zur Charakterisierung der OLED werden Elektrolumineszenz-Spektren bei verschiedenen Strömen bzw. Spannungen aufgenommen. Weiterhin wird die Strom-Spannungs-Kennlinie in Kombination mit der abgestrahlten Lichtleistung gemessen. Die Lichtleistung kann durch Kalibrierung mit einem Luminanzmeter in photometrische Größen umgerechnet werden.

Für die beiden Ausführungsbeispiele einer weißen OLED ergeben sich die folgenden elektrooptischen Daten:

| Bsp | CIE | Im/W @ 1000nits | EQE^{*} @ 1000nits |
|---|---|---|---|
| **11ia** | 0.33, 0.36 | 14.8 Im/W | 13.8 % |
| **11ib** | 0.29, 0.36 | 16.4 Im/W | 12.9 % |
| **11ic** | 0.3,0.3 | 18.5 Im/W | 30.0 % |
| **11id** | 0.35, 0.37 | 21.5 Im/W | 29.3 % |

| | | | |
|---|---|---|---|
| ^{*}EQE - externe Quanteneffizienz. Gemessen in Vorwärtsrichtung unter der Annahme einer Lambertschen Lichtintensitätsverteilung. | | | |

### Beispiel 11j: flüssigprozessierte Emissionsschicht

Das als Anode verwendete ITO-Substrat wird zuerst mit kommerziellen Reinigungsmitteln für die LCD-Produktion (Deconex^{®} 20NS und Neutralisationsmittel 25ORGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs. Als nächstes wird die Lochinjektionsschicht AJ20-1000 der Firma Plexcore aus Lösung aufgesponnen.

Danach wird der Lochleiter und Excitonenblocker Ir(DPBIC)₃ aus der flüssigen Phase aufgebracht. Hierzu wird eine Lösung von Ir(DPBIC)₃ in THF mit einer Konzentration von 10mg/ml bei einer Geschwindigkeit von 5000 Umdrehungen pro Minute (U/min) aufgeschleudert (Spin-coating).

Anschließend wird die Emissionsschicht bestehend aus dem Emitter ***fac*-Em1** und dem Host **Ma7** ebenfalls aus flüssiger Phase aufgebracht. Hierzu wird eine Lösung von **fac-Em1** und **Ma7** in Methylenchlorid mit einer Konzentration von 10mg/ml bei einer Geschwindigkeit von 5000 U/min aufgeschleudert. Das Gewichtsverhältnis der Feststoffe zwischen Emitter und Host beträgt 30:70.

Danach werden die nachfolgend genannten organischen Materialien mit einer Rate von ca. 0.5-5 nm/min bei etwa 10⁻⁷-10⁻⁹ mbar auf die bereits vorhandenen Schichten im Vakuum aufgedampft.

Als erstes werden 10 nm reines **Ma7** als Excitonenblocker aufgedampft. Anschließend wird der Elektronenleiter **ETM1:Liq** (50:50) mit einer Schichtdicke von 20 nm aufgedampft.

Als Elektroneninjektor folgt dann 1 nm CsF und als Kathode werden 100nm Aluminium aufgedampft. Das Bauteil wird in einer inerten Stickstoffatmosphäre mit einem Glasdeckel verklebt und zeigt die Farbkoordinaten CIE x= 0.20, y=0.35.

### Beispiel 12:

### Einfluss einer gemischten Elektronenleiterschicht

Die elektronentransportierende Schicht des in Beispiel 9 beschriebenen Aufbaus für *fac*-**Em1** wird variiert. Es ergeben sich die folgenden elektrooptischen Daten:

| Elektronenleiter (20 nm) | CIE | Im/W @ 300nits | t_{1/2} @ 300 nits (normiert auf den Wert von BCP) |
|---|---|---|---|
| BCP | 0.16,0.22 | 3.2 | 100% |
| BCP:Liq 50% | 0.17,0.26 | 9.0 | 2780% |

### Beispiel 13:

### Emitter Em12:

### 2,3-Bis-(N-4'-methylphenylamino)pyrazin

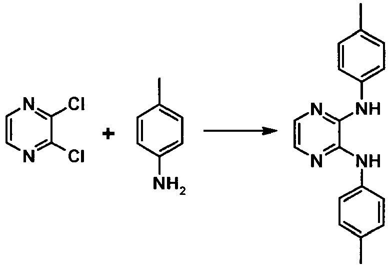

Eine Mischung von 2,3-Dichlorpyrazin (13.4 g, 90 mmol) in p-Toluidin (21.2 g, 198 mmol) wird bei 110 °C über Nacht gerührt. Nach Abkühlung auf Raumtemperatur wird die Mischung in Dichlormethan (200 mL) aufgenommen und mit 25%iger Natronlauge ausgeschüttelt. Die vereinten organischen Phasen werden über Natriumsulfat getrocknet und zur Trockene eingeengt. Der Rückstand wird mit Petrolether verrührt, abgesaugt und mit Cyclohexan gewaschen. Ausbeute: 15.7 g (60%).

¹H-NMR (CD₂Cl₂, 500 MHz): δ = 2.30 (s, 6H), 6.24 (br s, 2H), 7.10 (d, 4H), 7.18 (d, 4H), 7.67 (s, 2H).

### 2-Ethoxy-1,3-bis-4'-methylphenyl)pyrazinoimidazolin

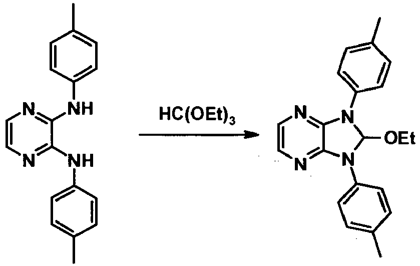

Eine Mischung von 2,3-Bis(N-4'-methylphenylamino)pyrazin (4.0 g, 14 mmol) in Triethylorthoformiat (65 mL) wird bei 75°C über Nacht gerührt. Nach Abkühlung auf Raumtemperatur wird die Mischung filtriert und das Filtrat zur Trockne eingeengt. Der Rückstand wird aus Methyl-tert-butylether umkristallisiert. Ausbeute: 3.3 g (70%).

¹H-NMR (CD₂Cl₂, 500 MHz): δ = 0.99 (t, 3H), 2.32 (s, 6H), 3.24 (q, 2H), 7.16 (s, 1H), 7.21 (d, 4H), 7.42 (s, 2H), 7.85 (d, 4H).

### Komplex fac-Em12:

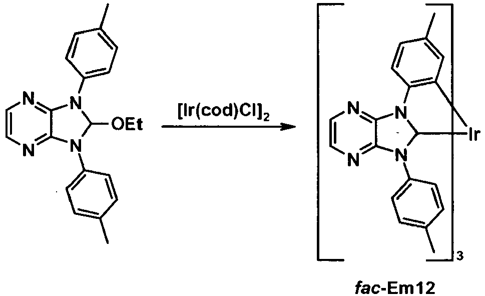

Eine Lösung von 2-Ethoxy-1,3-bis-(4'-methylphenyl)pyrazinoimidazolin (3.5 g, 10 mmol) in o-Xylol (60 mL) wird mit Molsieb 3Å (6 g) und Chloro-(1,5-cyclooctadiene)-iridium(I)-dimer (672 mg, 1.0 mmol) versetzt. Die Mischung wird über Nacht bei 115°C gerührt. Nach Abkühlung auf 80 °C wird der Rückstand abgesaugt und mit Dichlormethan gewaschen. Die vereinten Filtrate werden zur Trockene eingeengt und der Rückstand wird mit warmem Isopropanol gewaschen. Das Rohprodukt wird säulen-chromatographisch (Kieselgel, Toluol → Dichlormethan) gereinigt. Ausbeute: 0.8 g (37%).

¹H-NMR (CD₂Cl₂, 500 MHz): δ = 1.85 (s, 9H), 2.06 (s, 9H), 6.44 (s, 3H), 6.91 (d, 3H), 7.97 (d, 3H), 8.27 (d, 3H), 8.56 (d, 3H). Die 12 Protonen des nicht-cyclometallierten Toluyl-Ringes sind bei Raumtemperatur aufgrund der Rotation des Aromaten nur als sehr breite Erhebung im aromatischen Bereich erkennbar.

Photolumineszenz (2% im PMMA-Film):
λₘₐₓ = 485 nm, CIE: (0.18;0.36), QA = 85%

### Beispiel 14:

### Emitter Em13:

### Pyrazinverbindung (a)

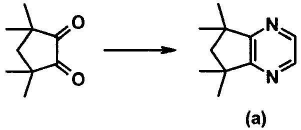

Eine Mischung von 3,3,5,5-Tetramethylcyclopentan-1,2-dion (Synthese siehe: T. Laitalainen, Finn. Chem. Lett. 1982, 10) (30.5 g, 188 mmol) und Ethylendiamin (15.9 mL, 235 mmol) in Ethanol (1.45 L) wird über Nacht unter Rückfluss gerührt. Nach Abkühlung auf 45°C wird Mangan(IV)oxid (36.0 g, 414 mmol) und Kaliumhydroxid (11.6 g, 207 mmol) zugegeben und 5 h unter Rückfluss gerührt. Nach Abkühlung auf 70°C wird die Mischung filtriert und das Filtrat bei Raumtemperatur mit 10%iger Salzsäure neutralisiert. Die Suspension wird filtriert und das Filtrat zur Trockne eingeengt. Der Rückstand wird mit Dichlormethan über Kieselgel säulenfiltriert. Ausbeute: 22.7 g (69%).

¹H-NMR (CD₂Cl₂, 500 MHz): δ = 1.33 (s, 12H), 1.98 (s, 2H), 8.30 (s, 2H).

### Pyrazinverbindung (b)

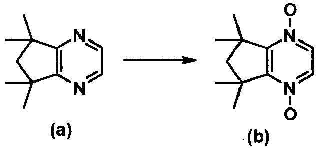

Eine Mischung von Pyrazinverbindung (a) (18.1 g, 98 mmol) und Wasser (750 mL) wird mit Kaliumperoxomonosulfat (Oxone, 144 g, 234 mmol) versetzt und bei 50°C über Nacht gerührt. Die wässrige Phase wird mit Dichlormethan extrahiert, zweimal mit Wasser gewaschen, über Natriumsulfat getrocknet und zur Trockne eingeengt. Ausbeute: 17.0 g (84%).

¹H-NMR (CD₂Cl₂, 500 MHz): δ = 1.51 (s, 12H), 1.97 (s, 2H), 7.78 (s, 2H).

### Pyrazinverbindung (c)

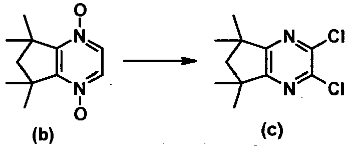

Eine Mischung von Pyrazinverbindung (b) (18.3 g, 83 mmol) in Phosphorylchlorid (325 mL) wird über Nacht unter Rückfluss gerührt. Nach Abkühlung auf Raumtemperatur wird die Lösung vorsichtig in eine Mischung aus Eiswasser (5 L) und Dichlormethan (1.5 L) getropft. Nach Neutralisation mit konzenrierter Natronlauge wird die organische Phase abgetrennt, dreimal mit Wasser gewaschen, über Natriumsulfat getrocknet und zur Trockne eingeengt. Man erhält eine Mischung aus Produkt (ca. 80%) und mono-chloriertem Nebenprodukt (ca. 20%), das ohne weitere Aufarbeitung eingesetzt wird. Reines Produkt kann durch Verrühren in Petrolether erhalten werden. Gesamtausbeute: 19.2 g (94%).

¹H-NMR (CD₂Cl₂, 500 MHz): δ = 1.29 (s, 12H), 1.99 (s, 2H).

### Pyrazinverbindung (d)

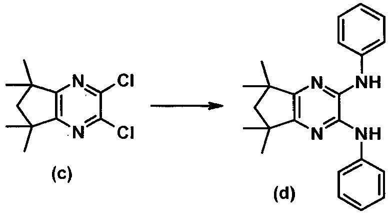

Eine Mischung von Pyrazinverbindung (c) (5.5 g, 22 mmol) und Anilin (24.6 mL, 270 mmol) in o-Xylol (65 mL) wird über Nacht bei 150°C gerührt. Nach Abkühlung auf Raumtemperatur wird der Niederschlag abgesaugt und mit Toluol gewaschen. Die vereinten Filtrate werden in Gegenwart von Kieselgel zur Trockne eingeengt. Der Rückstand wird mit einer Mischung aus Cyclohexan und Essigester über Kieselgel filtriert und das Filtrat zur Trockne eingeengt. Ausbeute: 6.6 g (81 %).

¹H-NMR (CD₂Cl₂, 500 MHz): δ = 1.28 (s, 12H), 1.95 (s, 2H), 6.25 (br s, 2H), 6.90-6.95 (m, 2H), 7.21-7.27 (m, 8H).

### Pyrazinverbindung (e)

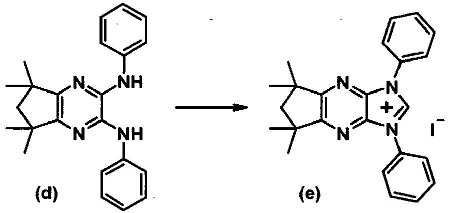

Eine Mischung von Pyrazinverbindung (d) (0.50 g, 0.7 mmol) in Triethylorthoformiat (3.5 mL) wird mit Ammoniumiodid (0.31 g, 2.1 mmol) versetzt und 2 h bei 85°C gerührt. Nach Abkühlung auf Raumtemperatur wird der Feststoff abgesaugt, mit Triethylorthoformiat, kaltem Ethanol und n-Heptan gewaschen und im Vakuumtrockenschrank bei 65°C getrocknet. Ausbeute: 0.16 g (46%).

¹H-NMR (d₆-DMSO, 500 MHz): δ = 1.41 (s, 12H), 2.19 (s, 2H), 7.74 (dd, 2H), 7.82 (dd, 4H), 8.01 (d, 4H), 10.98 (s, 1 H).

### Komplex fac-Em13:

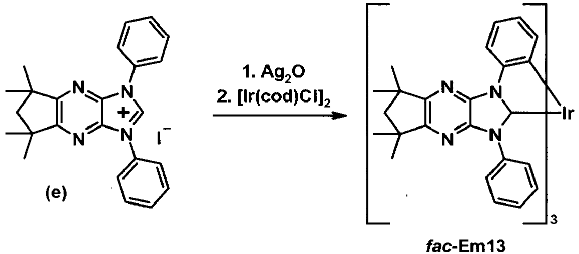

Eine Suspension von Pyrazinverbindung (e) (1.1 g, 2.1 mmol) in Dioxan (60 mL) wird mit Molsieb 4Å (11 g) und Silber(I)oxid (0.5 g, 2.1 mmol) versetzt und über Nacht bei Raumtemperatur gerührt. Anschließend wird eine Lösung von Chloro-(1,5-cyclooctadiene)-iridium(I)-dimer (142 mg, 0.2 mmol) in o-Xylol (75 mL) zugegeben. Die Mischung wird über Nacht bei Rückfluss gerührt. Nach Abkühlung auf 80 °C wird der Rückstand abgesaugt. Das Filtrat wird am Rotationsverdampfer bis zu einem Volumen von ca. 60 mL aufkonzentriert und über Nacht stehen gelassen. Der Niederschlag wird abfiltriert, mit Toluol und n-Hhexan gewaschen und im Vakuumtrockenschrank bei 85°C getrocknet. Ausbeute: 0.3 g (57%).

¹H-NMR (CD₂Cl₂, 500 MHz): δ = 1.15 (s, 9H), 1.35 (s, 9H), 1.46 (s, 9H), 1.59 (s, 9H), 2.11 (m_{c}, 6H), 6.67 (d, 3H), 6.73 (dd, 3H), 6.79 (dd, 3H), 7.14 (dd, 3H), 8.86 (d, 3H). 12 der 15 Protonen des nicht-cyclometallierten Phenyl-Ringes sind bei Raumtemperatur aufgrund der Rotation des Aromaten nur als sehr breite Erhebung im aromatischen Bereich erkennbar.

Photolumineszenz (2% im PMMA-Film):
λₘₐₓ = 474 nm, CIE: (0.14;0.24), QA = 91%

### Beispiel 15:

### Emitter Em14:

### 2,3-Bis(N-4'-fluorphenylamino)pyrazin

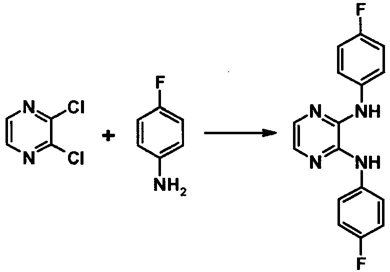

Eine Mischung von 2,3-Dichlorpyrazin (8.6 g, 58 mmol) und 4-Fluoanilin (66.3 g, 148 mmol) in o-Xylol (100 mL) wird über Nacht bei 130°C und 4h bei 140°C gerührt. Nach Abkühlung auf Raumtemperatur wird die Mischung zur Trockne eingeengt und der Rückstand wird in Dichlormethan (300 mL), Wasser (100 mL) und Ammoniak (25% in Wasser, 100 mL) aufgenommen. Die organische Phase wird abgetrennt, zweimal mit Wasser gewaschen, über Natriumsulfat getrocknet und zur Trockne eingeengt. Der Rückstand wird mit Petrolether verrührt, abgesaugt und mit Methyl-tert-butylether gewaschen. Ausbeute: 12.7 g (73%).

¹H-NMR (CD₂Cl₂, 500 MHz): δ = 6.30 (br s, 2H), 7.03 (dd, 4H), 7.31 (dd, 4H), 7.72 (s, 2H).

### 2-Ethoxy-1,3-bis-(4'-fluorphenyl)pyrazinoimidazolin

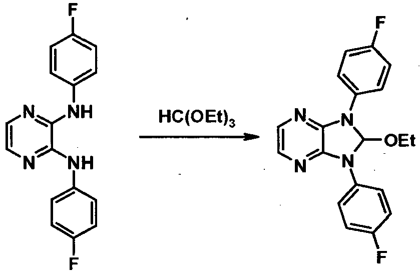

Eine Mischung von 2,3-Bis-(N-4'-fluorphenylamino)pyrazin (1.2 g, 4 mmol) in Triethylorthoformiat (36 mL) wird mit Natriumsulfat (4.7 g) und Molsieb 5Å (4.7 g) versetzt und 24 h bei 100°C gerührt. Nach Abkühlung auf Raumtemperatur wird die Mischung über Natriumsulfat und Watte filtriert und mit Methyl-tert-butylether gewaschen. Die vereinten Filtrate werden zur Trockne eingeengt. Der Rückstand wird mit n-Pentan verrührt und abgesaugt. Ausbeute: 0.6 g (42%).

¹H-NMR (CD₂Cl₂, 500 MHz): δ = 1.01 (t, 3H), 3.26 (q, 2H), 7.08-7.16 (m, 5H), 7.46 (s, 2H), 7.98 (dd, 4H).

### Komplex fac-Em14:

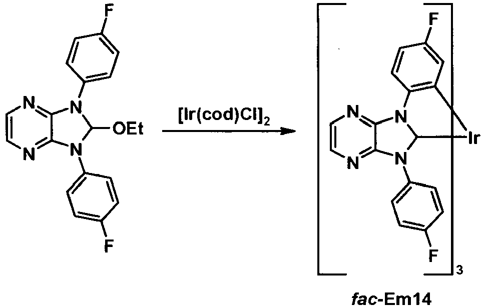

Eine Lösung von 2-Ethoxy-1,3-bis-(4'-fluorphenyl)pyrazinoimidazolin (560 mg, 1.60 mmol) in o-Xylol (60 mL) wird mit Natriumsulfat (4.7 g), Molsieb 5Å (4.7 g) und Chloro-(1,5-cyclooctadiene)-iridium(I)-dimer (107 mg, 0.16 mmol) versetzt. Die Mischung wird über Nacht bei 110°C gerührt. Nach erneuter Zugabe von Chloro-(1,5-cyclooctadiene)-iridium(I)-dimer (107 mg, 0.16 mmol) wird 5 h bei 110°C gerührt. Nach Abkühlung auf 80 °C wird der Rückstand abgesaugt und mit Dichlormethan gewaschen. Die vereinten Filtrate werden zur Trockene eingeengt und der Rückstand wird säulenchromatographisch (Kieselgel, Chloroform → Toluol) gereinigt. Ausbeute: 133 mg (19%),

¹H-NMR (CD₂Cl₂, 500 MHz): δ = 6.30 (dd, 3H), 6.84 (ddd, 3H), 8.09 (d, 3H), 8.36 (d, 3H), 8.71 (dd, 3H). Die 12 Protonen des nicht-cyclometallierten 4-Fluorphenyl-Ringes sind bei Raumtemperatur aufgrund der Rotation des Aromaten nur als sehr breite Erhebung im aromatischen Bereich erkennbar.

Photolumineszenz (2% im PMMA-Film):
λₘₐₓ = 458 nm, CIE: (0.17;0.17), QA = 58%

### Beispiel 16:

### Emitter Em15

### 2-Ethoxy-1,3-diphenyl-2,3-dihydro-1-H-imidazol[4,5-b]pyrazin

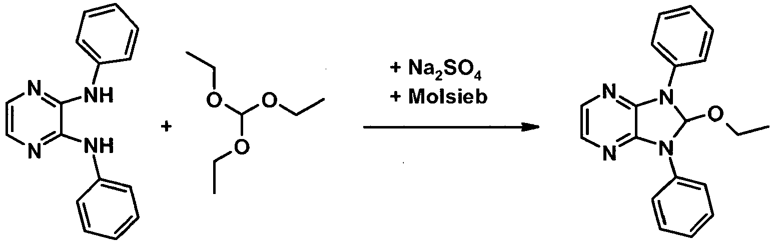

15.30 g (58.3 mmol) 2,3-Bis(N-phenylamino)pyrazin, 68.00 g Natriumsulfat und 68.00 g Molekularsieb (4A) werden in 270 ml Triethylorthoformiat 45h auf 90°C erhitzt. Nach dem Abkühlen wird abfiltriert und mit Methyl-tert-butyl-ether nachgewaschen. Das Filtrat wird im Vakuum vom Lösungsmittel befreit. Das erhaltene rotbraune Öl wird mehrfach mit etwas n-Heptan verrührt und erneut im Vakuum getrocknet. Der Rückstand wird säulen-chromatographisch aufgereinigt (Kieselgel, Eluent: Cyclohexan/Aceton = 4/1). Man erhält 11.39 g (61%) 2-Ethoxy-1,3-diphenyl-2,3-dihydro-1-H-imidazol[4,5-b]pyrazin.

¹H-NMR (DMSO-D₆, 500 MHz): δ = 0.88 (t, 3H), 3.17 (q, 2H), 7.16-7.20 (m, 2H), 7.45-7.48 (m, 4H), 7.52 (s, 2H), 7.76 (s, 1 H), 8.04-8.06 (m, 4H).

### Komplex Em15

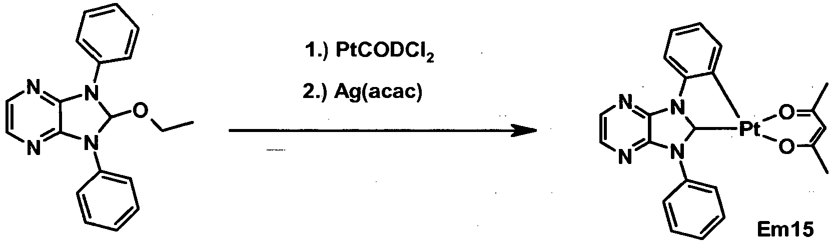

1.85 g (4.90 mmol) Dichloro(1,5-cyclooctadien)platin(II) werden bei Raumtemperatur zu einer Lösung von 1.85 g (5.82 mmol) 2-Ethoxy-1,3-diphenyl-2,3-dihydro-1-H-imidazol[4,5-b]pyrazin in 60 ml Butanon gegeben. Die Suspension wird 4 Stunden unter Rückfluss erhitzt. Dann werden bei Raumtemperatur 1.64 g (7.84 mmol) Silber(I)acetylacetonat zugegeben. Im Anschluss wird das Gemisch über Nacht unter Rückfluss erhitzt. Nach dem Abkühlen wird die Suspension filtriert. Das Filtrat wird im Vakuum vom Lösungsmittel befreit und säulenchromatographisch (Kieselgel, Eluent: Methylenchlorid) aufgereinigt. Man erhält 0.24 g Em15 (9%).

¹H-NMR (CD₂Cl₂, 500 MHz): δ =1.36 (s, 3H), 2.02 (s, 3H), 5.36 (s, 1H), 7.07 (dt, 1H), 7.17 (dt, 1 H), 7.57-7.65 (m, 5H), 7.81 (dd, 1 H), 8.20 (dd, 1 H), 8.30 (d, 1 H), 8.43 (d, 1 H).

Photolumineszenz (2% im PMMA-Film):
λₘₐₓ = 484 nm, CIE: (0.22;0.34)

### Beispiel 17:

### Emitter Em16

### Komplex K4

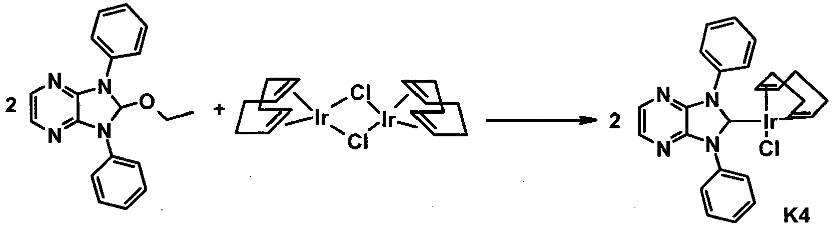

Eine Lösung von 0.63 g (0.94 mmol) Bis(1,5-cyclooctadien)diiridium-l-chlorid in 50 ml wasserfreiem Toluol wird bei Raumtemperatur zu einer Lösung von 0.60 g (1.88 mmol) 2-Ethoxy-1,3-diphenyl-2,3-dihydro-1-H-imidazol[4,5-b]pyrazin in 30 ml wasserfreiem Toluol getropft. Es wird 30 min bei 60°C und 24h bei 90°C gerührt. Nach dem Abkühlen wird das Lösungsmittel im Vakuum entfernt und der Rückstand säulenchromatographisch (Kieselgel, Eluent: Methylenchlorid/Methanol = 100/1) aufgereinigt. Man erhält 0.85 g (75%) **K4.**

¹H-NMR (CD₂Cl₂, 500 MHz): δ = 1.32-1.38 (m, 2H), 1.44-1.52 (m, 2H), 1.53-1.59 (m, 2H), 1.73-1.81 (m, 2H), 2.55-2.59 (m, 2H), 4.60-4.62 (m, 2H), 7.58-7.65 (m, 6H), 8.13-8.15 (m, 4H), 8.29 (s, 2H).

### Komplex Em16:

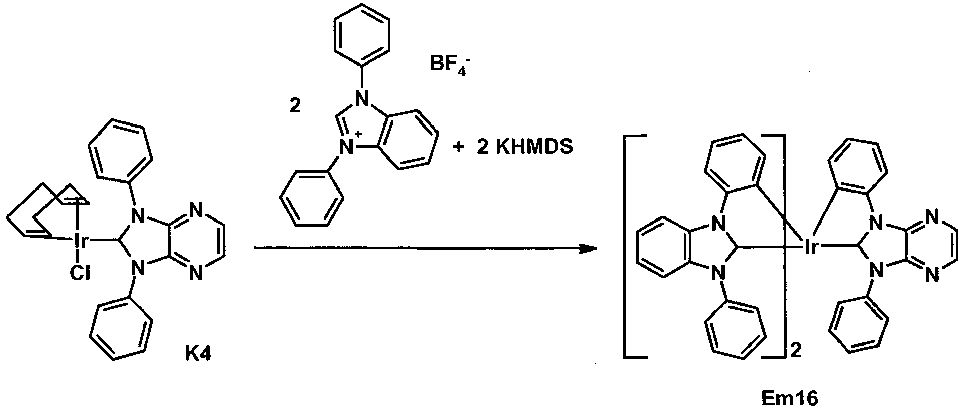

0.75 g (2.09 mmol) N,N'-Diphenylbenzimidazolium-tetrafluoroborat (Synthese analog WO2005/019373) in 30 ml wasserfreiem Toluol werden bei 0°C mit 4.20 ml (2.10 mmol) einer 0.5 molaren toluolischen Kaliumhexamethyldisilylamid(KHMDS)-Lösung versetzt. Man lässt auf Raumtemperatur auftauen und rührt eine Stunde. Dann wird eine Lösung von 0.61 g (0.99 mmol) **K4** in 150 ml wasserfreiem Toluol zugetropft und weitere 30 min gerührt. Anschließend wird eine Stunde am Rückfluss erhitzt. Nach dem Abkühlen wird das Reaktionsgemisch filtriert. Das Filtrat wird im Vakuum vom Lösungsmittel befreit und säulenchromatographisch aufgereinigt (Kieselgel, Eluent: Cyclohexan/Aceton = 4/1). Man erhält 0.30 g Em16 (30% Ausbeute).

¹H-NMR (CD₂Cl₂, 500 MHz): δ = 6.10-7.20 (sehr flaches, breites Signal, 4H), 6.19 (br. d, 1 H), 6.22-6.25 (m, 2H), 6.37-6.50 (m, 5H), 6.61 (br. d, 2H), 6.67 (br. d, 1H), 6.72-6.81 (m, 6H), 7.02-7.17 (m, 5H), 7.27-7.34 (m, 4H), 7.95 (d, 1 H), 8.00-8.02 (m, 2H), 8.14 (d, 1 H), 8.18 (d, 1 H), 8.26 (d, 1 H), 8.71 (d, 1 H).

Photolumineszenz (2% im PMMA-Film):
λₘₐₓ = 489 nm, CIE: (0.19;0.38), 98% QA

### Beispiel 18:

### Herstellung von 2,8-Di(dibenzofuran-2-yl)dibenzofuran, Ma14

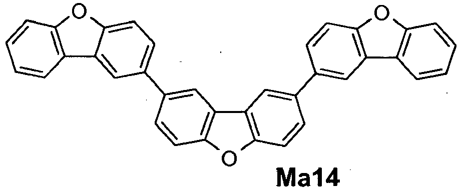

### Stufe 1:

10.4 g (42.09 mMol) 2-Bromdibenzofuran (hergestellt nach J. Med. Chem 52(7),1799-1802, 2009) werden in einem ausgeheizten 11-Dreihalsrundkolben, versehen mit Magnetrührer, Thermometer, Septum und Stickstoffüberleitung, eingewogen. Es werden 300 ml THF (absolutiert über Natrium) zugegeben und die klare, farblose Lösung unter Rühren und Argonatmosphäre auf -78°C abgekühlt. Innerhalb von 30 Minuten werden 17.1 ml (46.3 mMol) einer 2.7M Lösung von Butyllithium in Hexan zugetropft, wobei die Innentemperatur bei <73°C gehalten wird. Anschließend wird 30 Minuten bei dieser Temperatur nachgerührt. Danach werden innerhalb von 40 Minuten 8.61 g (46.3 mMol) 2-Isopropoxy-4,4,5,5-tetramethyl-1,3,2-dioxaborolane zu der gelben Suspension getropft, wobei die Innentemperatur bei <73°C gehalten wird. Danach wird 60 Minuten bei dieser Temperatur nachgerührt. Das Reaktionsgemisch wird auf RT erwärmt und die klare, gelbe Lösung auf 200 ml Puffer pH = 7 gegossen und mit 23ml 2N HCl versetzt. Das Lösungsmittel wird am Rotavap eingedampft und die wässrige Phase drei Mal mit 250 ml EtOAc extrahiert. Die vereinigten organischen Phasen werden ein Mal mit 100ml gesättigter NaCl-Lösung gewaschen, über Magnesiumsulfat getrocknet, filtriert und eingedampft. Man erhält 12.95 g eines weißen Feststoffes, welcher mit 50 ml MeOH versetzt und auf Rückfluss erhitzt wird. Die sich bildende klare Lösung wird unter Rühren auf Raumtemperatur und anschließend auf 0°C abgekühlt. Die sich bildende Suspension wird filtriert und der Rückstand zwei Mal mit 10 ml eiskaltem MeOH gewaschen und über Nacht im Vakuumtrockenschrank bei 50°C/120T getrocknet. Man erhält 8.97 g (71.6% d. Th.) 2-Dibenzofuran-2-yl-4,4,5,5-tetramethyl-1,3,2-dioxaborolane in einer Reinheit von 98.3%. Die NMR- und MS-Daten stimmen mit der vorgeschlagenen Struktur überein.

### Stufe 2:

4.09 g (9.73 mMol) 2,8-Diiododibenzofuran (hergestellt nach J. Amer. Chem. Soc. 124(40), 11900-11907, 2002), 6.30 g (21.04 mMol) 2-Dibenzofuran-2-yl-4,4,5,5-tetramethyl-1,3,2-dioxaborolane, 6.86 g (49.67 mMol) Kaliumcarbonat, 180 ml Toluol, 79 ml EtOH und 37 ml H₂O₂werden in einem 500 ml- Dreihalsrundkolben, versehen mit Magnetrührer, Thermometer, Rückflusskühler und Stickstoffüberleitung, vorgelegt, vier Mal evakuiert und mit Argon belüftet. Anschliessend werden 1.58 g (1.36 mMol) Tetrakis-triphenylphosphin-palladium zugegeben, vier Mal evakuiert und mit Argon belüftet. Das Reaktionsgemisch wird anschließend unter starkem Rühren vier Stunden auf Rückfluss erhitzt, danach mit 200 ml Toluol verdünnt und auf Raumtemperatur abgekühlt. Die Phasen werden getrennt und die organische Phase zwei Mal mit 150 ml Wasser gewaschen, über Magnesiumsulfat getrocknet, filtriert und eingedampft. Man erhält 5.72 g eines braunen Feststoffes. Eine Reinigung mittels Flashchromatographie mit Hexan/Toluol = 5:2 als Laufmittel ergibt 1.20 g 2,8-Di(dibenzofuran-2-yl)dibenzofuran als weißen Feststoff mit einer Reinheit von 99.3%. Die NMR- und MS-Daten stimmen mit der vorgeschlagenen Struktur überein.

### Beispiel 19

### Herstellung von Ma7

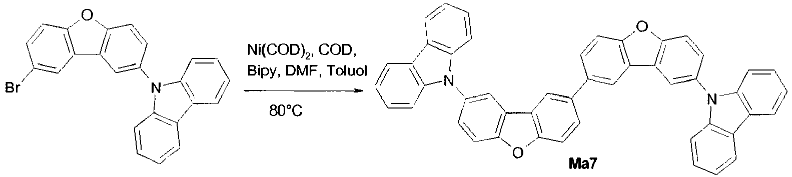

Ni(COD)₂ (9.03 g, 32.8 mmol), 1,5-Cyclooctadien (3.55 g, 32.8 mmol), und 2,2'-Bipyridin (5.12 g, 32.8 mmol) werden in einem Schlenkkolben (Glovebox) in trockenem DMF (140 mL) gelöst. Die Mischung wird 30 Min. bei 80 °C gerührt. Eine Lösung von 9-(8-brom-dibenzofuran-2yl)-9H-carbazol (11.75 g, 11.8 mmol) in trockenem Toluol (380 mL) wird langsam zugegeben. Nach 24 Std. rühren bei 80 °C unter Argon, wird die Mischung auf Raumtemperatur abgekühlt und auf MeOH/HCl (1:1, 2000 mL) gegeben und für 1 Std. gerührt. Die organische Phase wird mit Toluol extrahiert, getrocknet mit Na₂SO₄ und eingeengt. FC (SiO₂; Cyclohexan/CH₂Cl₂; 4/1) gibt **Ma7** (9.14 g, 87%).

¹H-NMR (400 MHz, CD₂Cl₂): 8.25 (2H, d, *J* = 2Hz), 8.19 (2H, d, *J* = 2Hz), 8.15 (4H, d, *J* = 8Hz), 7.88 (1 H, d, *J* = 2Hz), 7.83 (1 H, d, *J* = 2Hz), 7.77 (4H, AB, *J* = 8Hz), 7.65 (1 H, d, *J* = 2Hz), 7.62 (1 H, d, *J* = 2Hz), 7.43-7.38 (8H, m), 7.31-7.24 (4H, m).

### Beispiel 20

### Herstellung von Ma12

### Stufe 1:

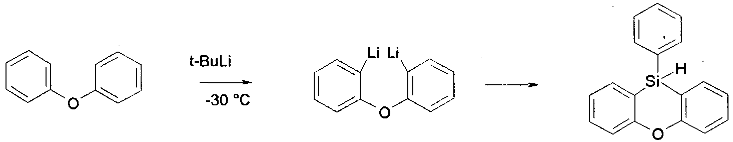

*t*-BuLi (1.7M in Pentan) (46.8 mL, 79.4 mmol) wird zu einer Lösung von Diphenylether (6 mL, 37.8 mmol) in trockenem THF (82 mL) bei -30 °C unter Argon zugetropft. Die Mischung wird 5.5 Std. bei -30 °C gerührt und dann wird Phenylsilan (4.64 g, 41.6 mmol) zugegeben. Die Mischung wird 1 Std. weiter bei -30 °C gerührt und dann übe Nacht auf Raumtemperatur aufgewärmt. Angesäurtes Eis (H₂SO₄) wird zugegeben und die organische Phase wird mit CH₂Cl₂ extrahiert, über Na₂SO₄ getrocknet, filtriert und eingeengt. Umkristallisation aus MeOH gibt 10-Phenylphenoxasilin (49 %).

¹H-NMR (CD₂Cl₂, 400 MHz): 7.57 (2H, dd, *J* = 8.0 Hz, *J* = 1.6 Hz), 7.48-7.35 (7H, m), 7.23 (2H, d, *J* = 8.4 Hz), 7.11 (2H, AB, *J* = 7.2 Hz, *J* = 0.8 Hz), 5.50 (1 H, s).

### Stufe 2:

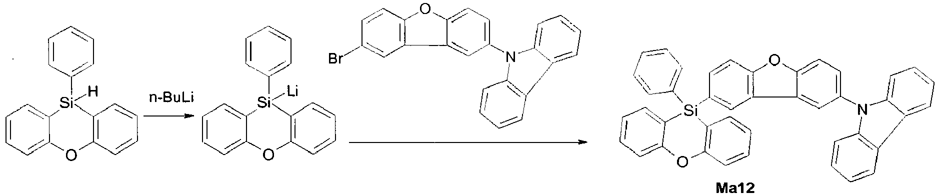

n-BuLi (1.6M in Hexan) (2.44 mL, 3.9 mmol) wird langsam zu einer Lösung von 9-(8-brom-dibenzofuran-2yl)-9H-carbazol (1.24 g, 3 mmol) in Diethylether (60 mL) bei 0 °C unter Ar gegeben. Nach 20 Min. rühren bei 0 °C wird eine Lösung von 10-Phenylphenoxasilin (0.99 g, 3.6 mmol) in Diethylether (10mL) bei 0 °C zugegeben. Die Mischung wird über Nacht unter Rühren auf Raumtemperatur aufgewärmt. Gesättigte NH₄Cl-Lösung wird zugegeben, die organische Phase mit Diethylether extrahiert, getrocknet über Na₂SO₄, filtriert und eingeengt. Umkristallisation aus Cyclohexane/CH₂Cl₂ gibt 9-(8-(10-phenyl-10H-dibenzo[*b*,*e*][1,4]oxasilin-10-yl)dibenzo[*b*,*d*]furan-2-yl)-9H-carbazol (0.92 g) in 51% Ausbeute.

¹H-NMR (CD₂Cl₂, 400 MHz): 8.16 (1H, s), 8.14 (2H, d, *J* = 8.0 Hz), 8.03 (1H, d, *J* = 2.0 Hz), 7.78 (1 H, d, *J* = 8.4 Hz), 7.72 (1 H, d, *J* = 8.0 Hz), 7.65 (1 H, d, *J* = 8.4 Hz), 7.62-7.58 (5H, m), 7.46 (2H, t, *J* = 7.4 Hz, *J* = 2.0 Hz), 7.40-7.33 (7H, m), 7.33-7.24 (4H, m), 7.13 (2H, t, *J* = 7.2Hz). ¹³C-NMR (CD₂Cl₂, 125 MHz): 110.0, 112.3, 113.3, 116.4, 118.6, 120.3, 120.5, 120.6, 123.4, 123.6, 124.4, 125.7, 126.4, 127.3, 128.6, 128.8, 129.3, 130.5, 132.2, 133.3, 134.3, 135.7, 136.2, 141.2, 155.6, 158.8, 160.8.

### Beispiel 21

### Synthese von Rot1

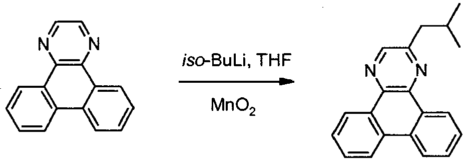

a) 3-Isobutylphenanthro[9,10-b]pyrazin wird gemäss **Beispiel 9c** in Patentanmeldung WO2009/100991 hergestellt, ausgehend von 32.2 g (0.14 mol) Dibenzo[f,h]quinoxalin, mit 90 ml (0.15 mol) einer 1.7M Lösung von Isobutyllithium in Heptan, und 50 g Mangan(IV)oxid. Das Rohprodukt wird heiss über Kieselgel filtriert und das Filtrat unter Vakuum eingeengt. Der resultierende Feststoff wird weiter in Ethanol während 18 h über Nacht verrührt. Filtration und Nachwaschen mit Ethanol liefert das Produkt als leicht beigen Feststoff (Ausbeute: 8.7 g, 31%). 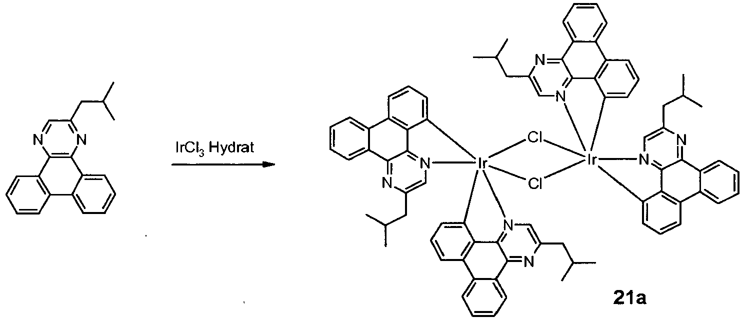
b) 8.0 g (28 mmol) 3-Isobutylphenanthro[9,10-b]pyrazin und 4.82 g (13.2 mmol) Iridium(III)chlorid Hydrat (53.01% Iridium-Gehalt) werden bei Raumtemperatur in 100 ml 2-Ethoxethanol vorgelegt. Die grau-schwarze Suspension wird während 24 h bei 123°C gerührt. Die resultierende rote Suspension wird filtriert, der Feststoff nachgewaschen mit Ethanol und danach weiter getrocknet unter Vakuum. Das Produkt 21a wird als rotes Pulver erhalten (Ausbeute: 10.1 g, 95%). 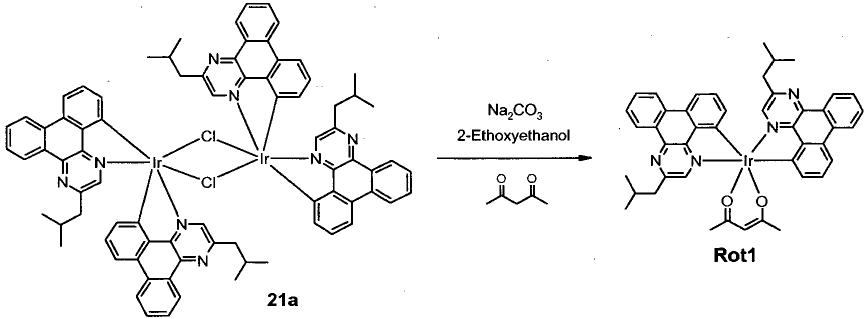
c) 4.95 g (3.1 mmol) Produkt **21a** und 3.3 g (3.1 mmol) Natriumcarbonat werden in 40 ml 2-Ethoxyethanol und 20 ml *N*,*N-*Dimethylformamid vorgelegt. Die rote Suspension wird mit 2.5 g (24.8 mmol) Acetylaceton versetzt und danach während 70 min bei 121°C gerührt. Die resultierende dunkelrote Suspension wird filtriert und der Feststoff danach einmal mit Ethanol, dann zweimal mit Wasser verrührt, und mit Hexan nachgewaschen. Das Produkt wird als rotes Pulver erhalten (Ausbeute: 4.3 g, 81%).

¹H-NMR (CDCl₃, 400 MHz): δ = 1.15 (t, 12H), 1.86 (s, 6H), 2.40-2.50 (m, 2H), 3.10-3.15 (m, 4H), 5.35 (s, 1H), 6.45 (d, 2H), 7.04 (6, 2H), 7.73-7.82 (m, 4H), 7.93 (d, 2H), 8.56 (d, 2H), 8.67 (s, 2H), 9.33 (d, 2H).

### Beispiel 22:

### Emitter Em17

### (2-Chloro-pyridin-3-yl)-isopropylamin:

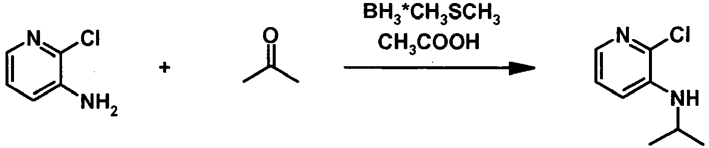

12.5 ml (9.7g, 167.0 mmol) Aceton und 75 ml Eisessig werden zu einer Lösung von 8.0 g (62.2 mmol) 3-Amino-2-chlorpyridin in 150 ml wasserfreien Dichlormethan gegeben. Bei 0°C werden 6.5 ml (5.2 g, 68.4 mmol) Borandimethylsulfidkomplex zugegeben. Nach beendeter Gasentwicklung wird auf Raumtemperatur aufgetaut und über Nacht weiter gerührt. Dann wird der pH-Wert durch Zugabe von 25%iger Ammoniaklösung auf 8 eingestellt. Es werden 50 ml Wasser zugegeben. Die wäßrige Phase wird dreimal mit je 50 ml Dichlormethan extrahiert. Die vereinigten organischen Phasen werden über Natriumsulfat getrocknet und vom Lösungsmittel befreit. Man erhält 10.5 g (99%) gelbes Öl.

¹H-NMR (DMSO-D₆, 500 MHz): δ = 1.18 (d, 6H), 3.64 (sept, 1H), 4.96 (d, 1H), 7.07 (d, 1 H), 7.17 (dd, 1H), 7.57 (dd, 1 H).

### 2-N-Phenylamino-3-N-isopropylaminopyridin:

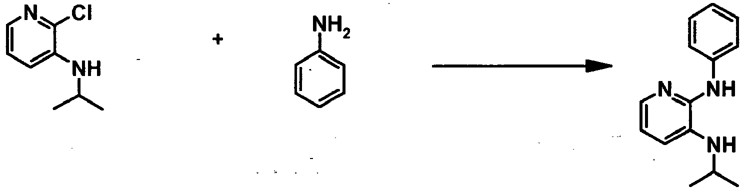

10.5 g (61.5 mmol) (2-Chloro-pyridin-3-yl)-isopropylamin werden mit 5.84 g (62.5 mmol) Anilin versetzt und 16 h bei 184°C gerührt. Nach Abkühlen auf Raumtemperatur werden 50 ml Wasser zugefügt. Das Gemisch wird 1 h gerührt und wäßriger NaOH-Lösung auf pH = 11 eingestellt. Es wird dreimal mit je 50 ml Dichlormethan extrahiert. Die vereinigten organischen Phasen werden über Natriumsulfat getrocknet und im Vakuum vom Lösungsmittel befreit. Der Rückstand wird säulenchromatographisch aufgereinigt (Kieselgel, Eluent: Cyclohexan/Ethylacetat = 5/1). Man erhält 7.5 g (53%) hellbraunen Feststoff.

¹H-NMR (CD₂Cl₂, 500 MHz): δ = 1.21 (d, 6H), 3.23 (s br, 1 H), 3.57 (sept, 1 H), 6.26 (s br, 1 H), 6.83 (dd, 1 H), 6.92-6.99 (m, 2H), 7.25-7.29 (m, 4H), 7.70 (dd, 1 H).

### 1-Phenyl-3-isopropyl-pyridino-imidazoliumiodid:

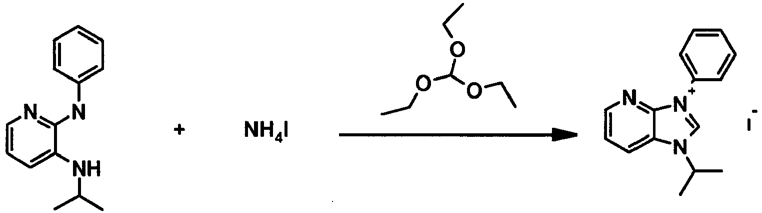

7.3 g (32.1 mmol) 2-N-Phenylamino-3-N-isopropylaminopyridin werden in 30 ml Triethylorthoformiat gelöst und mit 4.8 g (33.1 mmol) Ammoniumiodid versetzt. Das Reaktionsgemisch wird über Nacht bei 82°C gerührt. Nach dem Abkühlen wird der entstandene hellgelbe Feststoff abfiltriert, mit 3x15 ml Petrolether und 3x30 ml Dichlormethan gewaschen und getrocknet. Man erhält 10.2 g (86%) Imidazoliumsalz.

¹H-NMR (DMSO-D₆, 500 MHz): δ = 1.72 (d, 6H), 5.19 (sept, 1H), 7.67-7.70 (m, 1H), 7.73-7.76 (m, 2H), 7.86-7.88 (m, 1H), 7.95-7.97 (m, 2H), 8.81-8.82 (m, 2H), 10.43 (s, 1H).

### Komplex K5

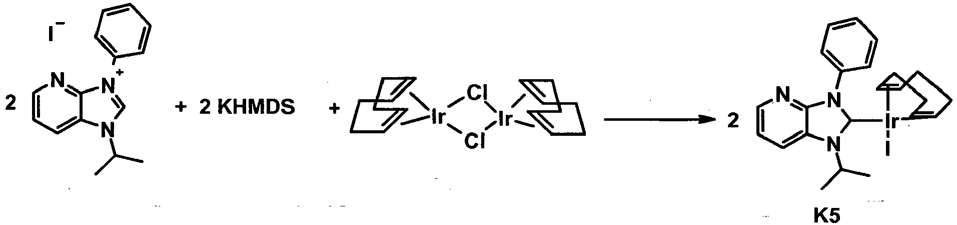

3.00 g (8.21 mmol) 1-Phenyl-3-isopropyl-pyridino-imidazoliumiodid werden in 45 ml wasserfreiem Toluol suspendiert. Bei -8°C werden 16.42 ml einer 0.5-molaren KHMDS-Lösung in Toluol (8.21 mmol) zugegeben. Es wird auf Raumtemperatur aufgetaut und eine Stunde gerührt. Dann wird die rote Suspension bei -78°C zu einer Lösung von 2.76 g (4.11 mmol) Bis(1,5-cyclooctadien)diiridium-l-chlorid in 75 ml wasserfreiem Toluol gegeben. Im Anschluß wird 1.5 Stunden bei Raumtemperatur und eine Stunde am Rückfluss erhitzt. Nach dem Abkühlen wird das Reaktionsgemisch filtriert. Das Filtrat wird im Vakuum vom Lösungsmittel befreit und säulenchromatographisch aufgereinigt (Kieselgel, Eluent: Methylenchlorid). Man erhält 3.70 g (68%) Komplex **K5** als gelbes Pulver.

¹H-NMR (CD₂Cl₂, 500 MHz): δ = 1.02-1.08 (m, 1H), 1.16-1.21 (m, 1H), 1.37-1.42 (m, 1H), 1.59-1.72 (m, 3H), 1.76 (dd, 6H), 2.07-2.18 (m, 2H), 2.51-2.55 (m, 1H),3.15-3.18 (m, 1 H), 4.65-4.69 (m, 1 H), 4.87-4.91 (m, 1 H), 6.06 (sept, 1 H), 7.23 (dd, 1 H), 7.49-7.56 (m, 3H), 7.85 (dd, 1 H), 8.04-8.06 (m, 2H), 8.22 (dd, 1 H).

### Komplex Em17:

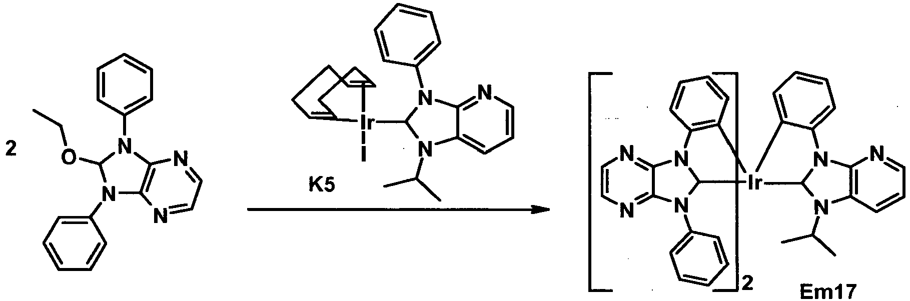

Eine Lösung von 0.90 g (2.83 mmol) 2-Ethoxy-1,3-diphenyl-2,3-dihydro-1-H-imidazol[4,5-b]pyrazin in 50 ml wasserfreiem o-Xylol werden nacheinander bei Raumtemperatur mit 10 g Molsieb und einer Lösung von 0.86 g (1.29 mmol) **K5** in 75 ml wasserfreiem o-Xylol versetzt. Es wird 22 Stunden bei 115°C gerührt. Nach dem Abkühlen wird filtriert. Das Filtrat wird im Vakuum vom Lösungsmittel befreit und säulenchromatographisch (Kieselgel, Eluent: Cyclohexan/Aceton = 4/1) gereinigt. Man erhält 0.53 g (42%) **Em17** als gelbes Pulver.

¹H-NMR (CD₂Cl₂, 500 MHz): δ = 0.62 (d, 3H), 1.06 (d, 3H), 4.28 (sept, 1 H), 6.35-7.53 (sehr flaches, breites Signal, 8H, ortho- und meta-H der beiden nicht cyclometallierten Phenylringe), 6.52 (dd, 1 H), 6.59 (dd, 1H), 6.64 (dd, 1H), 6.72-6.77 (m, 3H), 6.81 (dt, 1H), 6.97-7.01 (m, 1H), 7.05-7.08 (m, 2H), 7.14 (dt, 1H), 7.19 (dt, 1H), 7.41 (dd, 1H), 8.07 (d, 1 H), 8.12 (d, 1 H), 8.34 (dd, 2H), 8.37 (dd, 1 H), 8.69 (dd, 1 H), 8.80 (dd, 1 H), 8.95 (dd, 1 H).

Photolumineszenz (2% im PMMA-Film):
λₘₐₓ = 484 nm, CIE: (0.17;0.34), 95% QA

### Beispiel 23:

### Komplex Em18:

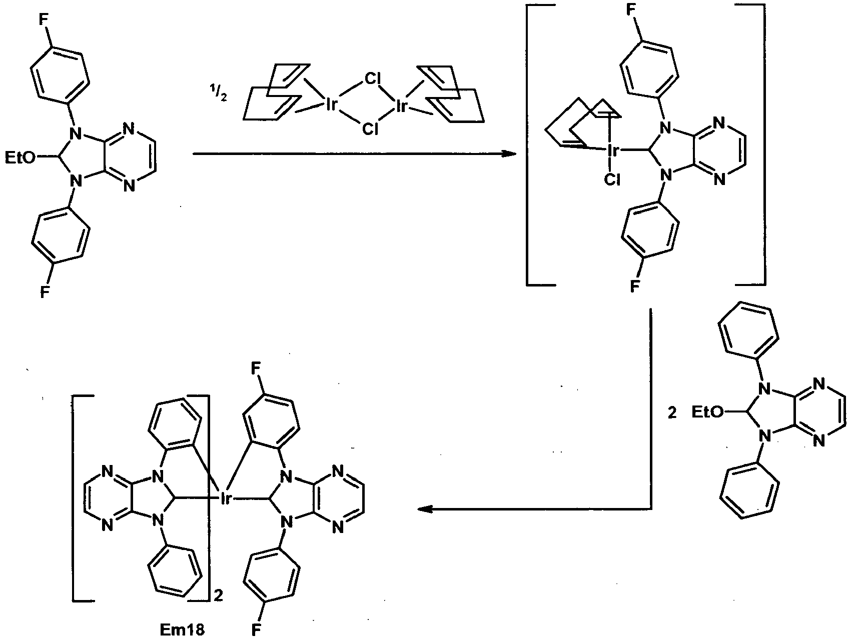

1.06 g (2.98 mmol) 2-Ethoxy-1,3-bis(4-fluorphenyl)-2,3-dihydro-1-H-imidazol[4,5-b]pyrazin (2-Ethoxy-1,3-bis-(4'-fluorphenyl)pyrazinoimidazolin) wurde zu einer Lösung von 1.00 g (1.49 mmol) [(µ-Cl)Ir(η⁴-1,5-COD)]₂ in 100 ml o-Xylol gegeben. Die resultierende Lösung wird anschließend 20 h bei 65 °C gerührt. Nach Zugabe von 1.80 g (5.66 mmol) 2-Ethoxy-1,3-diphenyl-2,3-dihydro-1-H-imidazol[4,5-b]pyrazin wird die Reaktionsmischung weitere 48 h bei 95 °C gerührt. Nach dem Abkühlen wird der Niederschlag abfiltriert und mit o-Xylol und Cyclohexan gewaschen. Die vereinigten organischen Phasen werden zur Trockene eingeengt und säulenchromatographisch (Kieselgel, Laufmittel Ethylacetat/Cyclohexan = 1/4) gereinigt. Man erhält 0.30 g (20 %) **Em18** als gelbes Pulver.

¹H-NMR (CD₂Cl₂, 500 MHz):
δ = 6.20-6.90 (sehr flaches breites Signal, 8H), 6.28 (dd, 1 H), 6.65 (ddd, 3H), 6.59 (d, 1H), 6.80-6.86 (m, 6H), 7.14-7.19 (m, 2H), 8.05 (d, 1H), 8.07 (dd, 2H), 8.32 (d, 1H), 8.35 (t, 2H), 8.72 (dd, 1 H), 8.76 (dd, 2H).

Photolumineszenz (2% im PMMA-Film):
λₘₐₓ = 466 nm, CIE: (0.15;0.19); QA = 95%

### Beispiel 24:

### Komplex Em19:

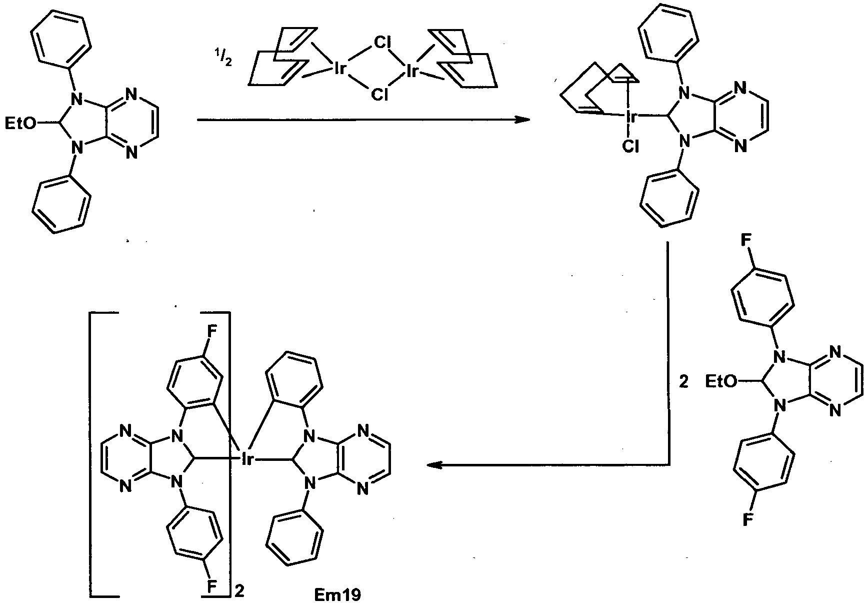

0.47 g (1.49 mmol) 2-Ethoxy-1,3-diphenyl-2,3-dihydro-1-H-imidazol[4,5-b]pyrazin wurde zu einer Lösung von 0.50 g (0.74 mmol) [(µ-Cl)Ir(η⁴-1,5-COD)]₂ in 50 ml o-Xylol gegeben. Die resultierende Lösung wird anschließend 22 h bei 60 °C gerührt. Nach Zugabe von 1.04 g (2.94 mmol) 2-Ethoxy-1,3-bis(4-fluorphenyl)-2,3-dihydro-1-H-imidazol[4,5-b]pyrazin wird die Reaktionsmischung weitere 48 h bei 95 °C gerührt. Nach dem Abkühlen wird die Reaktionsmischung zur Trockene eingeengt. Der feste Rückstand wird in Dichlormethyn/Cyclohexan gelöst und durch Celite filtriert. Das Filtrat wird wieder zur Trockene eingeengt und der Rückstand säulenchromatographisch (Kieselgel, Laufmittel Ethylacetat/Cyclohexan = 1/4) gereinigt. Man erhält 0.17 g (11 %) **Em19** als gelbes Pulver.

¹H-NMR (CD₂Cl₂, 500 MHz):
δ = 6.20-6.90 (sehr flaches breites Signal, 8H), 6.31 (ddd, 3H), 6.68 (dd, 2H), 6.83-6.88 (m, 4H), 7.21 (dt, 1 H), 8.10 (dd, 2H), 8.12 (d, 1 H), 8.37 (t, 2H), 8.40 (d, 1 H), 8.71-8.75 (m, 2H), 8.79 (dd, 1 H).

Photolumineszenz (2% im PMMA-Film):
λₘₐₓ = 461 nm, CIE: (0.15;0.16); QA = 90%

### Beispiel 25:

### Emitter Em20

### Pyrazinverbindung (f)

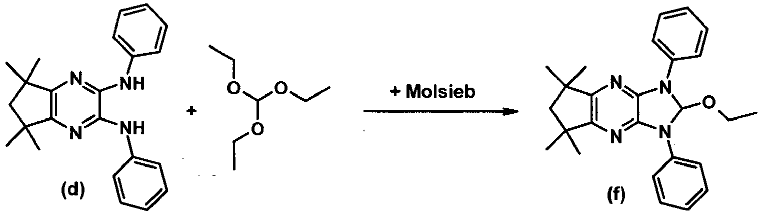

10.5 g (29.3 mmol) Pyrazinverbindung (d) und 15.0 g Molekularsieb (5A) werden in 230 ml Triethylorthoformiat 48h auf 120°C erhitzt. Nach dem Abkühlen wird abfiltriert und das Filtrat im Vakuum vom Lösungsmittel befreit. Das erhaltene braune Öl wird säulen-chromatographisch aufgereinigt (Kieselgel, Eluent: Cyclohexan/Ethylacetat = 9/1). Die Produktfraktionen werden vereinigt und aus Methyl-tertbutylether umkristallisiert. Man erhält 4.63 g (38%) Pyrazinverbindung (f).

¹H-NMR (CD₂Cl₂, 500 MHz): δ = 1.06 (t, 3H), 1.33 (s, 6H), 1.34 (s, 6H), 1.98 (s, 2H), 3.32 (q, 2H), 7.12-7.16 (m, 2H), 7.21 (s, 1H), 7.40-7.47 (m, 4H), 8.11-8.16 (m, 4H).

### Komplex Em20:

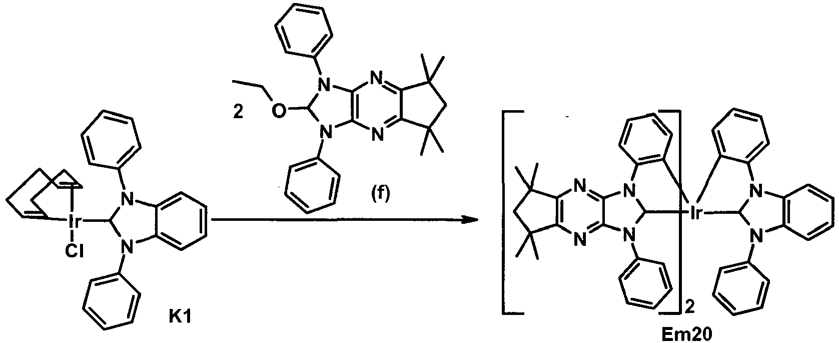

Eine Lösung von 1.9 g (1.8 mmol) Pyrazinverbindung (f) in 200 ml o-Xylol (wasserfrei) wird mit 20 g Molekularsieb vorgelegt und mit einer Lösung von 1.26 g (0.8 mmol) Komplex K1 in 150 ml o-Xylol (wasserfrei) versetzt. Das Reaktionsgemisch wird anschließend 18 h bei 115°C gerührt. Nach dem Abkühlen wird das Reaktionsgemisch filtriert. Das Filtrat wird zur Trockene eingeengt, säulenchromatographisch aufgereinigt (Kieselgel, Laufmittel Cyclohexan/Aceton = 4/1) und dann aus Methylenchlorid/Methanol umkristallisiert. Danach wird erneut säulenchromatographisch gereinigt (Kieselgel, Laufmittel Cyclohexan/Aceton = 10/1). Man erhält 0.75 g (30 %) Em20 als hellgelbes Pulver.

¹H-NMR (CD₂Cl₂, 500 MHz): δ = 1.13 (d, 6H), 1.36 (d, 6H), 1.45 (d, 6H), 1.58 (d, 6H), 2.03-2.16 (m, 4H), 6.18-6.43 (m, 5H), 6.44-6.97 (m, breit, 15H), 6.98-7.22 (m, 6H), 7.32 (t, 1H), 7.99 (d, 1H), 8.17 (d, 1H), 8.78 (d, 1H), 8.90 (d, 1H).

Photolumineszenz (2% im PMMA-Film):
λₘₐₓ = 479 nm, CIE: (0.14; 0.29), 95% QA

### Beispiel 26:

### Emitter Em21

### 2,3-Di-(N-phenylamino)pyridin:

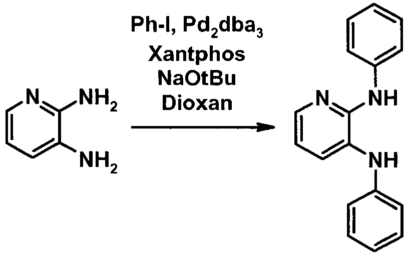

Eine Suspension aus 2,3-Diaminopyridin (8.9 g, 9 mmol) und lodbenzol (17.8 mL, 18 mmol) in Dioxan (270 mL) wird mit Tris(dibenzylidenaceton)dipalladium (838 mg, 0.1 mmol), 9,9-Dimethyl-4,5-bis-(diphenylphosphin)xanthen (1.4 g, 0.3 mmol), Natrium-*tert*-butylat (15.4 g, 18 mmol) und Wasser (2.3 g) versetzt. Die Mischung wird über Nacht unter Rückfluß gerührt. Nach Abkühlung auf Raumtemperatur wird der Niederschlag abgesaugt und mit Dichlormethan gewaschen. Die vereinten Filtrate werden zur Trockne eingeengt und der Rückstand wird in Dichlormethan (125 mL) und Cyclohexan (150 mL) gelöst und säulenfiltriert. Die Produktfraktionen werden aufkonzentriert und ausgefallenes Produkt wird abfiltriert. Ausbeute: 14.2 g (67%).

¹H-NMR (CD₂Cl₂, 500 MHz, 0698403439): δ = 5.19 (br s, 1 H), 6.71-6.76 (m, 3H), 6.84 (dd, 1 H), 6.89-6.96 (m, 2H), 7.19 (dd, 2H), 7.23 (dd, 2H), 7.39 (d, 1 H), 7.51 (d, 2H), 8.02 (d, 1H).

### 2,3-Di-(N-phenylamino)pyridin-hydrochlorid:

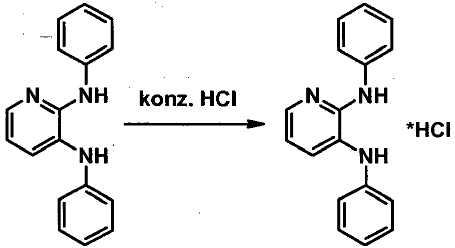

Eine Suspension von 2,3-Di-(*N-*phenylamino)pyridin (14.2 g, 54 mmol) in Salzsäure (200 mL) wird über Nacht bei Raumtemperatur gerührt. Die Mischung wird zur Trockne eingeengt. Ausbeute: 14.0 g (87%).

¹H-NMR (d₆-DMSO, 500 MHz, 0698403873): δ = 6.94-7.00 (m, 2H), 7.17 (d, 2H), 7.30 (m_{c}, 3H), 7.40-7.51 (m, 5H), 7.74 (dd, 1 H). Die NH-Protonen sind nicht detektierbar.

### 1,3-Diphenyl-4-azabenzimidazolium-chlorid:

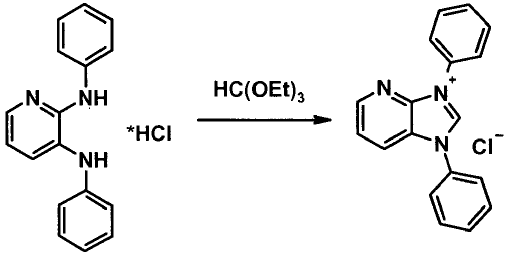

Eine Mischung von 2,3-Di-(*N-*phenylamino)pyridin-hydrochlorid (14.0 g, 47 mmol) in Triethylorthoformiat (160 mL) wird über Nacht bei 105°C gerührt. Nach Abkühlung auf Raumtemperatur wird der Feststoff abgesaugt und mit Triethylorthoformiat gewaschen. Ausbeute: 10.3 g (71%).

¹H-NMR (CD₂Cl₂, 500 MHz): δ = 7.55-7.73 (m, 7H), 8.08 (dd, 2H), 8.19 (dd, 1H), 8.33 (dd, 2H), 8.80 (dd, 1H), 12.24 (s, 1H).

### Komplex Em21

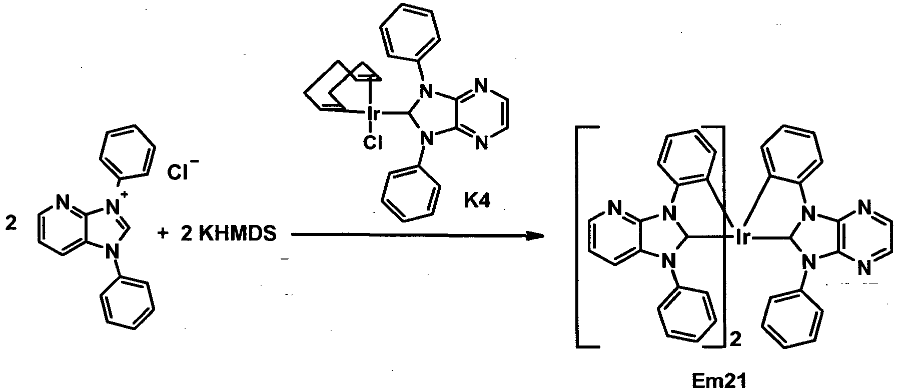

0.65 g 1,3-Diphenyl-4-azabenzimidazolium-chlorid (2.1 mmol) werden in 100 ml wasserfreiem Toluol suspendiert und auf 0°C abgekühlt. Dann werden langsam 4.2 ml Bis(trimethylsilyl)kaliumamid (KHMDS, 0.5M in Toluol, 2.1 mmol) zugesetzt. Das Gemisch wird 1 h bei Raumtemperatur gerührt und anschließend mit einer Lösung aus 0.61 g **K4** (1.0 mmol) in 100 ml wasserfreiem Toluol versetzt. Es wird 1h bei Raumtemperatur gerührt und danach 18 h am Rückfluss erhitzt. Nach dem Abkühlen wird zur Trockene eingeengt. Der Rückstand wird säulenchromatographisch gereinigt (Kieselgel, Laufmittel: Cyclohexan/ Aceton = 4/1). Man erhält 0.13 g (13%) Em21 als hellgelbes Pulver.

¹H-NMR (CD₂Cl₂, 500 MHz): δ = 6.23-6.32 (m, 3H), 6.38 (t, 1 H), 6.40-6.85 (m, 17H), 7.00-7.05 (m, 2H), 7.10-7.16 (m, 3H), 7.32 (t, 2H), 8.01 (d, 1H), 8.28 (d, 1H), 8.39 (dt, 2H), 8.72 (d, 1 H), 8.94 (d, 1 H), 8.98 (d, 1 H).

Photolumineszenz (2% im PMMA-Film):
λₘₐₓ = 480 nm, CIE: (0.17; 0.30), 95% QA

### Beispiel 27:

### Emitter Em22

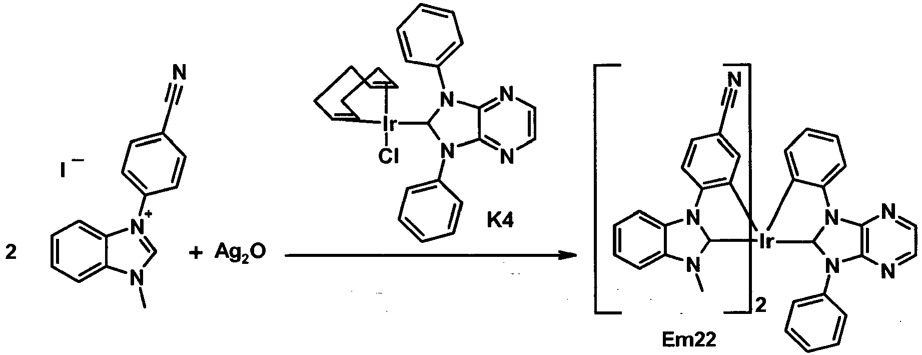

Eine Lösung von 2.30 g (6.4 mmol) 1-(4-Cyanophenyl)-3-methyl-benzimidazoliumiodid (Herstellung siehe WO2006/056418) in 200 ml 1,4-Dioxan (wasserfrei, ultratrocken) wird mit 1.11g (4.8 mmol) Silber(I)-oxid und 20 g Molsieb versetzt und über Nacht bei Raumtemperatur gerührt. Dann gibt man eine Lösung von 1.29 g (2.1 mmol) Komplex **K4** in 200 ml wasserfreiem o-Xylol zu und rührt 22h bei 110°C. Nach dem Abkühlen wird das Reaktionsgemisch filtriert. Das Filtrat wird im Vakuum vom Lösungsmittel befreit und anschließend säulenchromatographisch aufgetrennt (Kieselgel, Laufmittel: Cyclohexan/Aceton = 2/1). Man erhält 0.61 g (31%) **Em22-Isomer 1** (RF = 0.31), 0.45 g (23%) **Em22-Isomer 2** (RF = 0.25) und 0.25 g (13%) **Em22-Isomer 3** (RF = 0.20), die jeweils nochmal aus Methylenchlorid/Methanol umkristallisiert werden.

### Em22-Isomer 1:

¹H-NMR (CD₂Cl₂, 500 MHz): δ = 2.78 (s, 3H), 3.15 (s, 3H), 6.31 (d, sehr breit, 2H), 6.53 (d, 1H), 6.68 (t, 1H), 6.77 (t, 1H), 6.92 (d, 1H), 7.11-7.16 (m, 2H), 7.23-7.52 (m, 10H), 7.88 (d, 1H), 7.91 (d, 1H), 7.99 (d, 1H), 8.11 (d, 1H), 8.18 (d, 1H), 8.41 (d, 1H), 8.76 (d, 1 H).

Photolumineszenz (2% im PMMA-Film):
λₘₐₓ = 484 nm, CIE: (0.19; 0.33), 87% QA

### Em22-Isomer 2:

¹H-NMR (CD₂Cl₂, 500 MHz): δ = 3.20 (s, 3H), 3.37 (s, 3H), 5.67-6.92 (sehr flaches breites Signal, 2H), 6.51 (d, 1 H), 6.70-6.77 (m, 2H), 6.97-7.00 (m, 1 H), 7.06-7.12 (m, 3H), 7.20-7.47 (m, 10 H), 7.90 (d, 1 H), 8.06 (t, 2H), 8.24 (d, 1 H), 8.45 (d, 1 H), 8.80 (d, 1 H).

Photolumineszenz (2% im PMMA-Film):
λₘₐₓ = 494 nm, CIE: (0.22; 0.41), 87% QA

### Em22-Isomer 3:

¹H-NMR (CD₂Cl₂, 500 MHz): δ = 2.75 (s, 3H), 3.56 (s, 3H), 6.02-7.26 (sehr flaches breites Signal, 4H), 6.48 (dd, 1 H), 6.68 (d, 1 H), 6.69-6.73 (m, 1 H), 6.78 (dt, 1 H), 6.86 (d, 1 H), 6.90 (d, breit, 1 H), 7.14 (dt, 1 H), 7.24 (dt, 1 H), 7.30-7.39 (m, 5H), 7.49 (dd, 1H), 7.87 (d, 1H), 7.94 (d, 1H), 8.03 (d, 1H), 8.08 (d, 1H), 8.17 (d, 1H), 8.41 (d, 1H), 8.79 (dd, 1H).

Photolumineszenz (2% im PMMA-Film):
λₘₐₓ = 468 nm, CIE: (0.16; 0.21), 90% QA

### Beispiel 28

### Herstellung von dotierten OLEDs

Das als Anode verwendete ITO-Substrat wird zuerst mit kommerziellen Reinigungsmitteln für die LCD-Produktion (Deconex^{®} 20NS und Neutralisationsmittel 25ORGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs. Als nächstes wird die Lochinjektionsschicht AJ20-1000 der Firma Plexcore aus Lösung aufgesponnen.

### Beispiel 28a:

Nach der Lochinjektionsschicht werden die nachfolgend genannten organischen Materialien mit einer Rate von ca. 0.5-5 nm/min bei etwa 10⁻⁷-10⁻⁹ mbar auf das gereinigte Substrat aufgedampft. Als Lochleiter wird 20 nm der Mischung aus **Ir(DPBIC)₃** mit 4% des p-Dotierstoffs **F6-TNAP** aufgedampft. Als Excitonenblocker wird **Ir(DPBIC)₃** mit einer Dicke von 10 nm auf das Substrat aufgebracht. 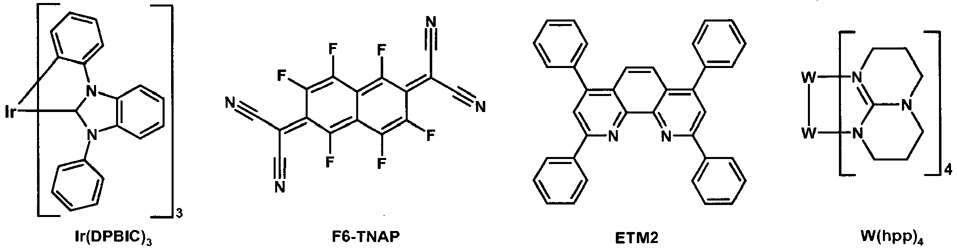 (zur Herstellung von Ir(DPBIC)₃ siehe Ir-Komplex (7) in der Anmeldung WO 2005/019373 A2).

Anschließend wird eine Mischung aus 30% Emitter **fac-Em1,** 60% der Verbindung **Ma7** und 10% der Verbindung **Ir(DPBIC)₃** mit einer Dicke von 30 nm als emissive Schicht aufgedampft, wobei letztere beide Verbindungen als Matrixmaterial fungieren.

Als folgende Elektronenleiterschicht dient eine mit **8% W(hpp)₄** (siehe EP1786050) dotierte **ETM2-**Schicht (siehe Compound No. 28 in EP1097981) mit einer Schichtdicke von 25 nm.

Eine Aluminiumkathode von 100nm Dicke schließt die Diode ab.

Alle Bauteile werden in einer inerten Stickstoffatmosphäre mit einem Glasdeckel verkapselt.

### Beispiel 28b:

Die OLED aus dem Beispiel 28b ist gleich aufgebaut wie die OLED aus Beispiel 28a, nur mit dem Unterschied, dass die emissive Schicht aus 30% **Em8** und 70% Matrixmaterial **Ma7** besteht.

Zur Charakterisierung der OLEDs werden Elektrolumineszenz-Spektren bei verschiedenen Strömen bzw. Spannungen aufgenommen. Weiterhin wird die Strom-Spannungs-Kennlinie in Kombination mit der abgestrahlten Lichtleistung gemessen. Die Lichtleistung kann durch Kalibrierung mit einem Luminanzmeter in photometrische Größen umgerechnet werden.

Für die beiden Ausführungsbeispiele einer dotierten OLED ergeben sich die folgenden elektrooptischen Daten:

| Bsp | Emitter | Spannung [V] bei 300nits | EQE [%] bei 300nits |
|---|---|---|---|
| **28a** | **fac-m1** | 2.75 V | 10.9 % |
| **28b** | **Em8** | 3.38 V | 10.8 % |

EQE - externe Quanteneffizienz. Gemessen in Vorwärtsrichtung unter der Annahme einer Lambertschen Lichtintensitätsverteilung.

### Beispiel 29

### Dioden mit verschiedenen Emittern

Das als Anode verwendete ITO-Substrat wird zuerst mit kommerziellen Reinigungsmitteln für die LCD-Produktion (Deconex^{®} 20NS und Neutralisationsmittel 25ORGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs. Als nächstes wird die 40 nm dicke Loch-injektionsschicht AJ20-1000 der Firma Plexcore aus Lösung aufgesponnen.

Danach werden die nachfolgend genannten organischen Materialien mit einer Rate von ca. 0.5-5nm/min bei etwa 10⁻⁷-10⁻⁹ mbar auf das gereinigte Substrat aufgedampft:
Als Lochleiter und Excitonenblocker wird **Ir(DPBIC)₃** in einer Dicke von 20 nm aufgebracht, wovon die ersten 15 nm mit 5% ReO₃ dotiert sind. Danach wird eine 20 nm dicke Emissions-schicht bestehend aus einem Emitter und zwei Matrixmaterialien aufgedampft. Es folgt eine 5 nm dicke Schicht Excitonen- und Lochblocker. Als nächstes wird eine Mischung aus 50% Liq und 50% **ETM1** als Elektronentransportschicht in einer Dicke von 40 nm aufgebracht. Abschließend werden eine 4 nm dicke Schicht KF sowie eine 100 nm dicke Al-Elektrode aufgedampft.

### Beispiel 29a

Die Diode wird wie oben beschrieben aufgebaut mit einer Emissionsschicht bestehend aus 10% des Emitters **Em16** und jeweils 45% der Matrixmaterialien **Ir(DPBIC)₃** und **Ma13.** Als Excitonen- und Lochblocker dient **Ma13.**

Die Diode zeigt die Farbkoordinaten CIE 0.20, 0.43. Bei 300 cd/m² beträgt die Spannung 3.6 V und die externe Quanteneffizienz 17.7%.

### Beispiel 29b

Die Diode wird wie oben beschrieben aufgebaut mit einer Emissionsschicht bestehend aus 10% des Emitters **Em16** und jeweils 45% der Matrixmaterialien **Ir(DPBIC)₃** und **Ma7.** Als Excitonen- und Lochblocker dient **Ma7.**

Die Diode zeigt die Farbkoordinaten CIE 0.19, 0.43. Bei 300 cd/m² beträgt die Spannung 3.6 V und die externe Quanteneffizienz 14.7%.

### Beispiel 29c

Die Diode wird wie oben beschrieben aufgebaut mit einer Emissionsschicht bestehend aus 10% des Emitters **Em17** und jeweils 45% der Matrixmaterialien **Ir(DPBIC)₃** und **Ma13.** Als Excitonen- und Lochblocker dient **Ma13.**

Die Diode zeigt die Farbkoordinaten CIE 0.20, 0.44. Bei 300 cd/m² beträgt die Leuchteffizienz 45.5 Im/W und die externe Quanteneffizienz 19.6%.

### Beispiel 29d

Die Diode wird wie oben beschrieben aufgebaut mit einer Emissionsschicht bestehend aus 10% des Emitters **Em17** und jeweils 45% der Matrixmaterialien **Ir(DPBIC)₃** und **Ma7.** Als Excitonen- und Lochblocker dient **Ma7.**

Die Diode zeigt die Farbkoordinaten CIE 0.18, 0.39. Bei 300 cd/m² beträgt die Spannung 3.8 V und die Leuchteffizienz 40.4 Im/W.

### Beispiel 29e

Die Diode wird wie oben beschrieben aufgebaut mit einer Emissionsschicht bestehend aus 10% des Emitters **Em20** und jeweils 45% der Matrixmaterialien **Ir(DPBIC)₃** und **Ma7.** Als Excitonen- und Lochblocker dient **Ma7.**

Die Diode zeigt die Farbkoordinaten CIE 0.15, 0.29. Bei 300 cd/m² beträgt die Spannung 3.6 V und die externe Quanteneffizienz 19.9%.

### Beispiel 29f

Die Diode wird wie oben beschrieben aufgebaut mit einer Emissionsschicht bestehend aus 10% des Emitters **Em20** und jeweils 45% der Matrixmaterialien **Ir(DPBIC)₃** und **Ma13.** Als Excitonen- und Lochblocker dient **Ma13.**

Die Diode zeigt die Farbkoordinaten CIE 0.15, 0.29. Bei 300 cd/m² beträgt die Spannung 3.3 V und die externe Quanteneffizienz 19.0%.

### Beispiel 30

### Homojunction-OLEDs

### Beispiel 30a

Das als Anode verwendete ITO-Substrat wird zuerst mit kommerziellen Reinigungsmitteln für die LCD-Produktion (Deconex^{®} 20NS und Neutralisationsmittel 25ORGAN-ACID^{®}) und anschließend in einem Aceton/Isopropanol-Gemisch im Ultraschallbad gesäubert. Zur Beseitigung möglicher organischer Rückstände wird das Substrat in einem Ozonofen weitere 25 Minuten einem kontinuierlichen Ozonfluss ausgesetzt. Diese Behandlung verbessert auch die Lochinjektionseigenschaften des ITOs. Als nächstes wird die 40 nm dicke Loch-injektionsschicht AJ20-1000 der Firma Plexcore aus Lösung aufgesponnen.

Danach werden die nachfolgend genannten organischen Materialien mit einer Rate von ca. 0.5-5nm/min bei etwa 10⁻⁷-10⁻⁹ mbar auf das gereinigte Substrat aufgedampft:
Als Lochleiter und Excitonenblocker wird **Ma4** in einer Dicke von 20 nm aufgebracht, wovon die ersten 15 nm mit 5% ReO₃ dotiert sind. Danach wird eine 20 nm dicke Emissionsschicht bestehend aus 10% **fac-Em1,** 35% **Ir(DPBIC)₃** und 55% **Ma4** aufgedampft. Es folgt eine 5 nm dicke Schicht **Ma4** als Excitonen- und Lochblocker. Als nächstes wird eine Mischung aus 50% **Liq** und 50% **Ma4** als Elektronentransportschicht in einer Dicke von 40 nm aufgebracht. Abschließend werden eine 4 nm dicke Schicht KF sowie eine 100 nm dicke Al-Elektrode aufgedampft.

Die Diode zeigt die Farbkoordinaten CIE 0.15, 0.25. Bei 300 cd/m² beträgt die externe Quanteneffizienz 18.7%.

### Beispiel 30b

Die Diode wird analog dem Beispiel 30a aufgebaut mit dem Unterschied, daß die Emissionsschicht aus 30% **fac-Em1,** 35% **Ir(DPBIC)₃** und 35% **Ma4** besteht.

Die Diode zeigt die Farbkoordinaten CIE 0.16, 0.31. Bei 300 cd/m² beträgt die externe Quanteneffizienz 16.2%.

### Beispiel 30c

Die Diode wird analog dem Beispiel 30a aufgebaut mit dem Unterschied, daß in den jeweiligen Schichten **Ma4** durch **Ma7** ersetzt wird und die Emissionsschicht aus 10% **fac-Em1,** 45% **Ir(DPBIC)₃** und 45% **Ma7** besteht.

Die Diode zeigt die Farbkoordinaten CIE 0.16, 0.28. Bei 300 cd/m² beträgt die externe Quanteneffizienz 11.9%.

### Beispiel 30d

Die Diode wird analog dem Beispiel 30a aufgebaut mit dem Unterschied, daß in den jeweiligen Schichten **Ma4** durch **Ma7** ersetzt wird und die Emissionsschicht aus 30% **fac-Em1,** 35% **Ir(DPBIC)₃** und 35% **Ma7** besteht.

Die Diode zeigt die Farbkoordinaten CIE 0.18, 0.34. Bei 300 cd/m² beträgt die externe Quanteneffizienz 16.3%.

### Beispiel 31

### Synthese von ETM3

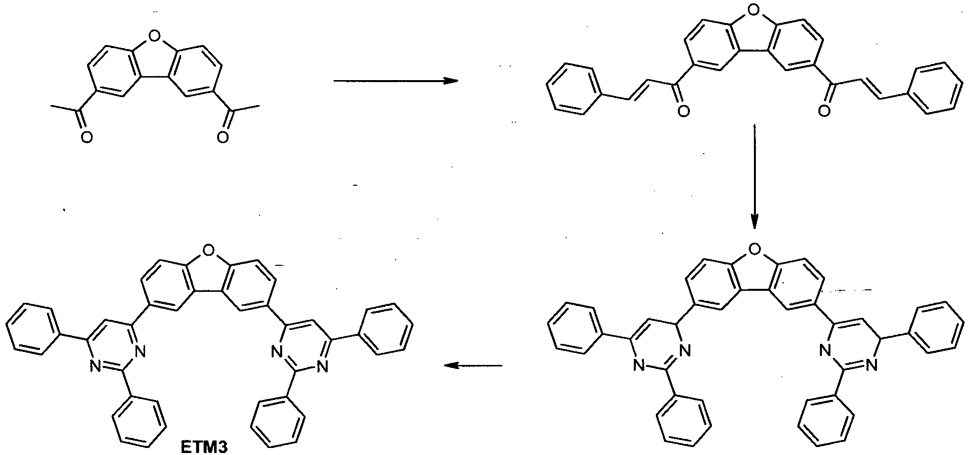

### Stufe 1

In einem 250 ml Dreihalsrundkolben, versehen mit Magnetrührer, Thermometer, Tropftrichter, Rückflusskühler und Stickstoffüberleitung, werden 150 ml Methanol vorgelegt und 3.8 g Kaliumhydroxid (≥85%) zugegeben. Die Mischung wird bei Raumtemperatur gerührt, bis sich eine klare, farblose Lösung bildet. Anschliessend wird mit einem Eisbad auf 0°C abgekühlt und 14,3 g (56,7 mMol) 1- (8-Acetyldibenzofuran-2-yl)-ethanon (hergestellt nach M. J. Bruce, Perkin Transactions I, 1789 (1995)) zugegeben. Die Suspension wird 1 h bei 0°C verrührt und dann 17.3 ml (170 mMol) Benzaldehyd innerhalb von 3 min. zugetropft. Das Reaktionsgemisch wird anschliessend auf Raumtemperatur erwärmt und 14 h verrührt. Die feine, beige-gelbe Suspension wird filtriert und der Rückstand mit 50 ml Ethanol gewaschen. Das Rohprodukt wird in 150 ml Ethanol suspendiert und 30 min unter Rückfluss verrührt. Danach wird auf Raumtemperatur und dann auf 0°C abgekühlt, filtriert, zwei Mal mit 10 ml eiskaltem Ethanol gewaschen und der Rückstand über Nacht bei 50°C/150 mbar getrocknet. Man erhält 21,6 g (88,9% d.Th) 3-Phenyl-1-{8-(3-phenyl-acryloyl)]-dibenzofuran-2-yl}-propenon als hellbeige Kristalle.

¹H-NMR (CDCl₃, 300 MHz):
8.67 (s, 2H), 8.25 (d, J=7Hz, 2H), 7.89 (s, J=12Hz, 2H), 7.8-7.65 (m, 8H), 7.55-7.4 (m, 6H).

### Stufe 2

In einem 100 ml Dreihalsrundkolben, versehen mit Magnetrührer, Thermometer Rückflusskühler und Stickstoffüberleitung, werden 60 ml Methanol vorgelegt und unter Rühren mit einem Eisbad auf 0°C abgekühlt. Anschliessend werden innerhalb von 10 min 15.7 g (240 mMol) Kalium-tert-butoxid portionenweise zugegeben. In einem 500 ml Dreihalsrundkolben, versehen mit Magnetrührer, Thermometer, Tropftrichter, Rückflusskühler und Stickstoffüberleitung, werden 90 ml Methanol vorgelegt und dann unter Rühren 8.6 g (20 mMol) 3-Phenyl-1-{8-(3-phenyl-acryloyl)]-dibenzofuran-2-yl}-propenon und 41.8 g (80 mMol) Benzamidin hydrochlorid (30% in Methanol) zugegeben. Die beige Suspension wird auf Rückfluss erhitzt und danach die vorher hergestellte Kalium-tert-butoxid-Lösung innerhalb von 20 Min. zugetropft. Die beigebraune Suspension wird über Nacht unter Rückfluss gerührt. Anschliessend wird auf Raumtemperatur abgekühlt und dann 150 ml Wasser zugetropft und die Mischung 4 h verrührt. Es bildet sich eine Suspension, welche filtriert wird. Der Rückstand wird in 200 ml Wasser suspendiert und 2 h auf Rückfluss erhitzt. Anschliessend wir auf Raumtemperatur abgekühlt, filtriert und der Rückstand mit 50 ml Wasser gewaschen und anschliessend über Nacht bei 60°C/150 mbar getrocknet. Man erhält 10,9 g 2,8-Bis-(2,4-diphenyl-1,4-dihydro-pyrimidin-dibenzofuran (85,8% d.Th.) als gelbe Kristalle. Das Rohprodukt wird ohne Reinigung weiterverwendet.

### Stufe 3

In einem 500 ml Dreihalsrundkolben, versehen mit Magnetrührer, Thermometer Rückflusskühler und Stickstoffüberleitung, werden 10,9 g (17 mMol) rohes 2,8-Bis-(2,4-diphenyl-1,4-dihydro-pyrimidin)-dibenzofuran in 100 ml o-Dichlorbenzol vorgelegt und unter Rühren auf 60°C erwärmt. Danach werden 19,1 g (78 mMol) Chloranil zugegeben und das dunkle Reaktionsgemisch 14 h auf Rückfluss erhitzt. Anschliessend werden 160 ml MeOH zugegeben und 15 min. unter Rückfluss gerührt. Danach wird mit einem Eisbad auf 0°C abgekühlt, die dunkelbraune Suspension filtriert und der Rückstand drei Mal mit je 50 ml MeOH und zwei Mal mit je 50 ml Wasser gewaschen. Nach Trocknung über Nacht bei 60°C/125 mbar erhält man 18,0 g braune Kristalle. Das Rohprodukt wird in 100 ml MeOH 1 h unter Rückfluss suspendiert, anschliessend auf 0°C abgekühlt, zwei Mal mit je 20 ml MeOH gewaschen und bei 60°C/125 mbar getrocknet. Man erhält 16.4 g braune Kristalle. 5.7 g dieser Ware wird in 50 ml EtOH 30 min. unter Rückfluss suspendiert, anschliessend auf 0°C abgekühlt, zwei Mal mit je 20 ml EtOH gewaschen und bei 60°C/125 mbar getrocknet. Man erhält 5.0 g braune Kristalle. Dieses Rohprodukt wird in 190 ml Toluol angeschlämmt und 30 min. unter Rückfluss gerührt. Danach wird mit einem Eisbad auf 0°C abgekühlt, die Suspension filtriert und der Rückstand drei Mal mit je 20 ml Toluol gewaschen. Nach Trocknung über Nacht bei 60°C/125 mbar erhält man 3,0 g (81% d. Th.) des gewünschten Produktes als weissbeige Kristalle.

HPLC-MS: Reinheit 99.5%, [M+1] = 629,5 m/z.

¹H-NMR (CDCl₃, 300 MHz):
8.97 (s, 2H), 8.73 (d, J=9Hz, 4H), 8,43 (d, J=10Hz, 2H), 8.42-8.28 (m, 4H), 8.11 (s, 2H), 7.73 (d, J=9Hz, 2H) 7.60-7.35 (m, 12H)

### Beispiel 32

Zunächst wird das als Anode verwendete ITO-Substrat wie in Beispiel 30a behandelt und mit einer 40 nm dicken Lochinjektionsschicht aus AJ20-1000 der Firma Plexcore wie oben beschrieben versehen.

Danach werden die nachfolgend genannten organischen Materialien mit einer Rate von ca. 0.5-5nm/min bei etwa 10⁻⁷-10⁻⁹ mbar auf das gereinigte Substrat aufgedampft:

### Beispiel 32a

Als Lochleiter und Excitonenblocker wird **Ir(DPBIC)₃** in einer Dicke von 20 nm aufgebracht, wovon die ersten 15 nm mit 5% ReO₃ dotiert sind. Danach wird eine 20 nm dicke Emissionsschicht bestehend aus 10% **fac-Em1,** 45% **Ir(DPBIC)₃** und 45% **Ma13** aufgedampft. Es folgt eine 5 nm dicke Schicht **Ma13** als Excitonen- und Lochblocker. Als nächstes wird eine Mischung aus 50% Liq und 50% **ETM3** als Elektronentransportschicht in einer Dicke von 40 nm aufgebracht. Abschließend werden eine 4 nm dicke Schicht KF sowie eine 100 nm dicke Al-Elektrode aufgedampft.

Die Diode zeigt die Farbkoordinaten CIE 0.16, 0.27. Bei 300 cd/m² beträgt die externe Quanteneffizienz 13.7%.

### Beispiel 32b

Die Diode wird aufgebaut wie in Beispiel 32a beschrieben mit dem Unterschied, daß die Elektronentransportschicht aus reinem **ETM3** besteht.

Die Diode zeigt die Farbkoordinaten CIE 0.16, 0.27. Bei 300 cd/m² beträgt die Spannung 3.4V.

### Beispiel 32c

Die Diode wird aufgebaut wie in Beispiel 32a beschrieben mit dem Unterschied, daß die Emissionsschicht aus 30% **fac-Em1,** 35% **Ir(DPBIC)₃** und 35% **Ma13** besteht.

Die Diode zeigt die Farbkoordinaten CIE 0.17, 0.32. Bei 300 cd/m² beträgt die Spannung 3.4V.

### Beispiel 32d

Die Diode wird aufgebaut wie in Beispiel 32c beschrieben mit dem Unterschied, daß die Elektronentransportschicht aus reinem **ETM3** besteht.

Die Diode zeigt die Farbkoordinaten CIE 0.17, 0.31. Bei 300 cd/m² beträgt die externe Quanteneffizienz 14.2%.

### Beispiel 33

### Synthese von ETM4

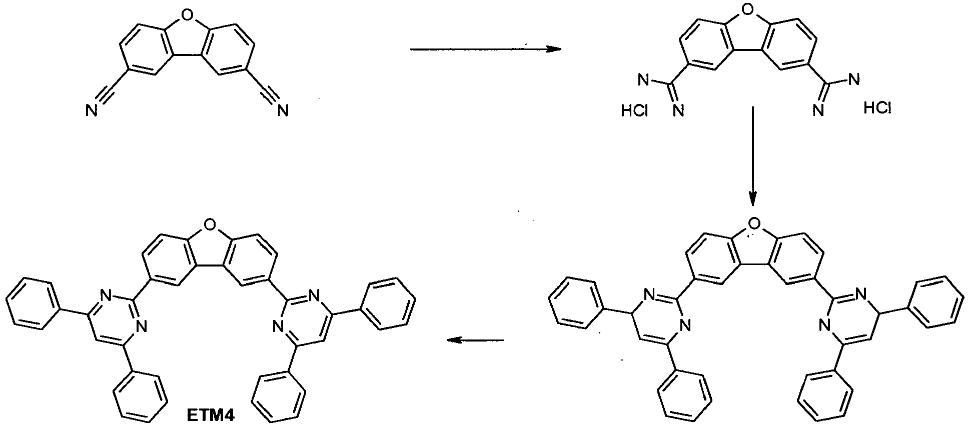

### Stufe 1

In einem 100 ml Dreihalsrundkolben, Magnetrührer, Thermometer, Rückflusskühler, Gaseinleitrohr, Stickstoffüberleitung und Gaswaschflaschen werden 1.09 g (5.0 mMol) Dibenzofuran-2,8-dicarbonitrile (hergestellt nach S. Wang, Eur. J. Med. Chem. 34, 215 (1999)) in einem Gemisch von 1.8 ml Ethanol und 50 ml 1,4-Dioxan vorgelegt und mit einem EtOH/CO₂-Bad auf 0°C abgekühlt und dann HCl-Gas bis zur Sättigung eingeleitet. Danach wird der Kolben verschlossen, die weisse Suspension auf RT erwärmt und 48 h gerührt. Anschliessend wird trockenes N₂ in die Suspension geblasen, bis im Abgas kein HCl mehr nachwiesen werden kann. Es werden 25 ml t-Butylmethylether zugegeben und die gelbliche Suspension filtriert. Der Rückstand wird zwei Mal mit je 25 ml t-Butylmethylether gewaschen, anschliessend in 40 ml 2M NH₃ in Ethanol suspendiert und 18 h bei 60°C gerührt. Anschliessend wird die Suspension abgekühlt und trockenes N₂ in die Suspension geblasen, bis im Abgas kein NH₃ mehr nachwiesen werden kann. Dann wird mit einem Eisbad gekühlt und wiederum HCl-Gas bis zur Sättigung eingeleitet. Danach wird wiederum mit trockenem N₂ ausgeblasen, die beige Suspension filtriert und portionenweise mit total 25 ml eiskaltem EtOH gewaschen. Der Rückstand wird über Nacht bei 50°C/125 mbar getrocknet. Man erhält 1.71 g (99,2% d.Th.) Dibenzofuran-2,8-bisamidine hydrochlorid als beige Kristalle.

¹H-NMR (DMSO, 300 MHz):
8.90-8.75 (m, 2H), 8.25-8.00 (m, 4H)

### Stufe 2

In einem 100 ml Dreihalsrundkolben, versehen mit Magnetrührer, Thermometer Rückflusskühler und Stickstoffüberleitung, werden 40 ml Methanol vorgelegt und unter Rühren mit einem Eisbad auf 0°C abgekühlt. Anschliessend werden innerhalb von 10 min 2.85 g (25,4 mMol) Kalium-tert-butoxid portionenweise zugegeben. In einem 250 ml Dreihalsrundkolben, versehen mit Magnetrührer, Thermometer, Tropftrichter, Rückflusskühler und Stickstoffüberleitung, werden 60 ml Methanol vorgelegt und dann unter Rühren 1.7 g Dibenzofuran-2,8-bisamidine hydrochlorid und 4.08 g (19 mMol) Benzylidenacetophenon zugegeben. Die beige Suspension wird auf Rückfluss erhitzt und danach die vorher hergestellte Kalium-tert-butoxid-Lösung innerhalb von 5 Min. zugetropft. Die beige Suspension wird über Nacht unter Rückfluss gerührt. Anschliessend wird auf 0°C abgekühlt, filtriert und der Rückstand mit 15 ml eiskaltem MeOH und drei Mal mit 60 ml Wasser gewaschen und über Nacht bei 60°C/150 mbar getrocknet. Man erhält 1.59 g Dibenzofuran-2,8-bis-(4,6-diphenyl-1,4-dihydro)-pyrimidin (39.6% d.Th.) als gelbe Kristalle. Das Rohprodukt wird ohne Reinigung weiterverwendet.

### Stufe 3

In einem 100 ml Dreihalsrundkolben, versehen mit Magnetrührer, Thermometer Rückflusskühler und Stickstoffüberleitung, werden 1.59 g 2,45 mMol) Dibenzofuran-2,8-di-yl-(4,6-diphenyl-1,4-dihydro-pyrimidine) in 15 ml o-Dichlorbenzol vorgelegt. Das Gemisch wird unter Rühren auf 50°C Innentemperatur erwärmt und dann 2,41 g (9.80 mMol) Chloranil zugegeben. Die beigebraune Suspension wird 4 h auf Rückfluss erhitzt, dann 30 ml MeOH und anschliessend 1 g NaOH gelöst in 5 ml Wasser zugetropft. Die dunkelbraune Suspension wird 30 min. unter Rückfluss gerührt, anschliessend mit einem Eisbad auf 0°C abgekühlt und filtriert. Der Rückstand wird drei Mal mit 20 ml MeOH und fünf Mal mit 20 ml heissem Wasser gewaschen. Der beige Feststoff wird in 50 ml Toluol suspendiert, und unter Rühren 30 min. rückflussiert. Danach wird auf eine Innentemperatur von 60°C abgekühlt, filtriert, der Rückstand zwei Mal mit 10 ml Toluol gewaschen und über Nacht bei 60°C/125 mbar getrocknet. Man erhält 0,85g (55.2% d.Th.) des gewünschten Produktes als weisse Kristalle.

HPLC-MS: Reinheit 99.3%, [M+1] = 629,5 m/z.

¹H-NMR (CDCl₃, 300 MHz):
9.41 (s, 2H), 8.89 (d, J=9Hz, 2H), 8.35-8.20 (m, 8H), 7.98 (s, 2H), 7.68 (d, J=9Hz, 2H) 7.70-7.45 (m, 12H)

## Patentansprüche

1. Metall-Carben-Komplex der allgemeinen Formel (I) 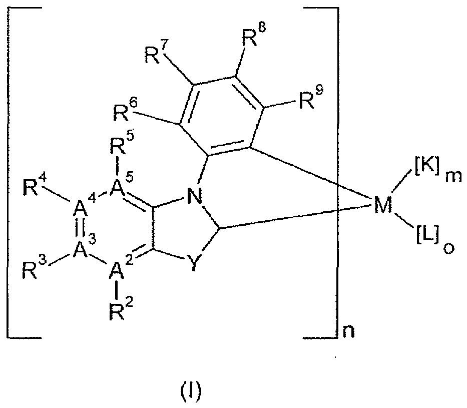 wobei M, n, Y, A², A³, A⁴, A⁵, R¹, R², R³, R⁴, R⁵, R⁶, R⁷, R⁸, R⁹, R¹⁰, K, L, m und o die folgenden Bedeutungen aufweisen:
M Ir oder Pt,
n ganze Zahl ausgewählt aus 1, 2 oder 3,
Y NR¹, O, S oder C(R¹⁰)₂,
A², A³, A⁴, A⁵ unabhängig voneinander N oder C, wobei 2 A = N-Atome sind und im Ring zwischen zwei N-Atomen wenigstens ein C-Atom vorliegt,
R¹ linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
R², R³, R⁴, R⁵ falls A², A³, A⁴ und/oder A⁵ N bedeuten, freies Elektronenpaar oder, falls A², A³, A⁴ und/oder A⁵ C bedeuten, unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung
oder
R³ und R⁴ bilden zusammen mit A³ und A⁴ einen gegebenenfalls von wenigstens einem weiteren Heteroatom unterbrochenen, gegebenenfalls substituierten, ungesättigten Ring mit insgesamt 5 bis 18 Kohlenstoff- und/oder Heteroatomen,
R⁶, R⁷, R⁸, R⁹ unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen mit einem Grundgerüst ausgewählt aus Pyridyl, Pyrimidyl, Pyrazyl, Triazolyt, Pyrrol, Furan, Thiophen, Pyrazol, Triazol, Oxazol und Thiazol, Gruppe mit Donor- oder Akzeptorwirkung, ausgewählt aus Halogenresten, Silylresten, Siloxyresten, Alkoxyresten, Aryloxyresten, Carbonylresten, Esterresten, CH₂F-Gruppen, CHF₂-Gruppen, CF₃-Gruppen, CN-Gruppen, Thiogruppen und SCN-Gruppen,
oder
R⁶ und R⁷, R⁷ und R⁸ oder R⁸ und R⁹ bilden zusammen mit den C-Atomen, an welche Sie gebunden sind, einen gegebenenfalls von wenigstens einem Heteroatom unterbrochenen gesättigten, ungesättigten oder aromatischen, gegebenenfalls substituierten, Ring mit insgesamt 5 bis 18 Kohlenstoff- und/oder Heleroatomen,
und/oder
falls A⁵ C bedeutet, bilden R⁵ und R⁶ zusammen eine gesättigte oder ungesättigte, lineare oder verzweigte, gegebenenfalls Heteroatome, aromatische Einheit, heteroaromatische Einheit und/oder funktionelle Gruppen enthaltende Verbrückung mit insgesamt 1 bis 30 Kohlenstoff- und/oder Heteroatomen, an die gegebenenfalls ein substituierter oder unsubstituierter, fünf- bis achtgliedriger, Kohlenstoff- und/oder Heteroatome enthaltender Ring, annelliert ist,
R¹⁰ unabhängig voneinander linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
K neutraler mono- oder bidentater Ligand,
L mono- oder dianionischer Ligand, bevorzugt monoanionischer Ligand, der mono- oder bidentat sein kann,
m 0, 1 oder 2, wobei bei m gleich 2 die Liganden K gleich oder verschieden sein können,
o 0, 1 oder 2, wobei bei o gleich 2 die Liganden L gleich oder verschieden sein können.

2. Metall-Carben-Komplex nach Anspruch 1, **dadurch gekennzeichnet, dass** M, n, Y, A², A³, A⁴, A⁵, R¹ R², R³, R⁴, R⁵, R⁶, R⁷, R⁸, R⁹, L, m und o die folgenden Bedeutungen aufweisen:
M Ir,
n 1, 2 oder 3,
Y NR¹,
A², A³, A⁴, A⁵ unabhängig voneinander N oder C, wobei 2 A = N-Atome sind und im Ring zwischen zwei N-Atomen wenigstens ein C-Atom vorliegt,
R¹ linearer oder verzweigter Alkylrest mit 1 bis 6 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
R², R³, R⁴, R⁵ falls A², A³, A⁴ und/oder A⁵ N bedeuten freies Elektronenpaar, oder falls A², A³, A⁴ und/oder A⁵ C bedeuten, unabhängig voneinander Wasserstoff, linearer oder verzweigter Alkylrest mit 1 bis 6 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
oder
R³ und R⁴ bilden zusammen mit A³ und A⁴ einen gegebenenfalls von wenigstens einem weiteren Heteroatom unterbrochenen, gegebenenfalls substituierten, ungesättigten Ring mit insgesamt 5 bis 18 Kohlenstoff- und/oder Heteroatomen,
R⁶, R⁷, R⁸, R⁹ unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, mit einem Grundgerüst ausgewählt aus Pyridyl, Pyrimidyl, Pyrazyl, Triazolyl, Pyrrol, Furan, Thiophen, Pyrazol, Triazol, Oxazol und Thiazol,
L monoanionischer bidentater Ligand,
m 0,
o 0, 1 oder 2.

3. Metall-Carben-Komplex nach Anspruch 1 oder 2, **dadurch gekennzeichnet, dass** M, n, Y, A², A³, A⁴, A⁵, R¹, R², R³, R⁴, R⁵, R⁶, R⁷, R⁸, R⁹, L, m und o die folgenden Bedeutungen aufweisen:
M Ir,
n 2 oder 3,
Y NR¹,
A², A³, A⁴, A⁵ A² und A⁵ sind N und A³ und A⁴ sind C
oder
A² und A⁴ sind N und A³ und A⁵ sind C
oder
A³ und A⁵ sind N und A² und A⁴ sind C,
R¹ linearer oder verzweigter Alkylrest mit 1 bis 6 Kohlenstoffatomen, substituierter oder unsubstituierter Phenylrest, substituierter oder unsubstituierter Heteroarylrest mit insgesamt 6 bis 18 Kohlenstoff- und/oder Heteroatomen,
R², R³, R⁴, R⁵ falls A², A³, A⁴ und/oder A⁵ N bedeuten, freies Elektronenpaar, oder, falls A², A³, A⁴ und/oder A⁵ C bedeuten, unabhängig voneinander Wasserstoff, linearer oder verzweigter Alkylrest mit 1 bis 6 Kohlenstoffatomen, substituierter, insbesondere o,o'-dialkylierter, oder unsubstituierter Phenylrest,
oder
R³ und R⁴ bilden zusammen mit A³ und A⁴ einen gegebenenfalls substituierten, einfach ungesättigten Ring mit insgesamt 5 bis 7 Kohlenstoffatomen,
R⁶, R⁷, R⁸, R⁹ unabhängig voneinander Wasserstoff, linearer oder verzweigter Alkylrest mit 1 bis 20 Kohlenstoffatomen, o,o'-dialkylierter Arylrest mit 6 bis 30 Kohlenstoffatomen,
L monoanionischer bidentater Ligand,
m 0,
o 0 oder 1.

4. Metall-Carben-Komplex nach einem der Ansprüche 1 bis 3, **dadurch gekennzeichnet, dass** in der allgemeinen Formel (I) L Carbenligand der allgemeinen Formel (II) 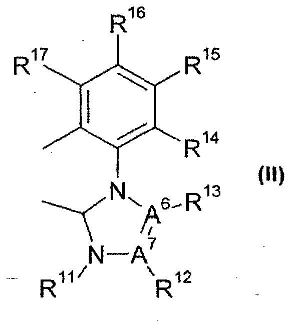 bedeutet, mit
A⁶, A⁷ unabhängig voneinander C oder N
R¹¹ linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen,
R¹², R¹³ unabhängig voneinander, wenn A gleich N, freies Elektronenpaar, oder wenn A gleich C, Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung,
R¹⁴ , R¹⁵ , R¹⁶, R¹⁷ unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung,
oder
R¹² und R¹³, R¹⁴ und R¹⁵, R¹⁵ und R¹⁶ oder R¹⁶ und R¹⁷ bilden zusammen mit A bzw. den C-Atomen, an welche sie gebunden sind, einen gegebenenfalls von wenigstens einem Heteroatom unterbrochenen ungesättigten oder aromatischen, gegebenenfalls substituierten, Ring mit insgesamt 5 bis 18 Kohlenstoff- und/oder Heteroatomen,
und/oder
falls A⁶ C bedeutet, bilden R¹³ und R¹⁴ zusammen eine gesättigte oder ungesättigte, lineare oder verzweigte, gegebenenfalls Heteroatome, aromatische Einheiten, heteroaromatische Einheiten und/oder funktionelle Gruppen enthaltende Verbrückung mit insgesamt 1 bis 30 Kohlenstoff- und/oder Heteroatomen, an die gegebenenfalls ein substituierter oder unsubstituierter, fünf- bis achtgliedriger, Kohlenstoff- und/oder Heteroatome enthaltender Ring, annelliert ist.

5. Metall-Carben-Komplex nach einem der Ansprüche 1 bis 3, **dadurch gekennzeichnet, dass** in der allgemeinen Formel (I) L
heterocyclischer Nicht-Carbenligand der allgemeinen Formel (III) 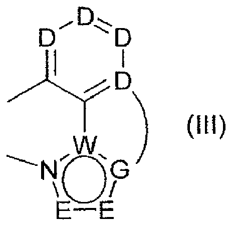 bedeutet, worin die Symbole in dem Liganden der allgemeinen Formel III die folgenden Bedeutungen aufweisen:
D unabhängig voneinander CR¹⁸ oder N, bevorzugt CR¹⁸;
W C, N, bevorzugt C;
E unabhängig voneinander CR¹⁹, N, NR²⁰, bevorzugt CR¹⁹ oder N;
G CR²¹, N, NR²², S, O, bevorzugt NR²¹
R¹⁸, R¹⁹ R²¹ unabhängig voneinander Wasserstoff, linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung,
oder jeweils 2 Reste R¹⁸, R¹⁹ und R²¹ bilden zusammen mit den C-Atomen, an welche sie gebunden sind, einen gegebenenfalls von wenigstens einem Heteroatom unterbrochenen gesättigten, ungesättigten oder aromatischen, gegebenenfalls substituierten, Ring mit insgesamt 5 bis 18 Kohlenstoff- und/oder Heteroatomen,
R²⁰, R²² unabhängig voneinander linearer oder verzweigter, gegebenenfalls von wenigstens einem Heteroatom unterbrochener, gegebenenfalls wenigstens eine funktionelle Gruppe tragender Alkylrest mit 1 bis 20 Kohlenstoffatomen, Cycloalkylrest mit 3 bis 20 Kohlenstoffatomen, Cycloheteroalkylrest mit 3 bis 20 Kohlenstoffatomen, substituierter oder unsubstituierter Arylrest mit 6 bis 30 Kohlenstoffatomen, substituierter oder unsubstituierter Heteroarylrest mit insgesamt 5 bis 18 Kohlenstoff- und/oder Heteroatomen, Gruppe mit Donor- oder Akzeptorwirkung; bevorzugt o,o'-dialkylierter Arylrest
wobei die durchgezogene, gebogene Linie eine optionale Verbrückung zwischen einer der Gruppen D und der Gruppe G bedeutet; wobei die Verbrückung die folgenden Bedeutungen aufweisen kann:
Alkylen, Arylen, Heteroarylen, Alkinylen, Alkenylen, NR²³, O, S, SiR²⁴R²⁵, und (CR²⁶R²⁷)_{d}, wobei eine oder mehrere nicht benachbarte Gruppen (CR²⁶R²⁷) durch NR²³, O, S, SiR²⁴R²⁵, ersetzt sein können, wobei
d 2 bis 10;
und
R²⁴, R²⁵, R²⁶, R²⁷ H, Alkyl, Aryl, Heteroaryl, Alkenyl, Alkinyl.

6. Verfahren zur Herstellung der Metall-Carben-Komplexe gemäß einem der Ansprüche 1 bis 5 durch Inkontaktbringen von geeigneten M enthaltenden Verbindungen mit den entsprechenden Liganden bzw. Ligandvorläufem.

7. Organisches Elektronikbauteil, enthaltend wenigstens einen Metall-Carben-Komplex nach einem der Ansprüche 1 bis 5.

8. Organisches Elektronikbauteil nach Anspruch 7, **dadurch gekennzeichnet, dass** es eine Organische Licht-Emittierende Diode (OLED), Organische Photovoltaik-Zelle (OPV), Organischer Feldeffekttransistor (OFET) oder Licht-Emittierende Elektrochemische Zelle (LEEC) ist.

9. Licht-emittierende Schicht, enthaltend mindestens einen Metall-Carben-Komplex nach einem der Ansprüche 1 bis 5.

10. OLED, enthaltend eine Licht-emittierende Schicht nach Anspruch 9.

11. OLED enthaltend mindestens einen Metall-Carben-Komplex nach einem der Ansprüche 1 bis 5, sowie mindestens eine Verbindung der Formel (X) 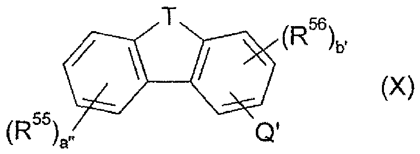 worin bedeuten
T NR⁵⁷, S, O oder PR⁵⁷, bevorzugt S oder O, besonders bevorzugt O;
R⁵⁷ Aryl, Heteroaryl, Alkyl, Cycloalkyl oder Heterocycloalkyl;
Q' -NR⁵⁸R⁵⁹, -P(O)R⁶⁰R⁶¹, -PR⁶²R⁶³, -S(O)₂R⁶⁴, -S(O)R⁶⁵, -SR⁶⁶ oder -OR⁶⁷, bevorzugt -NR⁵⁸R⁵⁹; besonders bevorzugt 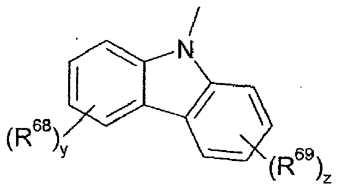 worin bedeuten
R⁶⁸, R⁶⁹ unabhängig voneinander Alkyl, Cycloalkyl, Heterocycloalkyl, Aryl oder Heteroaryl; bevorzugt Methyl, Carbazolyl, Dibenzofuryl oder Dibenzothienyl;
y, z unabhängig voneinander 0, 1, 2, 3 oder 4, bevorzugt 0 oder 1;
R⁵⁵, R⁵⁶ unabhängig voneinander Alkyl, Cycloalkyl, Heterocycloalkyl, Aryl, Heteroaryl, SiR⁷⁰R⁷¹R⁷², eine Gruppe Q' oder eine Gruppe mit Donor- oder Akzeptorwirkung;
a" 0, 1, 2, 3 oder 4;
b' 0, 1, 2 oder 3;
R⁵⁸, R⁵⁹ bilden gemeinsam mit dem N-Atom einen cyclischen Rest mit 3 bis 10 Ringatomen, der unsubstituiert oder mit einem oder mehreren Substituenten ausgewählt aus Alkyl, Cycloalkyl, Heterocycloalkyl, Aryl, Heteroaryl und einer Gruppe mit Donor- oder Akzeptorwirkung substituiert sein kann und/oder mit einem oder mehreren weiteren cyclischen Resten mit 3 bis 10 Ringatomen anelliert sein kann, wobei die anellierten Reste unsubstituiert oder mit einem oder mehreren Substituenten ausgewählt aus Alkyl, Cycloalkyl, Heterocycloalkyl, Aryl, Heteroaryl, und einer Gruppe mit Donor- oder Akzeptorwirkung substituiert sein können;
R⁷⁰, R⁷¹, R⁷², R⁶⁰, R⁶¹, R⁶², R⁶³, R⁶⁴, R⁶⁵, R⁶⁶, R⁶⁷ unabhängig voneinander Aryl, Heteroaryl, Alkyl, Cycloalkyl oder Heterocycloalkyl,
oder
zwei Einheiten der allgemeinen Formel (X) sind über eine lineare oder verzweigte, gesättigte oder ungesättigte, gegebenenfalls durch wenigstens ein Heteroatom unterbrochene Verbrückung, über eine Bindung oder über O miteinander verbrückt.

12. OLED nach Anspruch 11, **dadurch gekennzeichnet, dass** sie eine Emissionsschicht enthaltend mindestens einen Metall-Carben-Komplex nach einem der Ansprüche 1 bis 5, mindestens ein Matrixmaterial gemäß Formel (A) wie in Anspruch 11 definiert oder gemäß Formel (X) wie in Anspruch 11 definiert, und mindestens ein weiteres, bevorzugt lochtransportierendes, Matrixmaterial, enthält.

13. OLED nach einem der Ansprüche 10 bis 12, **dadurch gekennzeichnet, dass** sie eine Elektronen-transportierende Schicht enthaltend mindestens zwei verschiedene Materialien, wovon mindestens ein Material Elektronen-leitend ist, enthält.

14. Vorrichtung ausgewählt aus der Gruppe bestehend aus stationären Bildschirmen, mobilen Bildschirmen und Beleuchtungsmitteln, enthaltend mindestens eine OLED gemäß einem der Ansprüche 10 bis 13.

15. Verwendung eines Metall-Carben-Komplexes nach einem der Ansprüche 1 bis 5 in OLEDs.

## Claims

1. A metal-carbene complex of the general formula (I) 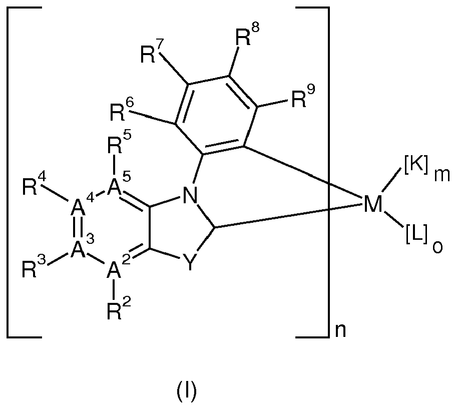 where M, n, Y, A², A³, A⁴, A⁵, R¹, R², R³, R⁴, R⁵, R⁶, R⁷, R⁸, R⁹, R¹⁰, K, L, m and o are each defined as follows:
M is Ir or Pt,
n is an integer selected from 1, 2 and 3,
Y is NR¹, 0, S or C(R¹⁰)₂,
A², A³, A⁴, A⁵ are each independently N or C, where 2 A = nitrogen atoms and at least one carbon atom is present between two nitrogen atoms in the ring,
R¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R², R³, R⁴, R⁵ are each, if A², A³, A⁴ and/or A⁵ is N, a free electron pair, or, if A², A³, A⁴ and/or A⁵ is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action,
or
R³ and R⁴ together with A³ and A⁴ form an optionally substituted, unsaturated ring optionally interrupted by at least one further heteroatom and having a total of 5 to 18 carbon atoms and/or heteroatoms,
R⁶, R⁷, R⁸, R⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group, having a total of 5 to 18 carbon atoms and/or heteroatoms and having a base skeleton selected from pyridyl, pyrimidyl, pyrazyl, triazolyl, pyrrole, furan, thiophene, pyrazole, triazole, oxazole and thiazole, group with donor or acceptor action selected from halogen radicals, silyl radicals, siloxy radicals, alkoxy radicals, aryloxy radicals, carbonyl radicals, ester radicals, CH₂F groups, CHF₂ groups, CF₃ groups, CN groups, thio groups and SCN groups,
or R⁶ and R⁷, R⁷ and R⁸ or R⁸ and R⁹,
together with the carbon atoms to which they are bonded, form a saturated, unsaturated or aromatic, optionally substituted ring optionally interrupted by at least one heteroatom and having a total of 5 to 18 carbon atoms and/or heteroatoms,
and/or
if A⁵ is C, R⁵ and R⁶ together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R¹⁰ is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m is 0, 1 or 2, where, when m is 2, the K ligands may be the same or different,
o is 0, 1 or 2, where, when o is 2, the L ligands may be the same or different.

2. A metal-carbene complex according to claim 1, wherein M, n, Y, A², A³, A⁴, A⁵, R¹, R², R³, R⁴, R⁵, R⁶, R⁷, R⁸, R⁹, L, m and o are each defined as follows:
M is Ir,
n is 1, 2 or 3,
Y is NR¹,
A², A³, A⁴, A⁵ are each independently N or C, where 2 A = nitrogen atoms and at least one carbon atom is present between two nitrogen atoms in the ring,
R¹ is a linear or branched alkyl radical having 1 to 6 carbon atoms, substituted or unsubstituted aryl radical having 6 to 30 carbon atoms, substituted or unsubstituted heteroaryl radical having a total of 5 to 18 carbon atoms and/or heteroatoms,
R², R³, R⁴, R⁵ are each, if A², A³, A⁴ and/or A⁵ is N, a free electron pair, or, if A², A³, A⁴ and/or A⁵ is C, each independently hydrogen, linear or branched alkyl radical having 1 to 6 carbon atoms, substituted or unsubstituted aryl radical having 6 to 30 carbon atoms, substituted or unsubstituted heteroaryl radical having a total of 5 to 18 carbon atoms and/or heteroatoms,
or
R³ and R⁴ together with A³ and A⁴ form an optionally substituted, unsaturated ring optionally interrupted by at least one further heteroatom and having a total of 5 to 18 carbon atoms and/or heteroatoms,
R⁶, R⁷, R⁸, R⁹ are each independently hydrogen, linear or branched alkyl radical optionally interrupted by at least one heteroatom, optionally bearing at least one functional group and having 1 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group, having a total of 5 to 18 carbon atoms and/or heteroatoms and having a base skeleton selected from pyridyl, pyrimidyl, pyrazyl, triazolyl, pyrrole, furan, thiophene, pyrazole, triazole, oxazole and thiazole,
L is a monoanionic bidentate ligand,
m is 0,
o is 0, 1 or 2.

3. A metal-carbene complex according to claim 1 or 2, wherein M, n, Y, A², A³, A⁴, A⁵, R¹, R², R³, R⁴, R⁵, R⁶, R⁷, R⁸, R⁹, L, m and o are each defined as follows:
M is Ir,
n is 2 or 3,
Y is NR¹,
A², A³, A⁴, A⁵ A² and A⁵ are each N and A³ and A⁴ are each C
or
A² and A⁴ are each N and A³ and A⁵ are each C
or
A³ and A⁵ are each N and A² and A⁴ are each C,
R¹ is a linear or branched alkyl radical having 1 to 6 carbon atoms, substituted or unsubstituted phenyl radical, substituted or unsubstituted heteroaryl radical having a total of 6 to 18 carbon atoms and/or heteroatoms,
R², R³, R⁴, R⁵ are each, if A², A³, A⁴ and/or A⁵ is N, a free electron pair or, if A², A³, A⁴ and/or A⁵ is C, each independently hydrogen, linear or branched alkyl radical having 1 to 6 carbon atoms, substituted, especially o,o'-dialkylated, or unsubstituted phenyl radical,
or
R³ and R⁴ together with A³ and A⁴ form an optionally substituted, monounsaturated ring having a total of 5 to 7 carbon atoms,
R⁶, R⁷, R⁸, R⁹ are each independently hydrogen, linear or branched alkyl radical having 1 to 20 carbon atoms, o,o'-dialkylated aryl radical having 6 to 30 carbon atoms,
L is a monoanionic bidentate ligand,
m is 0,
o is 0 or 1.

4. A metal-carbene complex according to any of claims 1 to 3, wherein, in the general formula (I), L is a carbene ligand of the general formula (II) 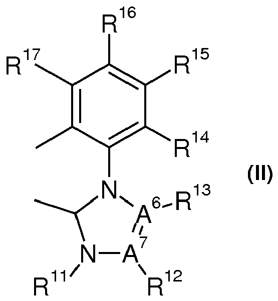 where
A⁶, A⁷ are each independently C or N
R¹¹ is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R¹², R¹³ are each independently, when A is N, a free electron pair, or, when A is C, hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action,
R¹⁴ , R¹⁵ R¹⁶, R¹⁷ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action,
or
R¹² and R¹³, R¹⁴ and R¹⁵, R¹⁵ and R¹⁶ or R¹⁶ and R¹⁷ form, together with A or the carbon atoms to which they are bonded, an unsaturated or aromatic, optionally substituted ring optionally interrupted by at least one heteroatom and having a total of 5 to 18 carbon atoms and/or heteroatoms,
and/or
if A6 is C, R13 and R14 together form a saturated or unsaturated, linear or branched bridge optionally comprising heteroatoms, aromatic units, heteroaromatic units and/or functional groups and having a total of 1 to 30 carbon atoms and/or heteroatoms, to which is optionally fused a substituted or unsubstituted, five- to eight-membered ring comprising carbon atoms and/or heteroatoms.

5. A metal-carbene complex according to any of claims 1 to 3, wherein L in the general formula (I) is a heterocyclic noncarbene ligand of the general formula (III) 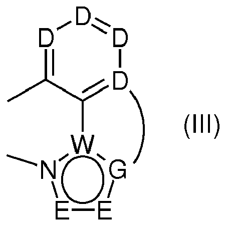 in which the symbols in the ligand of the general formula III are each defined as follows:
D are each independently CR¹⁸ or N, preferably CR¹⁸;
W is C, N, preferably C;
E are each independently CR¹⁹, N, NR²⁰ preferably CR¹⁹ or N;
G is CR²¹, N, NR²², S, O, preferably NR²¹
R¹⁸, R²¹ R¹⁹ are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action,
or in each case 2 R¹⁸, R¹⁹ and R²¹ radicals, together with the carbon atoms to which they are bonded, form a saturated, unsaturated or aromatic, optionally substituted ring optionally interrupted by at least one heteroatom and having a total of 5 to 18 carbon atoms and/or heteroatoms,
R²⁰, R²² are each independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cycloheteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action; preferably o,o'-dialkylated aryl radical,
where the solid curved line is an optional bridge between one of the D groups and the G group; where the bridge may be defined as follows:
alkylene, arylene, heteroarylene, alkynylene, alkenylene, NR²³, 0, S, SiR²⁴R²⁵ and (CR²⁶R²⁷)_{d}, where one or more nonadjacent (CR²⁶R²⁷) groups may be replaced by NR²³, 0, S, SiR²⁴R²⁵, where
d is 2 to 10;
and
R²⁴, R²⁵, R²⁶, R²⁷ are each H, alkyl, aryl, heteroaryl, alkenyl, alkynyl.

6. A process for preparing the metal-carbene complexes according to any of claims 1 to 5 by contacting suitable compounds comprising M with the appropriate ligands or ligand precursors.

7. An organic electronic component comprising at least one metal-carbene complex according to any of claims 1 to 5.

8. The organic electronic component according to claim 7, which is an organic light-emitting diode (OLED), organic photovoltaic cell (OPV), organic field-effect transistor (OFET) or light-emitting electrochemical cell (LEEC).

9. A light-emitting layer comprising at least one metal-carbene complex according to any of claims 1 to 5.

10. An OLED comprising a light-emitting layer according to claim 9.

11. An OLED comprising at least one metal-carbene complex according to any of claims 1 to 5, and at least one compound of the formula (X) 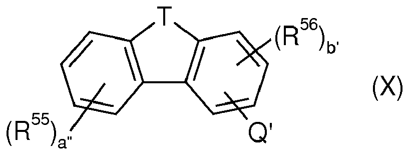 in which
T is NR⁵⁷, S, 0 or PR⁵⁷, preferably S or 0, more preferably 0;
R⁵⁷ is aryl, heteroaryl, alkyl, cycloalkyl or heterocycloalkyl;
Q' is -NR⁵⁸R⁵⁹, -P(O)R⁶⁰R⁶¹, -PR⁶²R⁶³, -S(O)₂R⁶⁴,-S(O)R⁶⁵, -SR⁶⁶ or -OR⁶⁷, preferably -NR⁵⁸R⁵⁹; more preferably 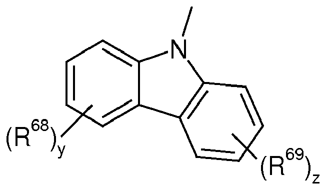 in which
R⁶⁸, R⁶⁹ are each independently alkyl, cycloalkyl, heterocycloalkyl, aryl or heteroaryl; preferably methyl, carbazolyl, dibenzofuryl or dibenzothienyl;
y, z are each independently 0, 1, 2, 3 or 4, preferably 0 or 1;
R⁵⁵, R⁵⁶ are each independently alkyl, cycloalkyl, heterocycloalkyl, aryl, heteroaryl, SiR⁷⁰R⁷¹R⁷², a Q' group or a group with donor or acceptor action;
a'' is 0, 1, 2, 3 or 4;
b' is 0, 1, 2 or 3;
R⁵⁸, R⁵⁹ form, together with the nitrogen atom, a cyclic radical which has 3 to 10 ring atoms and may be unsubstituted or substituted by one or more substituents selected from alkyl, cycloalkyl, heterocycloalkyl, aryl, heteroaryl and a group with donor or acceptor action, and/or may be fused to one or more further cyclic radicals having 3 to 10 ring atoms, where the fused radicals may be unsubstituted or substituted by one or more substituents selected from alkyl, cycloalkyl, heterocycloalkyl, aryl, heteroaryl and a group with donor or acceptor action;
R⁷⁰, R⁷¹, R⁷², R⁶⁰, R⁶¹, R⁶², R⁶³, R⁶⁴, R⁶⁵, R⁶⁶, R⁶⁷ are each independently aryl, heteroaryl, alkyl, cycloalkyl or heterocycloalkyl,
or
two units of the general formula (X) are bridged to one another via a linear or branched, saturated or unsaturated bridge optionally interrupted by at least one heteroatom, via a bond or via O.

12. The OLED according to claim 11, which comprises an emission layer comprising at least one metal-carbene complex according to any of claims 1 to 5, at least one matrix material of the formula (A) as defined in claim 11 or of the formula (X) as defined in claim 11, and at least one further, preferably hole-transporting matrix material.

13. The OLED according to any of claims 10 to 12, which comprises an electron-transporting layer comprising at least two different materials, of which at least one material is electron-conducting.

14. A device selected from the group consisting of stationary visual display units, mobile visual display units and illumination means, comprising at least one OLED according to any of claims 10 to 13.

15. The use of a metal-carbene complex according to any of claims 1 to 5 in OLEDs.

## Revendications

1. Complexe métal-carbène de formule générale (I) 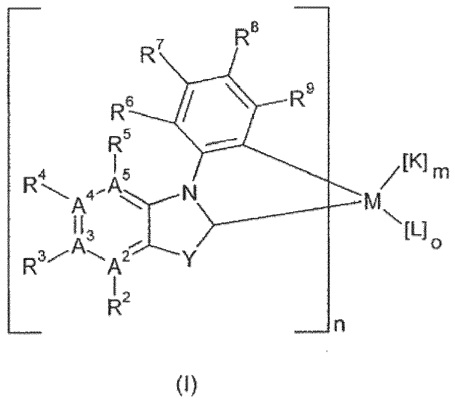 dans laquelle M, n, Y, A², A³, A⁴, A⁵, R¹, R², R³, R⁴, R⁵, R⁶, R⁷, R⁸, R⁹, R¹⁰, K, L, m et o ont les significations suivantes :
M Ir ou Pt,
n nombre entier choisi parmi 1, 2 ou 3,
Y NR¹, 0, S ou C(R¹⁰)₂,
A², A³, A⁴, A⁵ indépendamment les uns des autres N ou C, avec 2 A = atomes N et au moins un atome C étant présent dans le cycle entre deux atomes N,
R¹ radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes,
R², R³, R⁴, R⁵ si A², A³, A⁴ et/ou A⁵ C, signifient N, paire d'électrons libres ou, si A², A³, A⁴ et/ou A⁵ signifient indépendamment les uns des autres hydrogène, radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes, groupe à effet donneur ou accepteur,
ou
R³ et R⁴ forment ensemble avec A³ et A⁴ un cycle insaturé, éventuellement interrompu par au moins un hétéroatome supplémentaire, éventuellement substitué, contenant au total 5 à 18 atomes de carbone et/ou hétéroatomes,
R⁶, R⁷, R⁸, R⁹ indépendamment les uns des autres hydrogène, radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical cyclohétéroalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes, comprenant un squelette de base choisi parmi pyridyle, pyrimidyle, pyrazyle, triazolyle, pyrrole, furane, thiophène, pyrazole, triazole, oxazole et thiazole, groupe à effet donneur ou accepteur, choisi parmi les radicaux halogène, les radicaux silyle, les radicaux siloxy, les radicaux alcoxy, les radicaux aryloxy, les radicaux carbonyle, les radicaux ester, les groupes CH₂F, les groupes CHF₂, les groupes CF₃, les groupes CN, les groupes thio et les groupes SCN,
ou
R⁶ et R⁷, R⁷ et R⁸ ou R⁸ et R⁹
forment ensemble avec les atomes C auxquels ils sont reliés un cycle éventuellement interrompu par au moins un hétéroatome, saturé, insaturé ou aromatique, éventuellement substitué, contenant au total 5 à 18 atomes de carbone et/ou hétéroatomes,
et/ou
si A⁵ signifie C, R⁵ et R⁶ forment ensemble un pont saturé ou insaturé, linéaire ou ramifié, contenant éventuellement des hétéroatomes, une unité aromatique, une unité hétéroaromatique et/ou des groupes fonctionnels, contenant au total 1 à 30 atomes de carbone et/ou hétéroatomes, auquel un cycle substitué ou non substitué, de cinq à huit éléments, contenant des atomes de carbone et/ou des hétéroatomes, est éventuellement annelé,
les R¹⁰ indépendamment les uns des autres, radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes,
K ligand mono- ou bidentate neutre,
L ligand mono- ou dianionique, de préférence ligand monoanionique, qui peut être mono- ou bidentate,
m 0, 1 ou 2, les ligands K pouvant être identiques ou différents lorsque m = 2,
o 0, 1 ou 2, les ligands L pouvant être identiques ou différents lorsque o = 2.

2. Complexe métal-carbène selon la revendication 1, **caractérisé en ce que** M, n, Y, A², A³, A⁴, A⁵, R¹, R², R³, R⁴, R⁵, R⁶, R⁷, R⁸, R⁹, L, m et o ont les significations suivantes :
M Ir,
n 1, 2 ou 3,
Y NR¹,
A², A³, A⁴, A⁵ indépendamment les uns des autres N ou C, avec 2 A = atomes N et au moins un atome C étant présent dans le cycle entre deux atomes N,
R¹ radical alkyle linéaire ou ramifié, contenant 1 à 6 atomes de carbone, radical aryle substitué ou non substitué contenant 6 à 30 atomes de carbone, radical hétéroaryle substitué ou non substitué contenant au total 5 à 18 atomes de carbone et/ou hétéroatomes,
R², R³, R⁴, R⁵ si A², A³, A⁴ et/ou A⁵ signifient N, paire d'électrons libres ou, si A², A³, A⁴ et/ou A⁵ signifient C, indépendamment les uns des autres hydrogène, radical alkyle linéaire ou ramifié, contenant 1 à 6 atomes de carbone, radical aryle substitué ou non substitué contenant 6 à 30 atomes de carbone, radical hétéroaryle substitué ou non substitué contenant au total 5 à 18 atomes de carbone et/ou hétéroatomes,
ou
R³ et R⁴
forment ensemble avec A³ et A⁴ un cycle insaturé, éventuellement interrompu par au moins un hétéroatome supplémentaire, éventuellement substitué, contenant au total 5 à 18 atomes de carbone et/ou hétéroatomes,
R⁶, R⁷, R⁸, R⁹ indépendamment les uns des autres hydrogène, radical alkyle linéaire ou ramifié, éventuellement interrompu par au moins un hétéroatome, portant éventuellement au moins un groupe fonctionnel, contenant 1 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes, comprenant un squelette de base choisi parmi pyridyle, pyrimidyle, pyrazyle, triazolyle, pyrrole, furane, thiophène, pyrazole, triazole, oxazole et thiazole,
L ligand monoanionique bidentate,
m 0,
o 0, 1 ou 2.

3. Complexe métal-carbène selon la revendication 1 ou 2, **caractérisé en ce que** M, n, Y, A², A³, A⁴, A⁵, R¹, R², R³, R⁴, R⁵, R⁶, R⁷, R⁸, R⁹, L, m et o ont les significations suivantes :
M Ir,
n 2 ou 3,
Y NR¹,
A², A³, A⁴, A⁵ A² et A⁵ représentent N et A³ et A⁴ représentent C
ou
A² et A⁴ représentent N et A³ et A⁵ représentent C
ou
A³ et A⁵ représentent N et A² et A⁴ représentent C,
R¹ radical alkyle linéaire ou ramifié, contenant 1 à 6 atomes de carbone, radical phényle substitué ou non substitué, radical hétéroaryle substitué ou non substitué contenant au total 6 à 18 atomes de carbone et/ou hétéroatomes,
R², R³, R⁴, R⁵ si A², A³, A⁴ et/ou A⁵ signifient N, paire d'électrons libres ou, si A², A³, A⁴ et/ou A⁵ signifient C, indépendamment les uns des autres hydrogène, radical alkyle linéaire ou ramifié, contenant 1 à 6 atomes de carbone, radical phényle substitué, notamment o,o'-dialkylé, ou non substitué,
ou
R³ et R⁴ forment ensemble avec A³ et A⁴ un cycle monoinsaturé, éventuellement substitué, contenant au total 5 à 7 atomes de carbone,
R⁶, R⁷, R⁸, R⁹ indépendamment les uns des autres hydrogène, radical alkyle linéaire ou ramifié, contenant 1 à 20 atomes de carbone, radical aryle o,o'-dialkylé contenant 6 à 30 atomes de carbone,
L ligand monoanionique bidentate,
m 0,
o 0 ou 1.

4. Complexe métal-carbène selon l'une quelconque des revendications 1 à 3, **caractérisé en ce que**, dans la formule générale (I), L signifie un ligand carbène de formule générale (II) 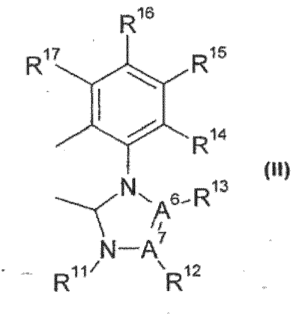 avec
A⁶, A⁷ indépendamment l'un de l'autre C ou N,
R¹¹ radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical cyclohétéroalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes,
R¹², R¹³ indépendamment l'un de l'autre, lorsque A représente N, paire d'électrons libres ou, lorsque A représente C, hydrogène, radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical cyclohétéroalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes, groupe à effet donneur ou accepteur,
R¹⁴, R¹⁵, R¹⁶, R¹⁷ indépendamment les uns des autres, hydrogène, radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical cyclohétéroalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes, groupe à effet donneur ou accepteur,
ou
R¹² et R¹³, R¹⁴ et R¹⁵, R¹⁵ et R¹⁶ ou R¹⁶ et R¹⁷ forment ensemble avec A ou les atomes C auxquels ils sont reliés, un cycle éventuellement interrompu par au moins un hétéroatome, insaturé ou aromatique, éventuellement substitué, contenant au total 5 à 18 atomes de carbone et/ou hétéroatomes,
et/ou
si A⁶ signifie C, R¹³ et R¹⁴ forment ensemble un pont saturé ou insaturé, linéaire ou ramifié, contenant éventuellement des hétéroatomes, des unités aromatiques, des unités hétéroaromatiques et/ou des groupes fonctionnels, contenant au total 1 à 30 atomes de carbone et/ou hétéroatomes, auquel un cycle substitué ou non substitué, de cinq à huit éléments, contenant des atomes de carbone et/ou des hétéroatomes, est éventuellement annelé.

5. Complexe métal-carbène selon l'une quelconque des revendications 1 à 3, **caractérisé en ce que**, dans la formule générale (I), L signifie
un ligand non-carbène hétérocyclique de formule générale (III) 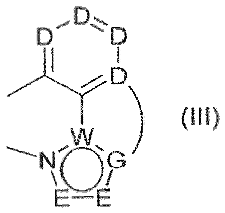 les symboles dans le ligand de formule générale (III) ayant les significations suivantes :
les D indépendamment les uns des autres CR¹⁸ ou N, de préférence CR¹⁸ ;
W C, N, de préférence C ;
les E indépendamment les uns des autres CR¹⁹, N, NR²⁰, de préférence CR¹⁹ ou N ;
G CR²¹, N, NR²², S, O, de préférence NR²¹ ;
R¹⁸, R¹⁹, R²¹ indépendamment les uns des autres hydrogène, radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical cyclohétéroalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes, groupe à effet donneur ou accepteur,
ou 2 radicaux parmi R¹⁸, R¹⁹ et R²¹ formant ensemble avec les atomes C auxquels ils sont reliés un cycle éventuellement interrompu par au moins un hétéroatome, saturé, insaturé ou aromatique, éventuellement substitué, contenant au total 5 à 18 atomes de carbone et/ou hétéroatomes,
R²⁰, R²² indépendamment l'un de l'autre, radical alkyle linéaire ou ramifié, éventuellement interrompu par au moins un hétéroatome, portant éventuellement au moins un groupe fonctionnel, contenant 1 à 20 atomes de carbone, radical cycloalkyle contenant 3 à 20 atomes de carbone, radical cyclohétéroalkyle contenant 3 à 20 atomes de carbone, radical aryle substitué ou non substitué contenant 6 à 30 atomes de carbone, radical hétéroaryle substitué ou non substitué contenant au total 5 à 18 atomes de carbone et/ou hétéroatomes, groupe à effet donneur ou accepteur ; de préférence radical aryle o,o'-dialkylé,
la ligne incurvée continue signifiant un pont optionnel entre un des groupes D et le groupe G ; le pont pouvant avoir les significations suivantes : alkylène, arylène, hétéroarylène, alcynylène, alcénylène, NR²³, 0, S, SiR²⁴R²⁵ et (CR²⁶R²⁷)_{d}, un ou plusieurs groupes (CR²⁶R²⁷) non voisins pouvant être remplacés par NR²³, O, S, SiR²⁴R²⁵,
d signifiant 2 à 10 ;
et
R²⁴, R²⁵, R²⁶, R²⁷ signifiant H, alkyle, aryle, hétéroaryle, alcényle, alcynyle.

6. Procédé de fabrication des complexes métal-carbène selon l'une quelconque des revendications 1 à 5 par mise en contact de composés appropriés contenant M avec les ligands ou précurseurs de ligands correspondants.

7. Composant électronique organique, contenant au moins un complexe métal-carbène selon l'une quelconque des revendications 1 à 5.

8. Composant électronique organique selon la revendication 7, **caractérisé en ce qu'**il s'agit d'une diode électroluminescente organique (OLED), d'une cellule photovoltaïque organique (OPV), d'un transistor à effet de champ organique (OFET) ou d'une cellule électrochimique électroluminescente (LEEC).

9. Couche électroluminescente, contenant au moins un complexe métal-carbène selon l'une quelconque des revendications 1 à 5.

10. OLED, contenant une couche électroluminescente selon la revendication 9.

11. OLED, contenant au moins un complexe métal-carbène selon l'une quelconque des revendications 1 à 5 et au moins un composé de formule (X) 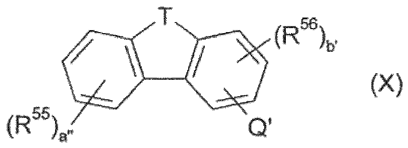 dans laquelle
T signifie NR⁵⁷, S, 0 ou PR⁵⁷, de préférence S ou 0, de manière particulièrement préférée 0 ;
R⁵⁷ signifie aryle, hétéroaryle, alkyle, cycloalkyle ou hétérocycloalkyle ;
Q' signifie -NR⁵⁸R⁵⁹, -P(O)R⁶⁰R⁶¹, -PR⁶²R⁶³, -S(O)₂R⁶⁴,-S(O)R⁶⁵, -SR⁶⁶ ou -OR⁶⁷, de préférence -NR⁵⁸R⁵⁹, de manière particulièrement préférée 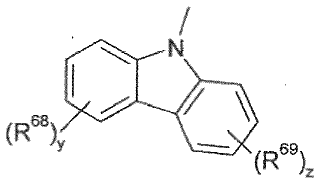
R⁶⁸, R⁶⁹ signifiant indépendamment l'un de l'autre alkyle, cycloalkyle, hétérocycloalkyle, aryle ou hétéroaryle ; de préférence méthyle, carbazolyle, dibenzofuryle ou dibenzothiényle ;
y, z signifiant indépendamment l'un de l'autre 0, 1, 2, 3 ou 4, de préférence 0 ou 1 ;
R⁵⁵, R⁵⁶ signifiant indépendamment l'un de l'autre alkyle, cycloalkyle, hétérocycloalkyle, aryle, hétéroaryle, SiR⁷⁰R⁷¹R⁷², un groupe Q' ou un groupe à effet donneur ou accepteur ;
a'' signifiant 0, 1, 2, 3 ou 4 ;
b' signifiant 0, 1, 2 ou 3 ;
R⁵⁸, R⁵⁹ formant ensemble avec l'atome N un radical cyclique contenant 3 à 10 atomes de cycle, qui peut être non substitué ou substitué avec un ou plusieurs substituants choisis parmi alkyle, cycloalkyle, hétérocycloalkyle, aryle, hétéroaryle et un groupe à effet donneur ou accepteur, et/ou qui peut être annelé avec un ou plusieurs radicaux cycliques supplémentaires contenant 3 à 10 atomes de cycle, les radicaux annelés pouvant être non substitués ou substitués avec un ou plusieurs substituants choisis parmi alkyle, cycloalkyle, hétérocycloalkyle, aryle, hétéroaryle et un groupe à effet donneur ou accepteur ;
R⁷⁰, R⁷¹, R⁷², R⁶⁰, R⁶¹, R⁶², R⁶³, R⁶⁴, R⁶⁵, R⁶⁶, R⁶⁷ signifiant indépendamment les uns des autres aryle, hétéroaryle, alkyle, cycloalkyle ou hétérocycloalkyle,
ou
deux unités de formule générale (X) sont pontées l'une avec l'autre par un pont linéaire ou ramifié, saturé ou insaturé, éventuellement interrompu par au moins un hétéroatome, par une liaison ou par O.

12. OLED selon la revendication 11, **caractérisée en ce qu'**elle contient une couche d'émission contenant au moins un complexe métal-carbène selon l'une quelconque des revendications 1 à 5, au moins un matériau de matrice selon la formule (A) telle que définie dans la revendication 11 ou selon la formule (X) telle que définie dans la revendication 11, et au moins un matériau de matrice supplémentaire, de préférence transporteur de trous.

13. OLED selon l'une quelconque des revendications 10 à 12, **caractérisée en ce qu'**elle contient une couche transporteuse d'électrons contenant au moins deux matériaux différents, parmi lesquels au moins un matériau est conducteur d'électrons.

14. Dispositif choisi dans le groupe constitué par les écrans stationnaires, les écrans mobiles et les moyens d'éclairage, contenant au moins une OLED selon l'une quelconque des revendications 10 à 13.

15. Utilisation d'un complexe métal-carbène selon l'une quelconque des revendications 1 à 5 dans des OLED.
